# EUROPEAN PATENT SPECIFICATION

(11) **EP 3 356 369 B1**
(45) Date of publication and mention of the grant of the patent: **04.05.2022**
(21) Application number: 16784964.5
(22) Date of filing: 30.09.2016
(51) Int. Cl.: C07D 487/04, C07D 519/00, C09K 11/06, H01L 51/00, H01L 51/50

(54) **BENZIMIDAZOLO[1,2-A]BENZIMIDAZOLE CARRYING TRIAZINE GROUPS FOR ORGANIC LIGHT EMITTING DIODES**
TRIAZINGRUPPEN TRAGENDE BENZIMIDAZOLO[1,2-A]BENZIMIDAZOL FÜR ORGANISCHE LEUCHTDIODEN
BENZIMIDAZOL[1,2-A]BENZIMIDAZOLE À GROUPES TRIAZINE POUR DIODES ÉLECTROLUMINESCENTES ORGANIQUES

(30) Priority: 01.10.2015 EP 15187931
(43) Date of publication of application: 08.08.2018
(73) Proprietor: Idemitsu Kosan Co., Ltd, Tokyo 100-8321 (JP)
(72) Inventor: SCHÄFER, Thomas, 4410 Liestal (CH); KAWAMURA, Masahiro, Sodegaura-shi Chiba 299-0293 (JP); BARDON, Kristina, 79761 Waldshut-Tiengen (DE); NAGASHIMA, Hideaki, 4057 Basel (CH)
(74) Representative: Hollah, Dorothee
(86) International application number: PCT/IB2016/055872
(87) International publication number: WO 2017/056055

(56) References cited:
- WO-A1-2013/068376
- WO-A1-2014/009317
- WO-A1-2014/044722
- WO-A1-2015/014791
- WO-A1-2015/063046

## Description

The present invention relates to compounds of formula (1) and their use in electronic devices, especially electroluminescent devices. When used as charge transport material, charge blocker material and/or host material in electroluminescent devices, the compounds of formula (1) may provide improved lifetime, driving voltage, efficiency, stability, manufacturability, or spectral characteristics of electroluminescent devices and reduced driving voltage of electroluminescent devices. Preferably, the compounds of formula (1) may provide improved lifetime and/or reduced driving voltage of electroluminescent devices.

Khan, Misbahul Ain; Ribeiro, Vera Lucia Teixeira, Pakistan Journal of Scientific and Industrial Research 43 (2000) 168-170 describes the synthesis of benzimidazo[1,2-a]benzimadozoles 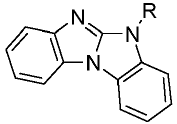 (R = H, Me, Et) by trialkyl phosphite-induced deoxygenation and thermolysis of 1-(o-nitrophenyl)- and 1-(o-azidophenyl)benzimidazoles.

Pedro Molina et al. Tetrahedron (1994) 10029-10036 reports that aza Wittig-type reaction of bis(iminophosphoranes), derived from bis(2-aminophenyl)amine with two equivalents of isocyanate directly provided benzimidazo[1,2,a]benzimidazole derivatives. 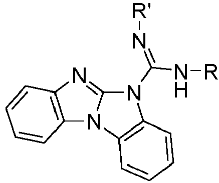 (R = R' = 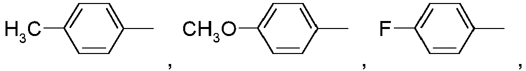 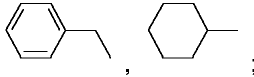 R = 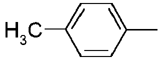 and R' = 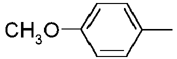 , R = iso-propyl and R' = ethyl)

Kolesnikova, I. V.; Zhurnal Organicheskoi Khimii 25 (1989) 1689-95 describes the synthesis of 5H-benzimidazo[1,2-a]benzimidazole 1,2,3,4,7,8,9,10-octafluoro-5-(2,3,4,5,6-pentafluorophenyl).

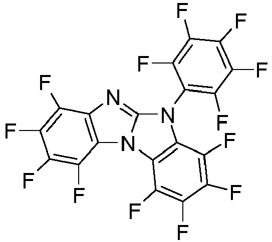

Achour, Reddouane; Zniber, Rachid, Bulletin des Societes Chimiques Belges 96 (1987) 787-92 describes the synthesis of benzimidazobenzimidazoles 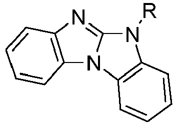 (R = H, - CH(CH₃)₂) which were prepared from benzimidazolinone derivatives.

Hubert, Andre J.; Reimlinger, Hans, Chemische Berichte 103 (1970) 2828-35 describes the synthesis of benzimidazobenzimidazoles 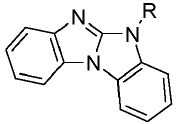 (R = H, CH₃,

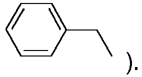

X. Wang et al. Org. Lett. 2012, 14, 452-455 discloses a highly efficient copper-catalyzed synthesis for compounds of formula 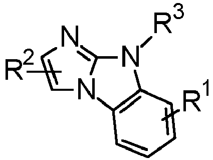 , wherein compounds of formula 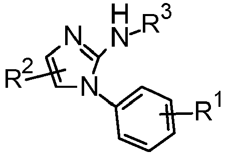 are reacted in the presence of copper acetate (Cu(OAc)₂)/PPh₃/1,10-phenathroline/sodium acetate and oxygen in m-xylene (1 atm) at elevated temperature. Among others the following compounds can be prepared by the described synthesis method: 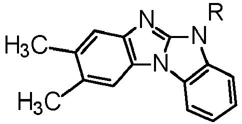 (R = 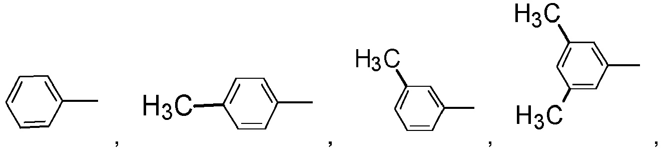 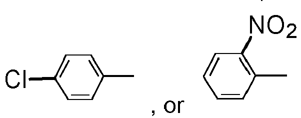 ).

In Eur. J. Org. Chem. 2014, 5986-5997 a new synthesis of benzimidazolo[1,2-a]benzimidazole is described.

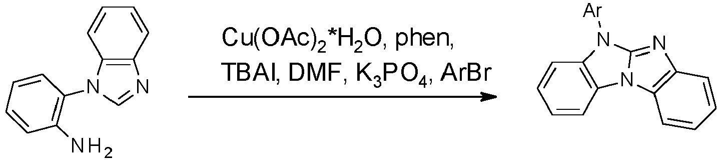

In RSC Advances 2014, 4, 21904-21908 a new synthesis of benzimidazolo[1,2-a]benzimidazole is described. 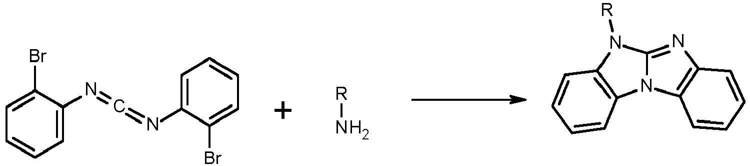 It is mentioned - as a general statement - that these polycyclic molecules have - besides other applications - also attracted great interest in the field of electroluminescent devices.

WO2011/160757 relates to an electronic device comprising an anode, cathode and at least one organic layer which contains a compound of formulae 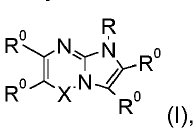 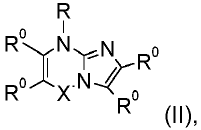 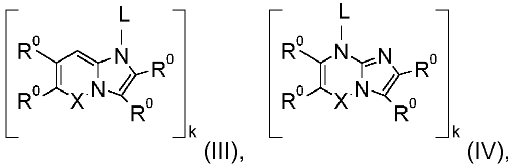 wherein X may be a single bond and L may be a divalent group. The following 4H-Imidazo[1,2-a]imidazole compounds are explicitly disclosed: 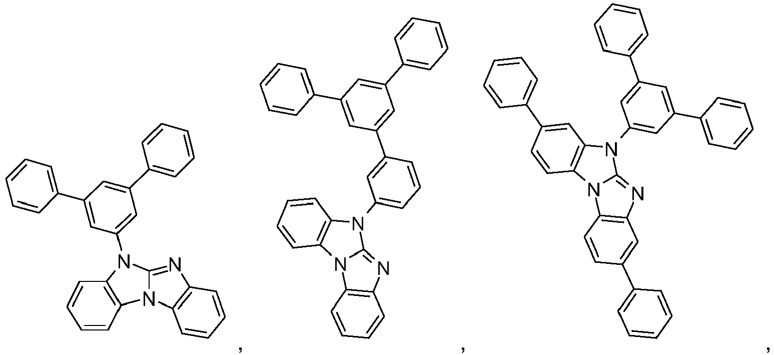 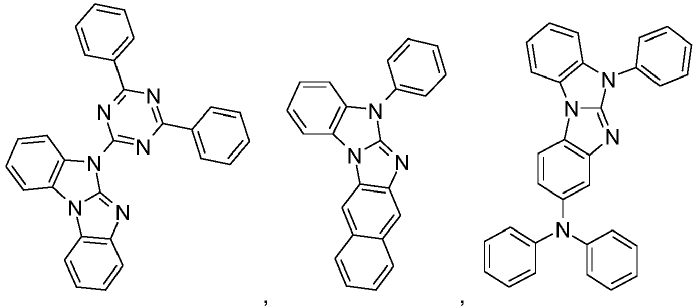 and

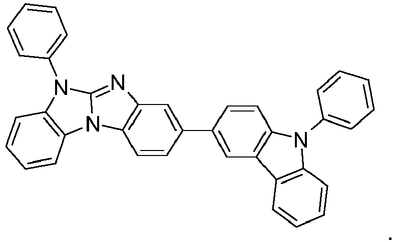

WO2012/130709 relates to 4H-Imidazo1,2-aimidazoles, 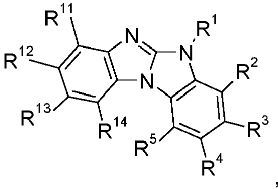 such as for example, 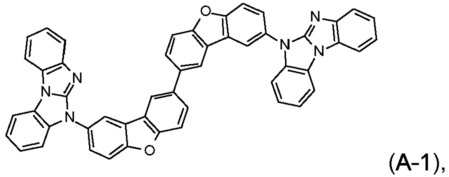 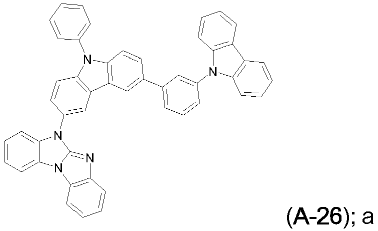 process for their production and their use in electronic devices, especially electroluminescent devices.

WO2013/068376 relates to compounds of formula 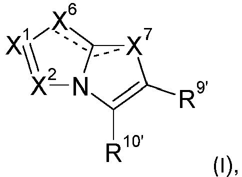 such as 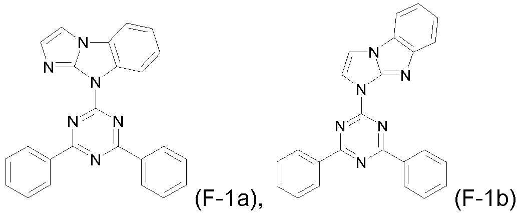 and electron transport materials(B-5).

WO2014/009317 relates to compounds of formula 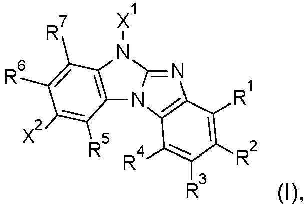 especially compounds of formula 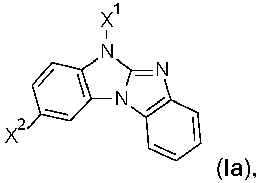 such as, for example, 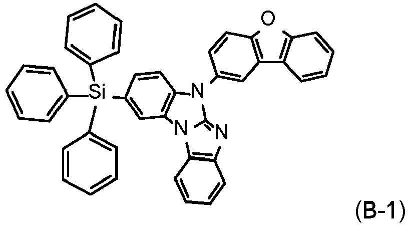 and 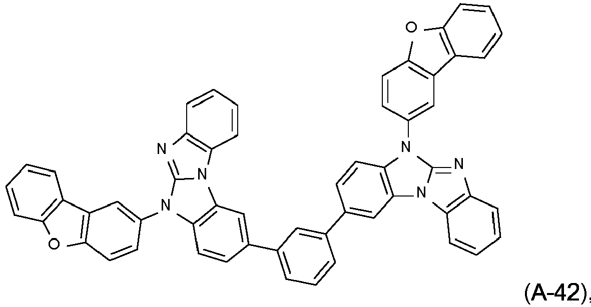 , a process for their production and their use in electronic devices, especially electroluminescent devices. The 2,5-disubstituted benzimidazo[1,2-a]benzimidazole derivatives are suitable hole transporting materials, or host materials for phosphorescent emitters.

Benzimidazo[1,2-a]benzimidazo-5-yl and benzimidazo[1,2-a]benzimidazo-2-ylsubstituted benzimidazolo[2,1-b][1,3]benzothiazole derivatives are described in WO2015/014791.

European patent application no. EP14197947.9 describes carbazol compounds carrying benzimidazolo[1,2-a]benzimidazole groups of the following structure. 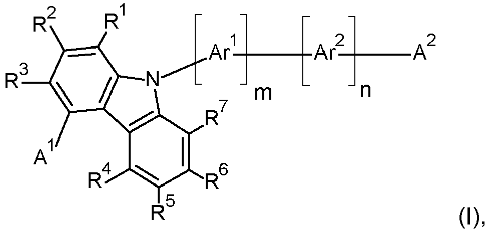 wherein m is 1, or 2, n is 0, 1, or 2,

Ar¹ and Ar² are independently of each other a C₆-C₂₄aryl group, which can optionally be substituted by G, a C₁₂-C₃₀heteroaryl group, which can optionally be substituted by G,
A¹ is a group of formula

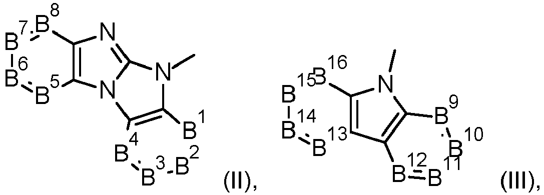

European patent application no. EP14197952.6 describes dibenzofurane compounds carrying benzimidazolo[1,2-a]benzimidazole groups of the following structure. 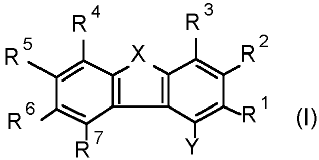 , wherein
X is O or S;
Y is a group of formula -[Ar¹]ₐ-[Ar²]_{b}-[Ar³]_{c}-A¹;
A¹ is a group of formula

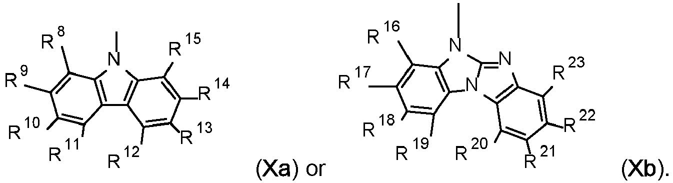

WO 2015/063046 A1 concerns aza-dibenzothiophene derivatives substituted with a benzimidazo[1,2-a]benzimidazo-5-yl group and/or a benzimidazo[1,2-a]benzimidazo-2,5-ylene group, suitable for use in organo-electroluminescent devices

WO 2014/044722 A1 relates to aza-dibenzofuran derivatives substituted with a benzimi-dazo[1,2-a]benzimidazo-5-yl group and/or a benzimidazo[1,2-a]benzimidazo-2,5-ylene group suitable for use in organo-electroluminescent devices.

Notwithstanding these developments, there remains a need for organic light emitting devices comprising new materials, especially host ( = matrix) materials, charge transport materials, i.e. hole transport materials and electron transport materials, preferably electron transport materials and/or electron injection materials to provide improved properties of electroluminescent device, such as lifetime, efficiency, stability, manufacturability, driving voltage and/or spectral characteristics, especially to provide improved lifetime, efficiency, stability, manufacturability, driving voltage and/or spectral characteristics of electroluminescent devices.

Accordingly, it is an object of the present invention, with respect to the aforementioned related art, to provide further materials suitable for use in OLEDs and further applications in organic electronics. More particularly, it should be possible to provide charge transport materials, i.e. hole transport materials and electron transport materials, preferably electron transport materials, electron injection materials and host (= matrix) materials for use in OLEDs. The materials should be suitable especially for OLEDs which comprise at least one emitter, which is preferably a phosphorescence emitter, for example at least one green, red or yellow emitter, especially at least one green emitter or at least one red emitter.

Furthermore, the materials should be suitable for providing OLEDs which ensure good efficiencies, good operative lifetimes and a high stability to thermal stress, and a low use and operating voltage of the OLEDs. Preferably, the materials should be suitable for providing OLEDs which ensure good operative lifetimes and/or a low use and operating voltage of the OLEDs.

Said object is solved by heterocyclic derivatives of formula (1);

A-[(B₁)ₒ-(B₂)p-(B₃)_{q}-(B₄)ᵣAz]_{z} (1)

wherein
B₁, B₂, B₃ and B₄
are independently of each other a C₆-C₂₄arylene group, which can optionally be substituted by G, or a C₁-C₂₄ heteroarylene group, which can optionally be substituted by G;
o is 0 or 1, p is 0 or 1, q is 0 or 1, r is 0 or 1;
Az is represented by one of the following formulae (16), (17) or (18) 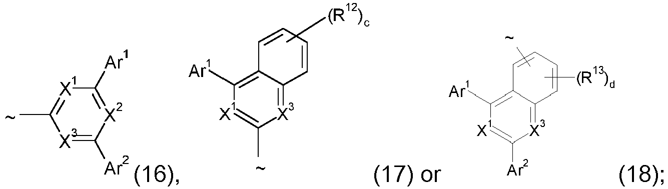
A is a heterocyclic group represented by formula (2) or formula (3); 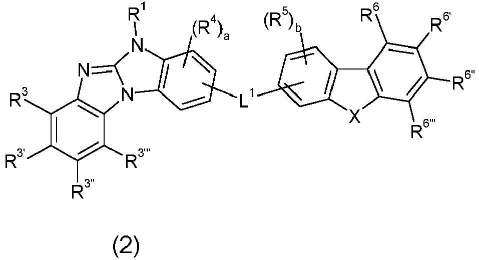 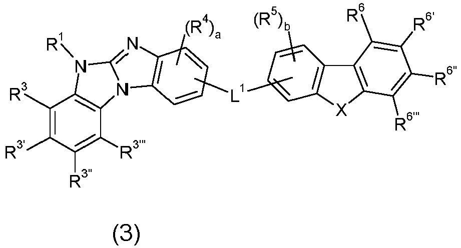 wherein X is NR⁷;
L¹ is single bond, a C₆-C₂₄arylene group, which can optionally be substituted by G, or a C₁-C₂₄heterocyclic group, which can optionally be substituted by G;
R¹ is phenyl or a group of the following formula 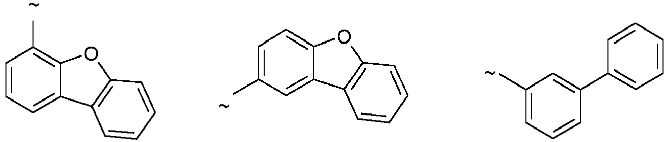 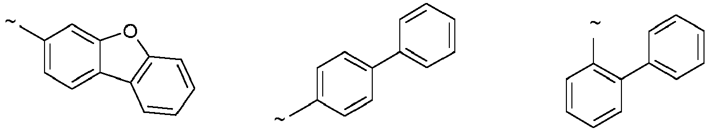 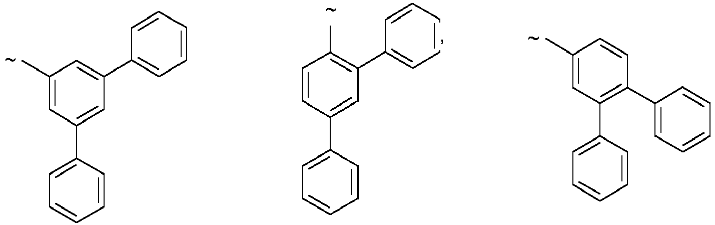 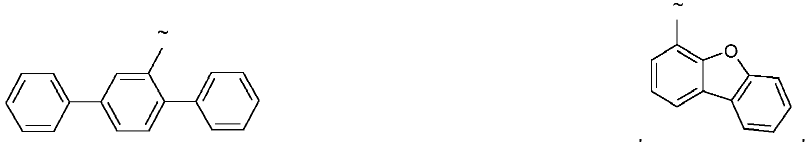 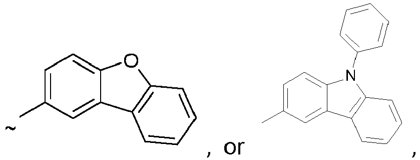 wherein ~ are bonding sites,
R3, R^{3'}, R^{3"}, R^{3‴}, R⁴, R⁵, R⁶, R^{6'}, R^{6"} and R^{6‴}
   are H;
G is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen, or a C₁-C₂₄alkyl group, a C₆-C₆₀aryl group, a C₆-C₆₀aryl group, which is substituted by F, C₁-C₂₄alkyl, or C₁-C₂₄alkyl which is interrupted by O; a C₂-C₆₀heteroaryl group, or a C₂-C₆₀heteroaryl group, which is substituted by F, C₁-C₁₈alkyl, or C₁-C₁₈alkyl which is interrupted by O;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ may form together with the atom to which they are bonded a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by-O-,
R⁷⁰ is a C₁-C₁₈alkyl group, a C₆-C₁₈aryl group, or a C₆-C₁₈aryl group, which is substituted by C₁-C₁₈alkyl, and
z is 1;
X¹, X² and X³ are independently of each other CR¹¹ or N, wherein in formula (16) at least one of X¹ to X³ is N, and wherein in formulae (17) and (18) at least one of X¹ and X³ is N;
Ar₁ and Ar² are independently of each other a C₆-C₂₄ aryl group, which is optionally substituted by G, or a C₁-C₂₄ heteroaryl group, which is optionally substituted by G;
R¹¹, R¹² and R¹³ are independently of each other H, a C₆-C₂₄ aryl group which can be substituted by G, a C₁-C₂₄ heteroaryl group which can be substituted by G or a C₁-C₂₅alkyl group, which can optionally be substituted by E and/or interrupted by D; preferably, H;
D is -CO-, -COO-, -S-, -SO-, -SO₂-, -O-, -NR65-, -SiR⁷⁰R⁷¹-, -POR⁷²-, -CR⁶³=CR⁶⁴-, or-C=C_{;}
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ may form together with the atom to which they are bonded a five or six membered ring,
R⁷¹ is a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl;
c is 0, 1, 2, 3 or 4; and
d is 0, 1, 2 or 3;
wherein R⁷ is replaced by -(B₁)ₒ-(B₂)ₚ-(B₃)_{q}-(B₄)ᵣ-Az.

The combination of the benzimidazo[1,2-a]benzimidazo-yl group with the carbazoloyl group and the group Az gives rise to materials that are highly suitable in devices that emit green, red or yellow light, preferably green or red light, more preferably green light. Moreover, a balanced electron transport and/or electron injection in devices is achieved resulting in low voltages and high external quantum efficiencies (EQE's) and/or long lifetimes.

The compounds of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescent devices, such as, for example, organic light-emitting diodes (OLEDs).

Accordingly, a further subject of the present invention is directed to an electronic device, comprising a compound according to the present invention. The electronic device is preferably an electroluminescent device, such as an organic light-emitting diode (OLED).

The compounds of formula (1) can in principal be used in any layer of an EL device, but are preferably used as host, electron transport and/or electron injection material. Particularly, the compounds of formula (1) are used as host material for green, red and yellow, preferably green and red, more preferably green light emitting phosphorescent emitters.

Hence, a further subject of the present invention is directed to an electron transport layer, comprising a compound of formula (1) according to the present invention.

A further subject of the present invention is directed to an emitting layer, comprising a compound of formula (1) according to the present invention. In said embodiment a compound of formula (1) is preferably used as host material or as co-host material together with one or more, preferably one, further host materials. More preferably, a combination of a compound of formula (1) and a co-host material together with a phosphorescent emitter is used.

A further subject of the present invention is directed to an electron injection layer, comprising a compound of formula (1) according to the present invention.

The terms halogen, alkyl, alkoxy, cycloalkyl, aryl, aryloxy, aralkyl, heteroaryl, arylene, heteroarylene generally have the following meaning, if said groups are not further specified in specific embodiments mentioned below:
Halogen is fluorine, chlorine, bromine and iodine, preferably fluorine.

C₁-C₂₅alkyl, preferably C₁-C₂₄alkyl and more preferably C₁-C₁₈alkyl are typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

The alkyl groups mentioned above can optionally be substituted by E and/or interrupted by D. Preferably, the alkyl groups mentioned above are unsubstituted or can optionally be substituted by E.

C₁-C₂₅alkoxy groups and preferably C₁-C₁₈alkoxy groups are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

The term "cycloalkyl group" is preferably C₅-C₁₂cycloalkyl, such as cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted by G.

C₆-C₃₀aryl, preferably C₆-C₂₄aryl and more preferably C₆-C₁₈aryl, which is unsubstituted or optionally can be substituted by G, is most preferably phenyl, 4-methylphenyl, 4-methoxyphenyl, naphthyl, especially 1-naphthyl, or 2-naphthyl, biphenylyl, triphenylyl, fluoranthenyl, terphenylyl, pyrenyl, 2- or 9-fluorenyl, phenanthryl, or anthryl, which may be unsubstituted or substituted by G. Phenyl, 1-naphthyl and 2-naphthyl are examples of a C₆-C₁₀aryl group.

C₂-C₆₀heteroaryl, preferably C₂-C₃₀heteroaryl, more preferably C₂-C₁₃ heteroaryl represents a ring with five, six or seven ring atoms or a condensed ring system, wherein nitrogen, oxygen or sulfur are the possible heteroatoms, and is typically a heterocyclic group with five to 60 atoms, preferably with five to 30 atoms, more preferably with five to 13 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, benzimidazolo[2,1-b][1,3]benzothiazolyl, carbazolyl, azatriphenylyl, azadibenzofuryl, azadibenzothiophenyl, azacarbazolyl, quinolonyl, isoquinolinyl, quinoxalinyl, quinazolinyl, phenanthrolinyl, phenanthridinyl, benzo[h]quinolonyl, benz[h]isoquinolinyl, benzo[f]isoquinolinyl, benzo[f]quinolinyl, benzo[h]quinazolinyl, benzo[f]quinazolinyl, dibenzo[f,h]quinolonyl, dibenzo[f,h]isoquinolonyl, dibenzo[f,h]quinoxalinyl, dibenzo[f,h]quinazolinyl or phenoxazinyl, which can be unsubstituted or substituted by G. Benzimidazo[1,2-a]benzimidazo-5-yl, benzimidazo[1,2-a]benzimidazo-2-yl, carbazolyl and dibenzofuranyl are examples of a C₂-C₁₄heteroaryl group.

The group C1-C₆₀heteroaryl, preferably C₁-C₃₀heteroaryl, more preferably C₁-C₂₄heteroaryl, most preferably C₂-C₁₃ heteroaryl, even more preferably C₂-C₆₀heteroaryl, C₂-C₃₀heteroaryl, C₂-C₂₄heteroaryl, C₂-C₁₃heteroaryl may be unsubstituted or substituted by G.

A C₂-C₁₃heteroaryl group is for example, benzimidazo[1,2-a]benzimidazo-5-yl ( 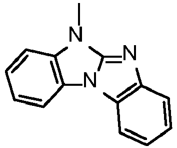 ), benzimidazo[1,2-a]benzimidazo-2-yl ( 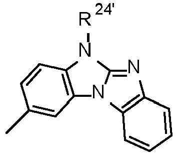 ), benzimidazolo[2,1-b][1,3]benzothiazolyl, benzimidazolo[2,1-b][1,3]benzoxazole, carbazolyl, dibenzofuranyl, or dibenzotihophenyl, which can be unsubstituted or substituted by G, especially by C₆-C₁₀aryl, or C₆-C₁₀aryl, which is substituted by C₁-C₄alkyl; or C₂-C₁₃heteroaryl.

C₁-C₆₀heteroaryl, preferably C₁-C₃₀heteroaryl, more preferably C₁-C₂₄heteroaryl, most preferably C₂-C₁₃ heteroaryl, even more preferably C₂-C₆₀heteroaryl, C₂-C₃₀heteroaryl, C₂-C₂₄heteroaryl, C₂-C₁₃heteroaryl means that the heteroaryl residue comprises at least one, preferably at least 2 carbon atoms and at most 60 carbon atoms in the base skeleton (without substituents). The further atoms in the heteroaryl base skeleton are heteroatoms (N, O and/or S).

R^{24'} is in each case independently C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, phenanthronyl, triphenylenyl, fluoranthenyl or biphenylyl.

C₁-C₂₄heterocyclic group, preferably C₁-C₁₃heterocyclic group, more preferably C₂-C₁₃ heterocyclic group represents a ring with five, six or seven ring atoms or a condensed ring system, wherein nitrogen, oxygen or sulfur are the possible heteroatoms, and is typically a heterocyclic group with five to 24 atoms, preferably with five to 13 atoms. The heterocyclic group may be a C₁-C₂₄heteroaryl group as defined above or a C₁-C₂₄heterocycloalkyl group which may be unsubstituted or substituted by G. Typical C₁-C₂₄heterocycloalkyl groups are oxetan, tetrahydrofuran, tetrahydropyran, oxepane, dioxane, azetidine, pyrrolidine, piperidine, hexahydroazepine, hexahydrodiazepin, tetrahydrothiophene, thietan, tetrahydrothiopyran, thiepan, morpholine as well as bridged heterocycloalkyl systems such as oxabicyclo[4.4.0]decane and azabicyclo[2,2,1]undecane.

C₆-C₂₄arylene groups, preferably C₆-C₁₀arylene groups, which optionally can be substituted by G, preferably C₆-C₁₀arylene groups, which optionally can be substituted by G, are more preferably phenylene, 4-methylphenylene, 4-methoxyphenylene, naphthylene, especially 1-naphthylene, or 2-naphthylene, biphenylylene, triphenylylene, fluoranthenylene, terphenylylene, pyrenylene, 2- or 9-fluorenylene, phenanthrylene, or anthrylene, which may be unsubstituted or substituted by G. Preferred C₆-C₂₄arylen groups, preferably C₆-C₁₀arylene groups are 1,3-phenylene, 3,3'-biphenylylene, 3,3'-m-terphenylene, 2- or 9-fluorenylene, phenanthrylene, which may be unsubstituted or substituted by G.

C₂-C₃₀heteroarylene groups, preferably C₂-C₁₄heteroarylene groups, which are unsubstituted or optionally can be substituted by G, represent a ring with five to seven ring atoms or a condensed ring system, wherein nitrogen, oxygen or sulfur are the possible heteroatoms, and is typically a heterocyclic group with five to 30 atoms having at least six conjugated -electrons such as thienylene, benzothiophenylene, dibenzothiophenylene, thianthrenylene, furylene, furfurylene, 2H-pyranylene, benzofuranylene, isobenzofuranylene, dibenzofuranylene, phenoxythienylene, pyrrolylene, imidazolylene, pyrazolylene, pyridylene, bipyridylene, triazinylene, pyrimidinylene, pyrazinylene, pyridazinylene, indolizinylene, isoindolylene, indolylene, indazolylene, purinylene, quinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, acridinylene, pyrimidinylene, phenanthrolinylene, phenazinylene, isothiazolylene, phenothiazinylene, isoxazolylene, furazanylene, carbazolylene, benzimidazo[1,2-a]benzimidazo-2,5-ylene, or phenoxazinylene, which can be unsubstituted or substituted by G. Preferred C₂-C₃₀heteroarylen groups are pyridylene, triazinylene, pyrimidinylene, carbazolylene, dibenzofuranylene, azatriphenylylene, azadibenzofurylene, azadibenzothiophenylene, azacarbazolylene, quinolonylene, isoquinolinylene, quinoxalinylene, quinazolinylene, phenanthrolinylene, phenanthridinylene, benzo[h]quinolonylene, benz[h]isoquinolinylene, benzo[f]isoquinolinylene, benzo[f]quinolinylene, benzo[h]quinazolinylene, benzo[f]quinazolinylene, dibenzo[f,h]quinolonylene, dibenzo[f,h]isoquinolonylene, dibenzo[f,h]quinoxalinylene, dibenzo[f,h]quinazolinylene and benzimidazo[1,2-a]benzimidazo-2,5-ylene ( 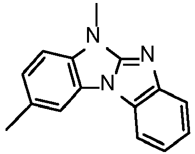 ), which can be unsubstituted or substituted by G, preferably substituted by C₆-C₁₀aryl, C₆-C₁₀aryl which is substituted by C₁-C₄alkyl; or C₂-C₁₃heteroaryl.

If a substituent occurs more than one time in a group, it can be different in each occurrence.

Halo-C₁-C₈alkyl is an alkyl group (as defined above) where at least one of the hydrogen atoms is replaced by a halogen atom. Examples are -CF₃, -CF₂CF₃, -CF₂CF₂CF₃,-CF(CF₃)₂, -(CF₂)₃CF₃, and -C(CF₃)₃.

The wording "substituted by G" means that one, or more, especially one, two or three substituents G might be present. Preferred substituents G are mentioned below.

The wording "substituted by E" means that one, or more, especially one, two or three substituents E might be present. Preferred substituents E are mentioned below.

As described above, the aforementioned alkyl groups may be substituted by E and/or, if desired, interrupted by D. Interruptions are of course possible only in the case of groups containing at least 2 carbon atoms connected to one another by single bonds; C₆-C₁₈aryl is not interrupted; interrupted arylalkyl contains the unit D in the alkyl moiety. C₁-C₁₈alkyl substituted by one or more E and/or interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y'})-CH₂-OR^{y}, where R^{y} is C₁-C₁₈alkyl, C₅-C₁₂cycioaikyi, phenyl, C₇-C₁₅phenylalkyl, and R^{y}' embraces the same definitions as R^{y} or is H.

An alkyl group substituted by E is, for example, an alkyl group where at least one of the hydrogen atoms is replaced by F. Examples are -CF₃, -CF₂CF₃, -CF₂CF₂CF₃, -CF(CF₃)₂, -(CF₂)₃CF₃, and -C(CF₃)₃.

D is -CO-, -COO-, -S-, -SO-, -SO₂-, -O-, -NR65-, -SiR⁷⁰R⁷¹-, -POR⁷²-, -CR⁶³=CR⁶⁴- or-C=C. Suitable residues R63, R⁶⁴, R⁶⁵, R⁷⁰ R⁷¹ and R⁷² are mentioned above. D is preferably -CO-, - COO-, -S-, -SO-, -SO₂-, -O-, -NR⁶⁵-, wherein R⁶⁵ is preferably C₁-C₁₈alkyl, such as methyl, ethyl, n-propyl, iso-propyl, n-butyl, isobutyl, or sec-butyl, or C₆-C₁₄aryl, such as phenyl, tolyl, naphthyl, triphenylyl or biphenylyl, or C₂-C₃₀heteroaryl, such as, for example, benzimidazo[1,2-a]benzimidazo-2-yl ( 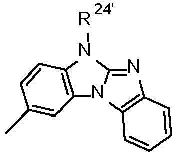 ), carbazolyl, dibenzofuranyl, which can be unsubstituted or substituted especially by C₆-C₁₀aryl, or C₆-C₁₀aryl, which is substituted by C₁-C₄alkyl; or C₂-C₁₃heteroaryl.

E is -OR69, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen. E is preferably-OR⁶⁹; -SR⁶⁹; -NR⁶⁵R⁶⁶; -COR⁶⁸; -COOR⁶⁷; -CON⁶⁵R⁶⁶; or-CN; wherein R⁶⁵, R⁶⁶, R⁶⁷, R⁶⁸ and R⁶⁹ are preferably independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, triphenylyl or biphenylyl.

G is E, or a C₁-C₂₄alkyl group, a C₆-C₃₀aryl group, a C₆-C₃₀aryl group, which is substituted by F, C₁-C₂₄alkyl, or C₁-C₂₄alkyl which is interrupted by O; a C₂-C₆₀heteroaryl group, or a C₂-C₆₀heteroaryl group, which is substituted by F, C₁-C₁₈alkyl, or C₁-C₁₈alkyl which is interrupted by O. G is preferably - OR69, -SR⁶⁹, -NR⁶⁵R⁶⁶; a C₁-C₁₈alkyl group, a C₆-C₁₈aryl group, a C₆-C₁₈aryl group, which is substituted by F, or C₁-C₁₈alkyl; a C₂-C₂₄heteroaryl group, or a C₂-C₂₄heteroaryl group, which is substituted by F, or C₁-C₁₈alkyl; wherein R⁶⁵, R⁶⁶ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl. More preferably, G is a C₆-C₁₈aryl group like phenyl, tolyl, triphenylyl or biphenylyl, or a C₆-C₂₄heteroaryl group like dibenzothiophenylyl, dibenzofuranyl, pyridyl, triazinyl, pyrimidinyl, azatriphenylyl, azadibenzofuryl, azadibenzothiophenyl, azacarbazolyl, quinolonyl, isoquinolinyl, quinoxalinyl, quinazolinyl, phenanthrolinyl, phenanthridinyl, benzo[h]quinolonyl, benz[h]isoquinolinyl, benzo[f]isoquinolinyl, benzo[f]quinolinyl, benzo[h]quinazolinyl, benzo[f]quinazolinyl, dibenzo[f,h]quinolonyl, dibenzo[f,h]isoquinolonyl, dibenzo[f,h]quinoxalinyl or dibenzo[f,h]quinazolinyl.

### Group A

A is a heterocyclic group represented by formula (2) or formula (3); 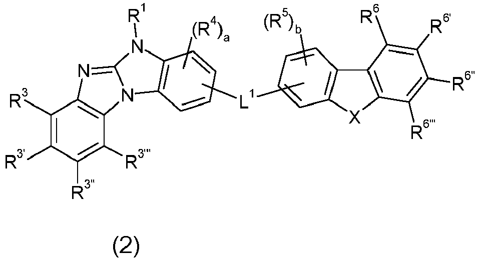 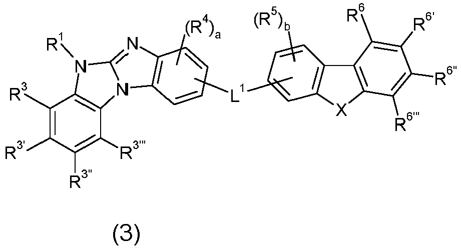 wherein X is O, S, NR⁷ or CR⁸R⁹, preferably NR⁷;
L¹ is single bond, a C₆-C₂₄arylene group, which can optionally be substituted by G, or a C₁-C₂₄heterocyclic group, which can optionally be substituted by G; preferably L¹ is a single bond, 1,2-phenylene, 1,3-phenylene or 1,4-phenylene, more preferably a single bond;
R¹ is phenyl or a group of the following formula 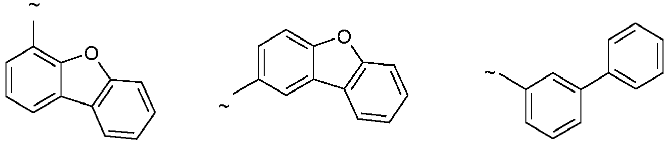 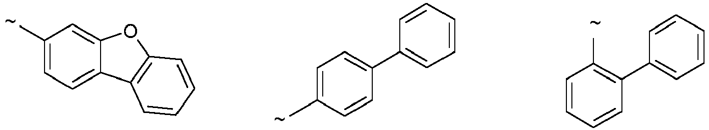 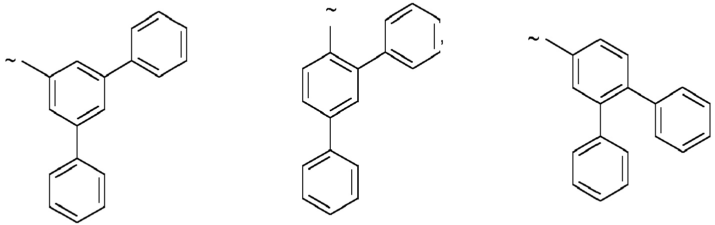 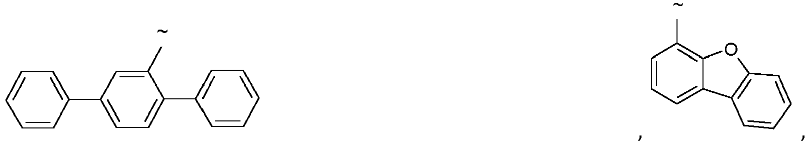 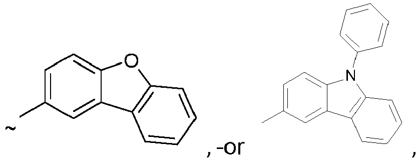 wherein ~ are bonding sites;
R³, R^{3'}, R^{3"}, R^{3‴}, R⁴, R⁵, R⁶, R⁶', R^{6"} and R^{6‴} are H;
G is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen, or a C₁-C₂₄alkyl group, a C₆-C₃₀aryl group, a C₆-C₃₀aryl group, which is substituted by F, C₁-C₂₄alkyl, or C₁-C₂₄alkyl which is interrupted by O; a C₂-C₆₀heteroaryl group, or a C₂-C₆₀heteroaryl group, which is substituted by F, C₁-C₁₈alkyl, or C₁-C₁₈alkyl which is interrupted by O;
R⁶⁵ and R⁶⁶ are independently of each other a C₆-C₁₈aryl group; a C₆-C₁₈aryl which is substituted by C₁-C₁₈alkyl or C₁-C₁₈alkoxy; a C₁-C₁₈alkyl group; or a C₁-C₁₈alkyl group, which is interrupted by-O-, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl; or
R⁶⁵ and R⁶⁶ may form together with the atom to which they are bonded a five or six membered ring;
R⁶⁷ is a C₆-C₁₈aryl group; a C₆-C₁₈aryl group, which is substituted by C₁-C₁₈alkyl or C₁-C₁₈alkoxy; a C₁-C₁₈alkyl group; or a C₁-C₁₈alkyl group, which is interrupted by -O-, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl;
R⁶⁸ is H; a C₆-C₁₈aryl group; a C₆-C₁₈aryl group, which is substituted by C₁-C₁₈alkyl or C₁-C₁₈alkoxy; a C₁-C₁₈alkyl group; or a C₁-C₁₈alkyl group, which is interrupted by -O-, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl;
R⁶⁹ is C₆-C₁₈aryl; a C₆-C₁₈aryl, which is substituted by C₁-C₁₈alkyl or C₁-C₁₈alkoxy; a C₁-C₁₈alkyl group; or a C₁-C₁₈alkyl group, which is interrupted by -O-, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl;
R⁷⁰ is a C₁-C₁₈alkyl group, a C₆-C₁₈aryl group, or a C₆-C₁₈aryl group, which is substituted by C₁-C₁₈alkyl, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl;
wherein R⁷ is replaced by -(B₁)ₒ-(B₂)ₚ-(B₃)_{q}-(B₄)ᵣ-Az.

In the case that the definition -(B₁)ₒ₋(B₂)ₚ₋(B₃)_{q}-(B₄)ᵣ₋Z is used instead of the definition -(B₁)ₒ-(B₂)ₚ-(B₃)_{q}-(B₄)ᵣ-Az, Z and Az have the identical meaning.

The heterocyclic groups represented by formula (2) or (3) are isomeric groups and can also be depicted as follows:

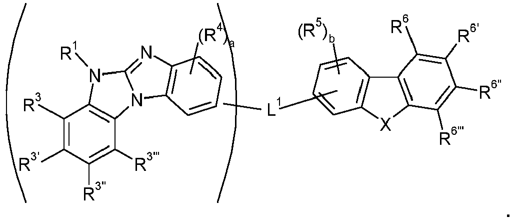

Preferred groups D, E and G are mentioned above.

Preferably, R¹ is phenyl or a group of the following formula 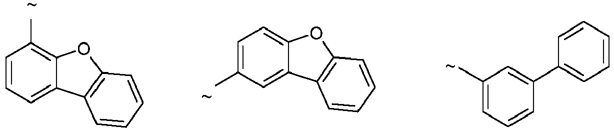 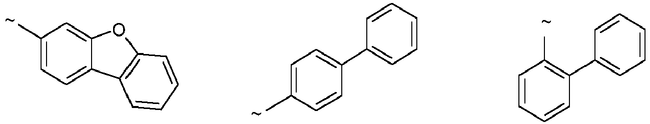 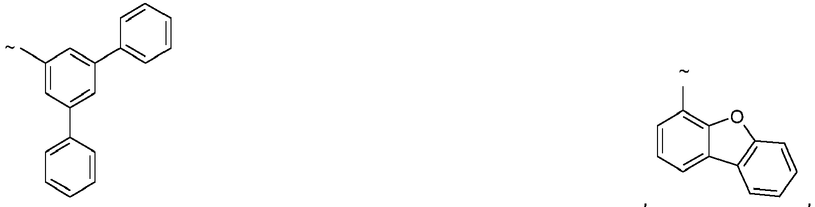 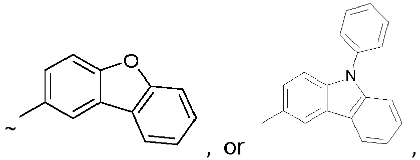 wherein ~ are bonding sites.

X in the heterocyclic derivative of formula (1) is NR⁷, R⁷ is replaced by-(B₁)ₒ-(B₂)ₚ-(B₃)_{q}-(B₄)ᵣ-Az.

L¹ in the heterocyclic derivative of formula (1) is a single bond, a C₆-C₂₄arylene group, which can optionally be substituted by G, or a C₁-C₂₄heterocyclic group, which can optionally be substituted by G; preferably L¹ is a single bond, 1,2-phenylene, 1,3-phenylene or 1,4-phenylene, more preferably a single bond.

Particularly preferred groups A are therefore represented by formula (4), formula (5), formula (6), formula (7), formula (8), formula (9), formula (10), formula (11), formula (12), formula (13), formula (14) or formula (15) and by formula (20), formula (21), formula (22), formula (23), formula (24), formula (25), formula (26), formula (27), formula (28), formula (29), formula (30), formula (31), formula (32), formula (33), formula (34), formula (35), formula (36), formula (37), formula (38) or formula (39): 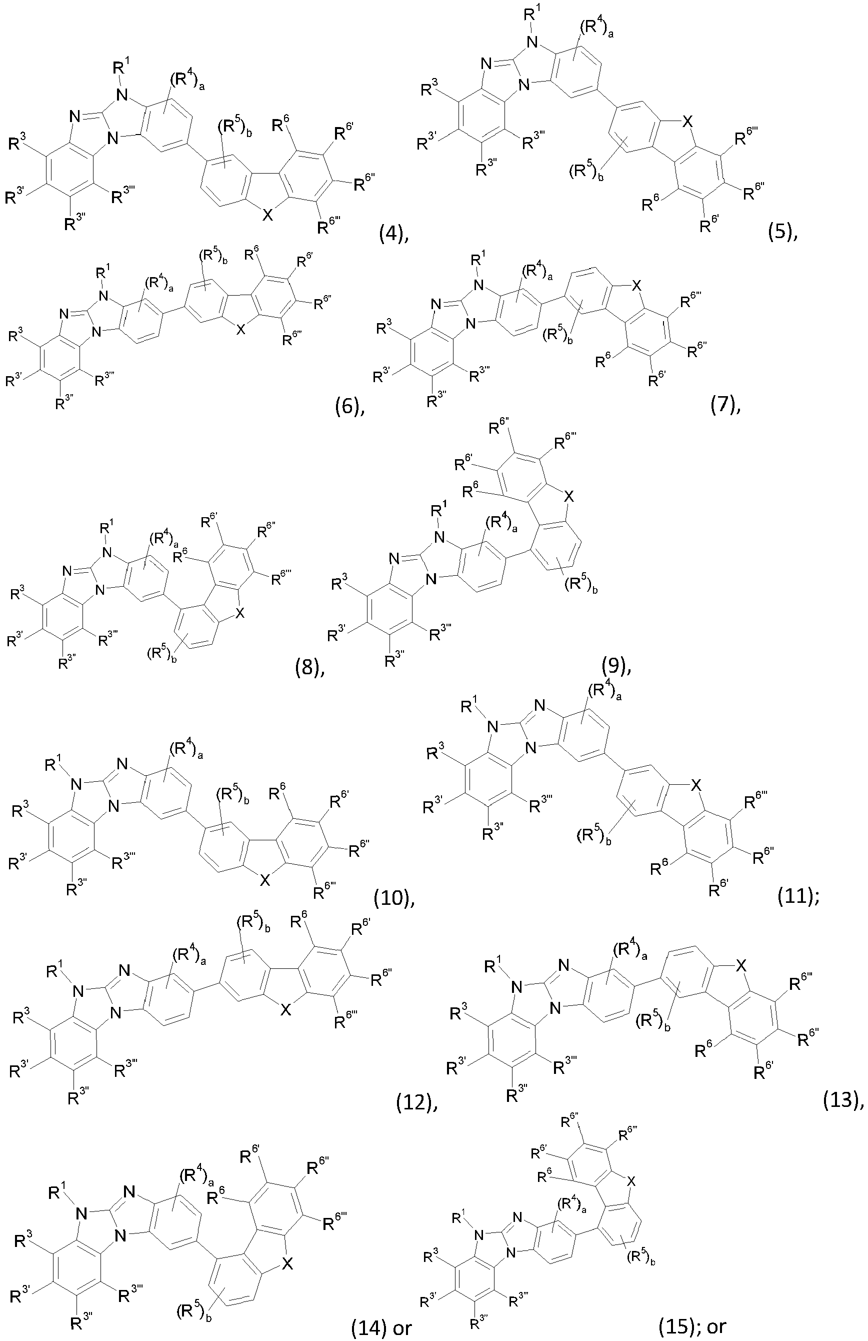 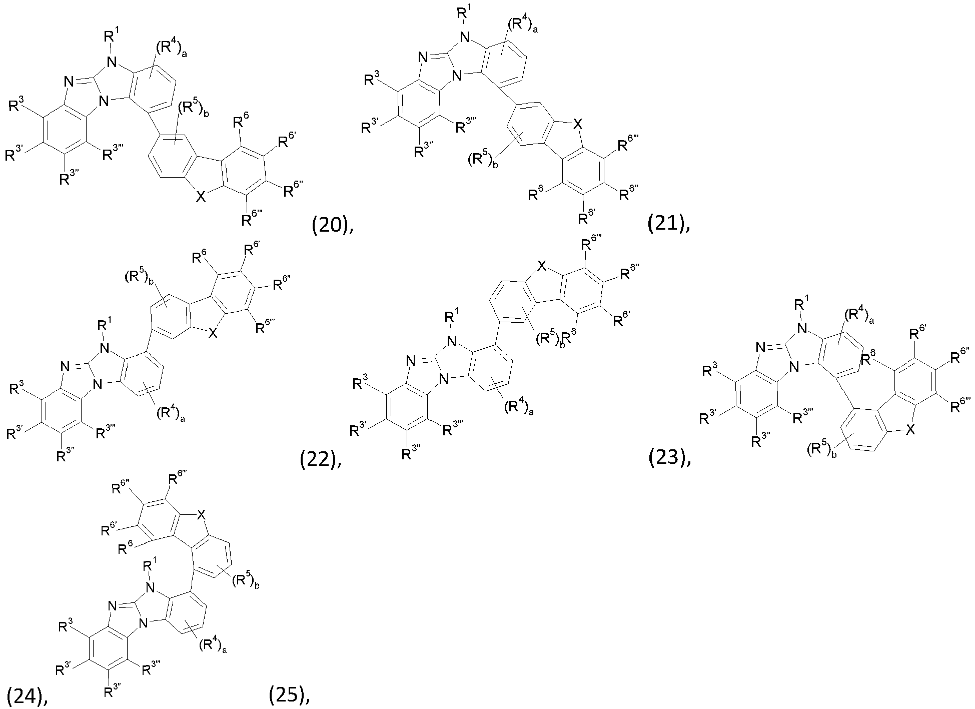 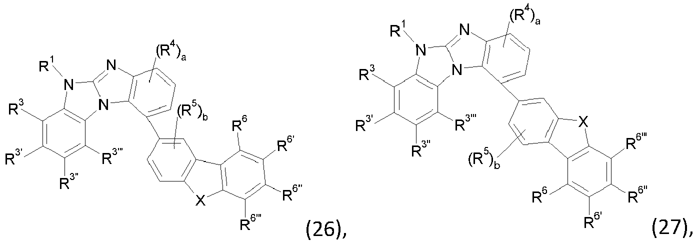 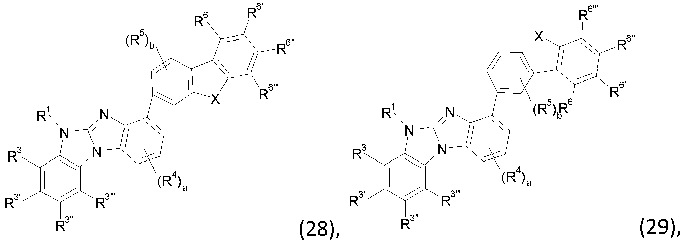 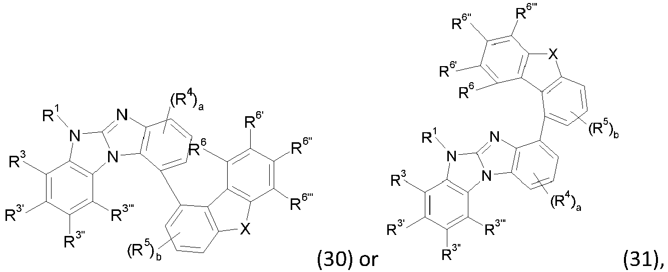 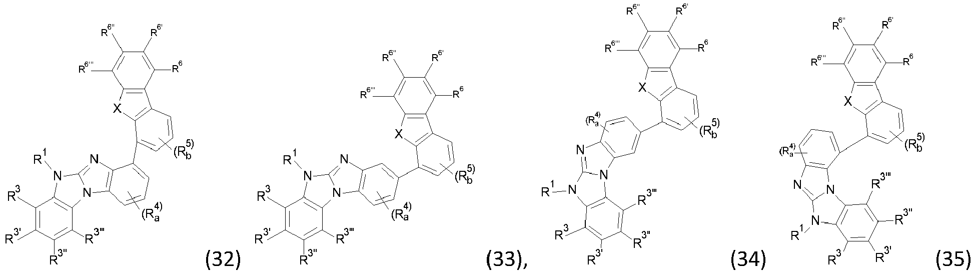 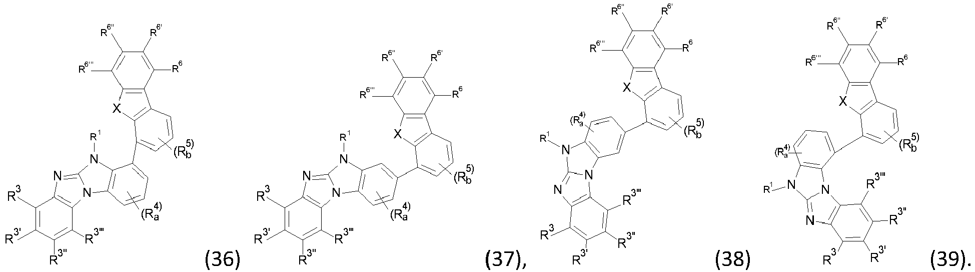 wherein X is NR⁷.

Preferred residues R¹ and R⁷ of the compounds of formula (4), formula (5), formula (6), formula (7), formula (8), formula (9), formula (10), formula (11), formula (12), formula (13), formula (14) or formula (15), formula (20), formula (21), formula (22), formula (23), formula (24), formula (25), formula (26), formula (27), formula (28), formula (29), formula (30), formula (31), formula (32), formula (33), formula (34), formula (35), formula (36), formula (37), formula (38) and formula (39) are mentioned before;
wherein R⁷ is replaced by -(B₁)ₒ-(B₂)ₚ-(B₃)_{q}-(B₄)ᵣ-Az.

Even more preferred groups A are represented by formula (4), formula (5), formula (6), formula (7), formula (8), formula (9), formula (10), formula (11), formula (12), formula (13), formula (14), formula (15) or formula (38); of said groups A, groups A are represented by groups A are represented by formula (4), formula (5), formula (8) and formula (38) are further preferred. Even further preferred are groups A are represented by formula (4) and formula (5).

Most preferred groups A are represented by formula (4), formula (5), formula (8) and formula (38). Even further preferred are groups A are represented by formula (4) and formula (5).

Further even more preferred groups A are: 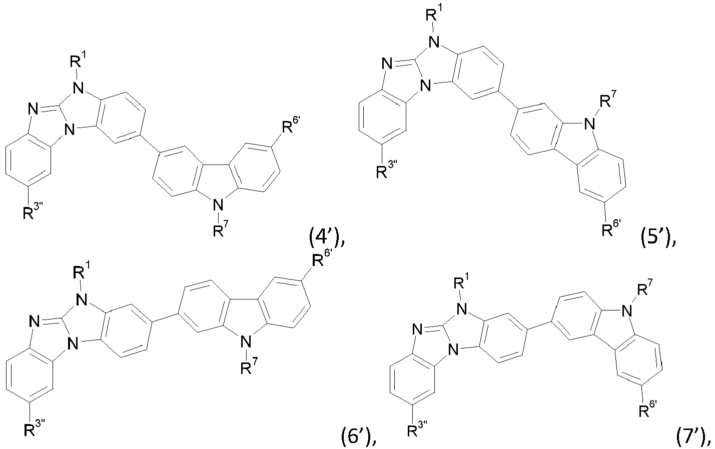 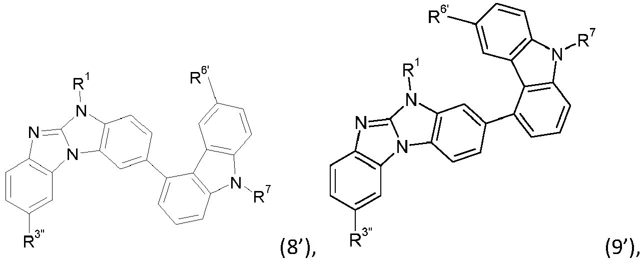 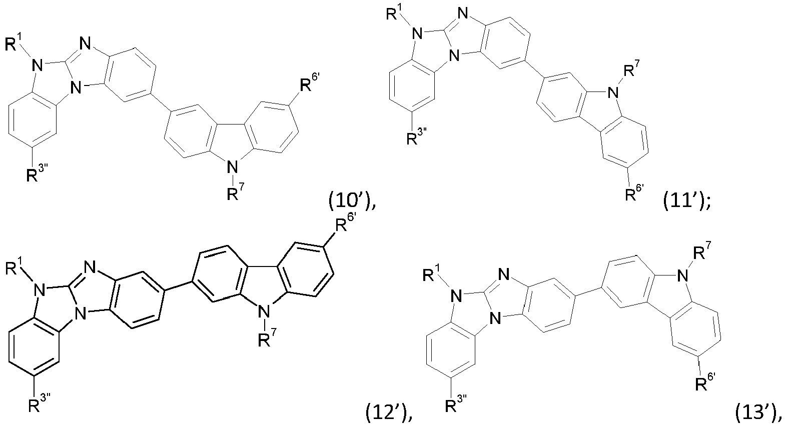 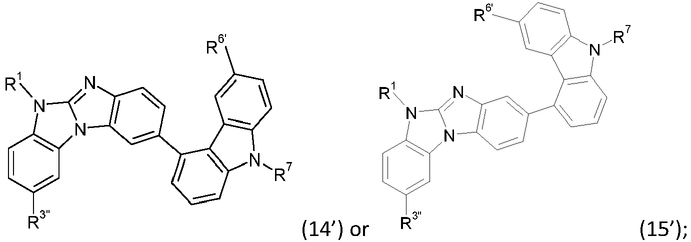 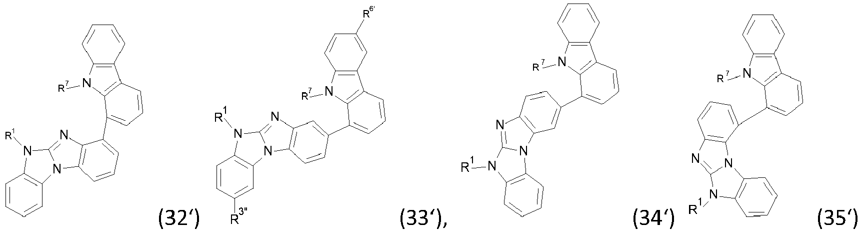 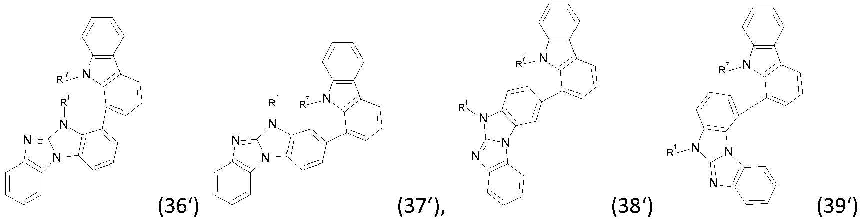 of said groups A, groups A are represented by formula (4'), formula (5'), formula (8') and formula (38') are further preferred. Even further preferred are groups A are represented by formula (4') and formula (5').

Preferred residues R¹ and R⁷ of the compounds of formula (4'), formula (5'), formula (6'), formula (7'), formula (8'), formula (9'), formula (10'), formula (11'), formula (12'), formula (13'), formula (14'), formula (15'), formula (32'), formula (33'), formula (34'), formula (35'), formula (36'), formula (37'), formula (38') and formula (39') are the residues mentioned before;
wherein R⁷ is replaced by -(B₁)ₒ-(B₂)ₚ-(B₃)_{q}-(B₄)ᵣ-Az.

Most preferably, in the compounds of formula (4'), formula (5'), formula (6'), formula (7'), formula (8'), formula (9'), formula (10'), formula (11'), formula (12'), formula (13'), formula (14') and formula (15'), R¹ is phenyl, biphenyl, triphenylyl and R⁷ is replaced by -(B₁)ₒ-(B₂)ₚ-(B₃)_{q}-(B₄)ᵣ-Az. Most preferably R¹ is phenyl and R⁷ is replaced by -(B₁)ₒ-(B₂)ₚ-(B₃)_{q}-(B₄)ᵣ-Az.
**Group -(B₁)ₒ-(B₂)ₚ-(B₃)_{q}-(B₄)ᵣ-**

B₁, B₂, B₃ and B₄ in group -(B₁)ₒ₋(B₂)ₚ₋(B₃)_{q}-(B₄)ᵣ- are independently of each other a C₆-C₂₄arylene group, which can optionally be substituted by G, or a C₁-C₂₄ heteroarylene group, which can optionally be substituted by G;

Preferred groups B₁, B₂, B₃ and B₄ are independently of each other a C₆-C₁₀arylene group, which can optionally be substituted by G, or a C₅-C₂₄heteroarylene group, which optionally can be substituted by G, characterized by a ring with five to seven ring atoms or a condensed ring system, wherein nitrogen, oxygen or sulfur are the possible heteroatoms, and having at least six conjugated-electrons.

Preferred groups B₁, B₂, B₃ and B₄ are independently of each other phenylene, naphthylene, especially 1-naphthylene, or 2-naphthylene, biphenylylene, triphenylylene, terphenylylene, pyrenylene, 2- or 9-fluorenylene, phenanthrylene or anthrylene, which are unsubstituted or can optionally be substituted by G;
benzothiophenylene, thianthrenylene, furylene, furfurylene, 2H-pyranylene, benzofuranylene, isobenzofuranylene, dibenzofuranylene ( 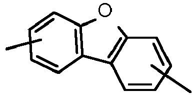 ), dibenzothiophenylene ( 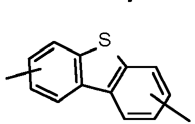 ), carbazolylene ( 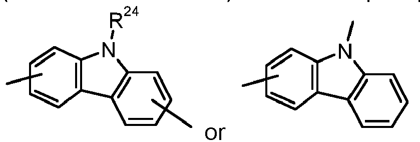 ), imidazolylene, pyrazolylene, pyridylene, bipyridylene, triazinylene, pyrimidinylene, pyrazinylene, pyridazinylene, indolizinylene, isoindolylene, indolylene, indazolylene, purinylene, quinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, pyrimidinylene, benzimidazo[1,2-a]benzimidazo-2,5-ylene ( 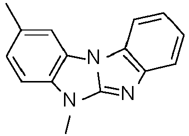 ),which can be unsubstituted or substituted by G; R²⁴ is a C₆-C₂₄aryl group, or a C₂-C₃₀heteroaryl group, which can optionally be substituted by G, wherein G is as defined in above; wherein the lines are bonding sites; 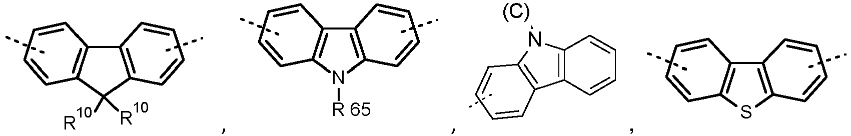 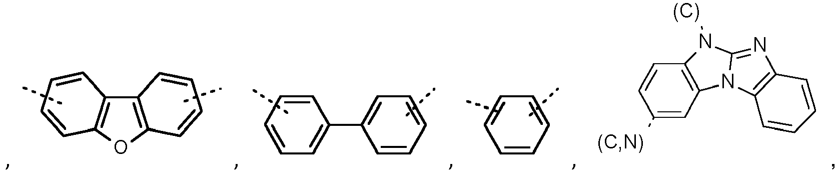 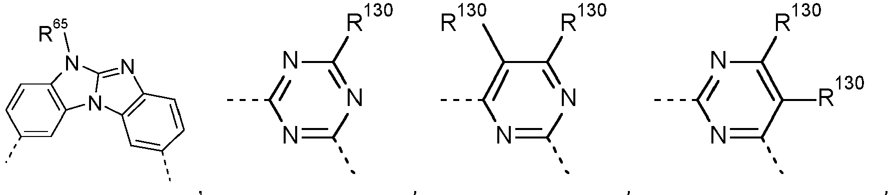 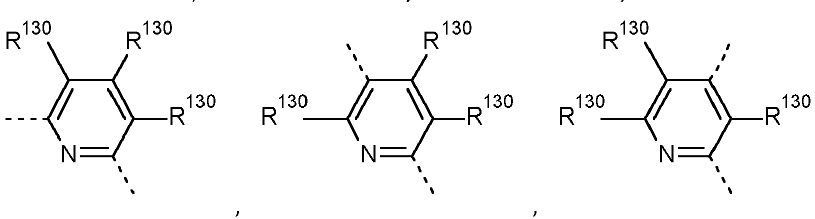 which can be unsubstituted or substituted by G. R⁶⁵ is a C₆-C₁₈aryl group; a C₆-C₁₈aryl which is substituted by C₁-C₁₈alkyl or C₁-C₁₈alkoxy; a C₁-C₁₈alkyl group; or a C₁-C₁₈alkyl group, which is interrupted by-O-, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl; R¹⁰ a C₁-C₂₅alkyl group, which can optionally be substituted by E and or interrupted by D; a C₆-C₂₄aryl group, which can optionally be substituted by G, or a C₁-C₂₄heteroaryl group, which can optionally be substituted by G; and/or two adjacent groups of the groups R¹⁰ may form together with the atom to which they are bonded a ring structure, which can optionally be substituted by G; R¹³⁰ is independently in each occurrence H or C₆-C₂₄arylene group, which can optionally be substituted by G, or a C₂-C₃₀heteroarylene group, which can optionally be substituted by G; wherein G is as defined in above; wherein the dotted lines are bonding sites;
wherein (C)- has the meaning that the bonding site of the group B₁, B₂, B₃ or B₄ is linked to a C-atom, and (N)- has the meaning that the bonding site of the group B₁, B₂, B₃ or B₄ is linked to a N-atom, and (C,N) has the meaning that the bonding site of the group B₁, B₂, B₃ or B₄ is linked to a C or N-atom.

B₁, B₂, B₃ and B₄ are most preferably in each occurrence independently of each other a group of the formula: 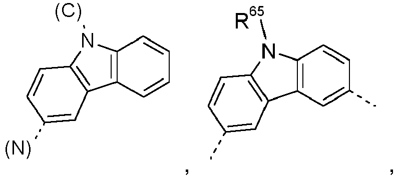 preferably 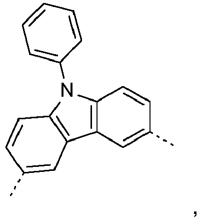 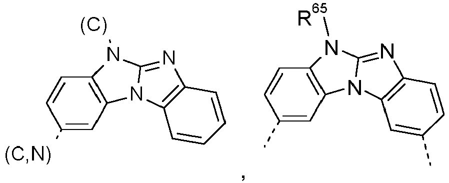 , preferably 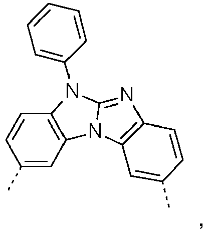 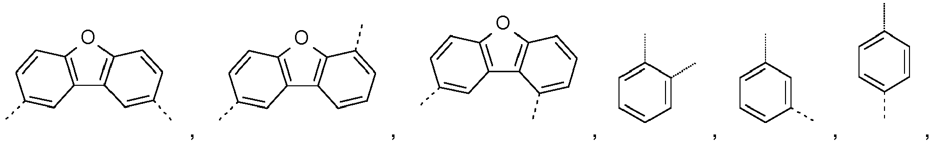 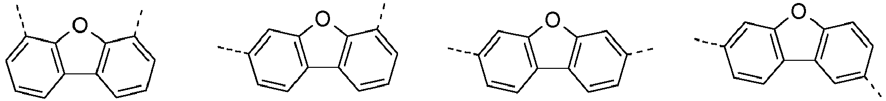 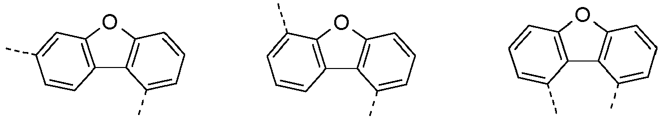 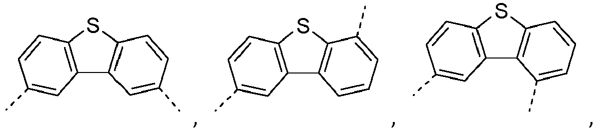 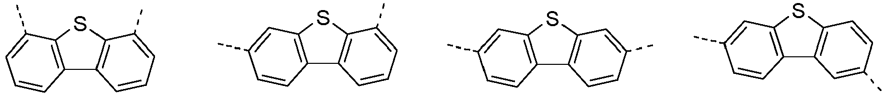 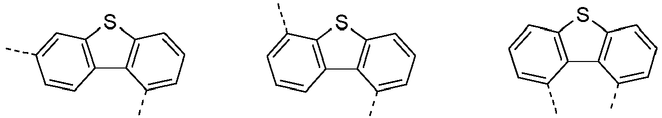 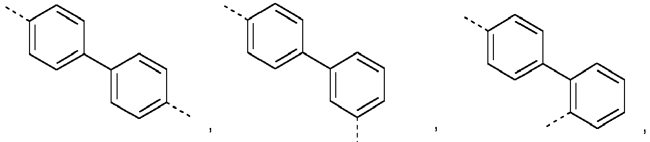 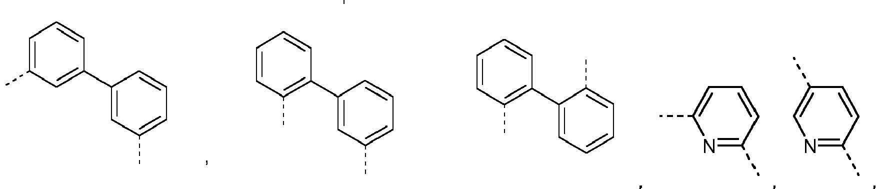 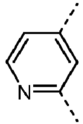 which can be unsubstituted or substituted by G;
wherein (C)- has the meaning that the bonding site of the group B₁, B₂, B₃ or B₄ is linked to a C-atom, and (N)- has the meaning that the bonding site of the group B₁, B₂, B₃ or B₄ is linked to a N-atom, and (C,N) has the meaning that the bonding site of the group B₁, B₂, B₃ or B₄ is linked to a C or N-atom; and the dotted lines are bonding sites.

Most preferred groups B₁, B₂, B₃ and B₄ are: 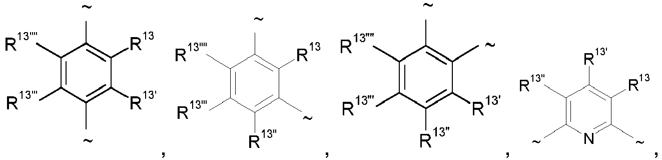 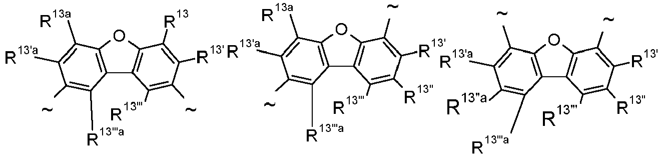 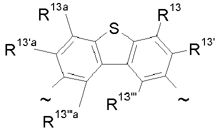 wherein
R13, R^{13'}, R^{13"}, R^{13‴}, R^{13""}, R^{13a}, R^{13'a}, R^{13"a} and R^{13‴a}
are independently of each other H, a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G;
D is -CO-, -COO-, -S-, -SO-, -SO₂-, -O-, -NR65-, -SiR⁷⁰R⁷¹-, -POR⁷²-, -CR⁶³=CR⁶⁴-, or-C=C_{;}
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
~ are bonding sites to the neighboring groups.

Suitable and preferred groups R⁶³, R⁶⁴, R⁶⁵, R⁶⁶, R⁶⁷, R⁶⁸, R⁶⁹, R⁷⁰, R⁷¹, R⁷² are mentioned above. Preferred alkyl groups, aryl groups, heteroaryl groups, and preferred groups D, E and G are mentioned above.

Most preferably, B₁, B₂, B₃ and B₄ are:
independently of each other a C₆-C₂₄arylene group, which can optionally be substituted by G, preferably phenylene, biphenylylene, triphenylylene, naphthylene, especially 1-naphthylene, or 2-naphthylene, terphenylylene, pyrenylene, 2- or 9-fluorenylene, phenanthrylene or anthrylene, which are unsubstituted or can optionally be substituted by G;
more preferably 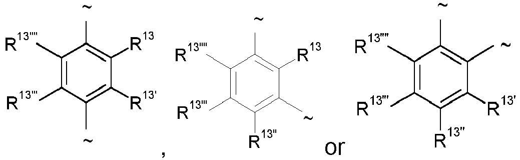 wherein
R¹³, R^{13'}, R^{13"}, R^{13‴} and R^{13""} are defined above, and
~ are bonding sites to the neighboring groups.

o is 0 or 1, p is 0 or 1, q is 0 or 1, r is 0 or 1, preferably, o is 0 or 1, p is 0 or 1 and q and r are 0, more preferably, o is 0 or 1 and p, q and r are 0, most preferably at least one of o, p, q and r is 1.

Examples for suitable groups -(B₁)ₒ₋(B₂)ₚ₋(B₃)_{q}-(B₄)ᵣ₋ are: 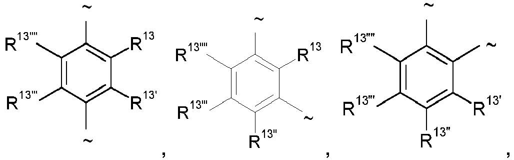 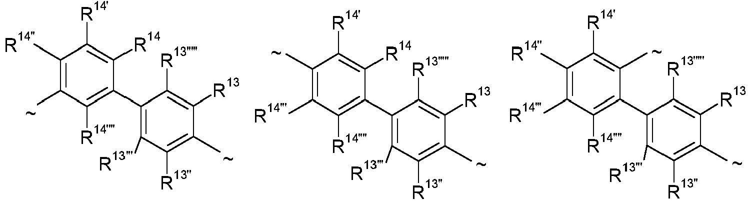 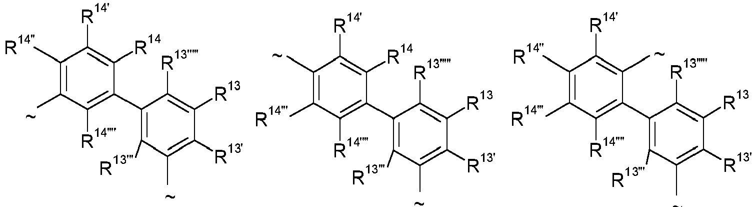 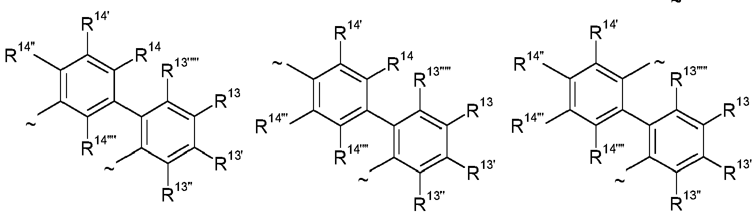 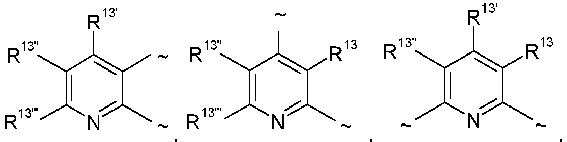 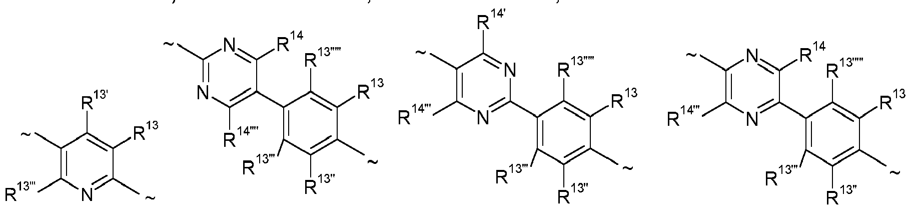 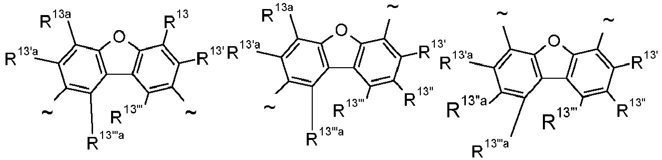 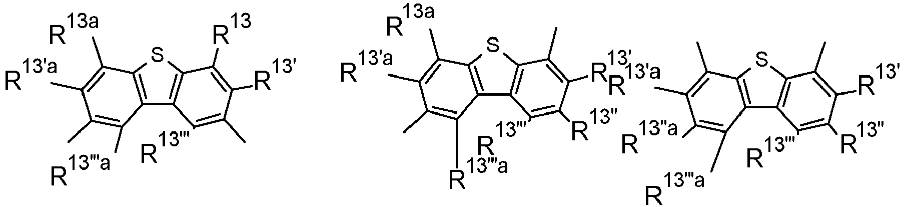 wherein
R¹³, R^{13'}, R^{13"}, R^{13‴}, R^{13""}, R^{13a}, R^{13'a}, R^{13"a} and R^{13‴a}, R14, R^{14'}, R^{14‴}, R^{14‴} and R^{14""}
are independently of each other H, a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G;
D is -CO-, -COO-, -S-, -SO-, -SO₂-, -O-, -NR65-, -SiR⁷⁰R⁷¹-, -POR⁷²-, -CR⁶³=CR⁶⁴-, or-C=C;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
~ are bonding sites to the neighboring groups.

Suitable and preferred groups R⁶³, R⁶⁴, R⁶⁵, R⁶⁶, R⁶⁷, R⁶⁸, R⁶⁹, R⁷⁰, R⁷¹, R⁷² are mentioned above. Preferred alkyl groups, aryl groups, heteroaryl groups, and preferred groups D, E and G are mentioned above.

Preferably, R¹³, R^{13'}, R^{13"}, R^{13‴}, R^{13""}, R^{13a}, R^{13'a}, R^{13"a}, R^{13‴a}, R¹⁴, R^{14'}, R^{14‴}, R^{14‴} and R^{14""} are H; and
~ are bonding sites to the neighboring groups.

Most preferred groups -(B₁)ₒ₋(B₂)ₚ₋(B₃)_{q}-(B₄)ᵣ₋ are: 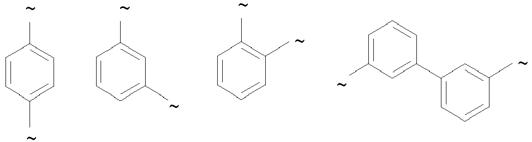 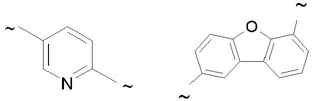 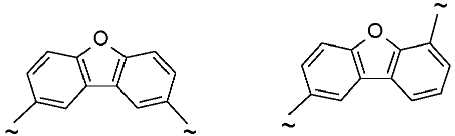 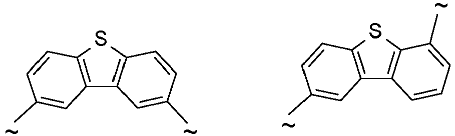 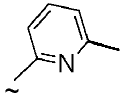 wherein
~ are bonding sites to the neighboring groups.

### Group Az

Az is represented by one of the following formulae (16), (17) or (18) 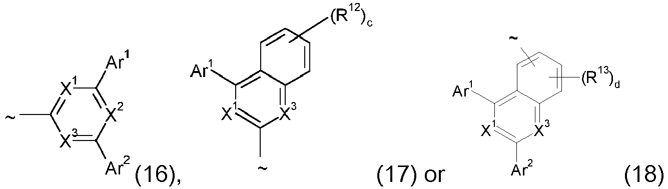 wherein
X¹, X² and X³ are independently of each other CR¹¹ or N, wherein in formula (16) at least one of X¹ to X³ is N, and wherein in formulae (17) and (18) at least one of X¹ and X³ is N;
Ar₁ and Ar₂ are independently of each other a C₆-C₂₄ aryl group, which is optionally substituted by G, or a C₁-C₂₄ heteroaryl group, which is optionally substituted by G;
R¹¹, R¹² and R¹³ are independently of each other H, a C₆-C₂₄ aryl group which can be substituted by G, a C₁-C₂₄ heteroaryl group which can be substituted by G or a C₁-C₂₅alkyl group, which can optionally be substituted by E and/or interrupted by D; preferably, H;
D is -CO-, -COO-, -S-, -SO-, -SO₂-, -O-, -NR65-, -SiR⁷⁰R⁷¹-, -POR⁷²-, -CR⁶³=CR⁶⁴-, or-C=C;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ may form together with the atom to which they are bonded a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by-O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl;
c is 0, 1, 2, 3 or 4; and
d is 0, 1, 2 or 3;
~ are bonding sites to the neighboring groups.

Preferably, Az is represented by one of the following formulae 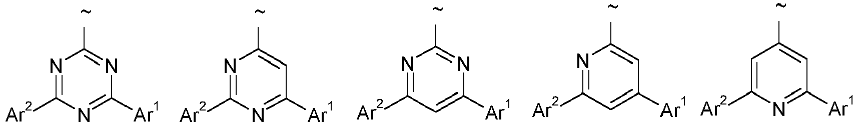 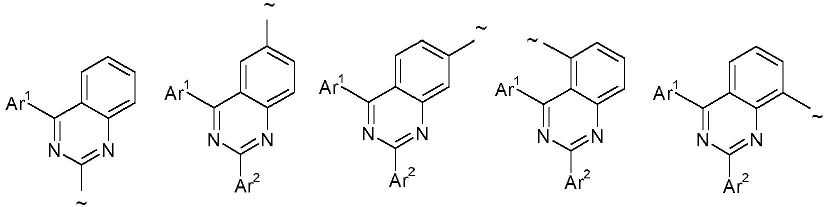 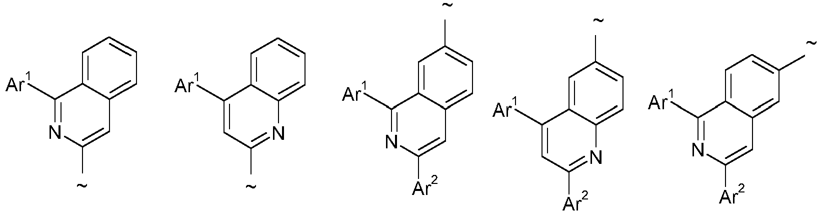 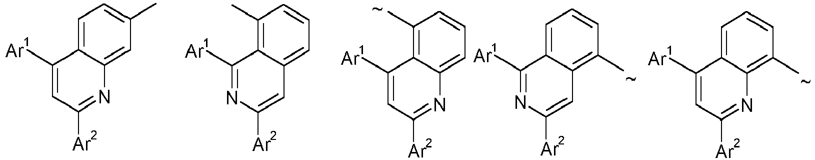 wherein
Ar₁ and Ar₂ are independently of each other a C₆-C₂₄ aryl group, which is optionally substituted by G, or a C₁-C₂₄ heteroaryl group, which is optionally substituted by G;
G is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃, halogen, a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ may form together with the atom to which they are bonded a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by-O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl;
∼ are bonding sites to the neighboring groups.

Preferably, Ar₁ and Ar₂ are unsubstituted phenyl or a group of the following formula 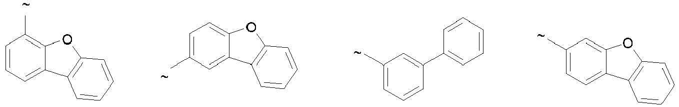 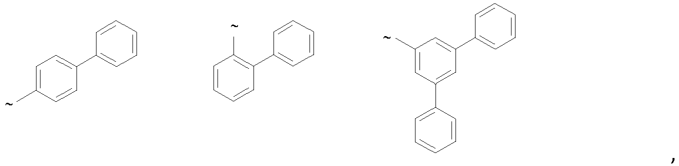 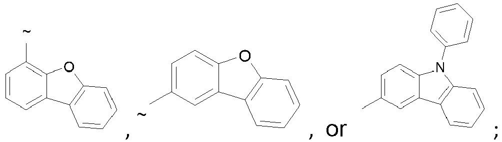 wherein
~ are bonding sites to the neighboring groups.
most preferably, Ar₁ and Ar₂ are unsubstituted phenyl.

### Heterocyclic derivative of formula (1)

The heterocyclic derivatives according to the present invention are represented by formula (1):

A-[(B₁)ₒ-(B₂)ₚ-(B₃)_{q}-(B₄)ᵣAz]_{z} (1)

wherein
z is 1; and
wherein R⁷ is replaced by -(B₁)ₒ-(B₂)ₚ-(B₃)_{q}-(B₄)ᵣ-Az.

The groups A, -(B₁)ₒ₋(B₂)ₚ₋(B₃)_{q}-(B₄)ᵣ₋ and Az have been defined before.

The heterocyclic derivatives of formula (1) are therefore heterocyclic derivatives of formula (1'):

A-(B₁)ₒ-(B₂)ₚ-(B₃)_{q}-(B₄)ᵣAz (1')

wherein
the groups A, -(B₁)ₒ₋(B₂)ₚ₋(B₃)_{q}-(B₄)ᵣ₋ and Az have been defined before.

Specific examples of the compounds represented by the formula (1) are given below. The compounds represented by the formula (1) are not limited to the following specific examples.

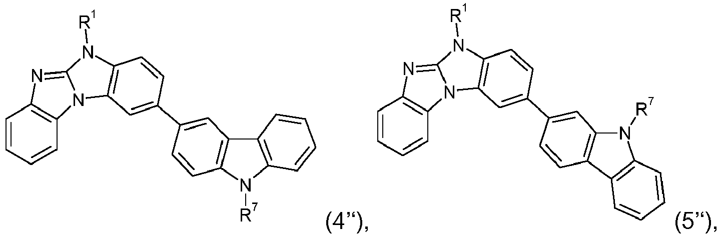

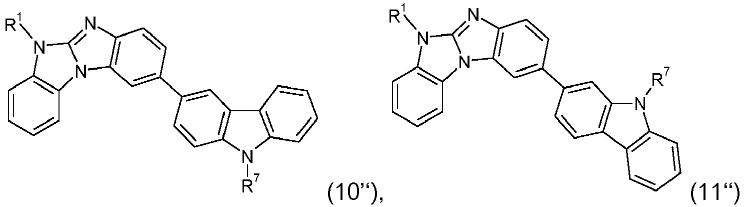

| Nr. | R¹ | R⁷ |
|---|---|---|
| 4"-1, 5"-1, 10"-1, 11"-1 | Ph | 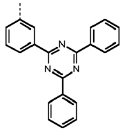 |
| 4"-2, 5"-2, 10"-2, 11"-2 | Ph | 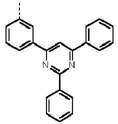 |
| 4"-3, 5"-3, 10"-3, 11 "-3 | Ph | 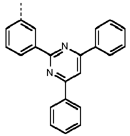 |
| 4"-4, 5"-4, 10"-4, 11 "-4 | Ph | 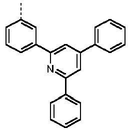 |
| 4"-5, 5"-5, 10"-5, 11 "-5 | Ph | 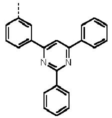 |
| 4"-6, 5"-6, 10"-6, 11 "-6 | Ph | 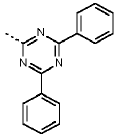 |
| 4"-7, 5"-7, 10"-7, 11 "-7 | Ph | 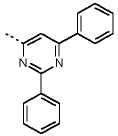 |
| 4"-8, 5"-8, 10"-8, 11 "-8 | Ph | 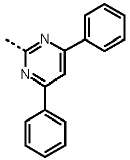 |
| 4"-9, 5"-9, 10"-9, 11"-9 | Ph | 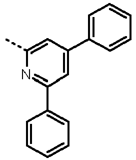 |
| 4"-10, 5"-10, 10"-10, 11"-10 | Ph | 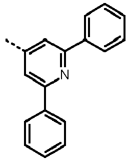 |
| 4"-11, 5"-11, 10"-11 , 11"-11 | Ph | 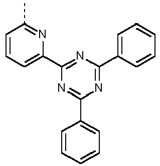 |
| 4"-12, 5"-12, 10"-12, 11"-12 | Ph | 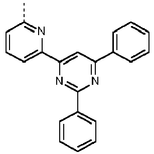 |
| 4"-13, 5"-13, 10"-13, 11"-13 | Ph | 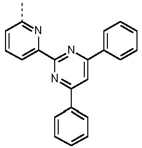 |
| 4"-14, 5"-14, 10"-14, 11"-14 | Ph | 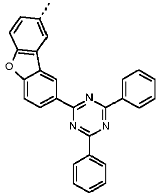 |
| 4"-15, 5"-15, 10"-15, 11"-15 | Ph | 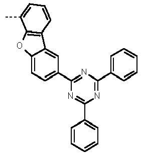 |
| 4"-16, 5"-16, 10"-16, 11"-16 | Ph | 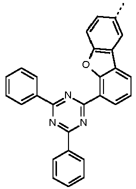 |
| 4"-17, 5"-17, 10"-17, 11"-17 | Ph | 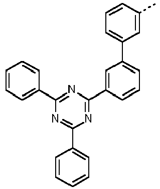 |
| 4"-18, 5"-18, 10"-18, 11"-18 | Ph | 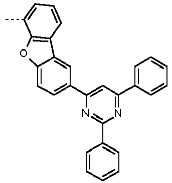 |
| 4"-19, 5"-19, 10"-19, 11"-19 | Ph | 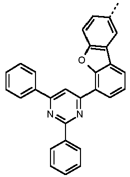 |
| 4"-20, 5"-20, 10"-20, 11"-20 | Ph | 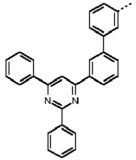 |
| 4"-21, 5"-21, 10"-21, 11"-21 | Ph | 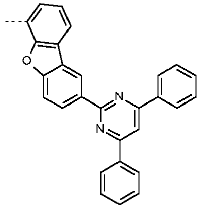 |
| 4"-22, 5"-22, 10"-22, 11"-22 | Ph | 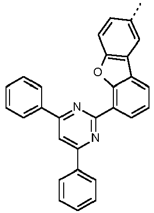 |
| 4"-23, 5"-23, 10"-23, 11"-23 | Ph | 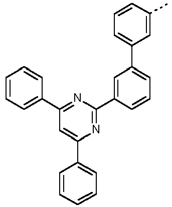 |
| 4"-24, 5"-24, 10"-24, 11"-24 | Ph | 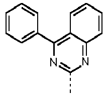 |
| 4"-25, 5"-25, 10"-25, 11"-25 | Ph | 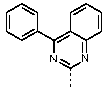 |
| 4"-26, 5"-26, 10"-26, 11"-26 | Ph | 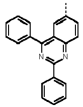 |
| 4"-27, 5"-27, 10"-27, 11"-27 | Ph | 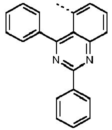 |
| 4"-28, 5"-28, 10"-28, 11"-28 | Ph | 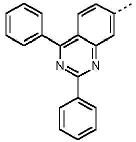 |
| 4"-29, 5"-29, 10"-29, 11"-29 | Ph | 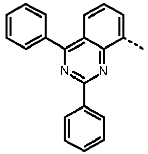 |
| 4"-30, 5"-30, 10"-30, 11"-30 | Ph | 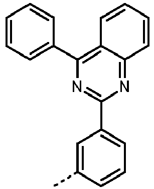 |
| 4"-31, 5"-31, 10"-31, 11"-31 | Ph | 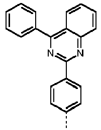 |
| 4"-32, 5"-32, 10"-32, 11"-32 | Ph | 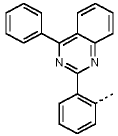 |
| 4"-33, 5"-33, 10"-33, 11"-33 | 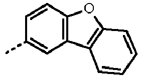 | 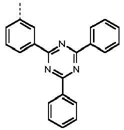 |
| 4"-34, 5"-34, 10"-34, 11"-34 | 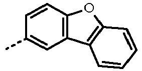 | 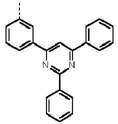 |
| 4"-35, 5"-35, 10"-35, 11"-35 | 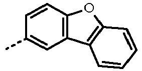 | 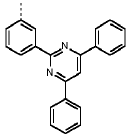 |
| 4"-36, 5"-36, 10"-36, 11"-36 | 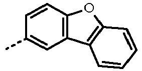 | 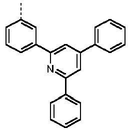 |
| 4"-37, 5"-37, 10"-37, 11"-37 | 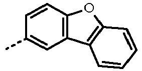 | 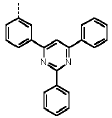 |
| 4"-38, 5"-38, 10"-38, 11"-38 | 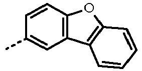 | 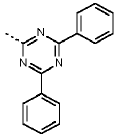 |
| 4"-39, 5"-39, 10"-39, 11"-39 | 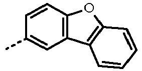 | 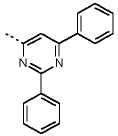 |
| 4"-40, 5"-40, 10"-40, 11"-40 | 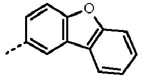 | 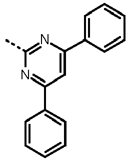 |
| 4"-41, 5"-41, 10"-41 , 11"-41 | 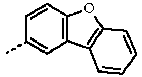 | 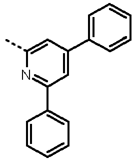 |
| 4"-42, 5"-42, 10"-42, 11"-42 | 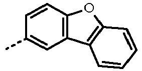 | 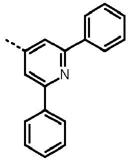 |
| 4"-43, 5"-43, 10"-43, 11"-43 | 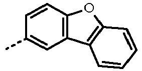 | 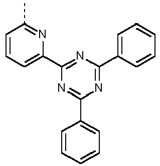 |
| 4"-44, 5"-44, 10"-44, 11"-44 | 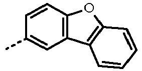 | 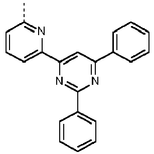 |
| 4"-45, 5"-45, 10"-45, 11"-45 | 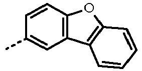 | 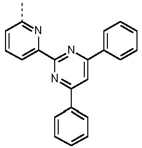 |
| 4"-46, 5"-46, 10"-46, 11"-46 | 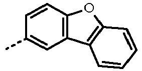 | 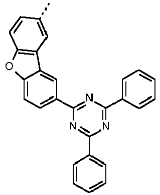 |
| 4"-47, 5"-47, 10"-47, 11"-47 | 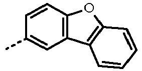 | 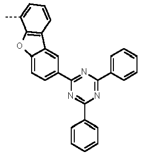 |
| 4"-48, 5"-48, 10"-48, 11"-48 | 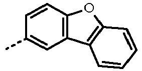 | 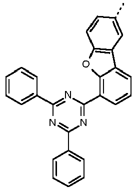 |
| 4"-49, 5"-49, 10"-49, 11"-49 | 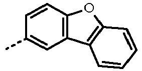 | 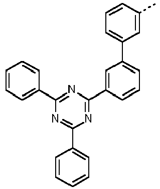 |
| 4"-50, 5"-50, 10"-50, 11"-50 | 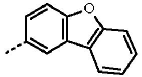 | 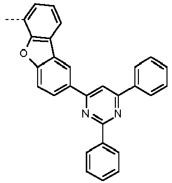 |
| 4"-51, 5"-51, 10"-51, 11"-51 | 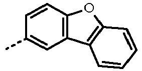 | 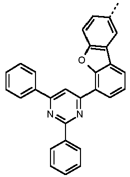 |
| 4"-52, 5"-52, 10"-52, 11"-52 | 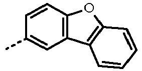 | 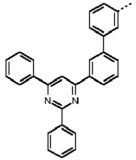 |
| 4"-53, 5"-53, 10"-53, 11"-53 | 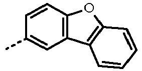 | 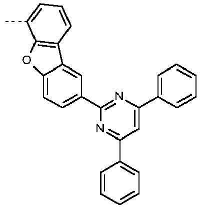 |
| 4"-54, 5"-54, 10"-54, 11"-54 | 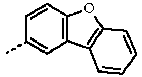 | 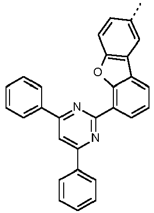 |
| 4"-55, 5"-55, 10"-55, 11"-55 | 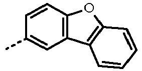 | 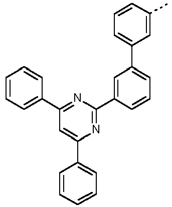 |
| 4"-56, 5"-56, 10"-56, 11"-56 | 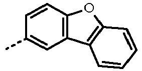 | 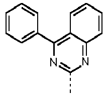 |
| 4"-57, 5"-57, 10"-57, 11"-57 | 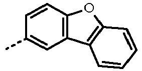 | 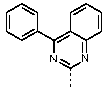 |
| 4"-58, 5"-58, 10"-58, 11"-58 | 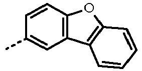 | 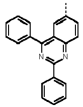 |
| 4"-59, 5"-59, 10"-59, 11"-59 | 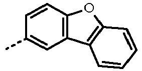 | 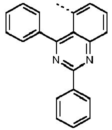 |
| 4"-60, 5"-60, 10"-60, 11"-60 | 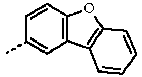 | 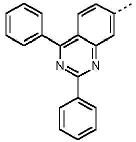 |
| 4"-61, 5"-61, 10"-61, 11"-61 | 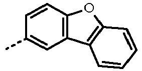 | 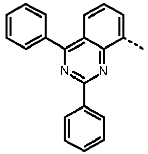 |
| 4"-62, 5"-62, 10"-62, 11"-62 | 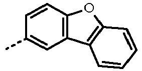 | 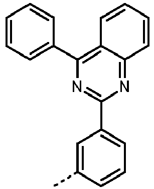 |
| 4"-63, 5"-63, 10"-63, 11"-63 | 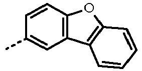 | 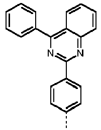 |
| 4"-64, 5"-64, 10"-64, 11"-64 | 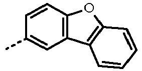 | 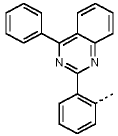 |
| 4"-65, 5"-65, 10"-65, 11"-65 | 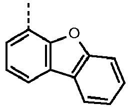 | 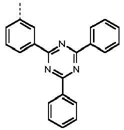 |
| 4"-66, 5"-66, 10"-66, 11"-66 | 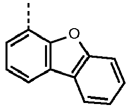 | 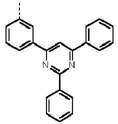 |
| 4"-67, 5"-67, 10"-67, 11"-67 | 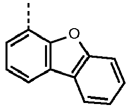 | 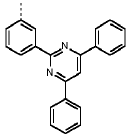 |
| 4"-68, 5"-68, 10"-68, 11"-68 | 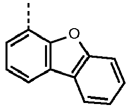 | 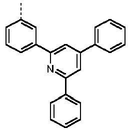 |
| 4"-69, 5"-69, 10"-69, 11"-69 | 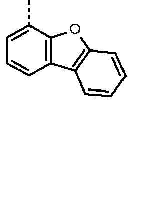 | 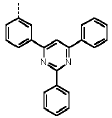 |
| 4"-70, 5"-70, 10"-70, 11"-70 | 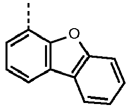 | 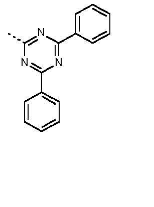 |
| 4"-71, 5"-71, 10"-71, 11"-71 | 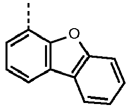 | 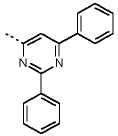 |
| 4"-72, 5"-72, 10"-72, 11"-72 | 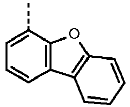 | 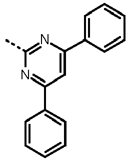 |
| 4"-73, 5"-73, 10"-73, 11"-73 | 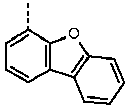 | 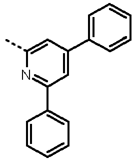 |
| 4"-74, 5"-74, 10"-74, 11"-74 | 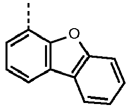 | 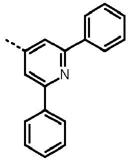 |
| 4"-75, 5"-75, 10"-75, 11"-75 | 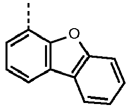 | 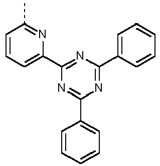 |
| 4"-76, 5"-76, 10"-76, 11"-76 | 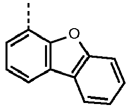 | 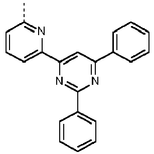 |
| 4"-77, 5"-77, 10"-77, 11"-77 | 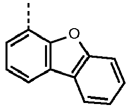 | 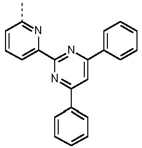 |
| 4"-78, 5"-78, 10"-78, 11"-78 | 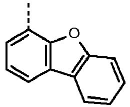 | 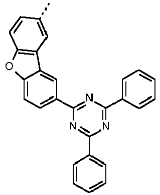 |
| 4"-79, 5"-79, 10"-79, 11"-79 | 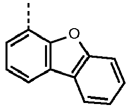 | 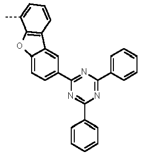 |
| 4"-80, 5"-80, 10"-80, 11"-80 | 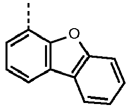 | 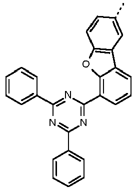 |
| 4"-81, 5"-81, 10"-81, 11"-81 | 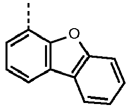 | 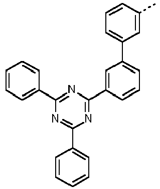 |
| 4"-82, 5"-82, 10"-82, 11"-82 | 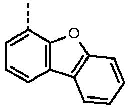 | 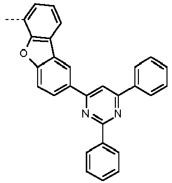 |
| 4"-83, 5"-83, 10"-83, 11"-83 | 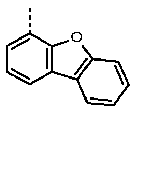 | 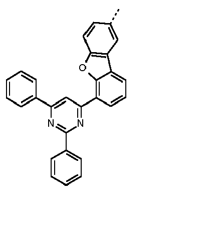 |
| 4"-84, 5"-84, 10"-84, 11"-84 | 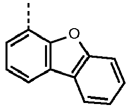 | 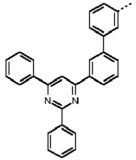 |
| 4"-85, 5"-85, 10"-85, 11"-85 | 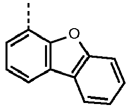 | 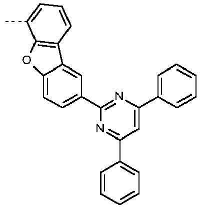 |
| 4"-86, 5"-86, 10"-86, 11"-86 | 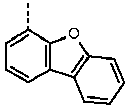 | 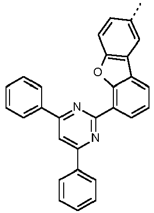 |
| 4"-87, 5"-87, 10"-87, 11"-87 | 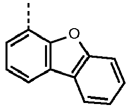 | 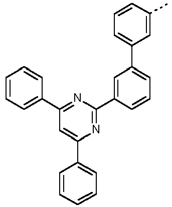 |
| 4"-88, 5"-88, 10"-88, 11"-88 | 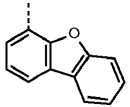 | 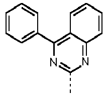 |
| 4"-89, 5"-89, 10"-89, 11"-89 | 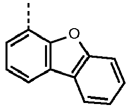 | 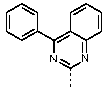 |
| 4"-90, 5"-90, 10"-90, 11"-90 | 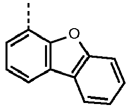 | 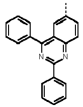 |
| 4"-91, 5"-91, 10"-91, 11"-91 | 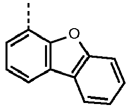 | 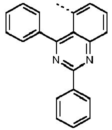 |
| 4"-92, 5"-92, 10"-92, 11"-92 | 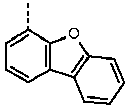 | 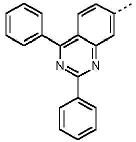 |
| 4"-93, 5"-93, 10"-93, 11"-93 | 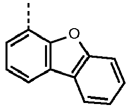 | 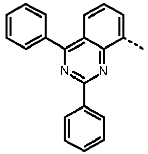 |
| 4"-94, 5"-94, 10"-94, 11"-94 | 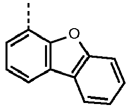 | 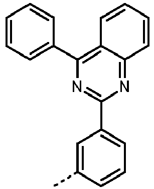 |
| 4"-95, 5"-95, 10"-95, 11"-95 | 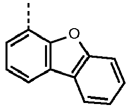 | 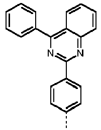 |
| 4"-96, 5"-96, 10"-96, 11"-96 | 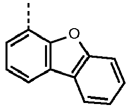 | 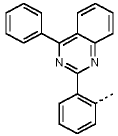 |
| 4"-97, 5"-97, 10"-97, 11"-97 | 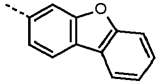 | 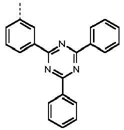 |
| 4"-98, 5"-98, 10"-98, 11"-98 | 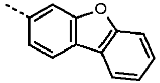 | 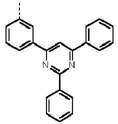 |
| 4"-99, 5"-99, 10"-99, 11"-99 | 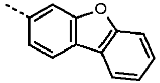 | 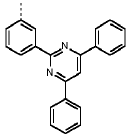 |
| 4"-100, 5"-100, 10"-100, 11"-100 | 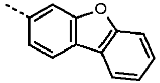 | 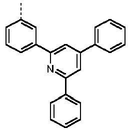 |
| 4"-101, 5"-101, 10"-101, 11"-101 | 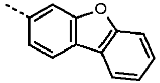 | 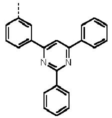 |
| 4"-102, 5"-102, 10"-102, 11"-102 | 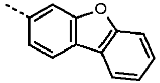 | 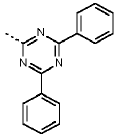 |
| 4"-103, 5"-103, 10"-103, 11"-103 | 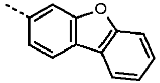 | 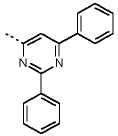 |
| 4"-104, 5"-104, 10"-104, 11"-104 | 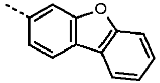 | 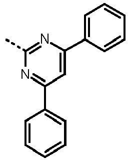 |
| 4"-105, 5"-105, 10"-105, 11"-105 | 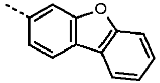 | 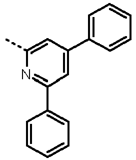 |
| 4"-106, 5"-106, 10"-106, 11"-106 | 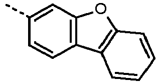 | 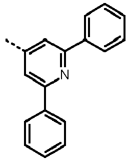 |
| 4"-107, 5"-107, 10"-107, 11"-107 | 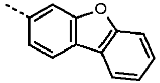 | 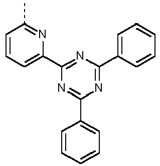 |
| 4"-108, 5"-108, 10"-108, 11"-108 | 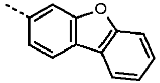 | 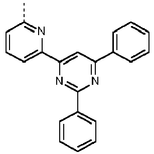 |
| 4"-109, 5"-109, 10"-109, 11"-109 | 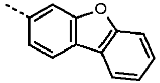 | 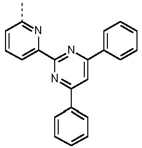 |
| 4"-110, 5"-110, 10"-110, 11"-110 | 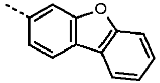 | 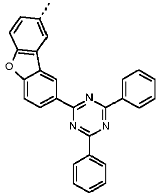 |
| 4"-111, 5"-111, 10"-111, 11"-111 | 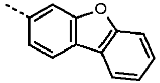 | 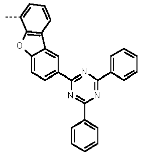 |
| 4"-112, 5"-112, 10"-112, 11"-112 | 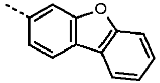 | 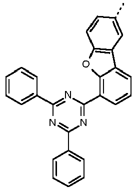 |
| 4"-113, 5"-113, 10"-113, 11"-113 | 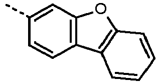 | 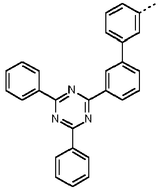 |
| 4"-114, 5"-114, 10"-114, 11"-114 | 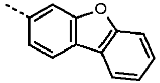 | 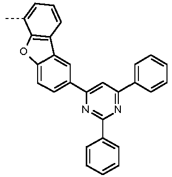 |
| 4"-115, 5"-115, 10"-115, 11"-115 | 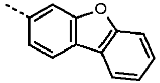 | 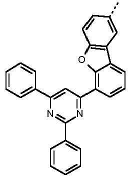 |
| 4"-116, 5"-116, 10"-116, 11"-116 | 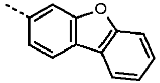 | 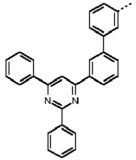 |
| 4"-117, 5"-117, 10"-117, 11"-117 | 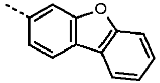 | 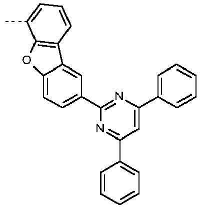 |
| 4"-118, 5"-118, 10"-118, 11"-118 | 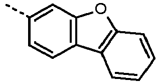 | 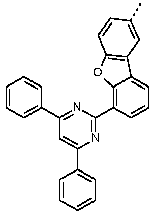 |
| 4"-119, 5"-119, 10"-119, 11"-119 | 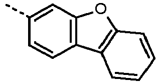 | 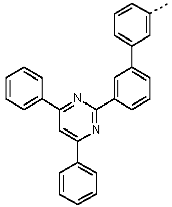 |
| 4"-120, 5"-120 10"-120, 11"-120 | 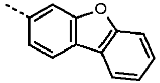 | 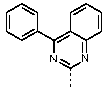 |
| 4"-121, 5"-121, 10"-121, 11"-121 | 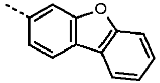 | 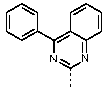 |
| 4"-122, 5"-122, 10"-122, 11"-122 | 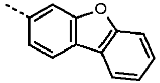 | 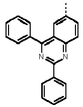 |
| 4"-123, 5"-123, 10"-123, 11"-123 | 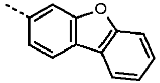 | 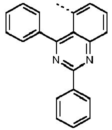 |
| 4"-124, 5"-124, 10"-124, 11"-124 | 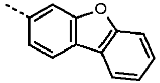 | 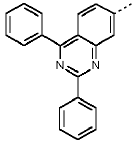 |
| 4"-125, 5"-125, 10"-125, 11"-125 | 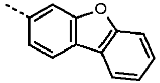 | 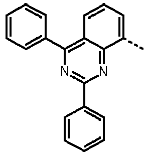 |
| 4"-126, 5"-126, 10"-126, 11"-126 | 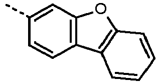 | 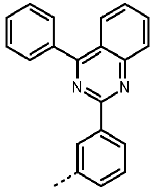 |
| 4"-127, 5"-127, 10"-127, 11"-127 | 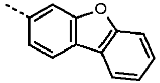 | 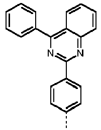 |
| 4"-128, 5"-128, 10"-128, 11"-128 | 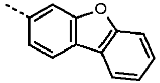 | 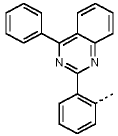 |
| 4"-129, 5"-129, 10"-129, 11"-129 | 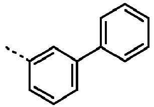 | 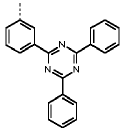 |
| 4"-130, 5"-130, 10"-130, 11"-130 | 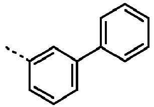 | 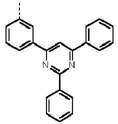 |
| 4"-131, 5"-131, 10"-131, 11"-131 | 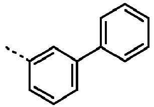 | 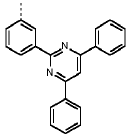 |
| 4"-132, 5"-132, 10"-132, 11"-132 | 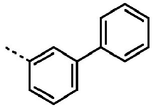 | 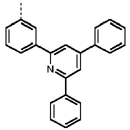 |
| 4"-133, 5"-133, 10"-133, 11"-133 | 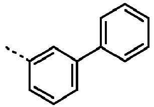 | 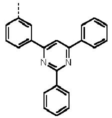 |
| 4"-134, 5"-134, 10"-134, 11"-134 | 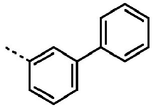 | 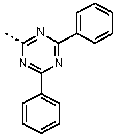 |
| 4"-135, 5"-135, 10"-135, 11"-135 | 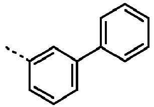 | 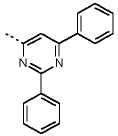 |
| 4"-136, 5"-136, 10"-136, 11"-136 | 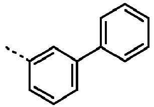 | 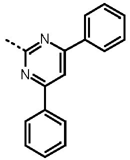 |
| 4"-137, 5"-137, 10"-137, 11"-137 | 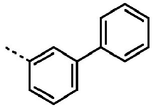 | 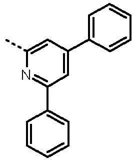 |
| 4"-138, 5"-138, 10"-138, 11"-138 | 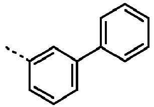 | 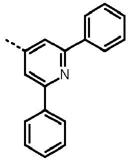 |
| 4"-139, 5"-139, 10"-139, 11"-139 | 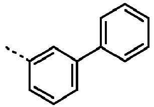 | 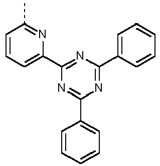 |
| 4"-140, 5"-140, 10"-140, 11"-140 | 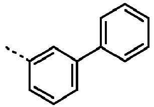 | 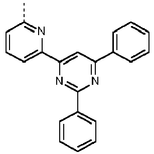 |
| 4"-141, 5"-141, 10"-141, 11"-141 | 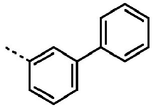 | 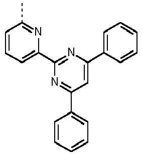 |
| 4"-142, 5"-142, 10"-142, 11"-142 | 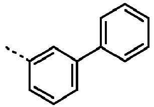 | 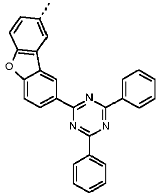 |
| 4"-143, 5"-143, 10"-143, 11"-143 | 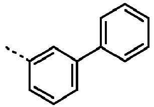 | 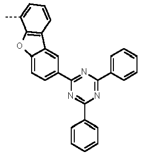 |
| 4"-144, 5"-144, 10"-144, 11"-144 | 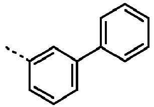 | 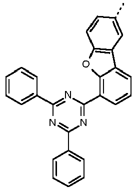 |
| 4"-145, 5"-145, 10"-145, 11"-145 | 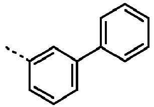 | 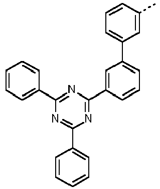 |
| 4"-146, 5"-146, 10"-146, 11"-146 | 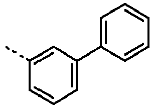 | 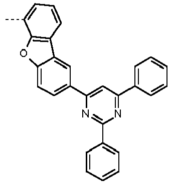 |
| 4"-147, 5"-147, 10"-147, 11"-147 | 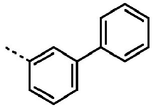 | 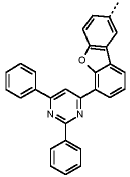 |
| 4"-148, 5"-148, 10"-148, 11"-148 | 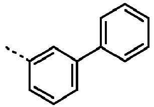 | 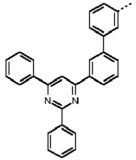 |
| 4"-149, 5"-149, 10"-149, 11"-149 | 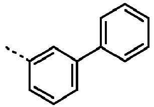 | 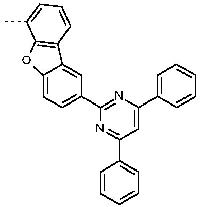 |
| 4"-150, 5"-150, 10"-150, 11"-150 | 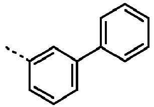 | 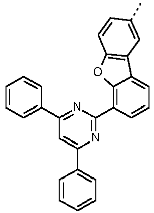 |
| 4"-151, 5"-151, 10"-151, 11"-151 | 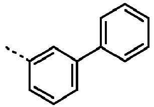 | 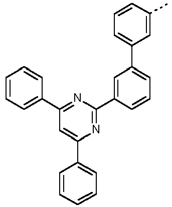 |
| 4"-152, 5"-152, 10"-152, 11"-152 | 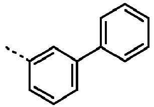 | 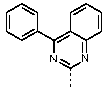 |
| 4"-153, 5"-153, 10"-153, 11"-153 | 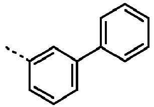 | 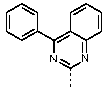 |
| 4"-154, 5"-154, 10"-154, 11"-154 | 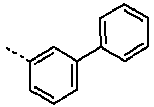 | 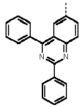 |
| 4"-155, 5"-155, 10"-155, 11"-155 | 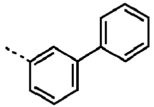 | 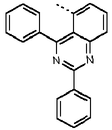 |
| 4"-156, 5"-156, 10"-156, 11"-156 | 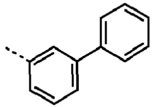 | 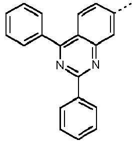 |
| 4"-157, 5"-157, 10"-157, 11"-157 | 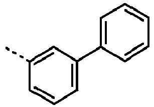 | 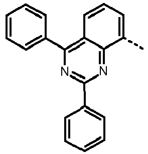 |
| 4"-158, 5"-158, 10"-158, 11"-158 | 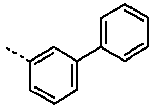 | 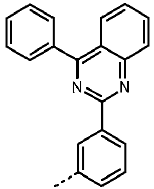 |
| 4"-159, 5"-159, 10"-159, 11"-159 | 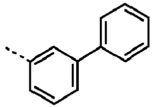 | 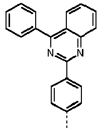 |
| 4"-160, 5"-160, 10"-160, 11"-160 | 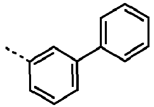 | 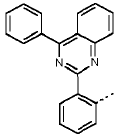 |
| 4"-161, 5"-161, 10"-161, 11"-161 | 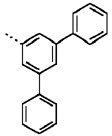 | 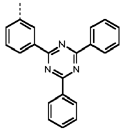 |
| 4"-162, 5"-162, 10"-162, 11"-162 | 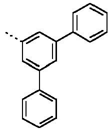 | 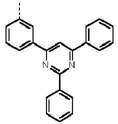 |
| 4"-163, 5"-163, 10"-163, 11"-163 | 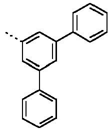 | 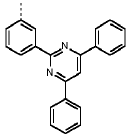 |
| 4"-164, 5"-164, 10"-164, 11"-164 | 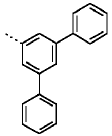 | 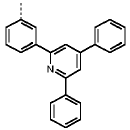 |
| 4"-165, 5"-165, 10"-165, 11"-165 | 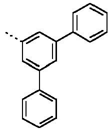 | 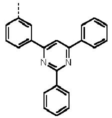 |
| 4"-166, 5"-166, 10"-166, 11"-166 | 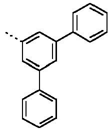 | 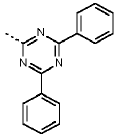 |
| 4"-167, 5"-167, 10"-167, 11"-167 | 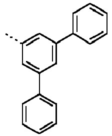 | 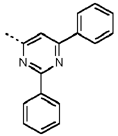 |
| 4"-168, 5"-168, 10"-168, 11"-168 | 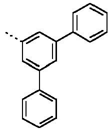 | 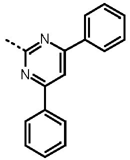 |
| 4"-169, 5"-169, 10"-169, 11"-169 | 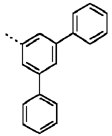 | 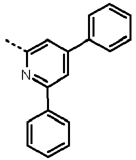 |
| 4"-170, 5"-170, 10"-170, 11"-170 | 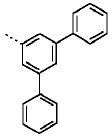 | 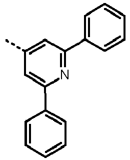 |
| 4"-171, 5"-171, 10"-171, 11"-171 | 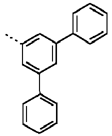 | 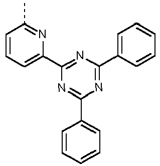 |
| 4"-172, 5"-172, 10"-172, 11"-172 | 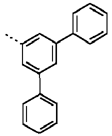 | 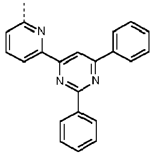 |
| 4"-173, 5"-173, 10"-173, 11"-173 | 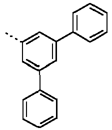 | 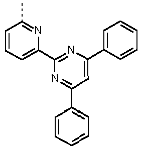 |
| 4"-174, 5"-174, 10"-174, 11"-174 | 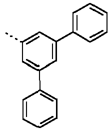 | 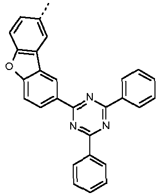 |
| 4"-175, 5"-175, 10"-175, 11"-175 | 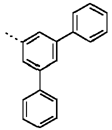 | 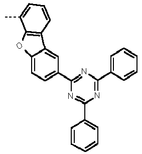 |
| 4"-176, 5"-176, 10"-176, 11"-176 | 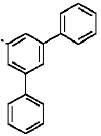 | 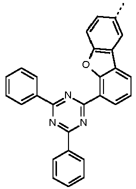 |
| 4"-177, 5"-177, 10"-177, 11"-177 | 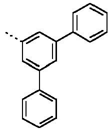 | 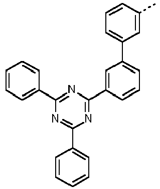 |
| 4"-178, 5"-178, 10"-178, 11"-178 | 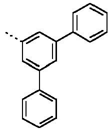 | 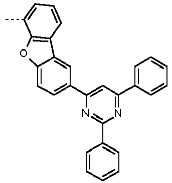 |
| 4"-179, 5"-179, 10"-179, 11"-179 | 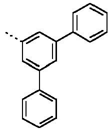 | 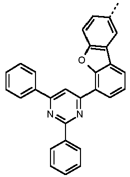 |
| 4"-180, 5"-180, 10"-180, 11"-180 | 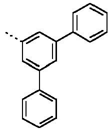 | 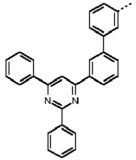 |
| 4"-181, 5"-181, 10"-181, 11"-181 | 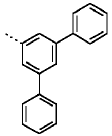 | 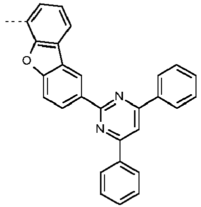 |
| 4"-182, 5"-182, 10"-182, 11"-182 | 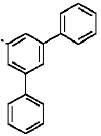 | 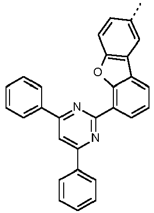 |
| 4"-183, 5"-183, 10"-183, 11"-183 | 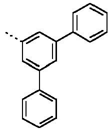 | 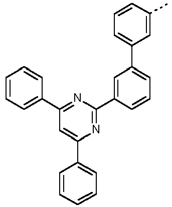 |
| 4"-184, 5"-184, 10"-184, 11"-184 | 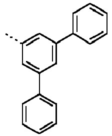 | 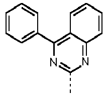 |
| 4"-185, 5"-185, 10"-185, 11"-185 | 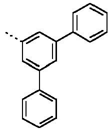 | 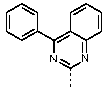 |
| 4"-186, 5"-186, 10"-186, 11"-186 | 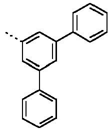 | 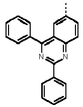 |
| 4"-187, 5"-187, 10"-187, 11"-187 | 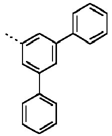 | 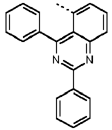 |
| 4"-188, 5"-188, 10"-188, 11"-188 | 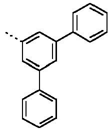 | 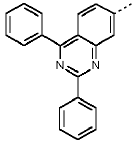 |
| 4"-189, 5"-189, 10"-189, 11"-189 | 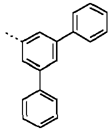 | 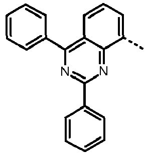 |
| 4"-190, 5"-190, 10"-190, 11"-190 | 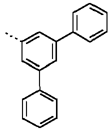 | 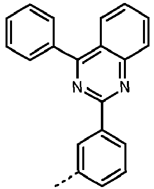 |
| 4"-191, 5"-191, 10"-191, 11"-191 | 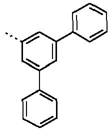 | 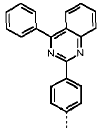 |
| 4"-192, 5"-192, 10"-192, 11"-192 | 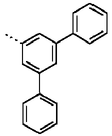 | 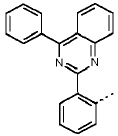 |

wherein the dotted lines in the groups R¹ and R⁷ are bonding sites to the neighboring groups.

The following compounds are reference compounds and not part of the invention:

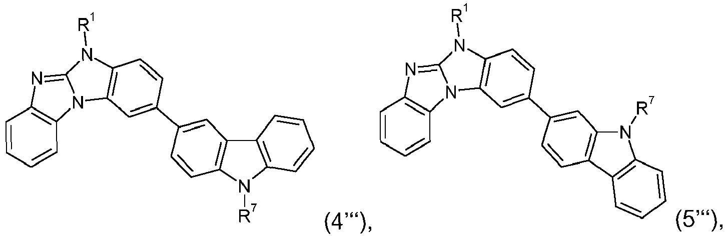

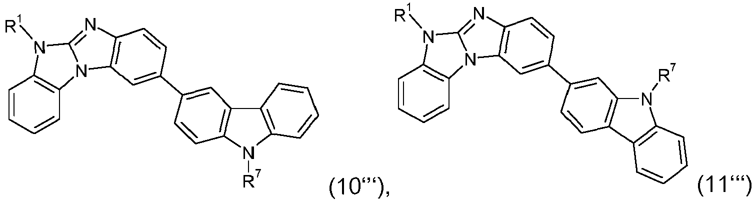

| Nr. | R⁷ | R¹ |
|---|---|---|
| 4‴-1, 5‴-1, 10‴-1, 11‴-1 | Ph | 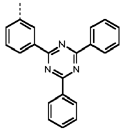 |
| 4‴-2, 5‴-2, 10‴-2, 11‴-2 | Ph | 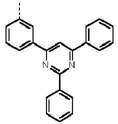 |
| 4‴-3, 5‴-3, 10‴-3, 11‴-3 | Ph | 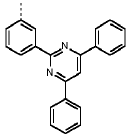 |
| 4‴-4, 5‴-4, 10‴-4, 11‴-4 | Ph | 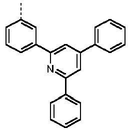 |
| 4‴-5, 5‴-5, 10‴-5, 11‴-5 | Ph | 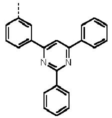 |
| 4‴-6, 5‴-6, 10‴-6, 11‴-6 | Ph | 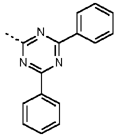 |
| 4‴-7, 5‴-7, 10"'-7, 11‴-7 | Ph | 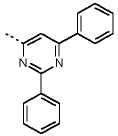 |
| 4‴-8, 5‴-8, 10‴-8, 11‴-8 | Ph | 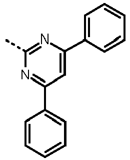 |
| 4‴-9, 5‴-9, 10‴-9, 11‴-9 | Ph | 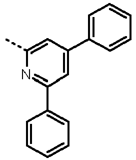 |
| 4‴-10, 5‴-10, 10‴-10, 11‴-10 | Ph | 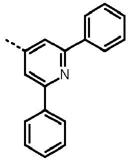 |
| 4‴-11, 5‴-11, 10‴-11, 11‴-11 | Ph | 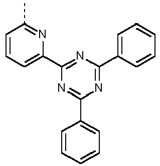 |
| 4‴-12, 5‴-12, 10"'-12, 11‴-12 | Ph | 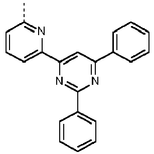 |
| 4"'-13, 5"'-13, 10‴-13, 11‴-13 | Ph | 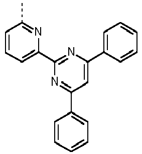 |
| 4‴-14, 5‴-14, 10"'-14, 11‴-14 | Ph | 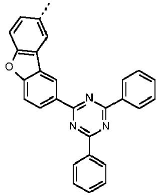 |
| 4"'-15, 5"'-15, 10"'-15, 11‴-15 | Ph | 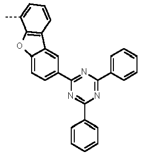 |
| 4"'-16, 5"'-16, 10"'-16, 11‴-16 | Ph | 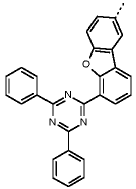 |
| 4‴-17, 5‴-17, 10"'-17, 11‴-17 | Ph | 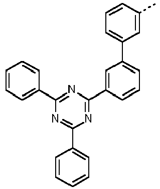 |
| 4"'-18, 5"'-18, 10"'-18, 11‴-18 | Ph | 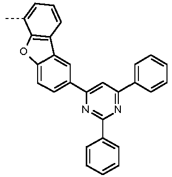 |
| 4"'-19, 5"'-19, 10‴-19, 11‴-19 | Ph | 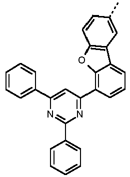 |
| 4‴-20, 5"'-20, 10"'-20, 11‴-20 | Ph | 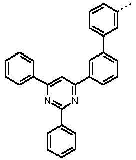 |
| 4‴-21, 5‴-21, 10"'-21, 11‴-21 | Ph | 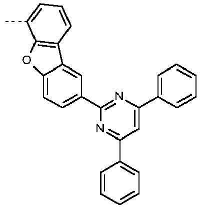 |
| 4"'-22, 5‴-22, 10‴-22, 11‴-22 | Ph | 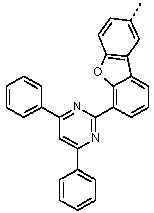 |
| 4‴-23, 5"'-23, 10"'-23, 11‴-23 | Ph | 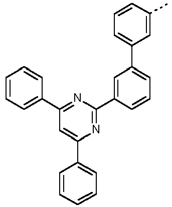 |
| 4"'-24, 5‴-24, 10‴-24, 11‴-24 | Ph | 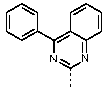 |
| 4‴-25, 5"'-25, 10"'-25, 11‴-25 | Ph | 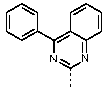 |
| 4‴-26, 5"'-26, 10"'-26, 11‴-26 | Ph | 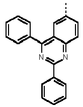 |
| 4"'-27, 5‴-27, 10‴-27, 11‴-27 | Ph | 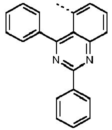 |
| 4"'-28, 5"'-28, 10"'-28, 11‴-28 | Ph | 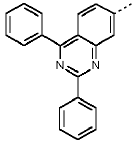 |
| 4"'-29, 5"'-29, 10‴-29, 11‴-29 | Ph | 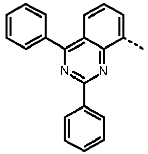 |
| 4"'-30, 5"'-30, 10"'-30, 11‴-30 | Ph | 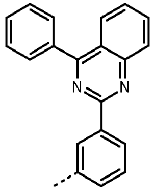 |
| 4‴-31, 5‴-31, 10‴-31, 11‴-31 | Ph | 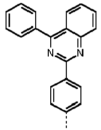 |
| 4"'-32, 5"'-32, 10‴-32, 11‴-32 | Ph | 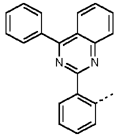 |
| 4‴-33, 5‴-33, 10"'-33, 11‴-33 | 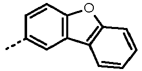 | 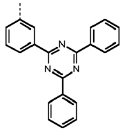 |
| 4"'-34, 5‴-34, 10‴-34, 11‴-34 | 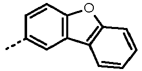 | 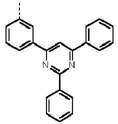 |
| 4‴-35, 5"'-35, 10‴-35, 11‴-35 | 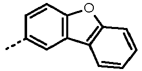 | 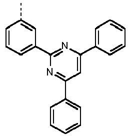 |
| 4‴-36, 5‴-36, 10"'-36, 11‴-36 | 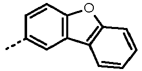 | 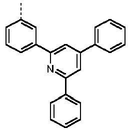 |
| 4"'-37, 5"'-37, 10"'-37, 11‴-37 | 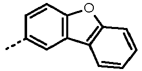 | 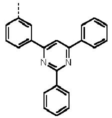 |
| 4"'-38, 5"'-38, 10"'-38, 11‴-38 | 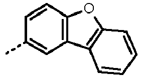 | 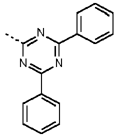 |
| 4‴-39, 5‴-39, 10"'-39, 11‴-39 | 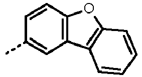 | 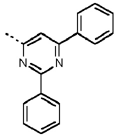 |
| 4"'-40, 5"'-40, 10"'-40, 11‴-40 | 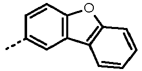 | 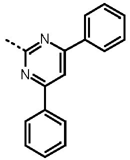 |
| 4‴-41, 5‴-41, 10"'-41, 11‴-41 | 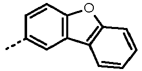 | 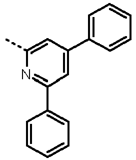 |
| 4‴-42, 5‴-42, 10"'-42, 11‴-42 | 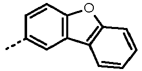 | 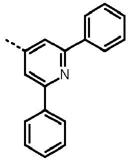 |
| 4‴-43, 5‴-43, 10"'-43, 11‴-43 | 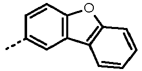 | 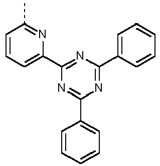 |
| 4‴-44, 5‴-44, 10"'-44, 11‴-44 | 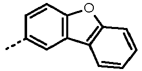 | 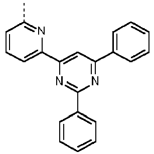 |
| 4‴-45, 5‴-45, 10"'-45, 11‴-45 | 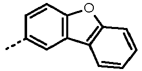 | 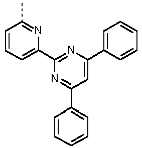 |
| 4‴-46, 5‴-46, 10"'-46, 11‴-46 | 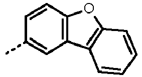 | 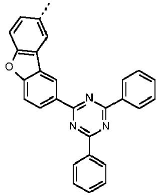 |
| 4‴-47, 5‴-47, 10"'-47, 11‴-47 | 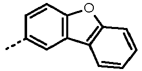 | 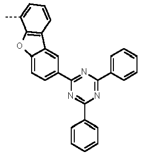 |
| 4"'-48, 5"'-48, 10"'-48, 11‴-48 | 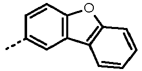 | 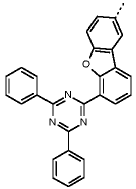 |
| 4‴-49, 5‴-49, 10"'-49, 11‴-49 | 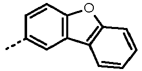 | 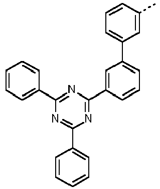 |
| 4"'-50, 5"'-50, 10"'-50, 11‴-50 | 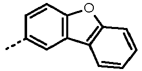 | 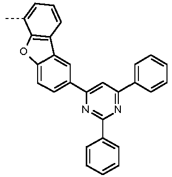 |
| 4‴-51, 5‴-51, 10‴-51, 11‴-51 | 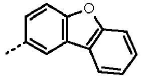 | 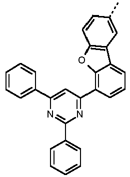 |
| 4"'-52, 5‴-52, 10"'-52, 11‴-52 | 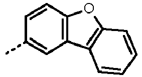 | 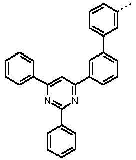 |
| 4"'-53, 5‴-53, 10‴-53, 11‴-53 | 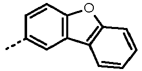 | 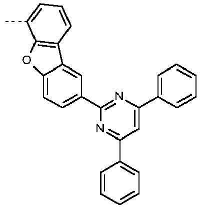 |
| 4‴-54, 5‴-54, 10"'-54, 11‴-54 | 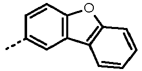 | 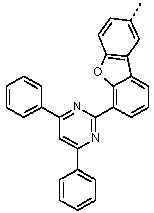 |
| 4"'-55, 5"'-55, 10‴-55, 11‴-55 | 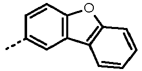 | 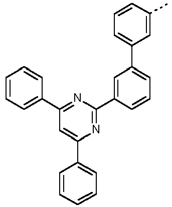 |
| 4‴-56, 5‴-56, 10"'-56, 11‴-56 | 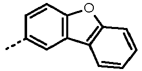 | 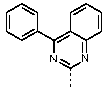 |
| 4"'-57, 5"'-57, 10"'-57, 11‴-57 | 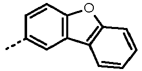 | 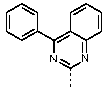 |
| 4"'-58, 5"'-58, 10"'-58, 11‴-58 | 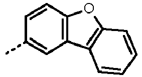 | 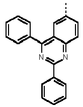 |
| 4"'-59, 5‴-59, 10‴-59, 11‴-59 | 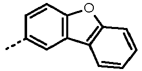 | 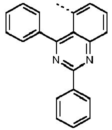 |
| 4"'-60, 5"'-60, 10"'-60, 11‴-60 | 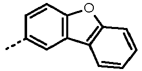 | 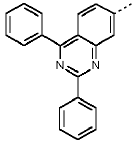 |
| 4‴-61, 5‴-61, 10‴-61, 11‴-61 | 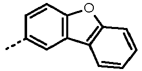 | 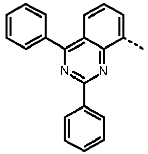 |
| 4‴-62, 5‴-62, 10"'-62, 11‴-62 | 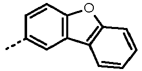 | 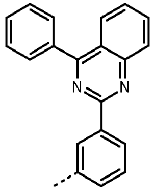 |
| 4‴-63, 5‴-63, 10"'-63, 11‴-63 | 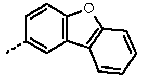 | 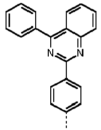 |
| 4‴-64, 5‴-64, 10"'-64, 11‴-64 | 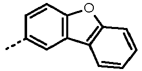 | 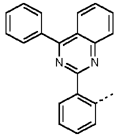 |
| 4‴-65, 5‴-65, 10"'-65, 11‴-65 | 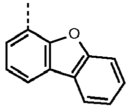 | 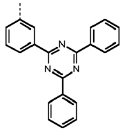 |
| 4"'-66, 5‴-66, 10‴-66, 11‴-66 | 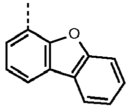 | 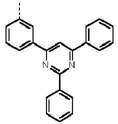 |
| 4"'-67, 5"'-67, 10"'-67, 11‴-67 | 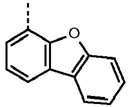 | 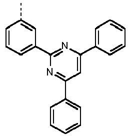 |
| 4"'-68, 5"'-68, 10"'-68, 11‴-68 | 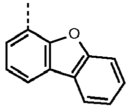 | 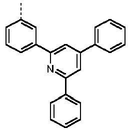 |
| 4‴-69, 5‴-69, 10"'-69, 11‴-69 | 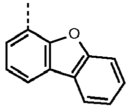 | 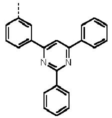 |
| 4‴-70, 5"'-70, 10"'-70, 11‴-70 | 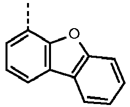 | 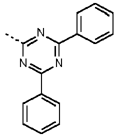 |
| 4"'-71, 5"'-71, 10"'-71, 11‴-71 | 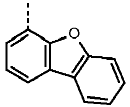 | 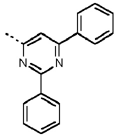 |
| 4"'-72, 5"'-72, 10‴-72, 11‴-72 | 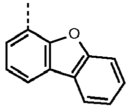 | 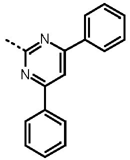 |
| 4"'-73, 5‴-73, 10‴-73, 11‴-73 | 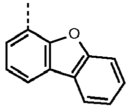 | 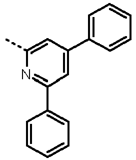 |
| 4‴-74, 5"'-74, 10‴-74, 11‴-74 | 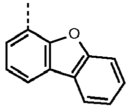 | 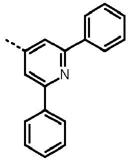 |
| 4‴-75, 5‴-75, 10"'-75, 11‴-75 | 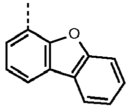 | 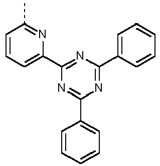 |
| 4‴-76, 5‴-76, 10"'-76, 11‴-76 | 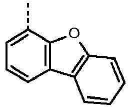 | 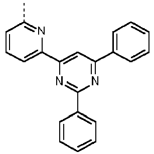 |
| 4‴-77, 5‴-77, 10"'-77, 11‴-77 | 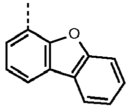 | 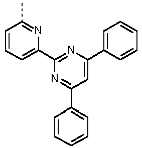 |
| 4"'-78, 5"'-78, 10"'-78, 11‴-78 | 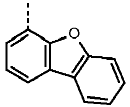 | 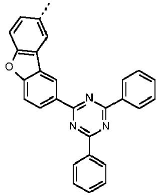 |
| 4‴-79, 5‴-79, 10‴-79, 11‴-79 | 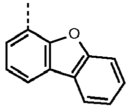 | 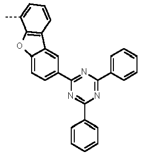 |
| 4"'-80, 5"'-80, 10"'-80, 11‴-80 | 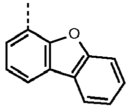 | 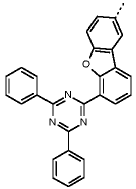 |
| 4‴-81, 5‴-81, 10‴-81, 11‴-81 | 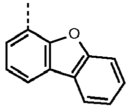 | 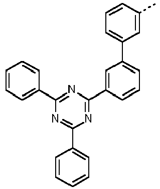 |
| 4"'-82, 5"'-82, 10‴-82, 11‴-82 | 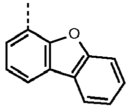 | 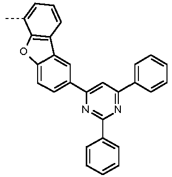 |
| 4"'-83, 5"'-83, 10‴-83, 11‴-83 | 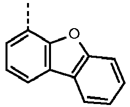 | 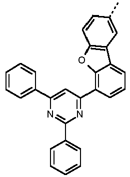 |
| 4‴-84, 5‴-84, 10"'-84, 11‴-84 | 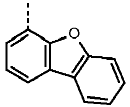 | 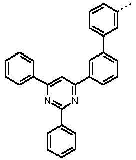 |
| 4"'-85, 5‴-85, 10‴-85, 11‴-85 | 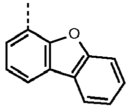 | 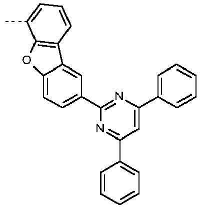 |
| 4‴-86, 5‴-86, 10"'-86, 11‴-86 | 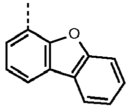 | 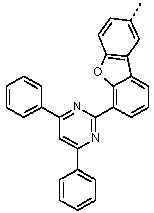 |
| 4"'-87, 5"'-87, 10"'-87, 11‴-87 | 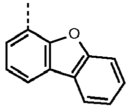 | 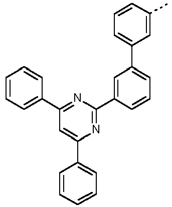 |
| 4"'-88, 5"'-88, 10"'-88, 11‴-88 | 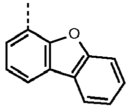 | 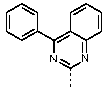 |
| 4‴-89, 5‴-89, 10"'-89, 11‴-89 | 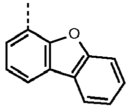 | 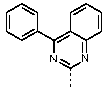 |
| 4"'-90, 5"'-90, 10"'-90, 11‴-90 | 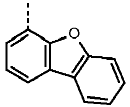 | 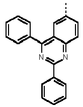 |
| 4‴-91, 5‴-91, 10‴-91, 11‴-91 | 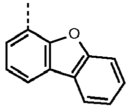 | 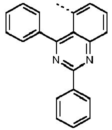 |
| 4"'-92, 5‴-92, 10"'-92, 11‴-92 | 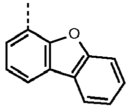 | 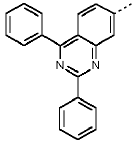 |
| 4‴-93, 5‴-93, 10"'-93, 11‴-93 | 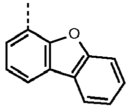 | 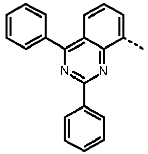 |
| 4‴-94, 5‴-94, 10"'-94, 11‴-94 | 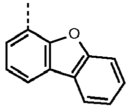 | 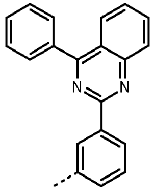 |
| 4‴-95, 5‴-95, 10"'-95, 11‴-95 | 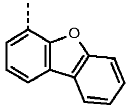 | 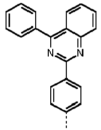 |
| 4‴-96, 5‴-96, 10"'-96, 11‴-96 | 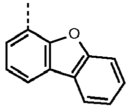 | 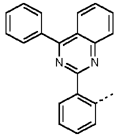 |
| 4"'-97, 5"'-97, 10"'-97, 11‴-97 | 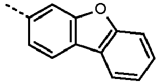 | 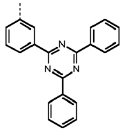 |
| 4"'-98, 5"'-98, 10"'-98, 11‴-98 | 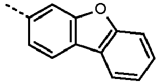 | 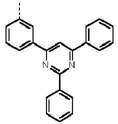 |
| 4"'-99, 5"'-99, 10‴-99, 11‴-99 | 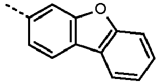 | 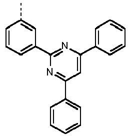 |
| 4‴-100, 5‴-100, 10‴-100, 11‴-100 | 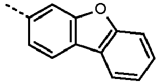 | 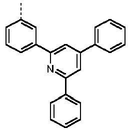 |
| 4"'-101, 5"'-101, 10‴-101, 11‴-101 | 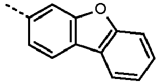 | 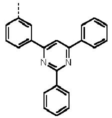 |
| 4"'-102, 5"'-102, 10"'-102, 11‴-102 | 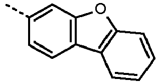 | 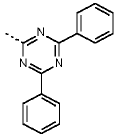 |
| 4‴-103, 5‴-103, 10‴-103, 11‴-103 | 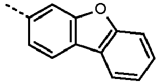 | 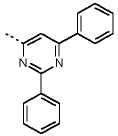 |
| 4‴-104, 5‴-104, 10‴-104, 11‴-104 | 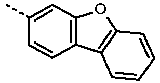 | 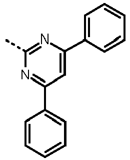 |
| 4‴-105, 5‴-105, 10‴-105, 11‴-105 | 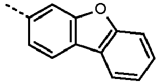 | 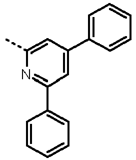 |
| 4‴-106, 5‴-106, 10‴-106, 11‴-106 | 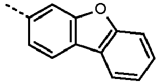 | 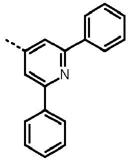 |
| 4"'-107, 5"'-107, 10"'-107, 11‴-107 | 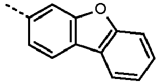 | 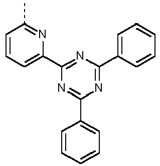 |
| 4‴-108, 5‴-108, 10‴-108, 11‴-108 | 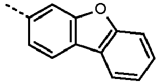 | 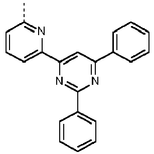 |
| 4‴-109, 5‴-109, 10‴-109, 11‴-109 | 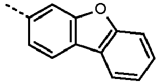 | 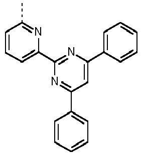 |
| 4"'-110, 5"'-110, 10"'-110, 11"'-110 | 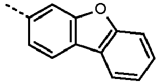 | 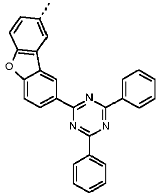 |
| 4"'-111, 5‴-111, 10‴-111, 11‴-111 | 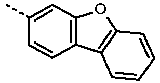 | 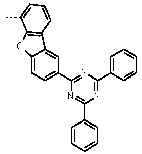 |
| 4"'-112, 5"'-112, 10"'-112, 11‴-112 | 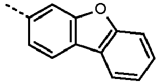 | 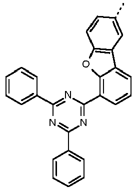 |
| 4"'-113, 5"'-113, 10"'-113, 11‴-113 | 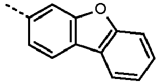 | 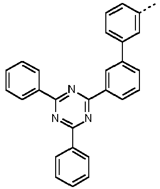 |
| 4‴-114, 5"'-114, 10"'-114, 11"'-114 | 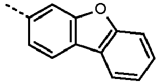 | 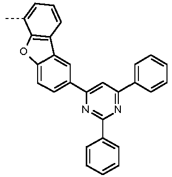 |
| 4‴-115, 5‴-115, 10"'-115, 11‴-115 | 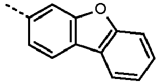 | 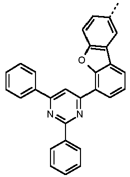 |
| 4"'-116, 5"'-116, 10"'-116, 11"'-116 | 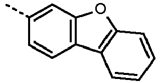 | 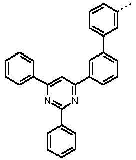 |
| 4"'-117, 5"'-117, 10"'-117, 11‴-117 | 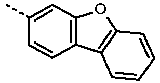 | 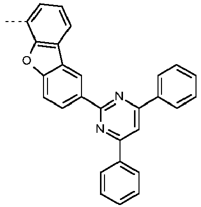 |
| 4"'-118, 5"'-118, 10"'-118, 11"'-118 | 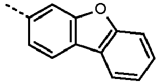 | 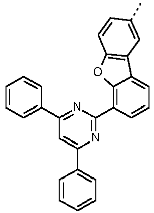 |
| 4"'-119, 5"'-119, 10"'-119, 11"'-119 | 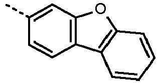 | 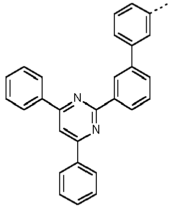 |
| 4"'-120, 5"'-120 10"'-120, 11‴-120 | 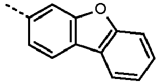 | 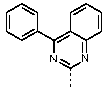 |
| 4"'-121, 5"'-121, 10"'-121, 11‴-121 | 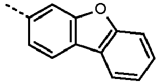 | 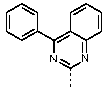 |
| 4"'-122, 5"'-122, 10"'-122, 11‴-122 | 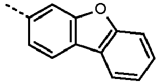 | 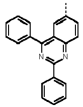 |
| 4"'-123, 5"'-123, 10"'-123, 11‴-123 | 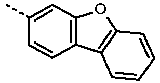 | 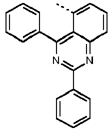 |
| 4"'-124, 5"'-124, 10"'-124, 11‴-1 24 | 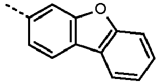 | 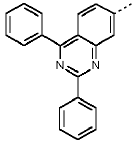 |
| 4"'-125, 5"'-125, 10"'-125, 11‴-125 | 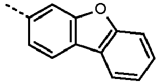 | 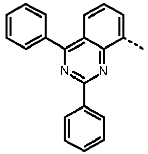 |
| 4"'-126, 5"'-126, 10"'-126, 11‴-126 | 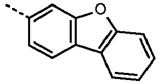 | 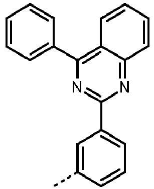 |
| 4"'-127, 5"'-127, 10"'-127, 11‴-127 | 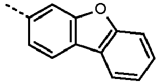 | 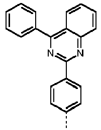 |
| 4"'-128, 5"'-128, 10"'-128, 11‴-128 | 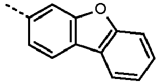 | 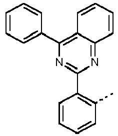 |
| 4"'-129, 5"'-129, 10"'-129, 11‴-129 | 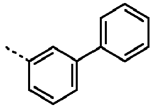 | 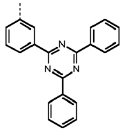 |
| 4‴-130, 5‴-130, 10‴-130, 11‴-130 | 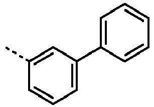 | 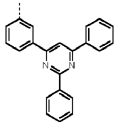 |
| 4‴-131, 5‴-131, 10‴-131, 11‴-131 | 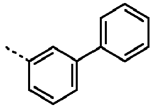 | 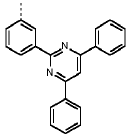 |
| 4‴-132, 5‴-132, 10‴-132, 11‴-132 | 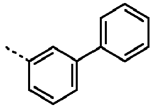 | 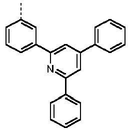 |
| 4"'-133, 5‴-133, 10‴-133, 11‴-133 | 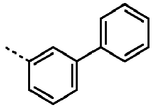 | 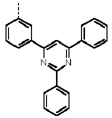 |
| 4"'-134, 5"'-134, 10‴-134, 11‴-134 | 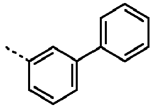 | 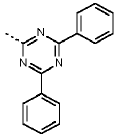 |
| 4‴-135, 5‴-135, 10‴-135, 11‴-135 | 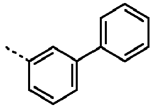 | 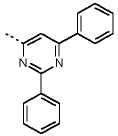 |
| 4‴-136, 5‴-136, 10‴-136, 11‴-136 | 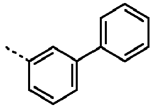 | 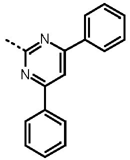 |
| 4"'-137, 5"'-137, 10"'-137, 11‴-137 | 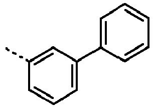 | 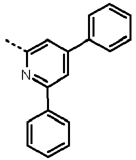 |
| 4"'-138, 5"'-138, 10‴-138, 11‴-138 | 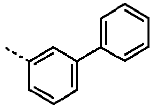 | 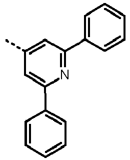 |
| 4"'-139, 5"'-139, 10‴-139, 11‴-139 | 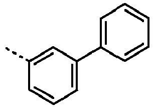 | 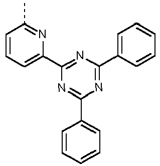 |
| 4"'-140, 5"'-140, 10"'-140, 11‴-140 | 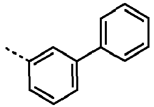 | 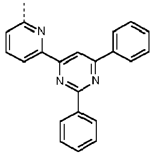 |
| 4"'-141, 5"'-141, 10"'-141, 11‴-141 | 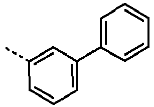 | 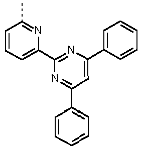 |
| 4"'-142, 5"'-142, 10"'-142, 11‴-142 | 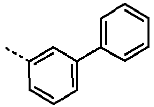 | 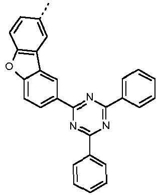 |
| 4"'-143, 5"'-143, 10"'-143, 11‴-143 | 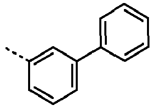 | 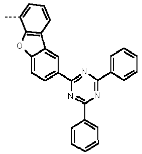 |
| 4"'-144, 5"'-144, 10"'-144, 11‴-144 | 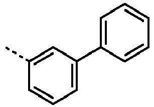 | 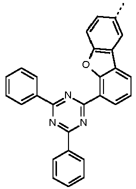 |
| 4"'-145, 5"'-145, 10"'-145, 11‴-145 | 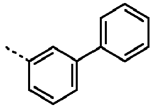 | 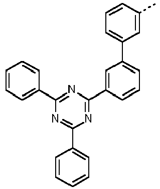 |
| 4"'-146, 5"'-146, 10"'-146, 11‴-146 | 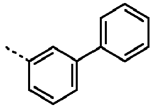 | 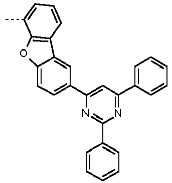 |
| 4"'-147, 5"'-147, 10"'-147, 11‴-147 | 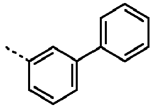 | 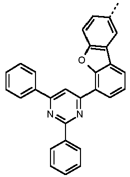 |
| 4"'-148, 5"'-148, 10"'-148, 11‴-148 | 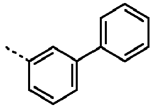 | 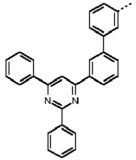 |
| 4"'-149, 5"'-149, 10"'-149, 11‴-149 | 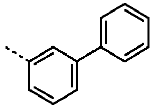 | 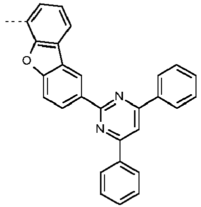 |
| 4"'-150, 5"'-150, 10"'-150, 11‴-150 | 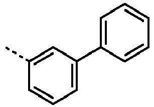 | 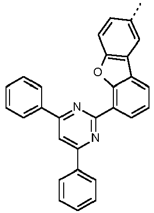 |
| 4"'-151, 5"'-151, 10‴-151, 11‴-151 | 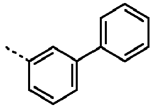 | 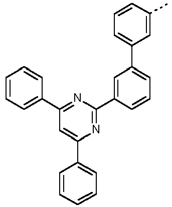 |
| 4"'-152, 5"'-152, 10"'-152, 11‴-152 | 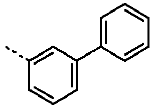 | 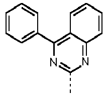 |
| 4"'-153, 5"'-153, 10"'-153, 11‴-153 | 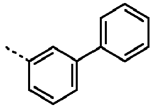 | 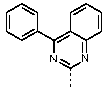 |
| 4"'-154, 5"'-154, 10"'-154, 11‴-154 | 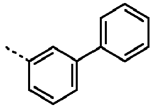 | 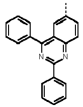 |
| 4"'-155, 5"'-155, 10"'-155, 11‴-155 | 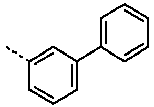 | 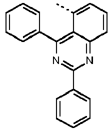 |
| 4"'-156, 5"'-156, 10"'-156, 11‴-156 | 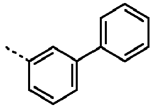 | 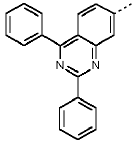 |
| 4"'-157, 5"'-157, 10"'-157, 11‴**-**157 | 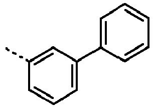 | 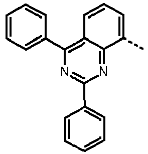 |
| 4"'-158, 5"'-158, 10"'-158, 11‴-158 | 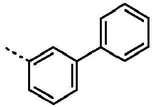 | 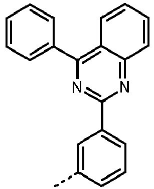 |
| 4"'-159, 5"'-159, 10"'-159, 11‴-159 | 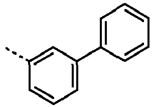 | 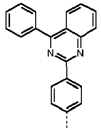 |
| 4"'-160, 5"'-160, 10"'-160, 11‴-160 | 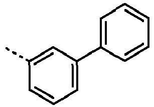 | 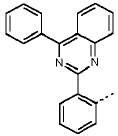 |
| 4"'-161, 5"'-161, 10"'-161, 11‴-161 | 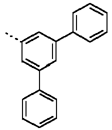 | 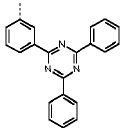 |
| 4"'-162, 5"'-162, 10"'-162, 11‴-162 | 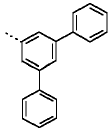 | 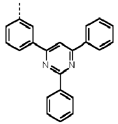 |
| 4"'-163, 5"'-163, 10"'-163, 11‴-163 | 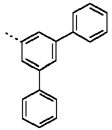 | 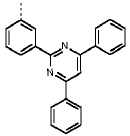 |
| 4"'-164, 5"'-164, 10"'-164, 11‴-164 | 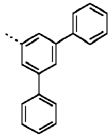 | 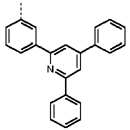 |
| 4"'-165, 5"'-165, 10"'-165, 11‴-165 | 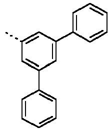 | 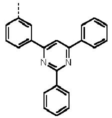 |
| 4"'-166, 5"'-166, 10"'-166, 11‴-166 | 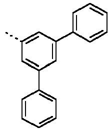 | 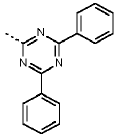 |
| 4"'-167, 5"'-167, 10"'-167, 11‴-167 | 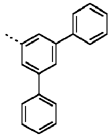 | 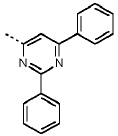 |
| 4"'-168, 5"'-168, 10"'-168, 11‴-168 | 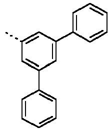 | 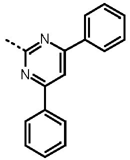 |
| 4"'-169, 5"'-169, 10"'-169, 11‴-169 | 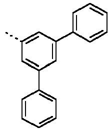 | 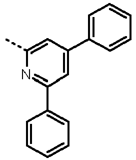 |
| 4‴-170, 5‴-170, 10‴-170, 11‴-170 | 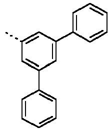 | 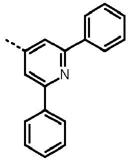 |
| 4‴-171, 5‴-171, 10"'-171, 11"'-171 | 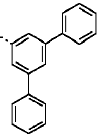 | 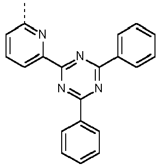 |
| 4"'-172, 5"'-172, 10"'-172, 11‴-1 72 | 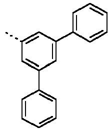 | 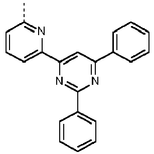 |
| 4"'-173, 5"'-173, 10"'-173, 11‴-173 | 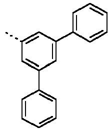 | 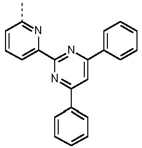 |
| 4‴-174, 5‴-174, 10‴-174, 11‴-1 74 | 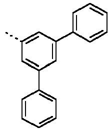 | 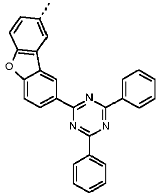 |
| 4‴-175, 5‴-175, 10‴-175, 11‴-175 | 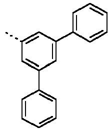 | 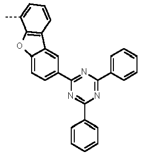 |
| 4‴-176, 5‴-176, 10‴-176, 11‴-176 | 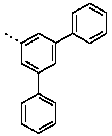 | 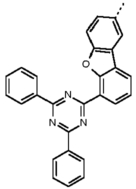 |
| 4"'-177, 5"'-177, 10"'-177, 11‴-177 | 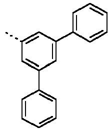 | 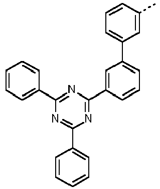 |
| 4"'-178, 5"'-178, 10‴-178, 11‴-178 | 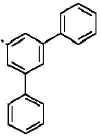 | 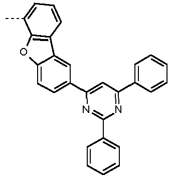 |
| 4"'-179, 5"'-179, 10‴-179, 11‴-179 | 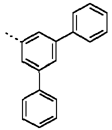 | 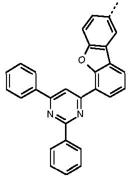 |
| 4"'-180, 5"'-180, 10"'-180, 11‴-180 | 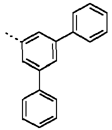 | 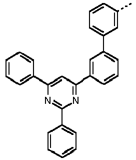 |
| 4"'-181, 5"'-181, 10"'-181, 11‴-181 | 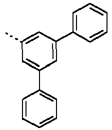 | 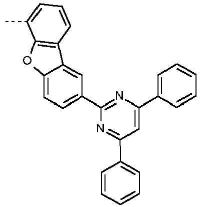 |
| 4"'-182, 5"'-182, 10"'-182, 11‴-182 | 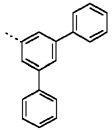 | 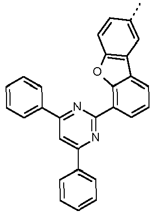 |
| 4"'-183, 5"'-183, 10"'-183, 11‴-183 | 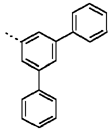 | 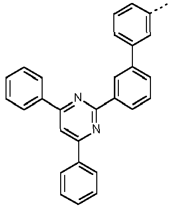 |
| 4"'-184, 5"'-184, 10"'-184, 11‴-1 84 | 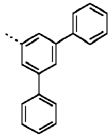 | 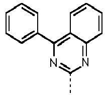 |
| 4"'-185, 5"'-185, 10"'-185, 11‴-185 | 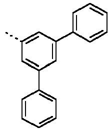 | 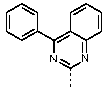 |
| 4"'-186, 5"'-186, 10"'-186, 11‴-186 | 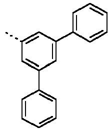 | 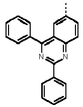 |
| 4"'-187, 5"'-187, 10"'-187, 11‴-187 | 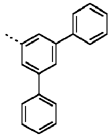 | 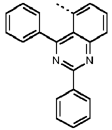 |
| 4"'-188, 5‴-188, 10"'-188, 11‴-188 | 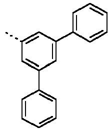 | 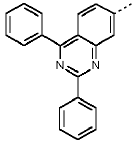 |
| 4"'-189, 5"'-189, 10"'-189, 11‴-189 | 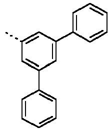 | 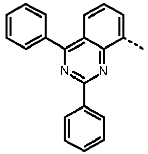 |
| 4"'-190, 5"'-190, 10"'-190, 11‴-190 | 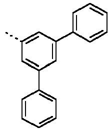 | 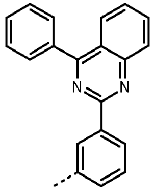 |
| 4"'-191, 5"'-191, 10"'-191, 11"'-191 | 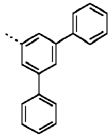 | 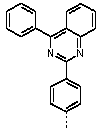 |
| 4"'-192, 5"'-192, 10"'-192, 11‴-192 | 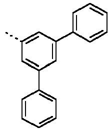 | 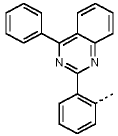 |

wherein the dotted lines in the groups R¹ and R⁷ are bonding sites to the neighboring groups.

The specific heterocyclic derivatives of formula (1) of the present invention are found to be suitable for use in organo-electroluminescent devices. In particular, the heterocyclic derivatives of formula (1) are suitable host materials, especially host materials for phosphorescent emitters, charge transport materials, i.e. hole transport materials and electron transport materials, preferably electron transport materials and/or electron injection materials with good efficiency and durability.

The combination of the benzimidazo[1,2-a]benzimidazo-yl group with the carbazoloyl group and the group Az gives rise to materials that are highly suitable in devices that emit green, red or yellow light, preferably green or red light, more preferably green light. Moreover, a balanced electron transport and/or electron injection in devices is achieved resulting in low voltages and high external quantum efficiencies (EQE's) and/or long lifetimes.

One key finding of the inventors of the present invention is the relevance of the combination of the benzimidazo[1,2-a]benzimidazo-yl group with the carbazoloyl group and the group Az to achieve better lifetimes or/and driving voltages in organic electronic devices compared with organic electronic devices comprising compounds of the related art.

The compounds of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescent devices (EL devices), such as, for example, organic light-emitting diodes (OLEDs). Preferably, the compounds of the present invention are used in electroluminescent devices, especially in OLEDs.

Accordingly, a further subject of the present invention is directed to an electronic device, comprising a compound according to the present invention. The electronic device is preferably an electroluminescent device (EL-device), especially an OLED.

The compounds of formula (1) can in principal be used in any layer of an EL device, but are preferably used as host, electron transport and/or electron injection material. Particularly, the compounds of formula (1) are used as host material for green, red or yellow, preferably green or red, more preferably green light emitting emitters, which are preferably phosphorescent emitters.

Hence, a further subject of the present invention is directed to a charge transport layer, i.e. an electron transport layer or a hole transport layer, preferably an electron transport layer comprising a compound of formula (1) according to the present invention.

A further subject of the present invention is directed to an emitting layer, comprising a compound of formula (1) according to the present invention. In said embodiment a compound of formula (1) is preferably used as host material in combination with an emitter, which is preferably a phosphorescent emitter. The compound of formula (1) is useful as a single host material in the light-emitting layer or as co-host together with one or more, preferably one further host material. Suitable further host materials which are useful with the compound of formula (1) as co-host are mentioned below.

A further subject of the present invention is directed to an electron injection layer, comprising a compound of formula (1) according to the present invention.

### Synthesis of the compounds of formula (1)

Generally, the heterocyclic derivatives of formula (1) are prepared in analogy to the preparation processes described in the related art, e.g. in WO2012/130709, WO2014/009317, WO2014/044722, European patent application no. 13191100.0, WO2015/014791, European patent application no. EP14197947.9 and European patent application no. EP14197952.6.

The present invention further relates to a process for the preparation of the heterocyclic derivatives of formula (1) comprising:
a) Coupling of a group 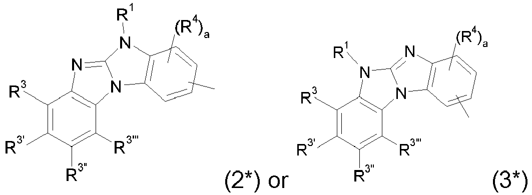 with a group 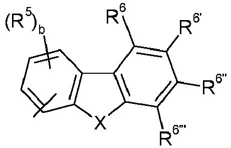 via a group L¹,
   whereby a heterocyclic group A of formula (2) or formula (3) is obtained 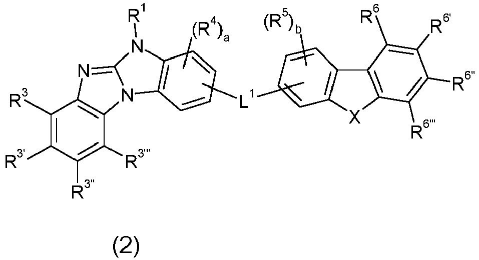 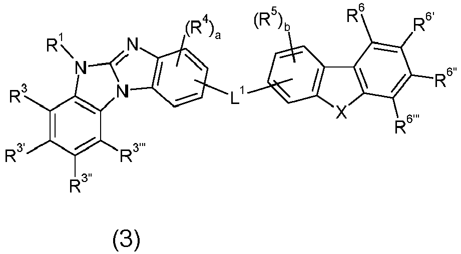 wherein the residues and groups R¹, R³, R^{3'}, R^{3"}, R^{3‴}, R⁴, R⁵, R⁶, R^{6'}, R^{6"}, R^{6‴}, X and L¹ and the indices a and b have been described above; and
b) Introduction of one group -(B₁)ₒ-(B₂)ₚ-(B₃)_{q}-(B₄)ᵣ-Az into the heterocyclic group A of formula (2) or formula (3),
   wherein the groups B₁, B₂, B₃, B₄ and Az and the indices o, p, q and r have been described above;
   whereby a heterocyclic derivative of formula (1)

      A-[(B₁)ₒ-(B₂)ₚ-(B₃)_{q}-(B₄)ᵣ-Az]_{z} (1)

      is obtained.

Preferred heterocyclic derivatives of formula (1) are mentioned above.

Specific reaction conditions of the steps a) and b) of the process according to the present invention are described below as well as in the example part of the present application.

The preparation of the group -(B₁)ₒ-(B₂)ₚ-(B₃)_{q}-(B₄)ᵣ-Az is known by a person skilled in the art. Specific reaction conditions for a functionalization of the group Az are additionally mentioned in the following documents: WO2011019156, WO2013187896, WO2013147205, WO2011132683, WO2014003440; WO2014014310, WO2013068376.Suitable reaction conditions are for example: NaOBu-t, t-Bu₃P, Pd(OAc)₂, PhMe, 10 h, reflux, as shown in the specific example below:

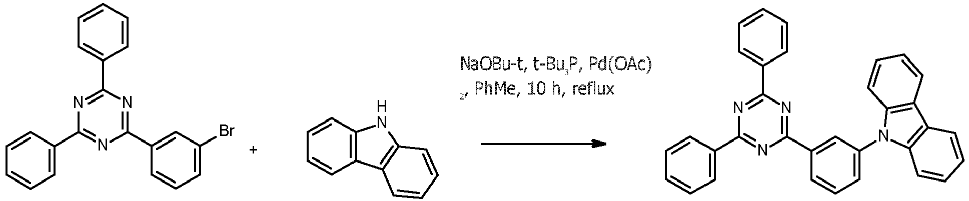

In the following, two examples for preparation processes for compounds of formula (1) are shown, wherein X is NR⁷ and R⁷ is -(B₁)ₒ-(B₂)ₚ-(B₃)_{q}-(B₄)ᵣ-Az, and the other group R¹ is as defined above, preferably phenyl:

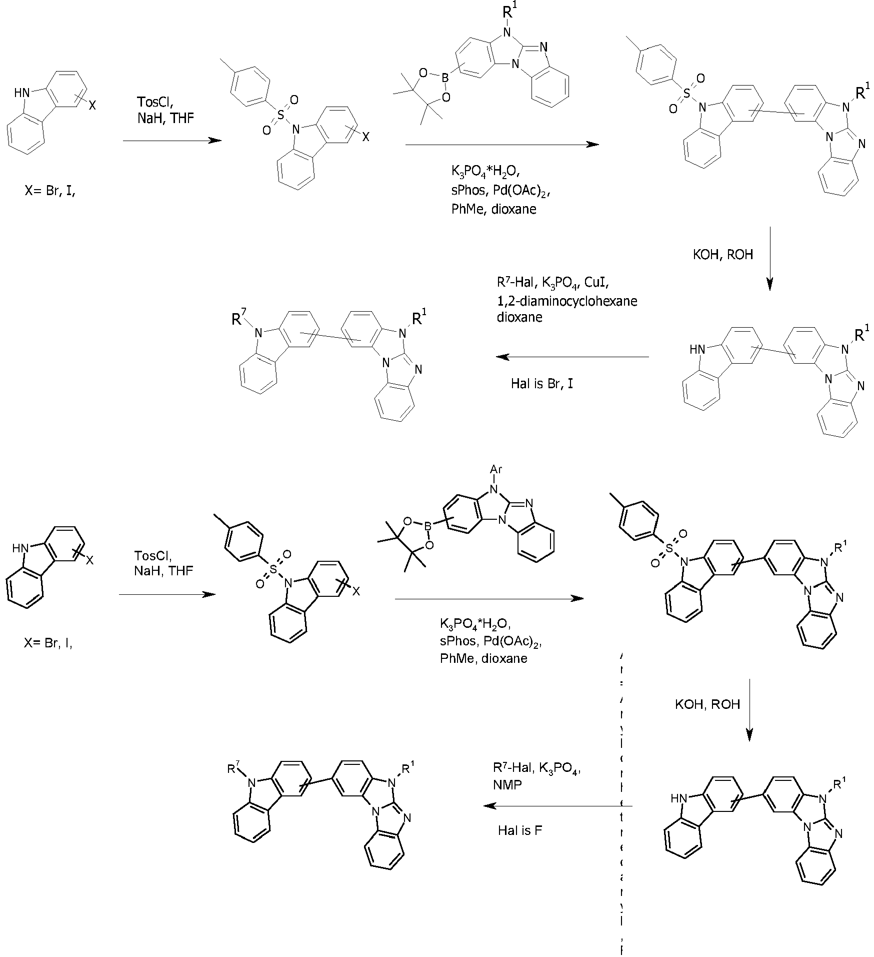

The compounds of formula (1) as well as benzimidazolo[1,2-a]benzimidazoles substituted by a group of formula -(B⁵)ₛ-(B⁶)ₜ-(B⁷)ᵤ-(B⁸)ᵥ-R¹⁰ in general, i.e having of one of the following formulae: 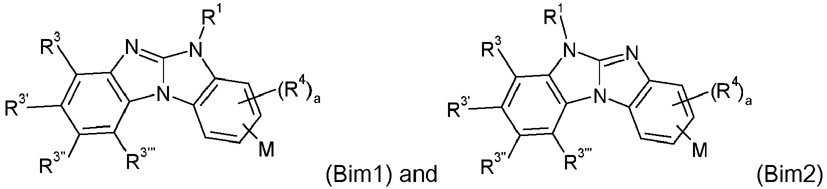 wherein M is a group of formula -(B⁵)ₛ-(B⁶)ₜ-(B⁷)ᵤ-(B⁸)ᵥ-R¹⁰,
are preferably prepared by coupling a compound of formula (1") or (1"') 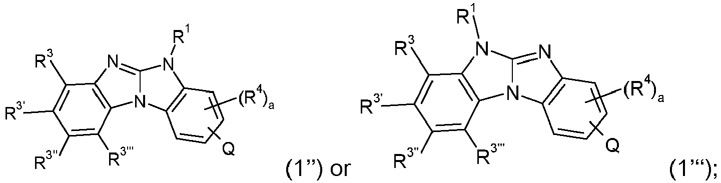 wherein
Q is H, F, Cl, Br, or I, preferably Cl or Br, more preferably Br;
R¹ is phenyl or a group of the following formula 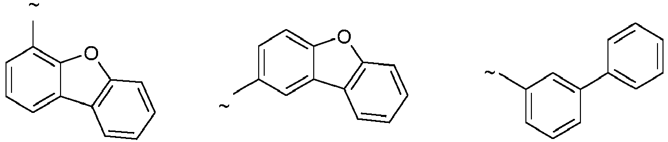 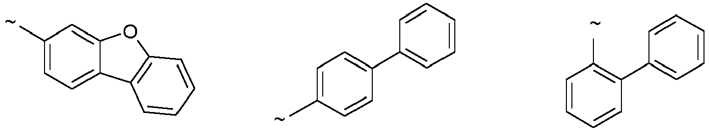 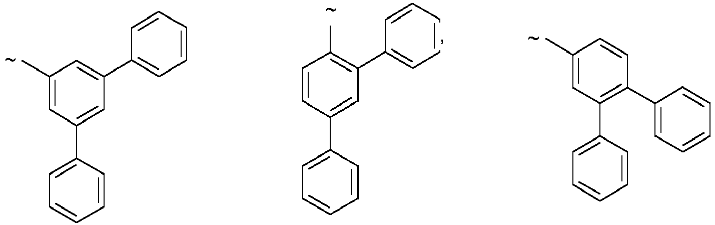 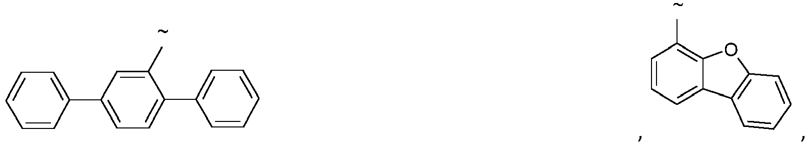 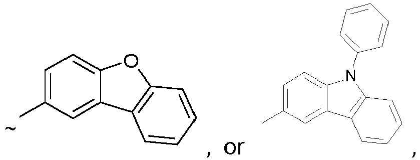 wherein ~ are bonding sites,
R³, R^{3'}, R^{3"}, R^{3‴} and R⁴
   are H;
preferably, R³, R^{3'}, R^{3"}, R^{3‴} and R⁴ are H and R¹ is phenyl or a group of the following formula 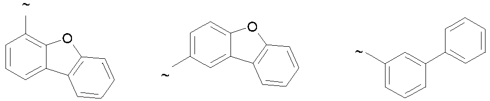 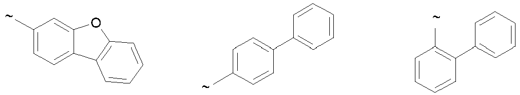 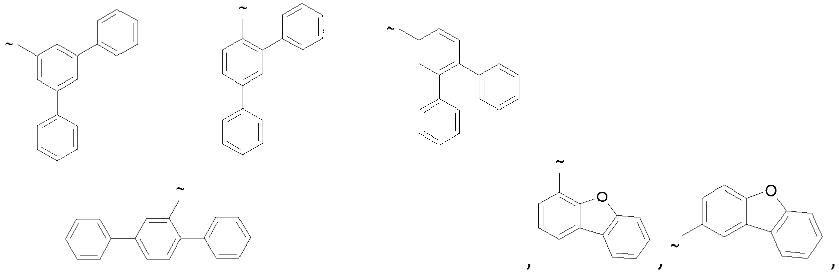 -or 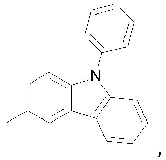
B⁵, B⁶, B⁷ and B⁸ are independently of each other a C₆-C₂₄arylene group, which can optionally be substituted by G, or a C₂-C₃₀heteroarylene group, which can optionally be substituted by G;
s is 0 or 1, t is 0 or 1, u is 0 or 1, v is 0 or 1;
R¹⁰ is H, a C₁-C₂₅alkyl group, which can optionally be substituted by E and or interrupted by D; a C₆-C₂₄aryl group, which can optionally be substituted by G, or a C₁-C₂₄heteroaryl group, which can optionally be substituted by G;
a is 1, 2 or 3;
D is -CO-, -COO-, -S-, -SO-, -SO₂-, -O-, -NR65-, -SiR⁷⁰R⁷¹-, -POR72-, -CR⁶³=CR⁶⁴-, or-C≡C;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₂₄alkyl group, a C₆-C₆oaryl group, a C₆-C₆₀aryl group, which is substituted by F, C₁-C₂₄alkyl, or C₁-C₂₄alkyl which is interrupted by O; a C₂-C₆₀heteroaryl group, or a C₂-C₆₀heteroaryl group, which is substituted by F, C₁-C₁₈alkyl, or C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ may form together with the atom to which they are bonded a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl.

Suitable compounds of formula (1") are therefore the following compounds:

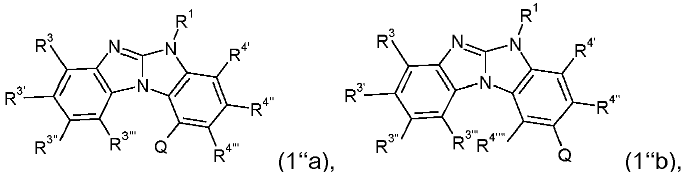

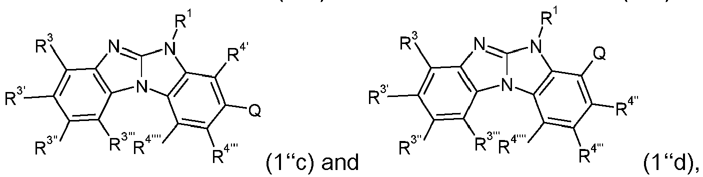

Suitable compounds of formula (1") are therefore the following compounds: 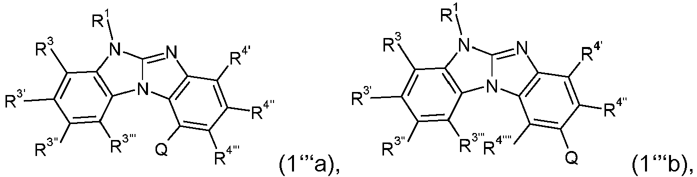 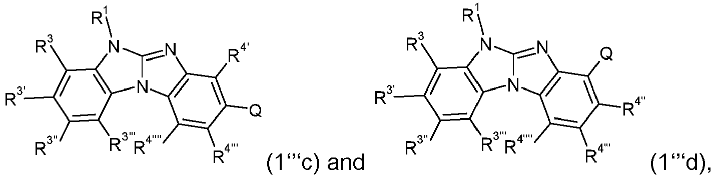 wherein
R^{4'}, R^{4"}, R^{4‴} and R^{4ʺʺ} are defined as R⁴, i.e. are H; and R¹, R³, R^{3'}, R^{3"}, R^{3‴} and Q are as defined in formulae (1") and (1‴).

### Preparation of the compounds of formula (1")

The compounds of formula (1"), i.e. (1"a), (1"b), (1"c) and (1"d), are preferably prepared by the following process:

Reaction of a compound of formula (31) with a compound of formula (32) in the presence of a base, whereby a compound of formula (1"), i.e. 1"a 1"b (1"c) and 1"d is obtained: 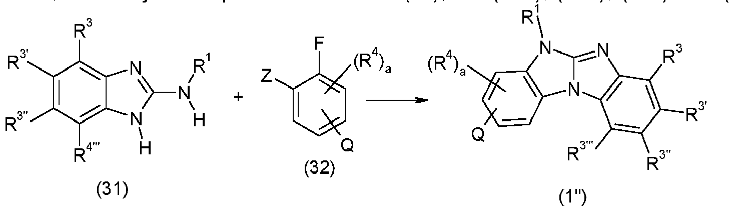 wherein
R¹, R³, R^{3'}, R^{3"}, R^{3‴}, R⁴, a and Q have the meanings as mentioned in the definition of formula (1") and Z is F, Cl, Br, or I, preferably Cl or Br, more preferably Br.

The molar ratio between the compound of formula (31) and the compound of formula (32) is usually 2:1 to 1:2, preferably 1.5:1 to 1:1.5, more preferably 1.3:1 to 1:1.3, most preferably 1.1:1 to 1:1.1 and further most preferably 1:1.

Suitable bases in the reaction mentioned above are preferably selected from the group consisting of potassium phosphate tribasic (K₃PO₄), K₂CO₃, Na₂CO₃, Cs₂CO₃, NaH, NaOtBu, KOtBu, preferably K₃PO₄. It is also possible to use a mixture of two or more bases.

The molar ratio between the compound of formula (31) and the base is usually 2:1 to 1:10, preferably 1:1 to 1:7, more preferably 1:1.5 to 1:5, most preferably 1:2 to 1:3.5.

The reaction mentioned above is preferably carried out in a solvent. Suitable solvents are for example (polar) aprotic solvents, preferably tertiary carboxylic acid amides like dimethyl acetamide (DMA), dimethyl formamide (DMF), dimethylsulfoxide (DMSO), N-methyl-2-pyrrolidone (NMP), 1,3-dimethyl imidazolidone (DMI), or mixtures thereof, preferably DMA.

The reaction temperature in the reaction mentioned above is usually 20°C to 220°C, preferably 50°C to 200°C, more preferably 70°C to 190°C, most preferably 90°C to 180°C.

The reaction time in the reaction mentioned above is usually 10 minutes to 72 hours, preferably 30 minutes to 24 hours, more preferably 2 hours to 16 hours.

The reaction pressure is not critical and usually atmospheric pressure.

### Preparation of the compound of formula (31)

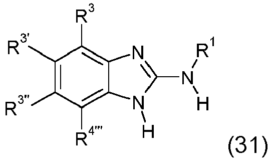

The compound of formula (31) is preferably prepared by reaction of a compound of formula (33) with R¹-NH₂: 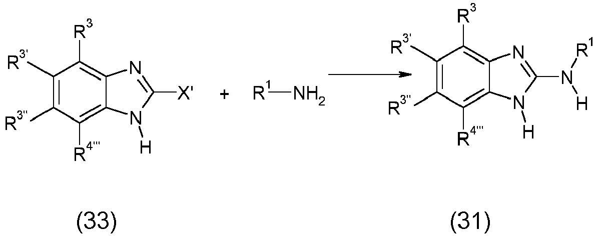 wherein
X' is Cl or Br, preferably Cl;
R¹, R³, R^{3'}, R^{3"} and R^{3‴} have the meanings as mentioned in the definition of formula (1").

The molar ratio of the compound of formula (33) to R¹-NH₂ is usually 2:1 to 1:2, preferably 1.5:1 to 1:1.5, more preferably 1.3:1 to 1:1.3, most preferably 1.1:1 to 1:1.1 and further most preferably 1:1.

The reaction mentioned above is preferably carried out in a solvent. Suitable solvents are alcohols, for example tert. butanol, (polar) aprotic solvents, for example tertiary carboxylic acid amides like dimethyl acetamide (DMA), dimethyl formamide (DMF), N-methyl-2-pyrrolidone (NMP), 1,3-dimethyl imidazolidone (DMI), nitrobenzene or mixtures thereof.

The reaction is further preferably carried out in the presence of an acid. Suitable acids are alkyl sulphonic acids like methane sulphonic acid, sulphonic acid, HCI (gas), p-toluene sulphonic acid, trifluoromethane sulphonic acid or mixtures thereof. The molar ratio of the acid to R¹-NH₂ is usually 2:1 to 1:3, preferably 1.5:1 to 1:2.5, more preferably 1.3:1 to 1:2, most preferably 1.1:1 to 1:1.8.

In a further embodiment, in the reaction mentioned above no acid is employed.

The reaction temperature in the reaction mentioned above is usually 20°C to 190°C, preferably 30°C to 180°C, more preferably 60°C to 140°C, most preferably 80°C to 120°C.

The reaction time in the reaction mentioned above is usually 10 minutes to 72 hours, preferably 2 hours to 48 hours, more preferably 3 hours to 24 hours.

The reaction pressure is not critical and usually atmospheric pressure.

The synthesis of 1-aryl-benzimidazol-2-amine is for example described in US 2009/0186879 (page 57).

### Preferred Br or Cl substituted compounds of formula (1")

Specific examples of Br or Cl substituted compounds of formula (1") are shown in the table below:
By using the aryl-amines and 1-fluoro-2-bromobenzens given in the table the intermediates in the table can be prepared according to the procedure mentioned above and described for example in US 2009/0186879.

| Nr. | Ar-NH₂ | F,Br-benzene | Intermediate |
|---|---|---|---|
| 1*-1 | 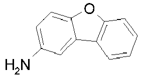 | 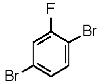 | 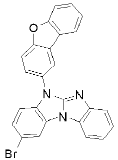 |
| 1*-2 | 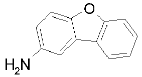 | 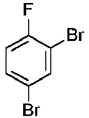 | 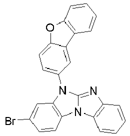 |
| 1*-3 | 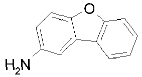 | 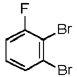 | 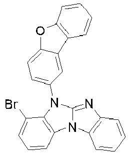 |
| 1*-4 | 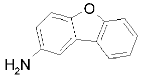 | 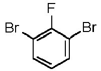 | 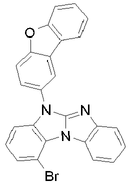 |
| 1*-5 | 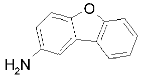 | 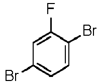 | 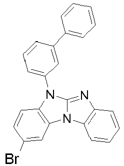 |
| 1*-6 | 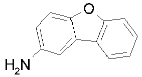 | 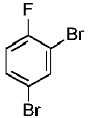 | 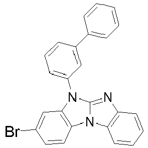 |
| 1*-7 | 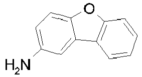 | 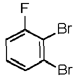 | 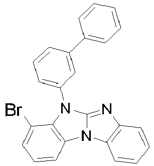 |
| 1*-8 | 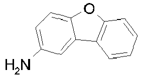 | 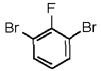 | 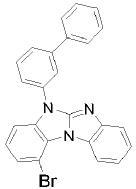 |
| 1*-9 | 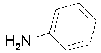 | 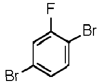 | 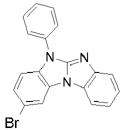 |
| 1*-10 | 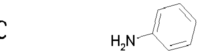 | 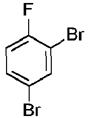 | 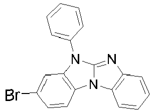 |
| 1*-1 | 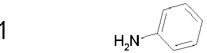 | 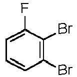 | 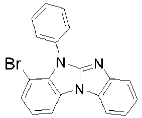 |
| 1*-12 | 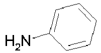 | 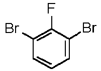 | 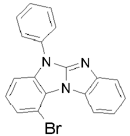 |
| 1*-13 | 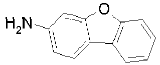 | 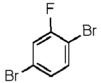 | 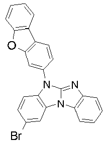 |
| 1*-14 | 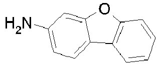 | 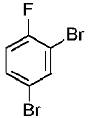 | 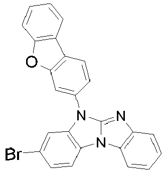 |
| 1*-15 | 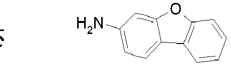 | 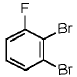 | 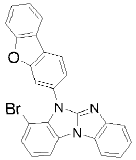 |
| 1*-16 | 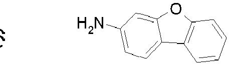 | 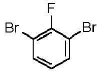 | 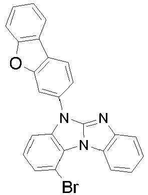 |
| 1*-17 | 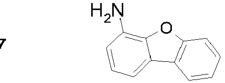 | 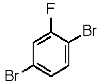 | 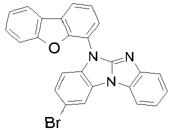 |
| 1*-18 | 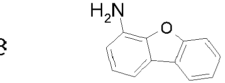 | 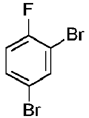 | 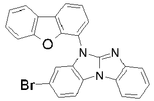 |
| 1*-19 | 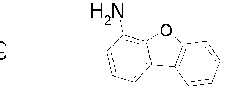 | 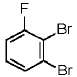 | 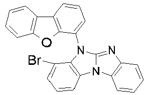 |
| 1*-20 | 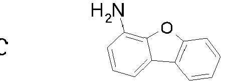 | 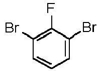 | 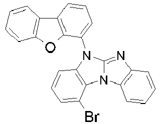 |
| 1*-21 | 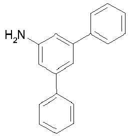 | 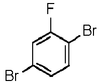 | 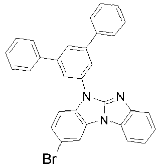 |
| 1*-22 | 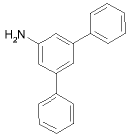 | 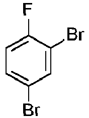 | 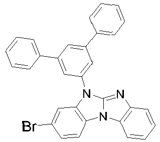 |
| 1*-23 | 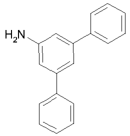 | 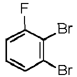 | 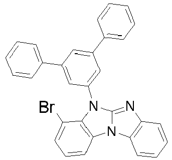 |
| 1*-24 | 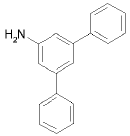 | 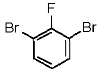 | 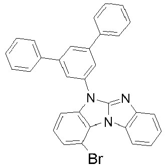 |
| 1*-25 | 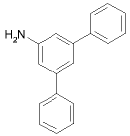 | 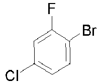 | 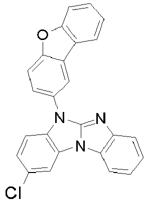 |
| 1*-26 | 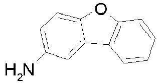 | 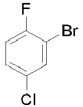 | 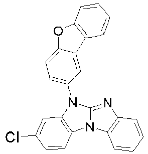 |
| 1*-27 | 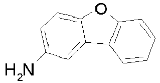 | 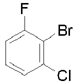 | 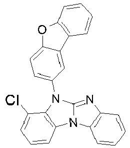 |
| 1*-28 | 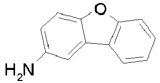 | 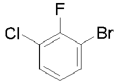 | 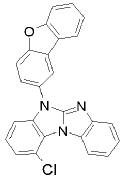 |
| 1*-29 | 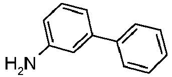 | 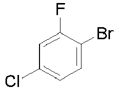 | 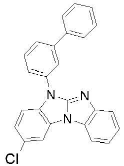 |
| 1*-30 | 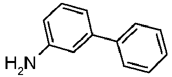 | 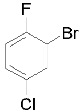 | 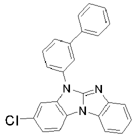 |
| 1*-31 | 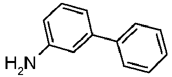 | 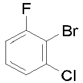 | 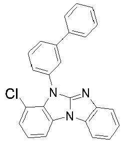 |
| 1*-32 | 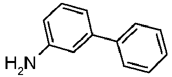 | 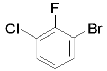 | 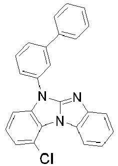 |
| 1*-33 | 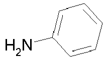 | 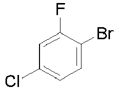 | 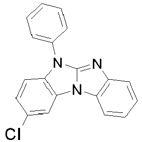 |
| 1*-34 | 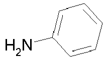 | 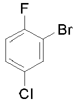 | 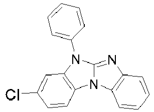 |
| 1*-35 | 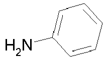 | 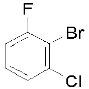 | 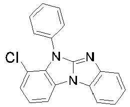 |
| 1*-36 | 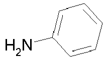 | 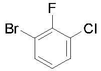 | 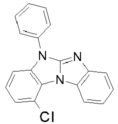 |
| 1*-37 | 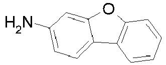 | 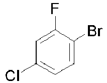 | 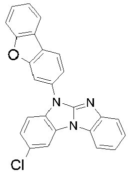 |
| 1*-38 | 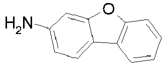 | 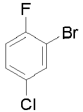 | 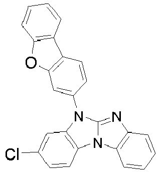 |
| 1*-39 | 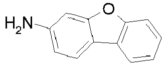 | 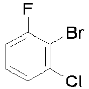 | 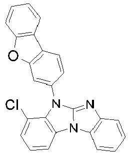 |
| 1*-40 | 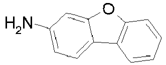 | 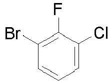 | 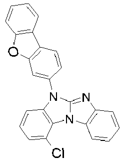 |
| 1*-41 | 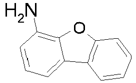 | 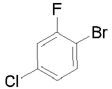 | 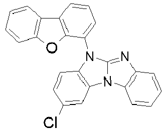 |
| 1*-42 | 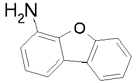 | 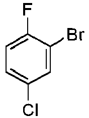 | 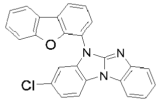 |
| 1*-43 | 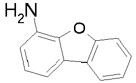 | 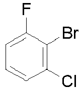 | 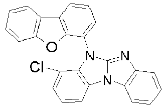 |
| 1*-44 | 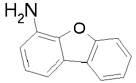 | 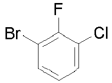 | 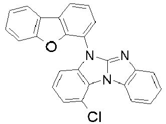 |
| 1*-45 | 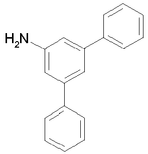 | 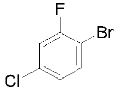 | 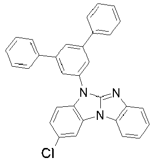 |
| 1*-46 | 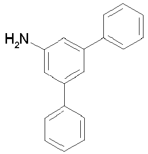 | 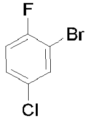 | 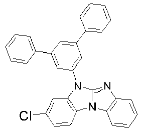 |
| 1*-47 | 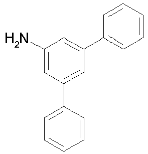 | 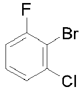 | 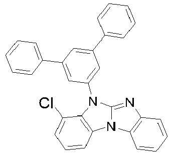 |
| 1*-48 | 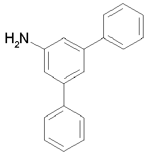 | 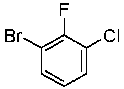 | 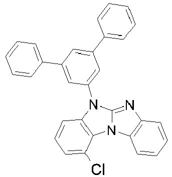 |

### Preparation of the compounds of formula (1‴)

The compounds of formula (1‴), i.e. (1‴a), (1‴b), (1‴c) and (1‴d), are preferably prepared by the following process:

Reaction of a compound of formula (31') with a compound of formula (32') in the presence of a base, whereby a compound of formula (1‴), i.e. (1‴a), (1‴b), (1‴c) and (1‴d), is obtained: 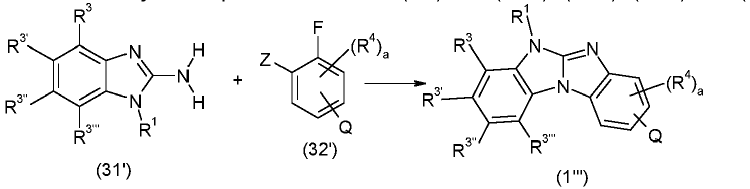 wherein
R¹, R³, R^{3'}, R^{3"}, R^{3‴}, R⁴, a and Q have the meanings as mentioned in the definition of formula (1") and Z is F, Cl, Br, or I, preferably Cl or Br, more preferably Br.

The molar ratio between the compound of formula (31') and the compound of formula (32') is usually 2:1 to 1:2, preferably 1.5:1 to 1:1.5, more preferably 1.3:1 to 1:1.3, most preferably 1.1:1 to 1:1.1 and further most preferably 1:1.

Suitable bases in the reaction mentioned above are preferably selected from the group consisting of potassium phosphate tribasic (K₃PO₄), K₂CO₃, Na₂CO₃, Cs₂CO₃, NaH, NaOtBu, KOtBu, preferably K₃PO₄. It is also possible to use a mixture of two or more bases.

The molar ratio between the compound of formula (31') and the base is usually 2:1 to 1:10, preferably 1:1 to 1:7, more preferably 1:1.5 to 1:5, most preferably 1:2 to 1:3.5.

The reaction mentioned above is preferably carried out in a solvent. Suitable solvents are for example (polar) aprotic solvents, preferably tertiary carboxylic acid amides like dimethyl acetamide (DMA), dimethyl formamide (DMF), dimethyl sulfoxide (DMSO), N-methyl-2-pyrrolidone (NMP), 1,3-dimethyl imidazolidone (DMI), or mixtures thereof, preferably DMA.

The reaction temperature in the reaction mentioned above is usually 20°C to 220°C, preferably 50°C to 200°C, more preferably 70°C to 190°C, most preferably 90°C to 180°C.

The reaction time in the reaction mentioned above is usually 10 minutes to 72 hours, preferably 30 minutes to 24 hours, more preferably 2 hours to 16 hours.

The reaction pressure is not critical and usually atmospheric pressure.

### Preparation of the compound of formula (31')

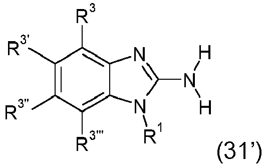

The compound of formula (31') is preferably prepared by reaction of a compound of formula (33') with R¹-X': 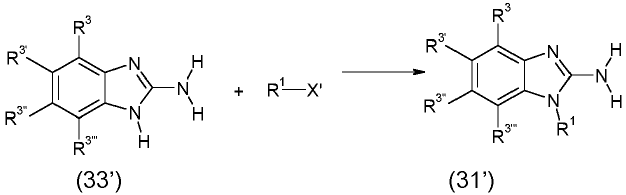 wherein
X' is Br or I, preferably I;
R¹, R³, R^{3'}, R^{3"} and R^{3‴} have the meanings as mentioned in the definition of formula (1").

The molar ratio of the compound of formula (33') to R¹-X' is usually 2:1 to 1:2, preferably 1.5:1 to 1:1.5, more preferably 1.3:1 to 1:1.3, most preferably 1.1:1 to 1:1.1 and further most preferably 1.1:1.

The reaction mentioned above is preferably carried out in a solvent. Suitable solvents are alcohols, for example tert. butanol, (polar) aprotic solvents, for example tertiary carboxylic acid amides like dimethyl acetamide (DMA), dimethyl formamide (DMF), dimethyl sulfoxide (DMSO), N-methyl-2-pyrrolidone (NMP), 1,3-dimethyl imidazolidone (DMI), nitrobenzene or mixtures thereof.

The reaction mentioned above is preferably carried out in the presence of a catalyst. More preferably, a Cu catalyst is employed, for example CuI, Cu₂O, CuO, CuBr, or mixtures thereof. The catalyst is usually used in an amount of 1 mol% to 30 mol%, preferably 3 mol% to 27 mol%, more preferably 4 mol% to 25 mol%, most preferably 5 mol% to 20 mol%, based on the compound of formula (33').

In addition to the catalyst which is preferably present, at least one ligand is preferably present. Said ligand is preferably selected from the group consisting of 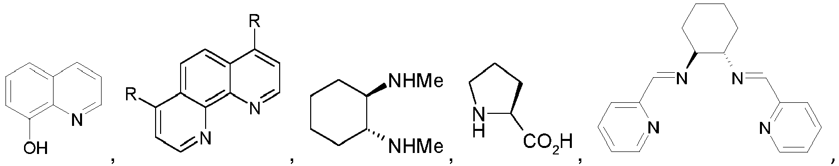 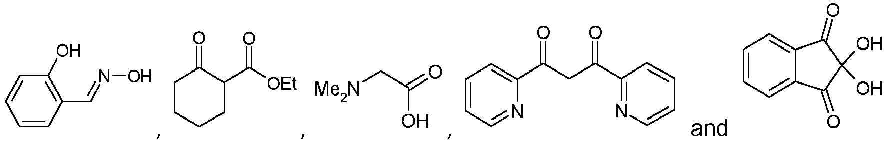 wherein
R is for example OMe. The ligand is usually used in an amount of 5 mol% to 25 mol%, preferably 8 mol% to 20 mol%, more preferably 10 mol% to 17 mol%, most preferably 12 mol% to 16 mol%, based on the compound of formula (33).

The reaction is further preferably carried out in the presence of a base. Suitable bases are K₃PO₄, K₂CO₃, Na₂CO₃, Cs₂CO₃, NaH, NaOtBu, KOtBu, or mixtures thereof, preferably K₃PO₄, K₂CO₃, Na₂CO₃, Cs₂CO₃, or mixtures thereof. The molar ratio of the base to R¹-X' is usually 2:1 to 1:3, preferably 1.5:1 to 1:2.5, more preferably 1.3:1 to 1:2, most preferably 1.1:1 to 1:1.8.

The reaction temperature in the reaction mentioned above is usually 20°C to 190°C, preferably 30°C to 180°C, more preferably 60°C to 170°C.

The reaction time in the reaction mentioned above is usually 10 minutes to 72 hours, preferably 2 hours to 48 hours, more preferably 3 hours to 24 hours.

The reaction pressure is not critical and usually atmospheric pressure.

The synthesis of 1-aryl-benzimidazol-2-amine is for example described in Angew. Chem. Int. Ed. 2012, 51, 10364 -10367.

### Preferred Br or Cl substituted compounds of formula (1‴)

Specific examples of Br or Cl substituted compounds of formula (1"') are shown in the table below:
By using the aryl-iodides and 1-fluoro-2-bromobenzens given in the table the intermediates in the table can be prepared according to the procedure mentioned above and described for example in Angew. Chem. Int. Ed. 2012, 51, 10364 -10367.

| Nr. | Ar-I | F,Br-benzene | Intermediate |
|---|---|---|---|
| 1*-49 | 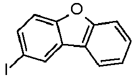 | 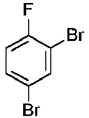 | 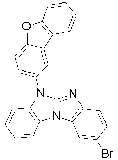 |
| 1*-50 | 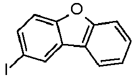 | 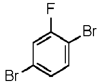 | 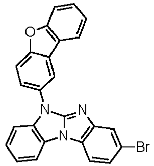 |
| 1*-51 | 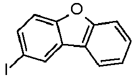 | 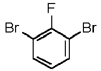 | 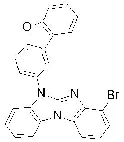 |
| 1*-52 | 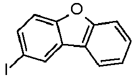 | 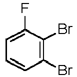 | 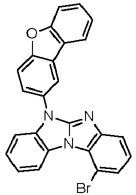 |
| 1*-53 | 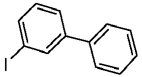 | 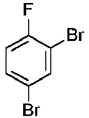 | 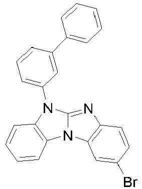 |
| 1*-54 | 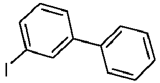 | 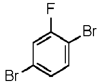 | 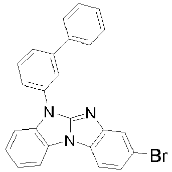 |
| 1*-55 | 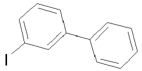 | 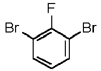 | 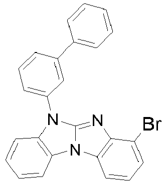 |
| 1*-56 | 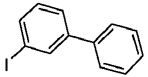 | 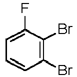 | 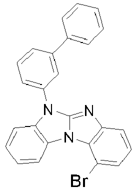 |
| 1*-57 | 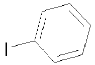 | 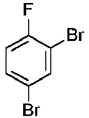 | 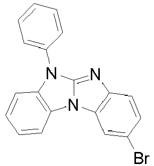 |
| 1*-58 | 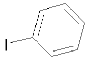 | 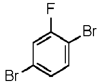 | 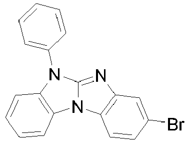 |
| 1*-59 | 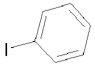 | 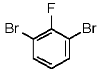 | 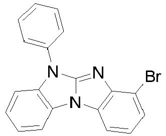 |
| 1*-60 | 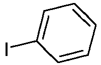 | 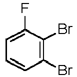 | 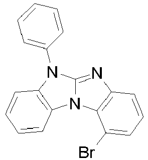 |
| 1*-61 | 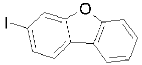 | 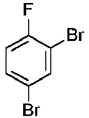 | 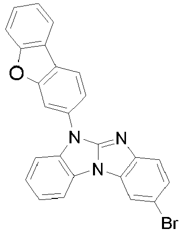 |
| 1*-62 | 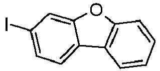 | 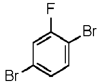 | 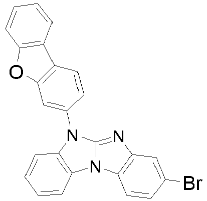 |
| 1*-63 | 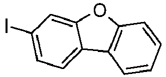 | 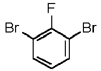 | 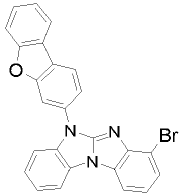 |
| 1*-64 | 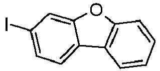 | 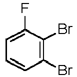 | 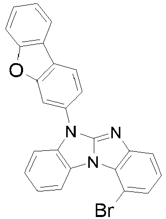 |
| 1*-65 | 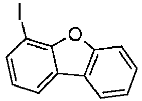 | 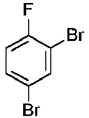 | 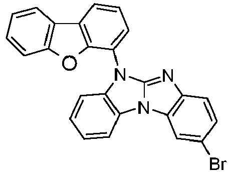 |
| 1*-66 | 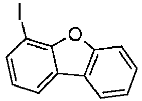 | 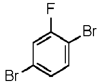 | 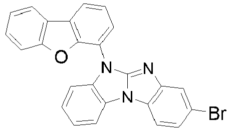 |
| 1*-67 | 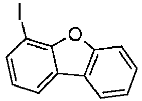 | 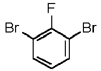 | 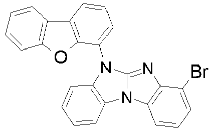 |
| 1*-68 | 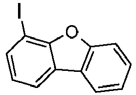 | 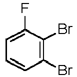 | 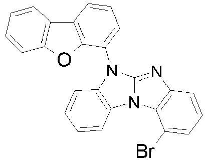 |
| 1*-69 | 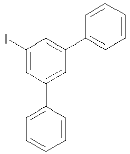 | 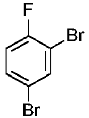 | 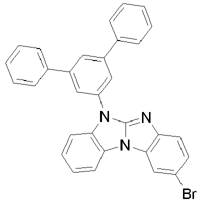 |
| 1*-70 | 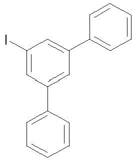 | 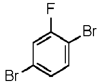 | 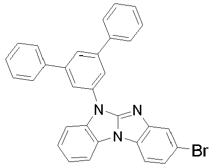 |
| 1*-71 | 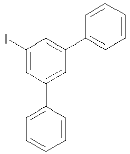 | 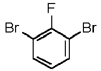 | 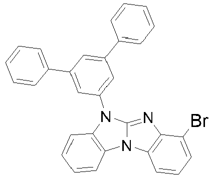 |
| 1*-72 | 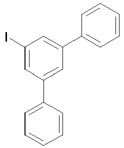 | 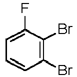 | 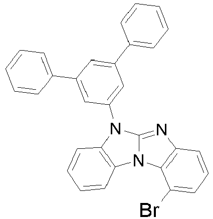 |
| 1*-73 | 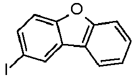 | 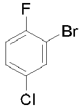 | 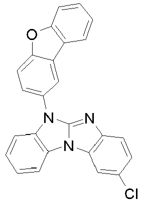 |
| 1*-74 | 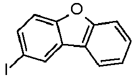 | 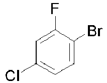 | 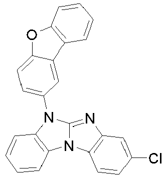 |
| 1*-75 | 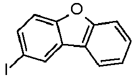 | 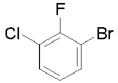 | 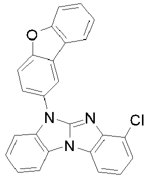 |
| 1*-76 | 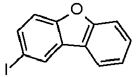 | 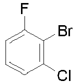 | 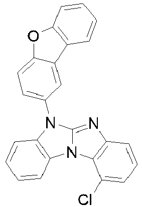 |
| 1*-77 | 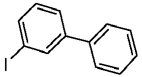 | 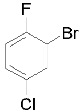 | 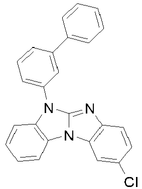 |
| 1*-78 | 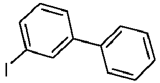 | 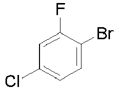 | 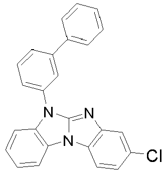 |
| 1*-79 | 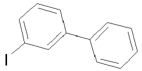 | 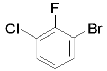 | 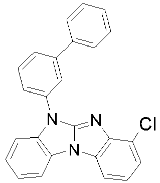 |
| 1*-80 | 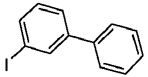 | 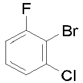 | 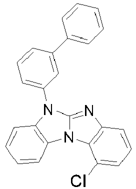 |
| 1*-81 | 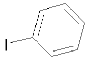 | 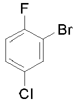 | 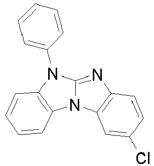 |
| 1*-82 | 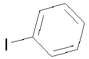 | 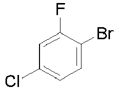 | 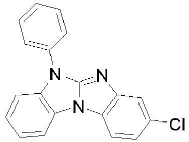 |
| 1*-83 | 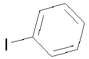 | 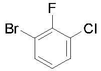 | 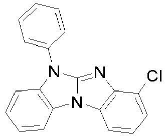 |
| 1*-84 | 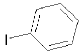 | 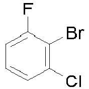 | 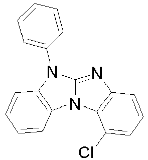 |
| 1*-85 | 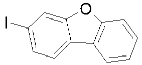 | 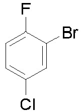 | 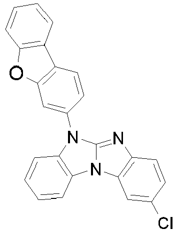 |
| 1*-86 | 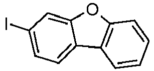 | 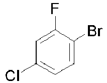 | 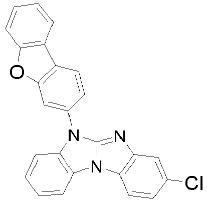 |
| 1*-87 | 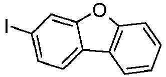 | 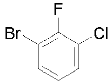 | 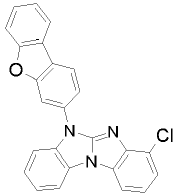 |
| 1*-88 | 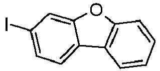 | 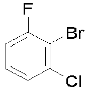 | 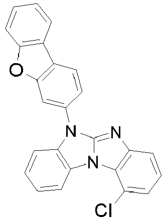 |
| 1*-89 | 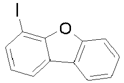 | 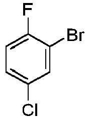 | 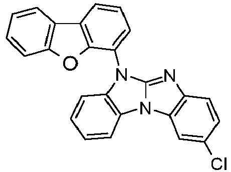 |
| 1*-90 | 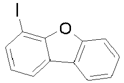 | 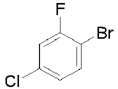 | 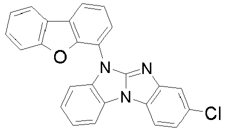 |
| 1*-91 | 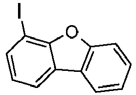 | 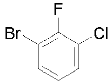 | 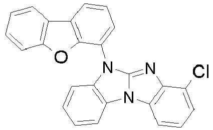 |
| 1*-92 | 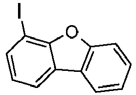 | 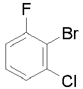 | 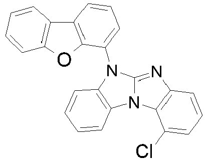 |
| 1*-93 | 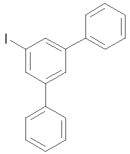 | 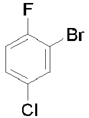 | 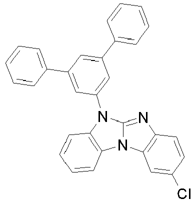 |
| 1*-94 | 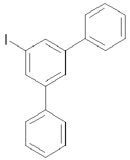 | 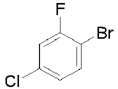 | 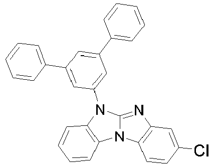 |
| 1*-95 | 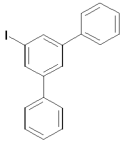 | 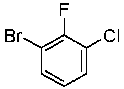 | 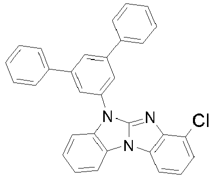 |
| 1*-96 | 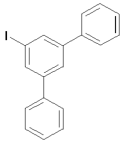 | 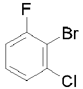 | 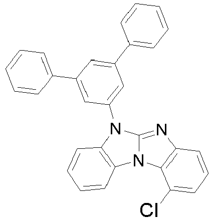 |

The compounds of formula (1") or (1‴) are then further coupled, in the case of formula (1), with at least one group of formula 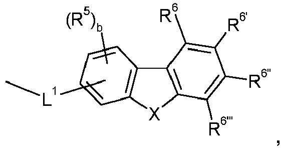 wherein L¹, X, R⁵, R⁶, R^{6'}, R^{6"}, R^{6‴} and b are defined above;
and in the case of formulae (Bim1) and (Bim2) with at least one group of formula -(B⁵)ₛ-(B⁶)ₜ-(B⁷)ᵤ-(B⁸)ᵥ-R¹⁰, wherein R¹⁰, B⁵, B⁶, B⁷, B⁸, s, t, u and v are defined above.

Specific examples are:
i) Coupling of a compound of formula (1") or (1‴) 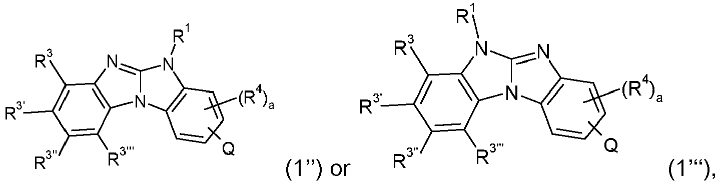 wherein the residues R¹, R3, R^{3'}, R^{3"}, R^{3‴} and R⁴ and the index a are described above, with a diboronic acid or diboronate of the group of formula 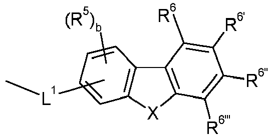 respectively -(B⁵)ₛ-(B⁶)ₜ-(B⁷)ᵤ-(B⁸)ᵥ-R¹⁰,
   wherein L¹, X, R⁵, R⁶, R^{6'}, R^{6"}, R^{6‴}' and b are defined above and R¹⁰, B⁵, B⁶, B⁷, B⁸, s, t, u and v are defined above; or
ii) Coupling of a compound of formula (1ʺʺ) or (1 ‴ʺ) 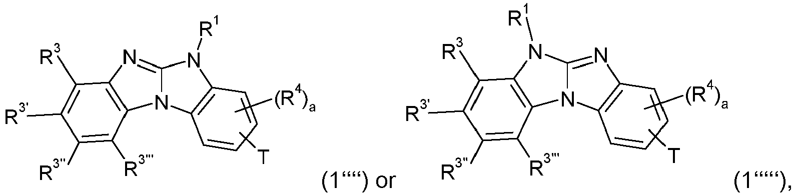 wherein the residues R¹, R³, R^{3'}, R^{3"}, R^{3‴} and R⁴ and the index a are described above, T is a diboronic acid group or diboronate group,
   with a halide i.e. iodide bromide or chloride preferably chloride or bromide more preferably bromide, of the group of of formula 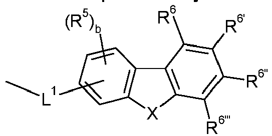 respectively -(B⁵)ₛ-(B⁶)ₜ-(B⁷)ᵤ-(B⁸)ᵥ-R¹⁰,
   wherein L¹, X, R⁵, R⁶, R^{6'}, R^{6"}, R^{6‴} and b are defined above and R¹⁰, B⁵, B⁶, B⁷, B⁸, s, t, u and v are defined above.

Diboronic acid or diboronate group containing groups of formula -(B⁵)ₛ-(B⁶)ₜ-(B⁷)ᵤ-(B⁸)ᵥ-R¹⁰can be readily prepared by an increasing number of routes. An overview of the synthetic routes is, for example, given in Angew. Chem. Int. Ed. 48 (2009) 9240 - 9261. General examples are mentioned below.

Halide group containing groups of formula -(B⁵)ₛ-(B⁶)ₜ-(B⁷)ᵤ-(B⁸)ᵥ-R¹⁰ can be readily prepared by an increasing number of routes. General examples are mentioned below.

Suitable reaction conditions for the Suzuki coupling are known by a person skilled in the art.

### Base skeleton

The synthesis of the compounds of formula (1) can be carried out in analogy to the synthesis of benzimidazolo[1,2-a]benzimidazoles mentioned in the related art.

The synthesis of 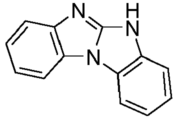 is described, for example, in Achour, Reddouane; Zniber, Rachid, Bulletin des Societes Chimiques Belges 96 (1987) 787-92, WO12130709, Org. Lett. 2012,14, 02, 452, Eur. J. Org. Chem. 2014, 5986-5997, and RSC Advances 2014, 4, 21904-21908

### N-arylation

The introduction of the group -R¹ (N-arylation) is generally carried out by reacting the base skeleton 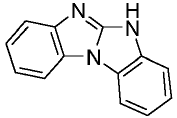 with a group Hal-R¹, wherein Hal is F, Cl, Br or I, preferably F, Br or I. Suitable groups R¹ are mentioned before.

The nucleophilic aromatic substitution (N-arylation) of 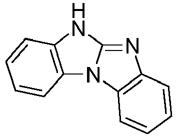 with F-R¹ is generally performed in the presence of a base (Angew. Chem. 2012, 124, 8136 -8140, Angew. Chem. Int. Ed. 2008, 47, 8104 -8107). Suitable bases are known to those skilled in the art and are preferably selected from the group consisting of alkali metal alkali metal and alkaline earth metal hydroxides such as NaOH, KOH, Ca(OH)₂, alkali metal hydrides such as NaH, KH, alkali metal amides such as NaNH₂, alkali metal or alkaline earth metal carbonates such as K₂CO₃ or Cs₂CO₃, alkaline metal phosphates such as K₃PO₄ alkaline metal fluorides such as KF, CsF and alkali metal alkoxides such as NaOMe, NaOEt. In addition, mixtures of the aforementioned bases are suitable. K₂CO₃ or Cs₂CO₃, K₃PO₄ are preferred.

The nucleophilic aromatic substitution (N-arylation) can be performed in solvent or in a melt. Preferably, the reaction is carried out in a solvent. Suitable solvents are, for example, (polar) aprotic solvents such as dimethyl sulfoxide (DMSO), dimethylformamide (DMF), N-methyl-2-pyrrolidone (NMP) or dimethylacetamide (DMA).

The reaction temperature is strongly dependent on the reactivity of the aryl fluoride. The reaction (N-arylation) is preferably carried out at a temperature of -10 to 220 °C, more preferably 60 to 150 °C.

Ullmann reaction (N-arylation) of 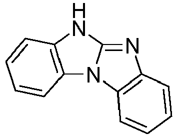 with Y-R¹ (Y is Cl, Br, or I) generally performed in the presence of a base and a catalyst.

Reaction conditions for Ullmann reactions are, for example, described in Angew Chem Int Ed Engl., 48 (2009) 6954-71 WO14009317, WO12130709, J. Am. Chem. Soc. 131 (2009) 2009-2251, J. Org. Chem, 70 (2005) 5165.

Typically the Ullmann coupling of the compound of formula 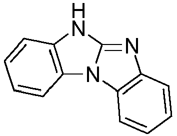 with a compound of formula Y-R¹ (Y is Cl, Br, or I, especially Br, I very especially I) is done in the presence of copper, or a copper salt, such as, for example, CuI, CuBr, Cu₂O, or CuO, and a ligand, such as, for example, L-proline, trans-cyclohexane-1,2-diamine (DACH), 1,10-phenanthroline in a solvent, such as, for example, dimethylsulfoxide (DMSO), dimethylformamide (DMF), dimethylacetamide (DMA), N-methylpyrrolidone (NMP) and dioxane, or a solvent mixture. The reaction temperature is dependent on the reactivity of the starting materials, but is generally in the range of 25 to 200 °C. If copper salt are used without a ligand the reaction temperatures are higher.

The N-arylation is, for example, disclosed in H. Gilman and D. A. Shirley, J. Am. Chem. Soc. 66 (1944) 888; D. Li et al., Dyes and Pigments 49 (2001) 181 - 186 and Eur. J. Org. Chem. (2007) 2147-2151.

Suitable base skeletons of the formula 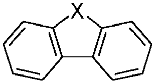 are either commercially available (especially in the cases when X is S, O, NH), or can be obtained by processes known to those skilled in the art. Reference is made to WO2010079051 and EP1885818.

The halogenation of said base skeletons 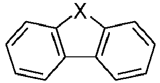 (carbazole, dibenzofuran or dibezothiophene, which is unsubstituted or substituted) can be performed by methods known to those skilled in the art. Preference is given to brominating or iodinating in the 3 and 6 positions (dibromination, diiodation or mixed bromination/iodation) or in the 3 or 6 positions (monobromination, monoiodation) of the base skeleton in the case of carbazole, respectively in the 2 and 8 positions (dibromination, diiodation) or in the 2 or 8 positions (monobromination, monoiodation) of the base skeleton in the case of dibenzofuran and dibenzothiophene.

Optionally substituted dibenzofurans, dibenzothiophenes and carbazoles can be dibrominated in the 2,8 positions (dibenzofuran and dibenzothiophene) or 3,6 positions (carbazole) with bromine or NBS in glacial acetic acid or in chloroform. For example, the bromination with Br₂ can be effected in glacial acetic acid or chloroform at low temperatures, e.g. 0°C. Suitable processes are described, for example, in M. Park, J.R. Buck, C.J. Rizzo, Tetrahedron, 54 (1998) 12707-12714 for X= NPh, and in W. Yang et al., J. Mater. Chem. 13 (2003) 1351 for X=S. In addition, 3,6-dibromocarbazole, 3,6-dibromo-9-phenylcarbazole, 2,8-dibromodibenzothiophene, 2,8-dibromodibenzofuran, 2-bromocarbazole, 3-bromodibenzothiophene, 3-bromodibenzofuran, 3-bromocarbazole, 2-bromodibenzothiophene and 2-bromodibenzofuran are commercially available.

Monobromination in the 4 position of dibenzofuran (and analogously for dibenzothiophene) is described, for example, in J. Am. Chem. Soc. 1984, 106, 7150. Dibenzofuran (dibenzothiophene) can be monobrominated in the 3 position by a sequence known to those skilled in the art, comprising a nitration, reduction and subsequent Sandmeyer reaction.

Monobromination in the 2 position of dibenzofuran or dibenzothiophene and monobromination in the 3 position of carbazole are effected analogously to the dibromination, with the exception that only one equivalent of bromine or NBS is added.

For the nucleophilic substitution, Cl- or F-substituted dibenzofurans, dibenzothiophenes and carbazoles are preferred. The chlorination is described, inter alia, in J. Heterocyclic Chemistry, 34 (1997) 891-900, Org. Lett., 6 (2004) 3501-3504; J. Chem. Soc. [Section] C: Organic, 16 (1971) 2775-7, Tetrahedron Lett. 25 (1984) 5363-6, J. Org. Chem. 69 (2004) 8177-8182. The fluorination is described in J. Org. Chem. 63 (1998) 878-880 and J. Chem. Soc., Perkin Trans. 2, 5 (2002) 953-957.

*Introduction of the* 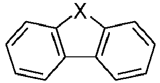 *skeleton*

The introduction of the 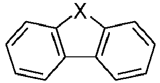 skeleton, can be affected, for example, by copper-catalyzed coupling (Ullmann reaction). Suitable reaction components and reaction conditions for carrying out the Ullmann reaction are mentioned above.

Alternatively, the introduction of the 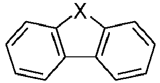 skeleton, especially in cases, wherein the 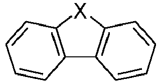 skeleton is substituted, e.g. by a group 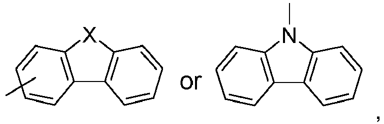 can be affected, for example, by Pd catalyzed coupling of diboronic acid or diboronate group containing dibenzofurans, dibenzothiophenes or carbazoles with halogenated aromatic groups, wherein the halogen is preferably I (Suzuki coupling).

An Example for a Suzuki coupling is shown in the example part of the present application:

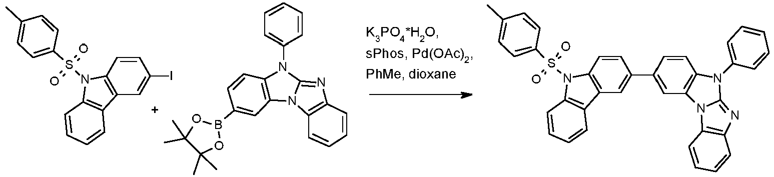

Diboronic acid or diboronate group containing dibenzofurans, dibenzothiophenes and carbazoles can be readily prepared by an increasing number of routes. An overview of the synthetic routes is, for example, given in Angew. Chem. Int. Ed. 48 (2009) 9240 - 9261.

By one common route diboronic acid or diboronate group containing dibenzofurans, dibenzothiophenes, and carbazoles can be obtained by reacting halogenated dibenzofurans, dibenzothiophenes and carbazoles with (Y1O)2B-B(OY1)2, 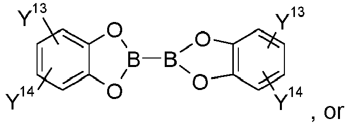 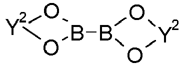 in the presence of a catalyst, such as, for example, [1,1'-bis(diphenylphosphino)ferrocene]dichloropalladium(II), complex (Pd(Cl)2(dppf)), and a base, such as, for example, potassium acetate, in a solvent, such as, for example, dimethyl formamide, dimethyl sulfoxide, dioxane and/or toluene (cf. Prasad Appukkuttan et al., Synlett 8 (2003) 1204), wherein Y1 is independently in each occurrence a C₁-C₁₈alkylgroup and Y² is independently in each occurrence a C₂-C₁₀alkylene group, such as -CY³Y⁴-CY⁵Y⁶-, or - CY⁷Y⁸-CY⁹Y¹⁰- CY¹¹Y¹²-, wherein Y³, Y⁴, Y⁵, Y⁶, Y⁷, Y⁸, Y⁹, Y¹⁰, Y¹¹ and Y¹² are independently of each other hydrogen, or a C₁-C₁₈alkylgroup, especially -C(CH₃)₂C(CH₃)₂-, - C(CH₃)₂CH₂C(CH₃)₂-, or -CH₂C(CH₃)₂CH₂-, and Y¹³ and Y¹⁴ are independently of each other hydrogen, or a C₁-C₁₈alkylgroup.

Diboronic acid or diboronate group containing dibenzofurans, dibenzothiophenes and carbazoles can also be prepared by reacting halogenated dibenzofurans, dibenzothiophenes and carbazoles with alkyl lithium reagents, such as, for example, n-butyl lithium, or t-buthyl lithium, followed by reaction with boronic esters, such as, for example, B(isopropoxy)₃, B(methoxy)₃, or 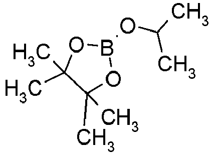 (cf. Synthesis (2000) 442-446).

Diboronic acid or diboronate group containing dibenzofurans, dibenzothiophenes and carbazoles can also be prepared by reacting dibenzofurans, dibenzothiophenes and carbazoles with lithium amides, such as, for example, lithium diisopropylamide (LDA) followed by reaction with boronic esters such as, for example, B(isopropoxy)₃, B(methoxy)₃, or 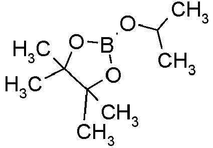 (J. Org. Chem. 73 (2008) 2176-2181).

### Compounds of formula (1) in organic electronics applications

It has been found that the compounds of the formula (1) are particularly suitable for use in applications in which charge carrier conductivity is required, especially for use in organic electronics applications, for example selected from switching elements such as organic transistors, e.g. organic FETs and organic TFTs, organic solar cells and organic light-emitting diodes (OLEDs).

The *organic transistor* generally includes a semiconductor layer formed from an organic layer with charge transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor. The layers with charge transport capacity may comprise the compounds of formula (1).

The *organic solar cell* (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or charge transport capacity. In addition, it is possible for further layers known to those skilled in the art to be present in the organic solar cell. The layers with charge transport capacity may comprise the compounds of formula (1).

The compounds of the formula (1) being particularly suitable in *OLEDs* for use as matrix material in a light-emitting layer and/or as electron and/or exciton blocker material and/or hole transport materials, especially in combination with a phosphorescence emitter.

The organic electronic device, which is preferably an organic electroluminescent device, wherein the organic electroluminescent device comprises an organic thin film layer between a cathode and an anode, wherein the organic thin film layer comprises one or more layers and comprises a light emitting layer, and at least one layer of the organic thin film layer comprises the compound of formula (1) according to the present invention. Preferably, the light emitting layer comprises the compound of formula (1) according to the present invention.

The organic electronic device preferably comprises a light emitting layer, wherein the light emitting layer comprises a phosphorescent material, which is an ortho-metallated complex comprising a metal atom selected from iridium (Ir), osmium (Os) and platinum (Pt).

In the case of use of the inventive compounds of the formula (1) in OLEDs, OLEDs which have good efficiencies and a long lifetime and which can be operated especially at a low use and operating voltage are obtained. Preferably, the inventive compounds of the formula (1) are suitable for providing OLEDs which ensure good operative lifetimes and/or a low use and operating voltage of the OLEDs.

The inventive compounds of the formula (1) are suitable especially for use as matrix and/or charge transport, preferably electron transport, and/or electron injection materials for green, red and yellow, preferably green and red, more preferably green emitters. Furthermore, the compounds of the formula (1) can be used as conductor/complementary materials in organic electronics applications selected from switching elements and organic solar cells. (In the sense of the present application, the terms matrix and host are used interchangeable).

In the emission layer or one of the emission layers of an OLED, it is also possible to combine an emitter material with at least one matrix material of the compound of the formula (1) and one or more, preferably one, further matrix materials (co-host). This may achieve a high quantum efficiency, low driving voltage and/or long lifetime of this devices.

It is likewise possible that the compounds of the formula (1) are present in two or three of the following layers: in the light-emitting layer (preferably as matrix material), in the injection layer (as electron injection material) and/or in the transport layer (as charge transport, preferably electron transport material).

When a compound of the formula (1) is used as matrix (host) material in an emission layer and additionally as electron injection material and/or ascharge transport, preferably electron transport material, owing to the chemical identity or similarity of the materials, an improved interface between the emission layer and the adjacent material, which can lead to a decrease in the voltage with equal luminance and to an extension of the lifetime of the OLED. Moreover, the use of the same material as charge transport, preferably electron transport material and/or as electron injection material and as matrix of an emission layer allows the production process of an OLED to be simplified, since the same source can be used for the vapor deposition process of the material of one of the compounds of the formula the compound of the formula (1).

Suitable structures of organic electronic devices, especially organic light-emitting diodes (OLED), are known to those skilled in the art and are specified below.

The present invention further provides an organic light-emitting diode (OLED) comprising an anode (a) and a cathode (i) and a light-emitting layer (e) arranged between the anode (a) and the cathode (i), and if appropriate at least one further layer selected from the group consisting of at least one blocking layer for holes/excitons, at least one blocking layer for electrons/excitons, at least one hole injection layer, at least one hole transport layer, at least one electron injection layer and at least one electron transport layer, wherein the at least one compound of the formula (1) is present in the light-emitting layer (e) and/or in at least one of the further layers. The at least one compound of the formula the compound of the formula (1) is preferably present in the light-emitting layer and/or the electron injection layer and/or the charge transport, preferably electron transport layer.

In a preferred embodiment of the present invention, at least one compound of the formula the compound of the formula (1) is used as charge transport, preferably electron transport material. Examples of preferred compounds of the formula (1) are shown above.

In another preferred embodiment of the present invention, at least one compound of the formula the compound of the formula (1) is used as electron injection material. Examples of preferred compounds of the formula (1) are shown above.

The present application further relates to a light-emitting layer comprising at least one compound of the formula (1), preferably as host material or co-host material. Examples of preferred compounds of the formula (1) are shown above.

### Structure of the inventive OLED

The inventive organic light-emitting diode (OLED) thus generally has the following structure: an anode (a) and a cathode (i) and a light-emitting layer (e) arranged between the anode (a) and the cathode (i).

The inventive OLED may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different than the aforementioned structure are also possible, and are known to those skilled in the art. For example, it is possible that the OLED does not have all of the layers mentioned; for example, an OLED with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. OLEDs which have layers (a), (c), (e) and (i), or layers (a), (e), (f), (g) and (i), are likewise suitable. In addition, the OLEDs may have a blocking layer for electrons/excitons (d) between the hole transport layer (c) and the Light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron transport layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used in accordance with the invention.

In a preferred embodiment the OLED according to the present invention comprises in this order:
(a) an anode,
(b) optionally a hole injection layer,
(c) optionally a hole transport layer,
(d) optionally an exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the OLED according to the present invention comprises in this order:
(a) an anode,
(b) optionally a hole injection layer,
(c) a hole transport layer,
(d) an exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the related art and are described in more detail below on basis of preferred embodiments.

### Anode (a):

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole injection layer (b):

Generally, injection layers are comprised of a material that may improve the injection of charge carriers from one layer, such as an electrode or a charge generating layer, into an adjacent organic layer. Injection layers may also perform a charge transport function. The hole injection layer may be any layer that improves the injection of holes from anode into an adjacent organic layer. A hole injection layer may comprise a solution deposited material, such as a spin-coated polymer, or it may be a vapor deposited small molecule material, such as, for example, CuPc or MTDATA. Polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore^{®} OC Conducting Inks commercially available from Plextronics), and copolymers such as poly(3,4-ethylenedioxythiophene)/poly(4-styrenesulfonate) also called PEDOT/PSS.

An example for a suitable hole injection material is: 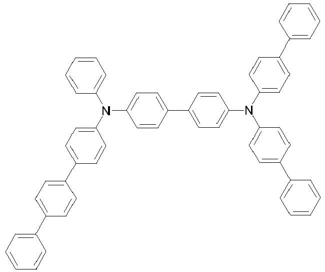 (see also hole-transporting molecules).

### Hole transport layer (c):

Either hole-transporting molecules or polymers may be used as the hole transport material. Suitable hole transport materials for layer (c) of the inventive OLED are disclosed, for example, in Kirk-Othmer Encyclopedia of Chemical Technology, 4th Edition, Vol. 18, pages 837 to 860, 1996, US20070278938, US2008/0106190, US2011/0163302 (triarylamines with (di)benzothiophen/(di)benzofuran; Nan-Xing Hu et al. Synth. Met. 111 (2000) 421 (indolocarbazoles), WO2010002850 (substituted phenylamine compounds) and WO2012/16601 (in particular the hole transport materials mentioned on pages 16 and 17 of WO2012/16601). Combination of different hole transport material may be used. Reference is made, for example, to WO2013/022419, wherein 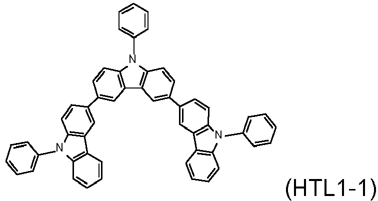 and 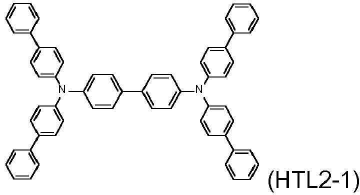 constitute the hole transport layer.

Customarily used hole-transporting molecules are selected from the group consisting of 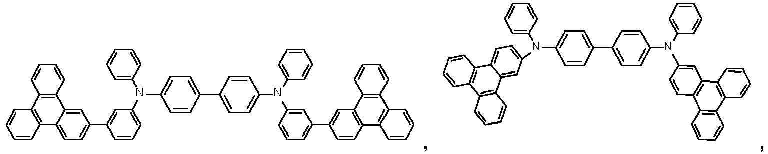 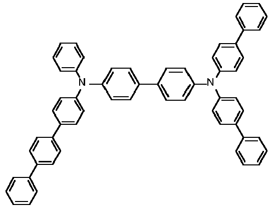 (4-phenyl-N-(4-phenylphenyl)-N-[4-[4-(N-[4-(4-phenyl-phenyl)phenyl]anilino)phenyl]phenyl]aniline), 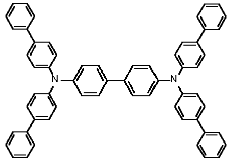 (4-phenyl-N-(4-phenylphenyl)-N-[4-[4-(4-phenyl-N-(4-phenylphenyl)anilino)phenyl]phenyl]aniline), 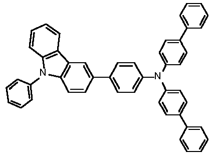 (4-phenyl-N-[4-(9-phenylcarbazol-3-yl)phenyl]-N-(4-phenylphenyl)aniline), 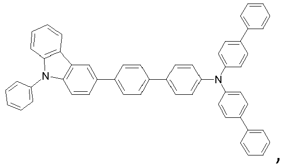 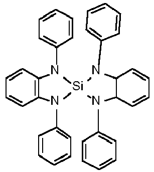 (1,1',3,3'-tetraphenylspiro[1,3,2-benzodiazasilole-2,2'-3a,7a-dihydro-1,3,2-benzodiazasilole]), 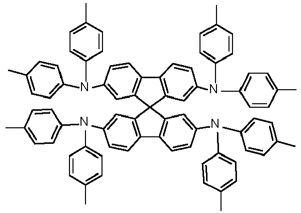 (N2,N2,N2',N2',N7,N7,N7',N7'-octakis(p-tolyl)-9,9'-spirobi[fluorene]-2,2',7,7'-tetramine), 4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)2-methylphenyl](4-methylphenyl)methane (MPMP), 1-phenyl-3-[p-(diethylamino)styryl]5-[p-(diethylamino)phenyl]pyrazoline (PPR or DEASP), 1,2-trans-bis(9H-carbazol9-yl)-cyclobutane (DCZB), N,N,N',N'-tetrakis(4-methylphenyl)-(1,1'-biphenyl)-4,4'-diamine (TTB), fluorine compounds such as 2,2',7,7'-tetra(N,N-di-tolyl)amino9,9-spirobifluorene (spiro-TTB), N,N'-bis(naphthalen-1-yl)-N,N'-bis(phenyl)9,9-spirobifluorene (spiro-NPB) and 9,9-bis(4-(N,N-bis-biphenyl-4-yl-amino)phenyl-9Hfluorene, benzidine compounds such as N,N'-bis(naphthalen-1-yl)-N,N'-bis(phenyl)benzidine and porphyrin compounds such as copper phthalocyanines. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore^{®} OC Conducting Inks commercially available from Plextronics), and copolymers such as poly(3,4-ethylenedioxythiophene)/poly(4-styrenesulfonate) also called PEDOT/PSS. Preferred examples of a material of the hole injecting layer are a porphyrin compound, an aromatic tertiary amine compound, or a styrylamine compound. Particularly preferable examples include an aromatic tertiary amine compound such as hexacyanohexaazatriphenylene (HAT).

The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂, MoO₃, WOₓ, ReO₃ and/or V₂O₅, preferably MoO₃ and/or ReO₃, more preferably MoO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetracyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6Hnaphthalen-2-ylidene)malononitrile (F₆-TNAP), Mo(tfd)₃ (from Kahn et al., J. Am. Chem. Soc. 2009, 131 (35), 12530-12531), compounds as described in EP1988587, US2008265216, EP2180029, US20100102709, WO2010132236, EP2180029 and quinone compounds as mentioned in EP2401254.

### Electron/exciton blocking layer (d):

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer.

Suitable metal complexes for use as electron/exciton blocker material are, for example, carbene complexes as described in WO2005/019373A2, WO2006/056418A2, WO2005/113704, WO2007/115970, WO2007/115981, WO2008/000727 and PCT/EP2014/055520.

### Emitting layer (e)

The light emitting layer is an organic layer having a light emitting function and is formed from one or more layers, wherein one of the layers comprises the host material and the light emitting material as described below.

Preferably, the light emitting layer of the inventive OLED comprises at least one compound of formula (1) as host material.

When the light emitting layer is composed of two or more layers, the light emitting layer or layers other than that mentioned above contains or contain a host material and a dopant material when a doping system is employed. The major function of the host material is to promote the recombination of electrons and holes and confine excitons in the light emitting layer. The dopant material causes the excitons generated by recombination to emit light efficiently.

In case of a phosphorescent device, the major function of the host material is to confine the excitons generated on the dopant in the light emitting layer.

The light emitting layer may be made into a double dopant layer, in which two or more kinds of dopant materials having high quantum yield are combinedly used and each dopant material emits light with its own color. For example, to obtain a yellow emission, a light emitting layer formed by co-depositing a host, a red-emitting dopant and a green-emitting dopant is used.

In a laminate of two or more light emitting layers, electrons and holes are accumulated in the interface between the light emitting layers, and therefore, the recombination region is localized in the interface between the light emitting layers, to improve the quantum efficiency.

The light emitting layer may be different in the hole injection ability and the electron injection ability, and also in the hole transporting ability and the electron transporting ability each being expressed by mobility.

The light emitting layer is formed, for example, by a known method, such as a vapor deposition method, a spin coating method, and LB method. Alternatively, the light emitting layer may be formed by making a solution of a binder, such as resin, and the material for the light emitting layer in a solvent into a thin film by a method such as spin coating.

The light emitting layer is preferably a molecular deposit film. The molecular deposit film is a thin film formed by depositing a vaporized material or a film formed by solidifying a material in the state of solution or liquid. The molecular deposit film can be distinguished from a thin film formed by LB method (molecular build-up film) by the differences in the assembly structures and higher order structures and the functional difference due to the structural differences.

The light-emitting layer (e) comprises at least one emitter material. In principle, it may be a fluorescence or phosphorescence emitter, suitable emitter materials being known to those skilled in the art. The at least one emitter material is preferably a phosphorescence emitter.

The emission wavelength of the phosphorescent dopant used in the light emitting layer is not particularly limited. In a preferred embodiment, at least one of the phosphorescent dopants used in the light emitting layer has the peak of emission wavelength of in general 430 nm or longer and 780 nm or shorter, preferably 490 nm or longer and 700 nm or shorter and more preferably 490 nm or longer and 650 nm or shorter. Most preferred are green emitter materials (490 nm to 570 nm).

The phosphorescent dopant (phosphorescent emitter material) is a compound which emits light by releasing the energy of excited triplet state and preferably a organometallic complex comprising at least one metal selected from Ir, Pt, Pd, Os, Au, Cu, Re, Rh and Ru and a ligand, although not particularly limited thereto as long as emitting light by releasing the energy of excited triplet state. A ligand having an ortho metal bond is preferred. In view of obtaining a high phosphorescent quantum yield and further improving the external quantum efficiency of electroluminescence device, a metal complex comprising a metal selected from Ir, Os, and Pt is preferred, with iridium complex, osmium complex, and platinum, particularly an ortho metallated complex thereof being more preferred, iridium complex and platinum complex being still more preferred, and an ortho metallated iridium complex being particularly preferred.

The compounds of the formula (1) can be used as the matrix in the light-emitting layer.

Suitable metal complexes for use in the inventive OLEDs, preferably as emitter material, are described, for example, in documents WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006121811 A1, WO 2007095118 A2, WO 2007/115970, WO 2007/115981, WO 2008/000727, WO2010129323, WO2010056669, WO10086089, US2011/0057559, WO2011/106344, US2011/0233528, WO2012/048266 and WO2012/172482.

Further suitable metal complexes are the commercially available metal complexes tris(2-phenylpyridine)iridium(III), iridium(III) tris(2-(4-tolyl)pyridinato-N,C^{2'}), bis(2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldi-benzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetyl-acetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable:
tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenyl-phenanthroline)europium(III), tris(dibenzoylmethane)mono(4,7-di-methyl-phenanthroline)europium(III), tris(dibenzoylmethane)mono(4,7-dimethylphenanthrolinedisulfonic acid)europium(III) disodium salt, tris[di(4-(2-(2-ethoxyethoxy)ethoxy)benzoylmethane)]mono-(phenanthroline)europium(III) and tris[di[4-(2-(2-ethoxyethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Particularly suitable metal complexes are described in US2012223295, US2014367667, US2013234119, US2014001446, US2014231794, US2014008633, WO2012108388 and WO2012108389. The emitters mentioned in US2013234119, paragraph [0222], are exemplified. Selected emitters, especially red emitters, of said emitters mentioned in US2013234119, paragraph [0222], are:

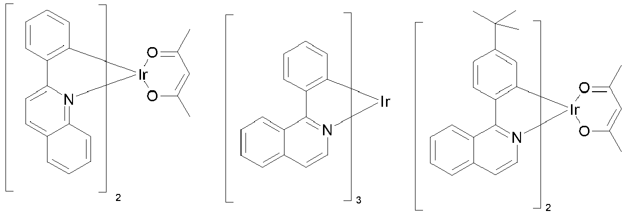

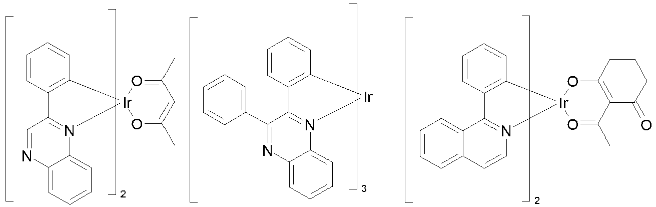

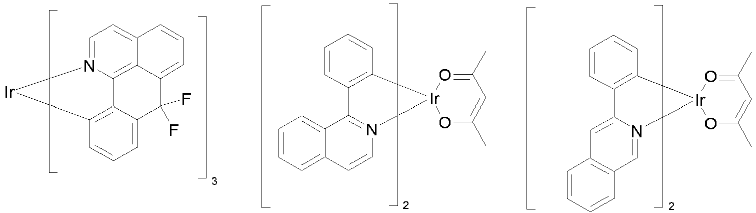

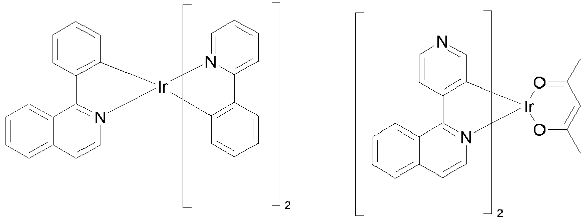

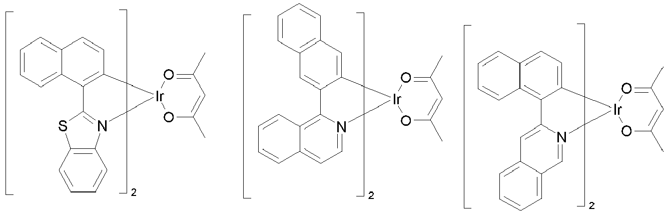

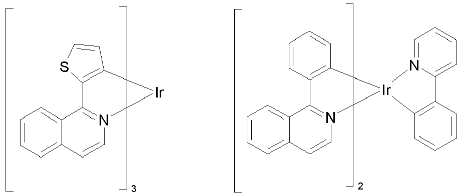

Further suitable Emitters are mentioned in: Mrs Bulletin, 2007, 32, 694:

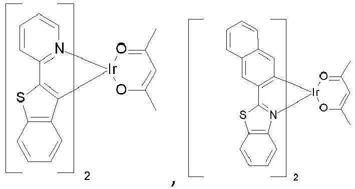

Further suitable Emitters are mentioned in: WO2009100991:

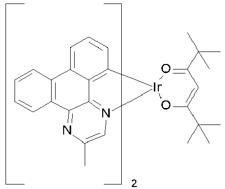

Further suitable Emitters are mentioned in: WO2008101842:

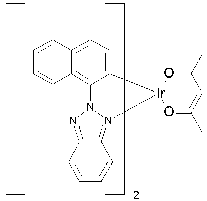

Further suitable Emitters are mentioned in: US 20140048784, especially in paragraph [0159]:

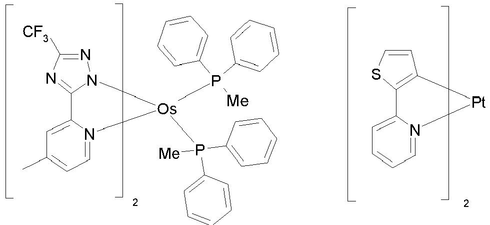

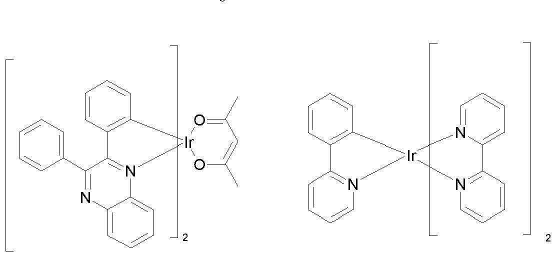

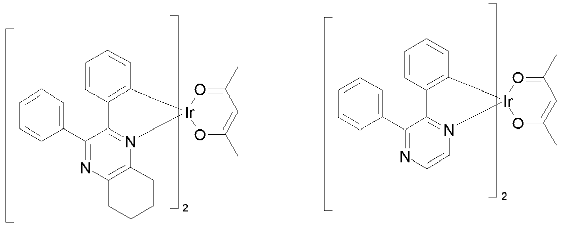

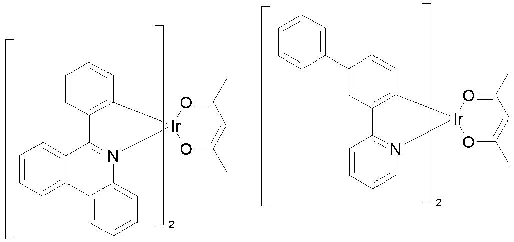

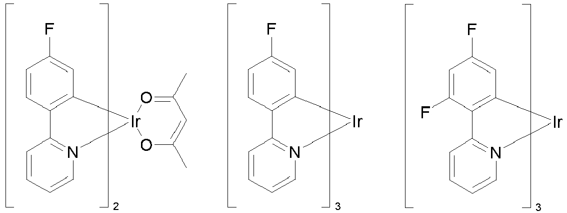

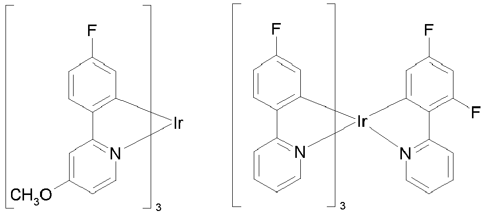

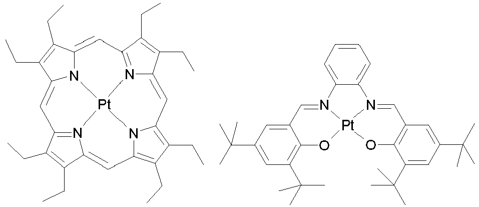

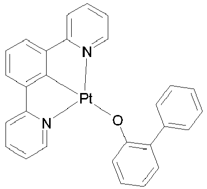

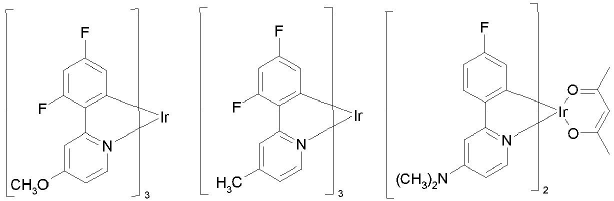

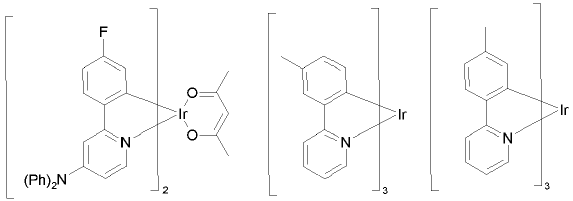

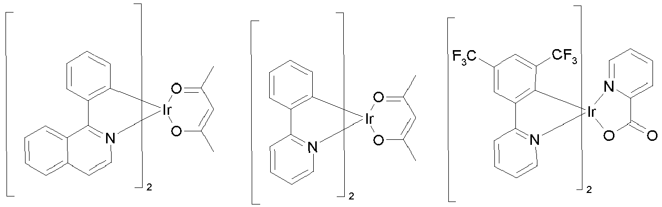

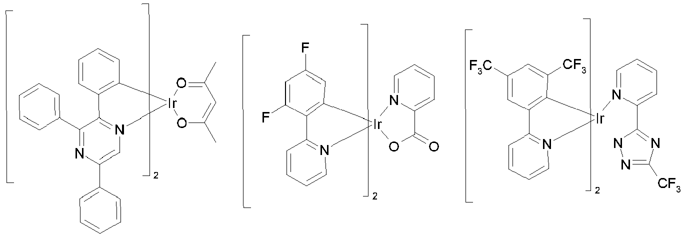

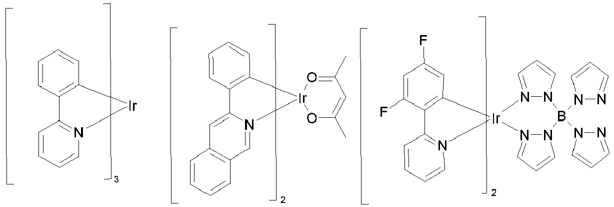

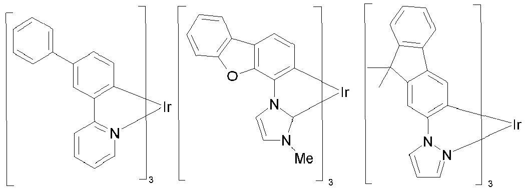

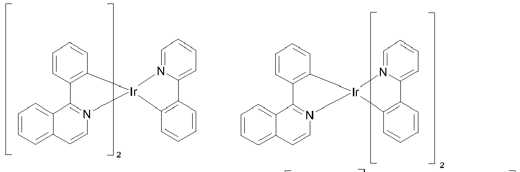

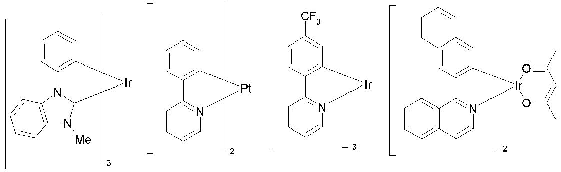

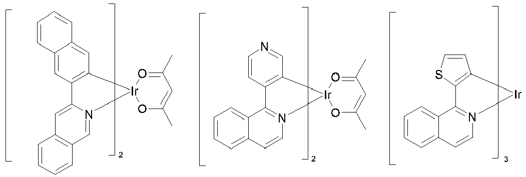

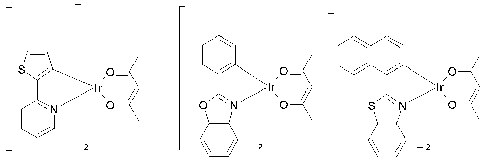

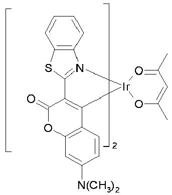

Suitable phosphorescent blue emitters are specified in the following publications: WO2006/056418A2, WO2005/113704, WO2007/115970, WO2007/115981, WO2008/000727, WO2009050281, WO2009050290, WO2011051404, US2011/057559 WO2011/073149, WO2012/121936A2, US2012/0305894A1, WO2012/170571, WO2012/170461, WO2012/170463, WO2006/121811, WO2007/095118, WO2008/156879, WO2008/156879, WO2010/068876, US2011/0057559, WO2011/106344, US2011/0233528, WO2012/048266, WO2012/172482, PCT/EP2014/064054 and PCT/EP2014/066272.

The light emitting layer (e) comprises for example at least one carbene complex as phosphorescence emitter. Suitable carbene complexes are, for example, compounds of the formula 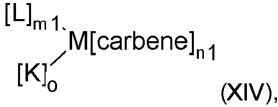 which are described in WO 2005/019373 A2, wherein the symbols have the following meanings:
M is a metal atom selected from the group consisting of Co, Rh, Ir, Nb, Pd, Pt, Fe, Ru, Os, Cr, Mo, W, Mn, Tc, Re, Cu, Ag and Au in any oxidation state possible for the respective metal atom;
carbene is a carbene ligand which may be uncharged or monoanionic and monodentate, bidentate or tridentate, with the carbene ligand also being able to be a biscarbene or triscarbene ligand;
L is a monoanionic or dianionic ligand, which may be monodentate or bidentate;
K is an uncharged monodentate or bidentate ligand, preferably selected from the group consisting of phosphines; phosphonates and derivatives thereof, arsenates and derivatives thereof; phosphites; CO; pyridines; nitriles and conjugated dienes which form a π complex with M¹;
n1 is the number of carbene ligands, where n1 is at least 1 and when n1 > 1 the carbene ligands in the complex of the formula I can be identical or different;
m1 is the number of ligands L, where m1 can be 0 or ≥ 1 and when m1 > 1 the ligands L can be identical or different;
o is the number of ligands K, where o can be 0 or ≥ 1 and when o > 1 the ligands K can be identical or different;
where the sum n1 + m1 + o is dependent on the oxidation state and coordination number of the metal atom and on the denticity of the ligands carbene, L and K and also on the charge on the ligands, carbene and L, with the proviso that n1 is at least 1.

More preferred are metal-carbene complexes of the general formula 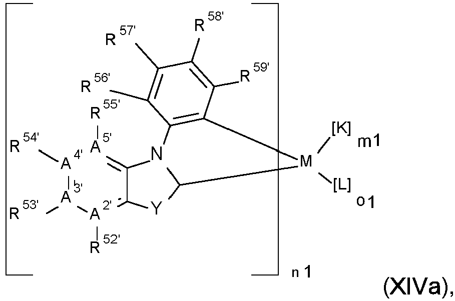 which are described in WO2011/073149, where M is Ir, or Pt,
n1 is an integer selected from 1, 2 and 3,
Y is NR^{51'}, O, S or C(R^{25'})₂,
A^{2'}, A^{3'}, A^{4'}, and A^{5'}are each independently N or C, where 2 A' = nitrogen atoms and at least one carbon atom is present between two nitrogen atoms in the ring,
R^{51'} is a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
R^{52'}, R^{53'}, R^{54'} and R^{55'}are each, if A^{2'}, A^{3'}, A^{4'} and/or A^{5'} is N, a free electron pair, or, if A^{2'}, A^{3'}, A^{4'} and/or A^{5'} is C, each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R^{53'} and R^{54'} together with A^{3'} and A^{4'} form an optionally substituted, unsaturated ring optionally interrupted by at least one further heteroatom and having a total of 5 to 18 carbon atoms and/or heteroatoms,
R^{56'}, R^{57'}, R^{58'} and R^{59'} are each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R^{56'} and R^{57'}, R^{57'} and R^{58'} or R^{58'} and R^{59'}, together with the carbon atoms to which they are bonded, form a saturated, unsaturated or aromatic, optionally substituted ring optionally interrupted by at least one heteroatom and having a total of 5 to 18 carbon atoms and/or heteroatoms, and/or
if A^{5'} is C, R^{55'} and R^{56'} together form a saturated or unsaturated, linear or branched bridge optionally comprising heteroatoms, an aromatic unit, heteroaromatic unit and/or functional groups and having a total of 1 to 30 carbon atoms and/or heteroatoms, to which is optionally fused a substituted or unsubstituted, five- to eight-membered ring comprising carbon atoms and/or heteroatoms,
R^{25'} is independently a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
K is an uncharged mono- or bidentate ligand,
L is a mono- or dianionic ligand, preferably monoanionic ligand, which may be mono- or bidentate,
m1 is 0, 1 or 2, where, when m1 is 2, the K ligands may be the same or different,
o1 is 0, 1 or 2, where, when o1 is 2, the L ligands may be the same or different.

The compound of formula XIV is preferably a compound of the formula:

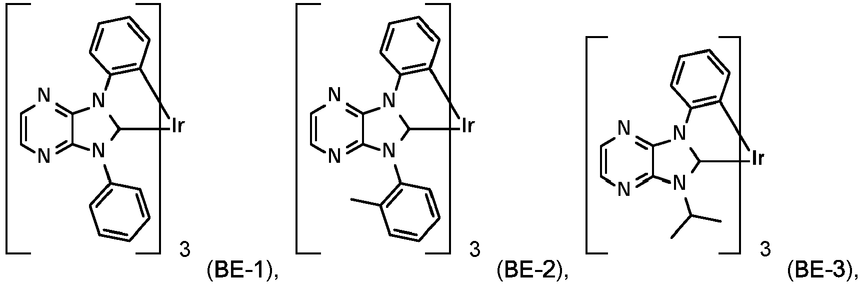

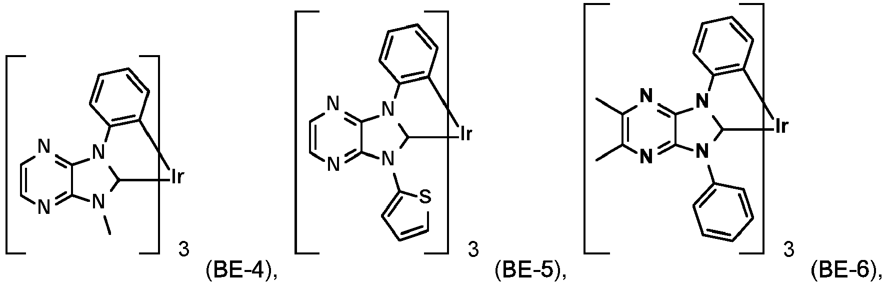

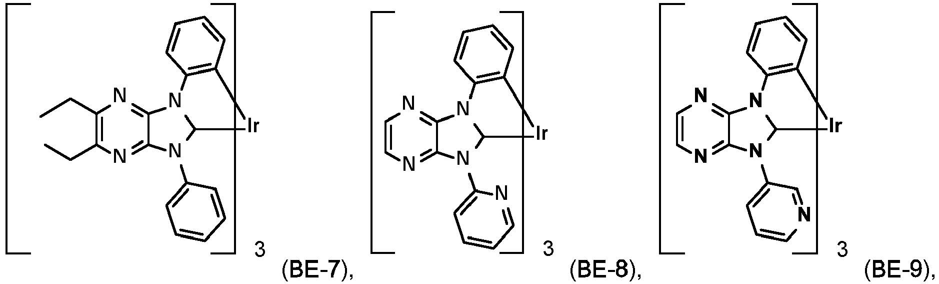

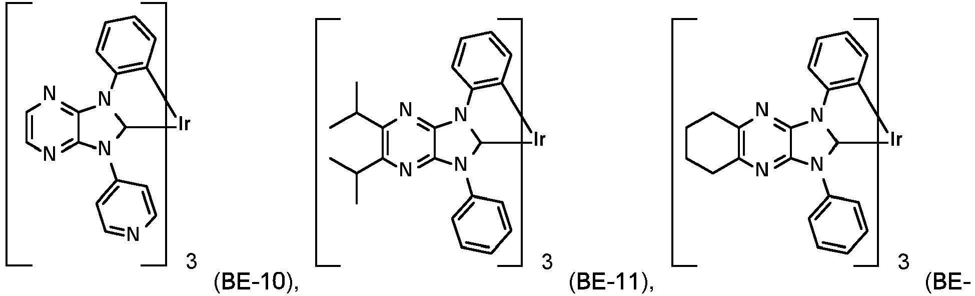

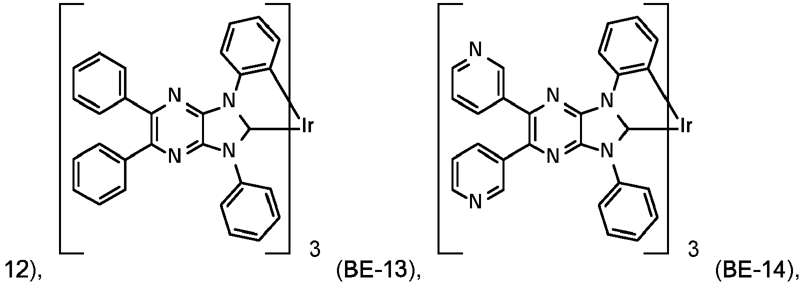

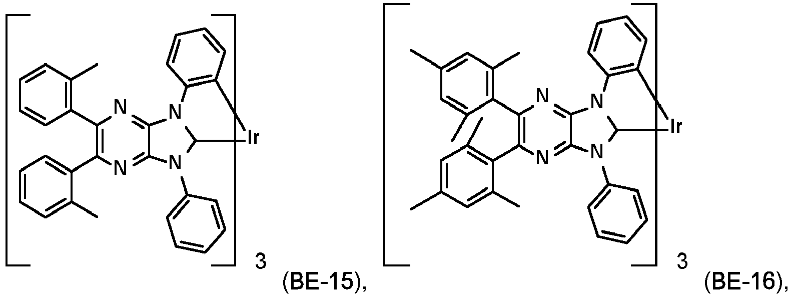

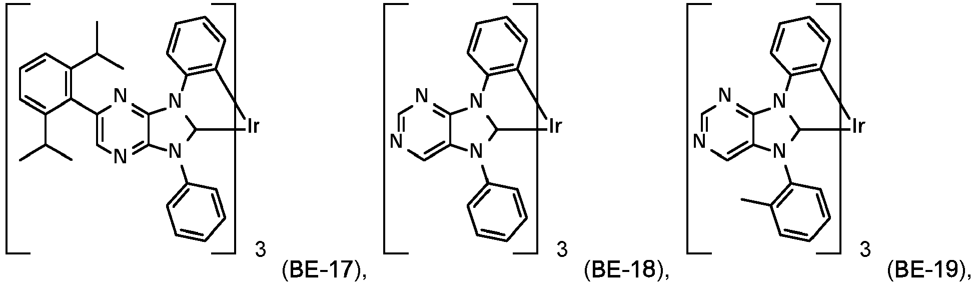

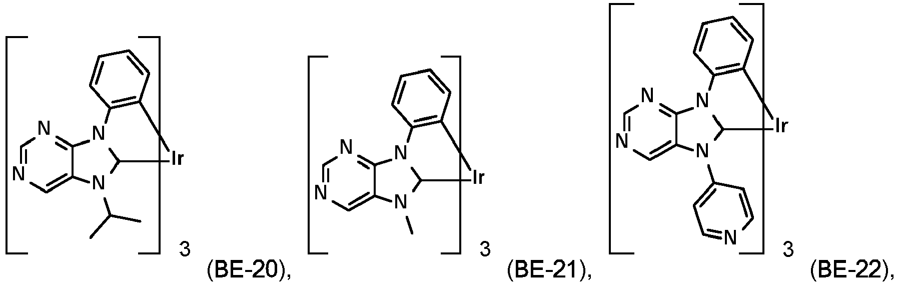

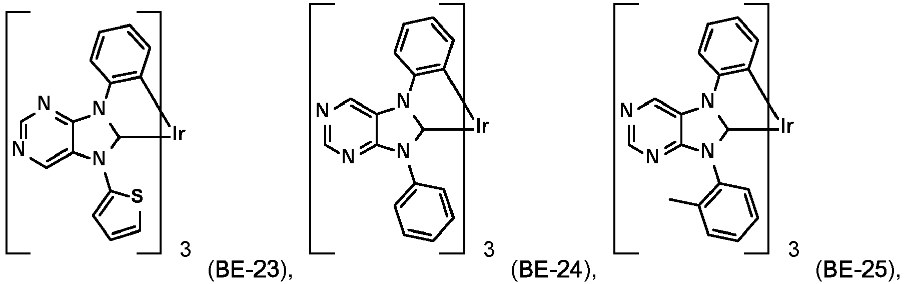

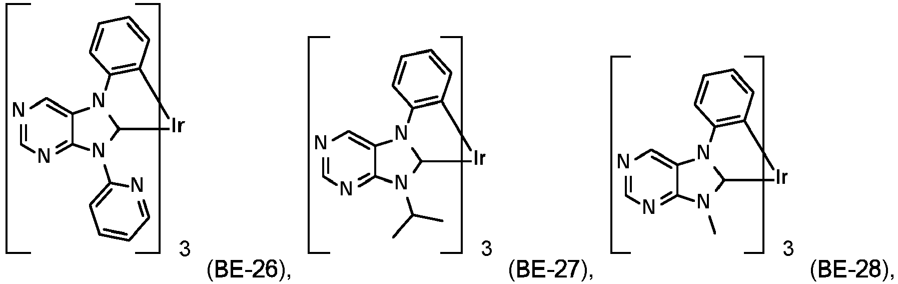

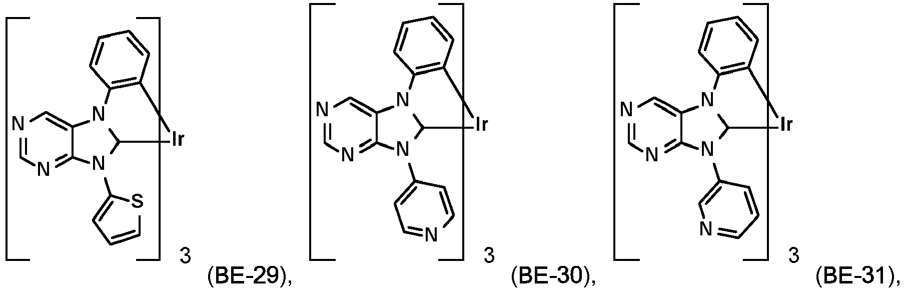

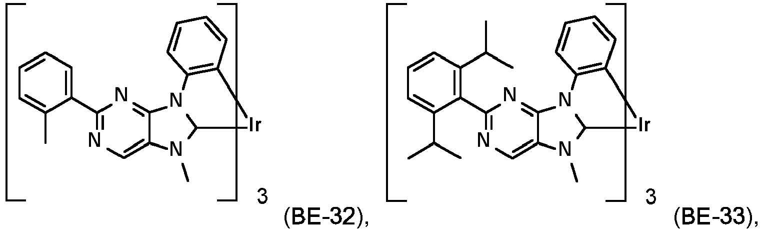

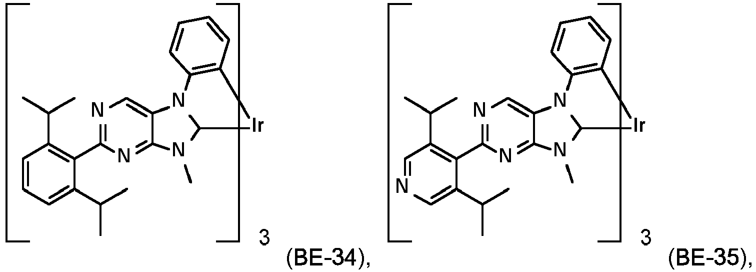

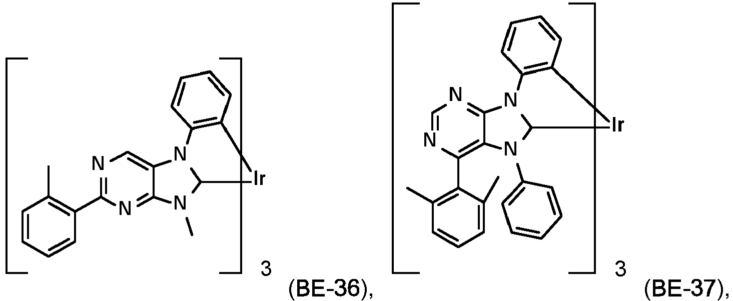

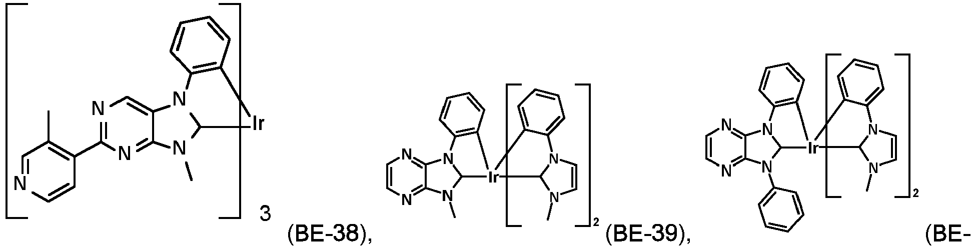

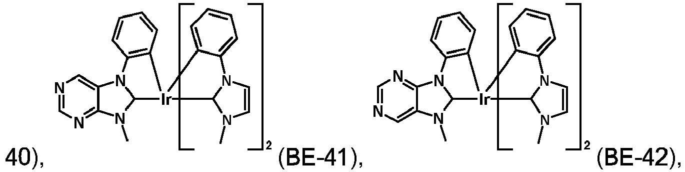

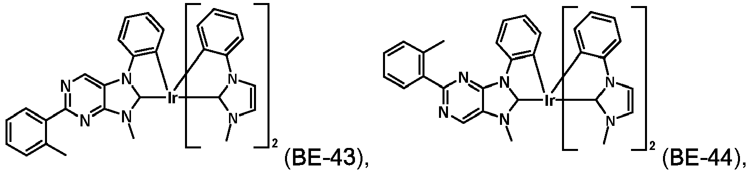

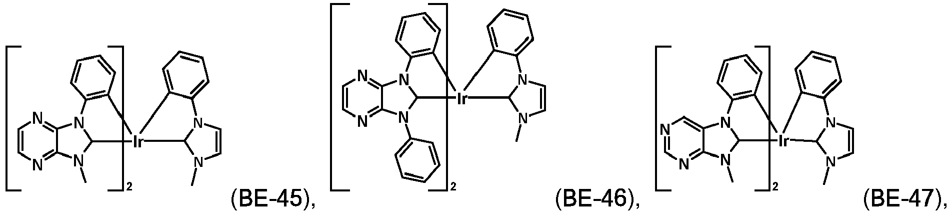

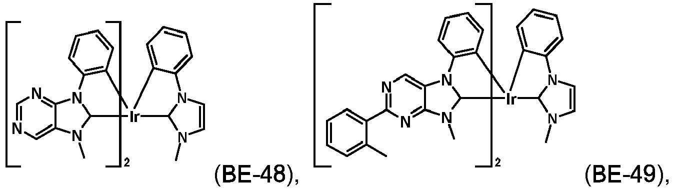

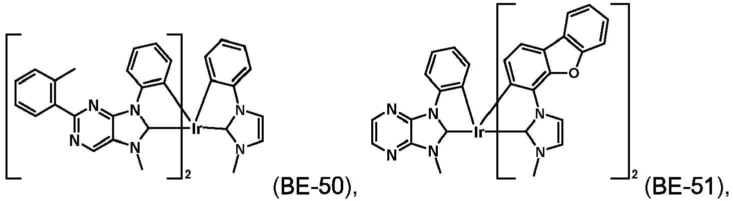

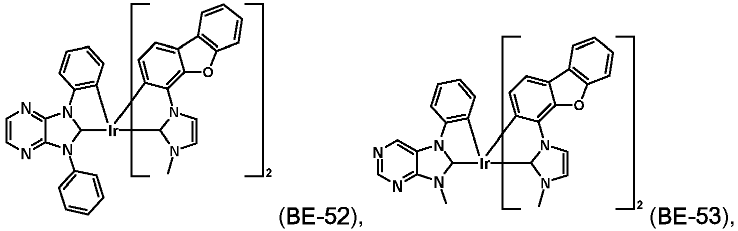

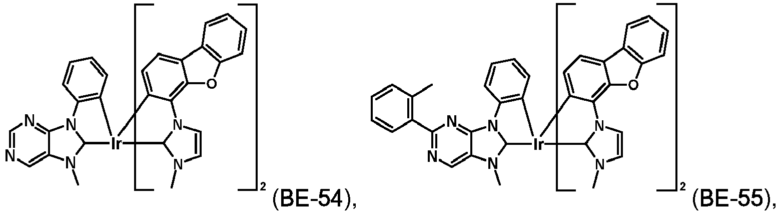

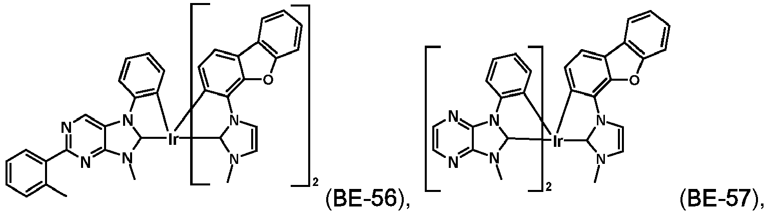

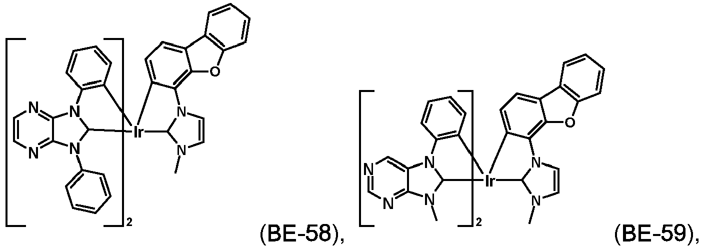

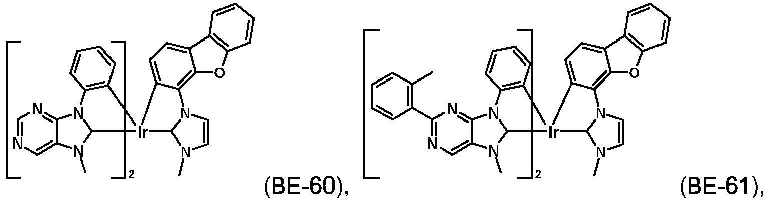

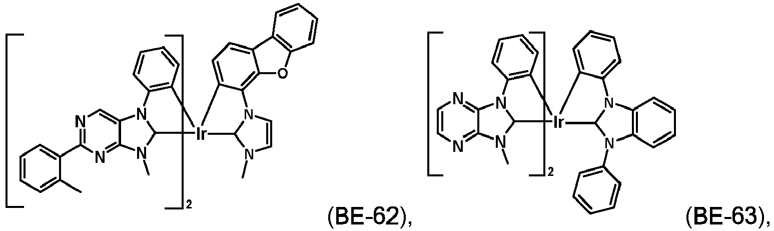

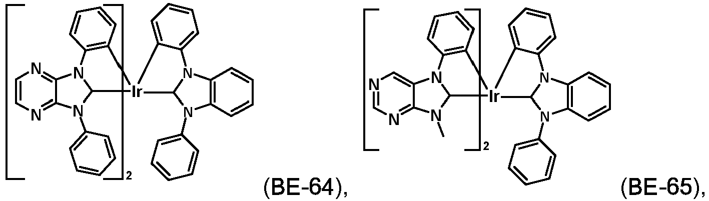

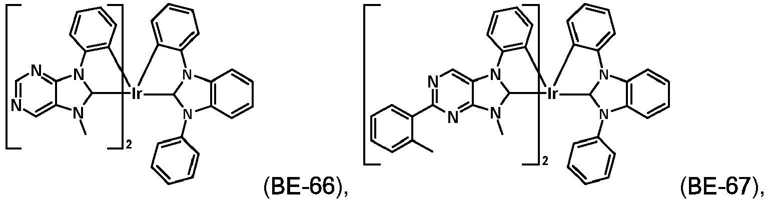

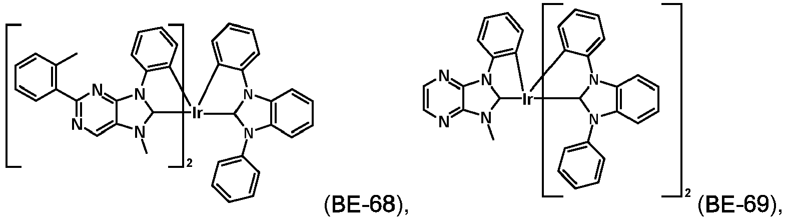

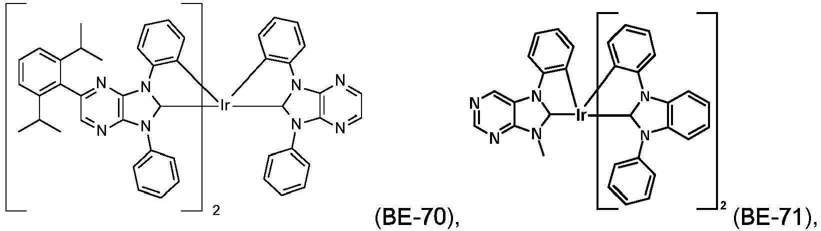

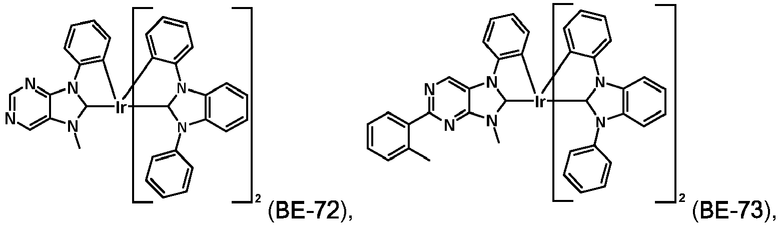

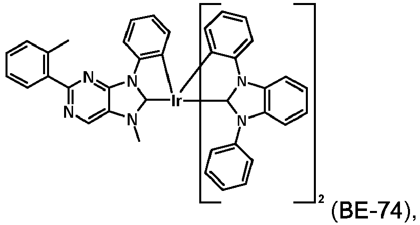

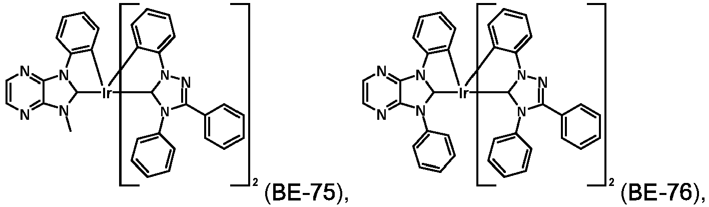

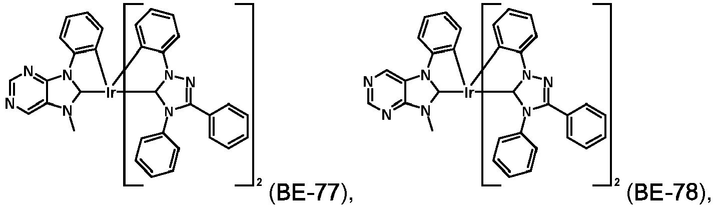

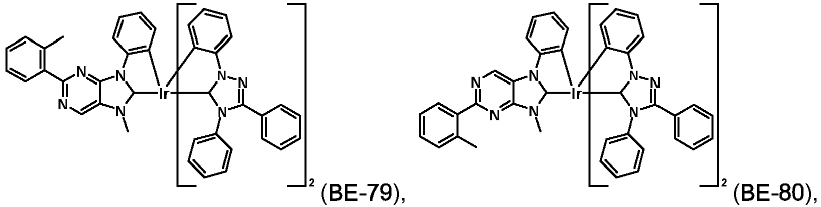

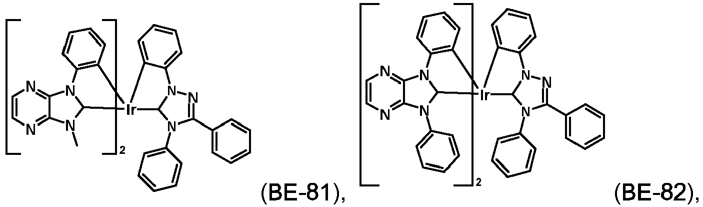

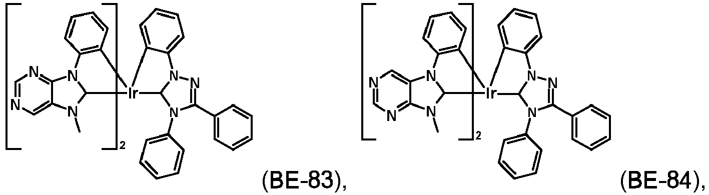

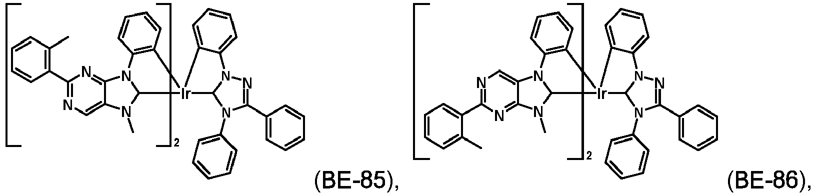

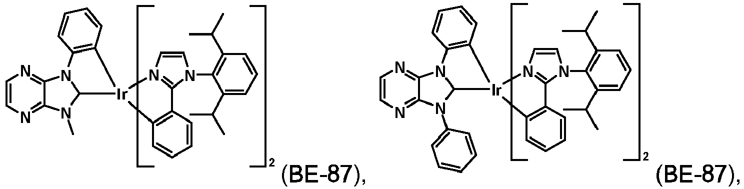

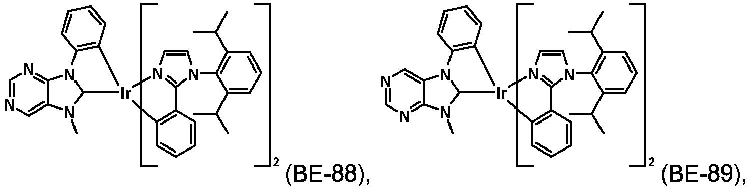

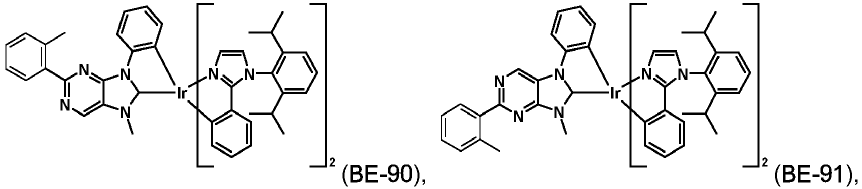

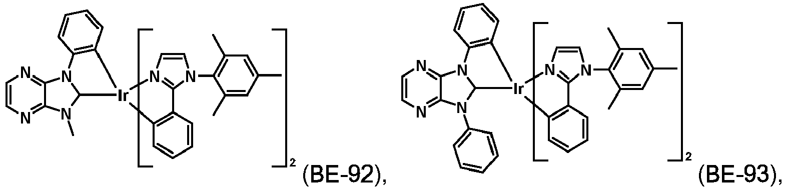

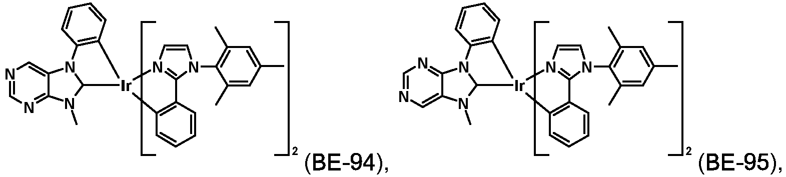

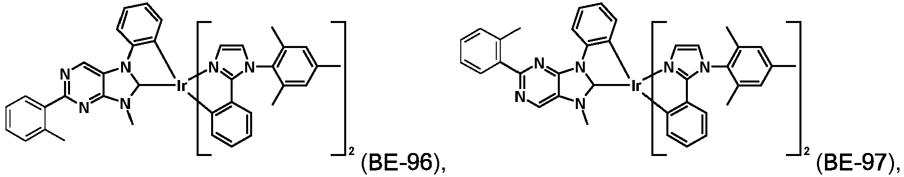

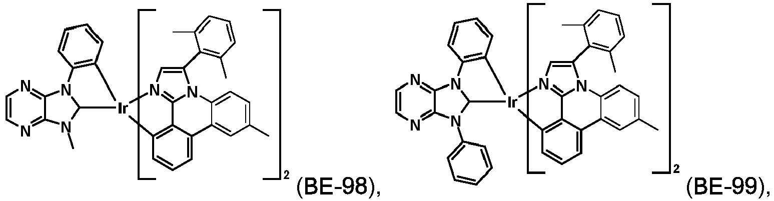

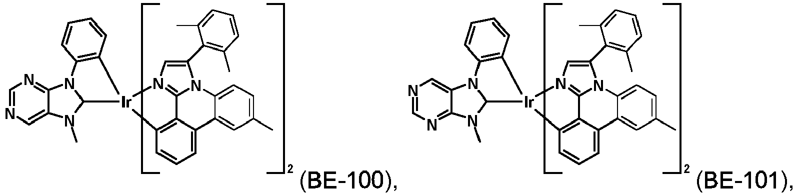

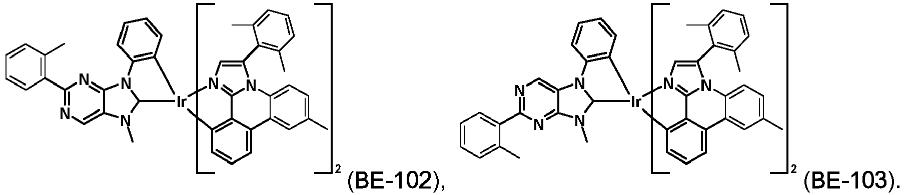

Further suitable non-carbene emitter materials are mentioned below:

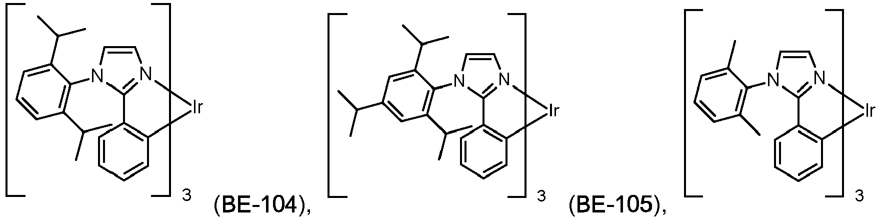

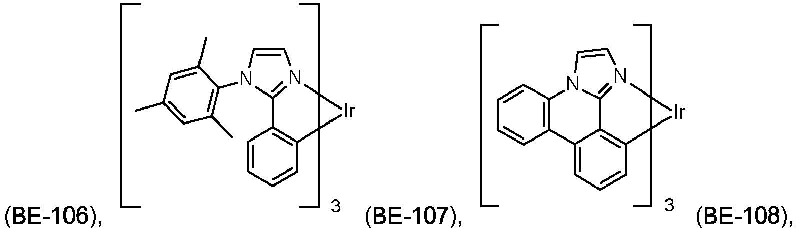

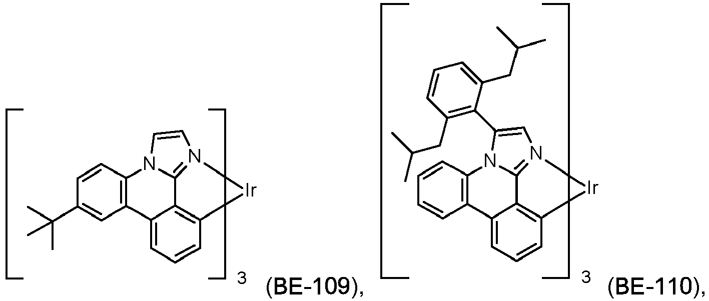

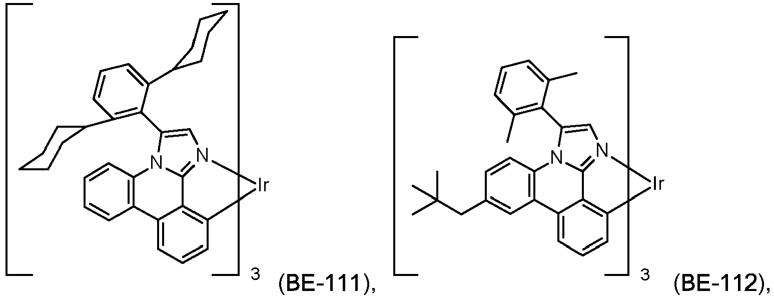

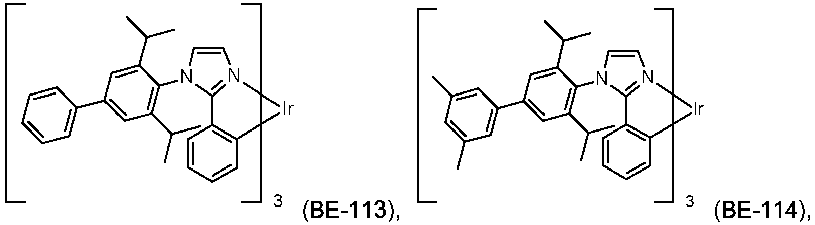

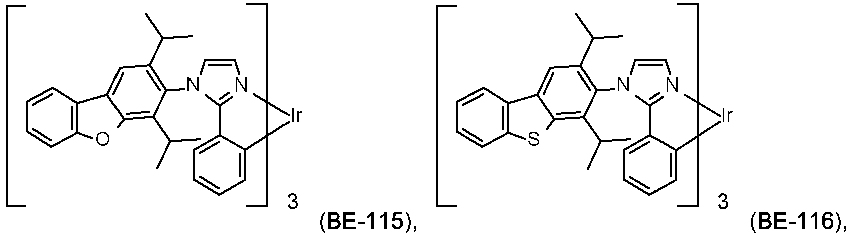

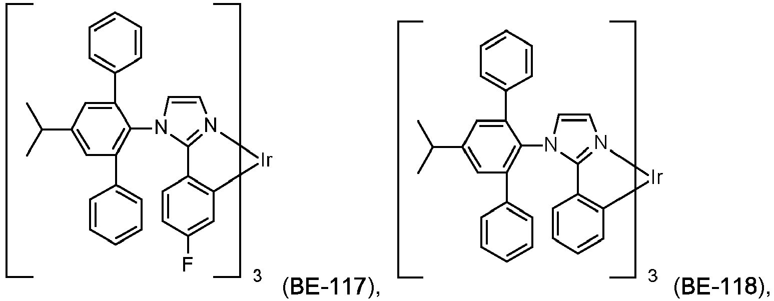

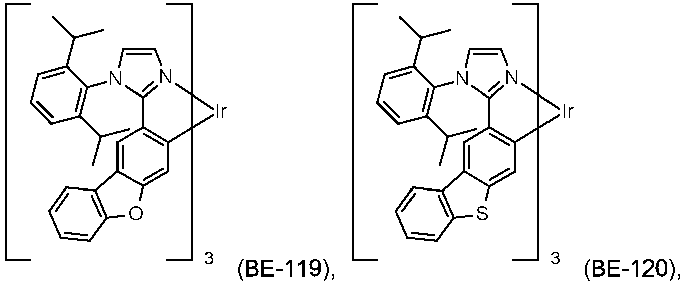

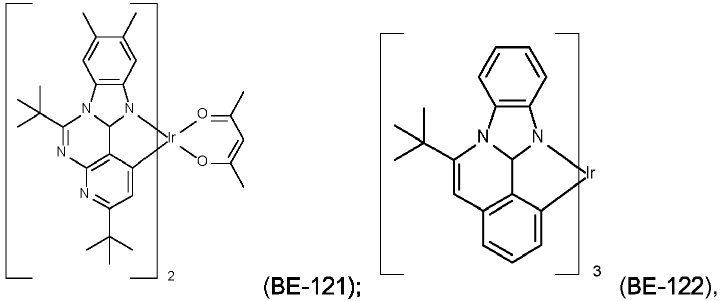

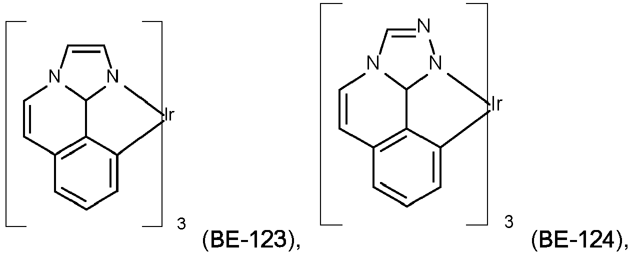

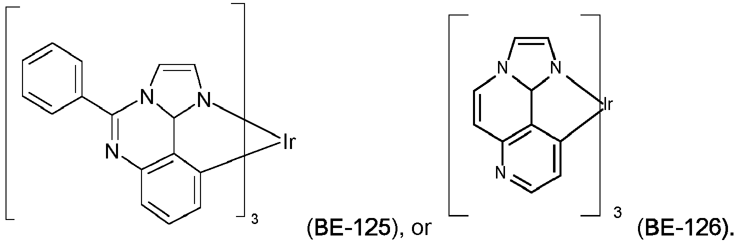

The compound of formula **XIV** is more preferably a compound **(BE-1), (BE-2), (BE-7), (BE-12), (BE-16), (BE-64),** or **(BE-70).** The most preferred phosphorescent blue emitters are compounds (**BE-1**) and **(BE-12).**

The homoleptic metal-carbene complexes may be present in the form of facial or meridional isomers or mixtures thereof, preference being given to the facial isomers.

Suitable carbene complexes of formula (XIV) and their preparation process are, for example, described in WO2011/073149.

The compounds of formula (1) the present invention can also be used as host for phosphorescent green emitters. Suitable phosphorescent green emitters are, for example, specified in the following publications: WO2006014599, WO20080220265, WO2009073245, WO2010027583, WO2010028151, US20110227049, WO2011090535, WO2012/08881, WO20100056669, WO20100118029, WO20100244004, WO2011109042, WO2012166608, US20120292600, EP2551933A1; US6687266, US20070190359, US20070190359, US20060008670; WO2006098460, US20110210316, WO2012053627; US6921915, US20090039776; JP2007123392 and European patent application no. 14180422.9.

Examples of suitable phosphorescent green emitters are shown below:

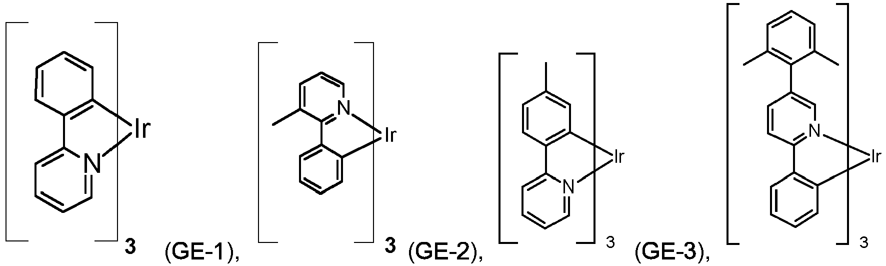

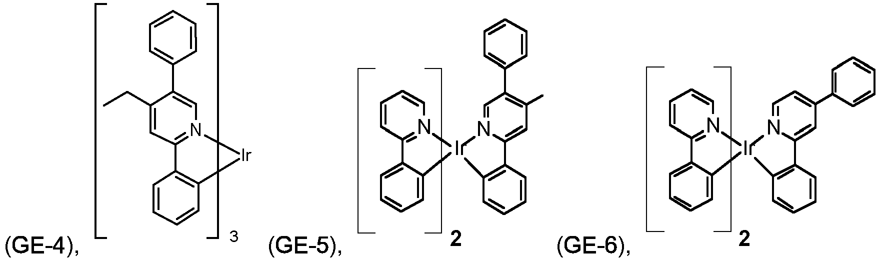

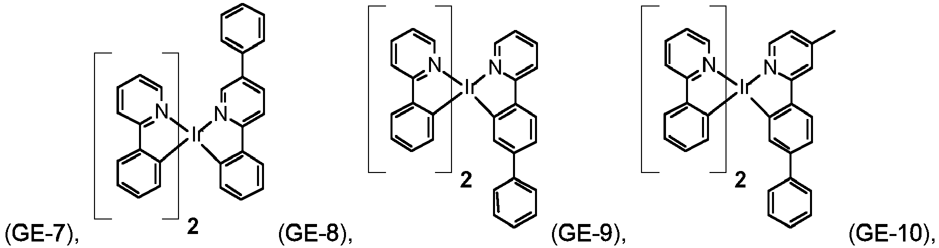

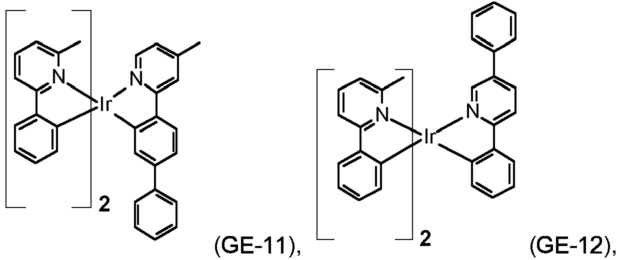

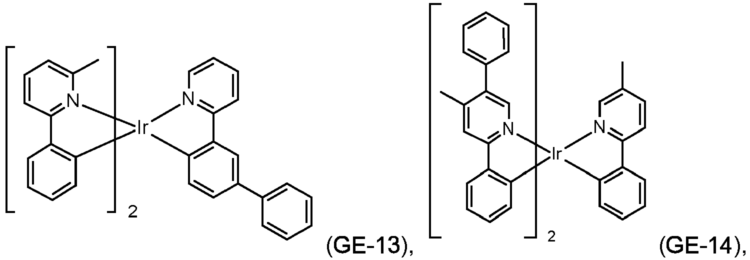

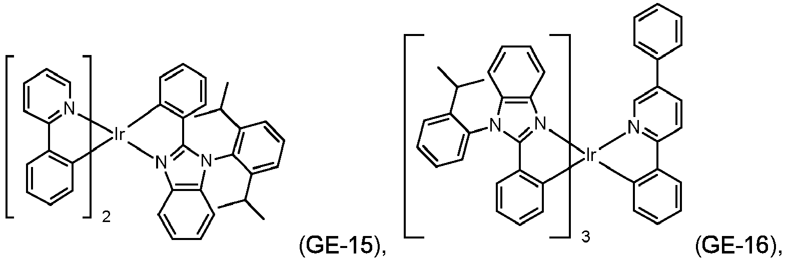

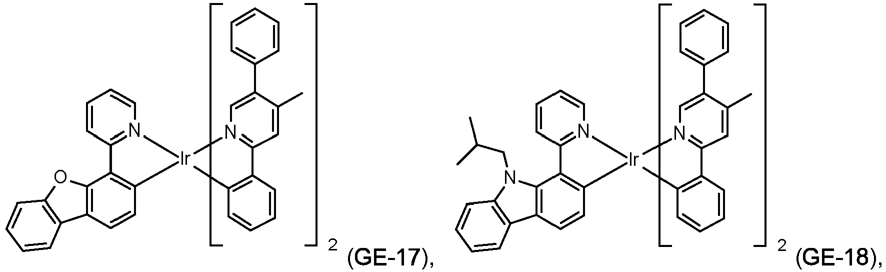

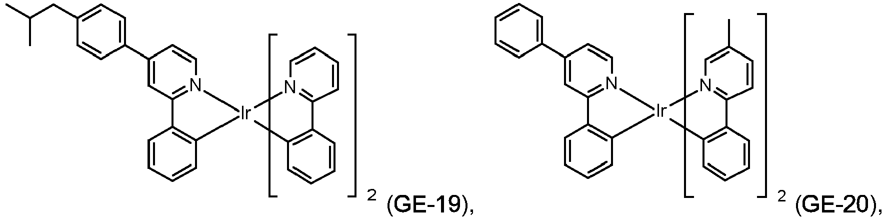

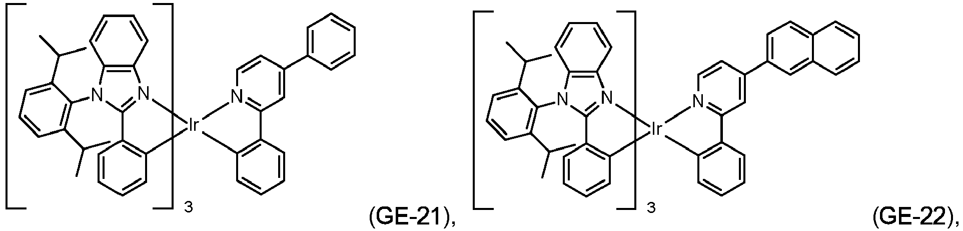

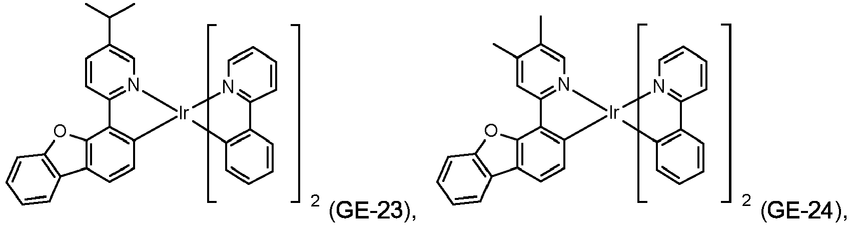

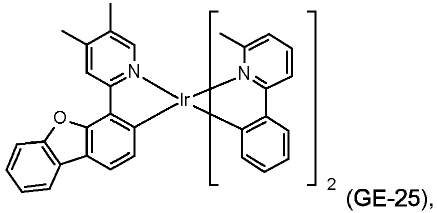

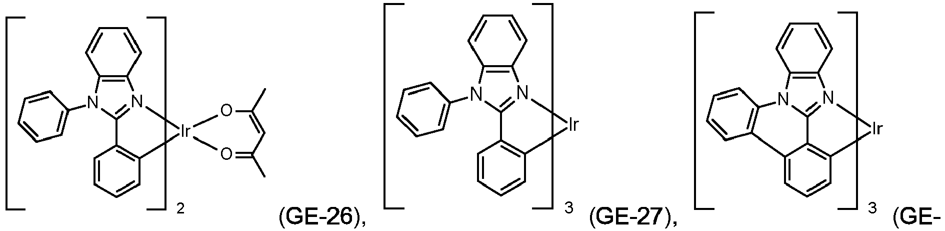

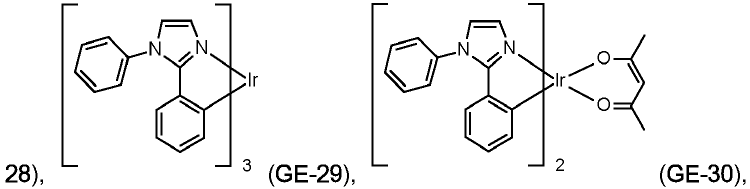

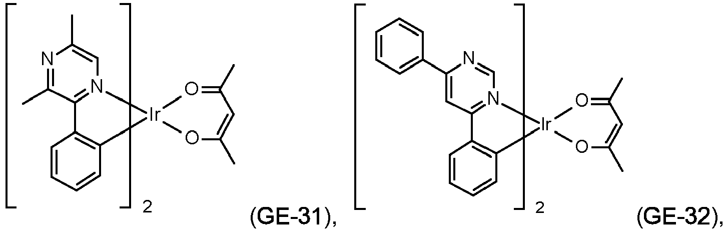

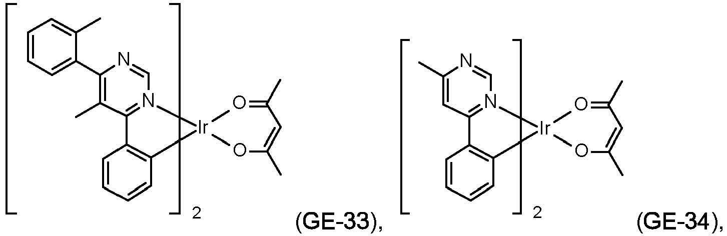

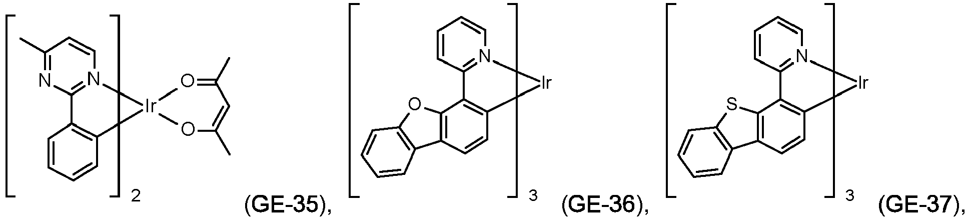

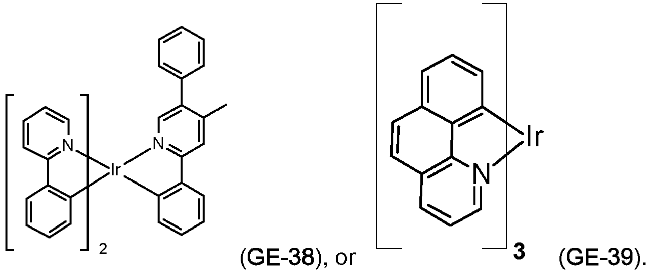

The emitter materials (dopants), preferably the phosphorescent emitter materials, may be used alone or in combination of two or more.

The content of the emitter materials (dopants), preferably the phosphorescent emitter materials, in the light emitting layer is not particularly limited and selected according to the use of the device, and preferably 0.1 to 70% by mass, and more preferably 1 to 30% by mass. If being 0.1% by mass or more, the amount of light emission is sufficient. If being 70% by mass or less, the concentration quenching can be avoided. The further component in the emitting layer is usually one or more host material, which is preferably present in an amount of 30 to 99.9 % by mass, more preferably 70 to 99% by mass, wherein the sum of the emitter material(s) and the host material(s) is 100% by mass.

### Host (matrix) materials

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA.

In the case that one or more phosphorescent emitter materials are used in the light emitting layer, one or more phosphorescent hosts are employed as host material. The phosphorescent host is a compound which confines the triplet energy of the phosphorescent dopant efficiently in the light emitting layer to cause the phosphorescent dopant to emit light efficiently.

In a preferred embodiment, the light-emitting layer is formed of at least one emitter material and of at least one of the matrix materials mentioned below- in one embodiment at least one compound of the formula (1) is used as matrix (host) material. In one embodiment, the light-emitting layer comprises at least one emitter material and at least two matrix materials, wherein one of the matrix materials is a compound of the formula (1) and the other matrix material(s) is/are used as co-host(s). Suitable other host materials than the compound of formula (1) (co-hosts) are mentioned below.

The compounds of the formula (1) are suitable as single host material as well as host material, together with one or more further host materials (co-host). Suitable further host materials are mentioned below. "Further host materials" means in the sense of the present application, host materials different from the compounds of formula (1). However, it is also possible to use two or more different compounds of formula (1) as host material in the light-emitting layer in an OLED of the present application.

In another preferred embodiment of the present invention, at least one compound of the formula (1) is used as host material. Examples of preferred compounds of formula (1) useful as host material are shown above.

In a more preferred embodiment, the light-emitting layer is formed from 0.1 to 70% by weight, preferably 1 to 30% by weight, of at least one of the aforementioned emitter materials and 30 to 99.9% by weight, preferably 70 to 99% by weight, of at least one of the matrix materials mentioned in the specification - in one embodiment at least one compound of the formula (1) - where the sum total of the emitter material and of the matrix material adds up to 100% by weight.

In a further more preferred embodiment, the light-emitting layer comprises a compound of formula (1) as matrix material, one further matrix material (co-host) and at least one emitter material. In said embodiment, the light-emitting layer is formed from 0.1 to 70% by weight, preferably 1 to 30% by weight, of the at least one emitter material and 30 to 99.9% by weight, preferably 70 to 99% by weight, of a compound of the formula (1) and the further matrix material, where the sum total of the at least one emitter material, the further matrix material and of the compound of formula (1) adds up to 100% by weight.

The content ratio of the compound of the formula (1) as first host material and the further matrix material as co-host in the light emitting layer is not particularly limited and may be selected accordingly, and the ratio of first host material:second host material (co-host) is preferably 1:99 to 99:1, more preferably 10:90 to 90:10, each based on mass.

Further suitable host materials, which may be small molecules or (co)polymers of the small molecules mentioned, are specified in the following publications: WO2007108459 (H-1 to H-37), preferably H-20 to H-22 and H-32 to H-37, most preferably H-20, H-32, H-36, H-37, WO2008035571 A1 (Host 1 to Host 6), JP2010135467 (compounds 1 to 46 and Host-1 to Host-39 and Host-43), WO2009008100 compounds No.1 to No.67, preferably No.3, No.4, No.7 to No. 12, No.55, No.59, No. 63 to No.67, more preferably No. 4, No. 8 to No. 12, No. 55, No. 59, No.64, No.65, and No. 67, WO2009008099 compounds No. 1 to No. 110, WO2008140114 compounds 1-1 to 1-50, WO2008090912 compounds OC-7 to OC-36 and the polymers of Mo-42 to Mo-51, JP2008084913 H-1 to H-70, WO2007077810 compounds 1 to 44, preferably 1, 2, 4-6, 8, 19-22, 26, 28-30, 32, 36, 39-44, WO201001830 the polymers of monomers 1-1 to 1-9, preferably of 1-3, 1-7, and 1-9, WO2008029729 the (polymers of) compounds 1-1 to 1-36, WO20100443342 HS-1 to HS-101 and BH-1 to BH-17, preferably BH-1 to BH-17, JP2009182298 the (co)polymers based on the monomers 1 to 75, JP2009170764, JP2009135183 the (co)polymers based on the monomers 1-14, WO2009063757 preferably the (co)polymers based on the monomers 1-1 to 1-26, WO2008146838 the compounds a-1 to a-43 and 1-1 to 1-46, JP2008207520 the (co)polymers based on the monomers 1-1 to 1-26, JP2008066569 the (co)polymers based on the monomers 1-1 to 1-16, WO2008029652 the (co)polymers based on the monomers 1-1 to 1-52, WO2007114244 the (co)polymers based on the monomers 1-1 to 1-18, JP2010040830 the compounds HA-1 to HA-20, HB-1 to HB-16, HC-1 to HC-23 and the (co)polymers based on the monomers HD-1 to HD-12, JP2009021336, WO2010090077 the compounds 1 to 55, WO2010079678 the compounds H1 to H42, WO2010067746, WO2010044342 the compounds HS-1 to HS-101 and Poly-1 to Poly-4, JP2010114180 the compounds PH-1 to PH-36, US2009284138 the compounds 1 to 111 and H1 to H71, WO2008072596 the compounds 1 to 45, JP2010021336 the compounds H-1 to H-38, preferably H-1, WO2010004877 the compounds H-1 to H-60, JP2009267255 the compounds 1-1 to 1-105, \WO2009104488 the compounds 1-1 to 1-38, WO2009086028, US2009153034, US2009134784, WO2009084413 the compounds 2-1 to 2-56, JP2009114369 the compounds 2-1 to 2-40, JP2009114370 the compounds 1 to 67, WO2009060742 the compounds 2-1 to 2-56, WO2009060757 the compounds 1-1 to 1-76, WO2009060780 the compounds 1-1 to 1-70, WO2009060779 the compounds 1-1 to 1-42, WO2008156105 the compounds 1 to 54, JP2009059767 the compounds 1 to 20, JP2008074939 the compounds 1 to 256, JP2008021687 the compounds 1 to 50, WO2007119816 the compounds 1 to 37, WO2010087222 the compounds H-1 to H-31, WO2010095564 the compounds HOST-1 to HOST-61, WO2007108362, WO2009003898, WO2009003919, WO2010040777, US2007224446, WO06128800, WO2012014621, WO2012105310, WO2012/130709 and European patent applications EP12175635.7, EP12185230.5 and EP12191408.9 (in particular page 25 to 29 of EP12191408.9).

The above-mentioned small molecules are more preferred than the above-mentioned (co)polymers of the small molecules.

Further suitable host materials, are described in WO2011137072 (for example, 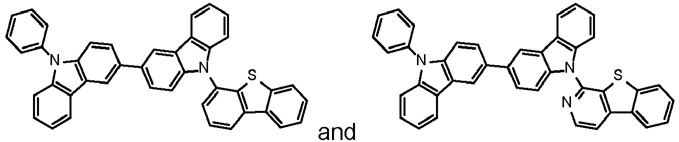 ; best results are achieved if said compounds are combined with 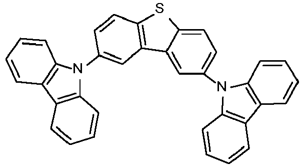 ); WO2012048266 (for example, 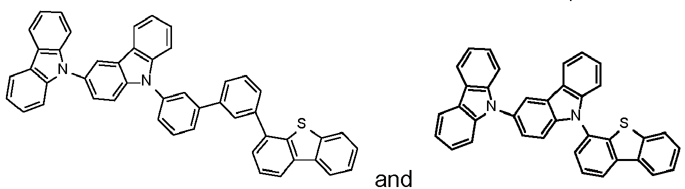 ).

The host materials mentioned above may be used in the OLED of the present invention a alone or in combination with the compound of formula (1) as host material. In this case, the compound of formula (1) is the host and the host materials mentioned above are the co-hosts.

Further examples of the compounds which are suitable as phosphorescent host, alone or in combination with the compound of formula (1) as host material, include a carbazole derivative, a triazole derivative, a oxazole derivative, an oxadiazole derivative, an imidazole derivative, a polyarylalkane derivative, a pyrazoline derivative, a pyrazolone derivative, a phenylenediamine derivative, an arylamine derivative, an amino-substituted chalcone derivative, a styrylanthracene derivative, a fluorenone derivative, a hydrazone derivative, a stilbene derivative, a silazane derivative, an aromatic tertiary amine compound, a styrylamine compound, an aromatic methylidene compound, a porphyrin compound, an anthraquinodimethane derivative, an anthrone derivative, a diphenylquinone derivative, a thiopyran dioxide derivative, a carbodiimide derivative, a fluorenylidenemethane derivative, a distyrylpyrazine derivative, a tetracarboxylic anhydride of fused ring such as naphthalene and perylene, a phthalocyanine derivative, a metal complex of 8-quinolinol derivative, metal phthalocyanine, metal complexes having a ligand such as benzoxazole and benzothiazole, an electroconductive oligomer, such as a polysilane compound, a poly(N-vinylcarbazole) derivative, an aniline copolymer, thiophene oligomer, and a polythiophene, and a polymer such as a polythiophene derivative, a polyphenylene derivative, a polyphenylenevinylene derivative, and a polyfluorene derivative. These phosphorescent hosts may be used alone or in combination of two or more. Specific examples thereof are shown below:

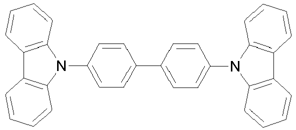

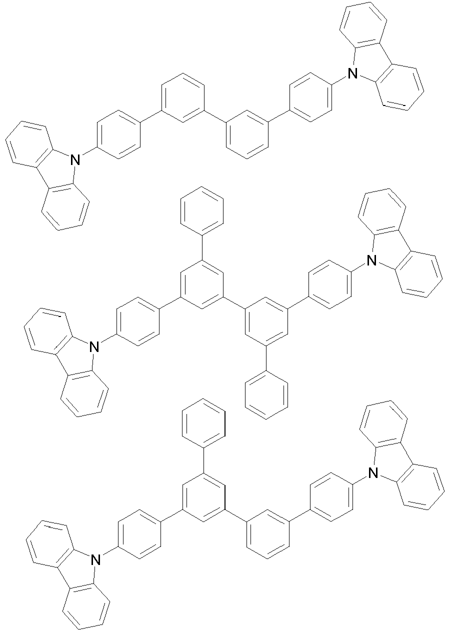

Further suitable hosts, which are especially useful as co-host together with at least one compound of formula (1) are the hosts described in US2012223295, US2014367667, US2013234119, US2014001446, US2014231794, US2014008633, WO2012108388, WO2014009317 and WO2012108389, as well as the compounds of formula (1) described in the EP application filed at the same day as the present application, i.e. October 1, 2015, with the title "Benzimidazolo[1,2-a]benzimidazole carrying benzofurane or benzothiophene groups for Organic Light Emitting Diodes".

Especially preferred are the second host materials mentioned in US2013234119 and the compounds of formula (1) described in the EP application filed at the same day as the present application , i.e. October 1, 2015, with the title "Benzimidazolo[1,2-a]benzimidazole carrying benzofurane or benzothiophene groups for Organic Light Emitting Diodes".

The first host material mentioned in US2013234119 which is preferably used as co-host together with at least one compound of formula (1) in the light emitting layer of an OLED according to the present invention is represented by formula (A). The lifetime of an OLED is increased by combinedly using as a first host material at least one compound of formula (1) and as co-host the host material represented by formula (A) in the light emitting layer. 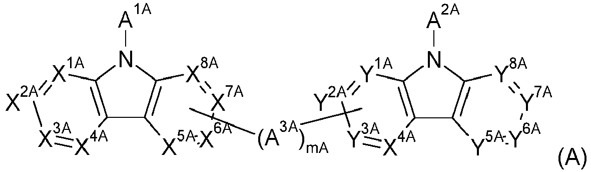 wherein
each of A^{1A} and A^{2A} independently represents an aryl group having 6 to 30 ring carbon atoms, which may be unsubstituted or substituted; or a heterocyclic group having 5 to 30 ring atoms, which may be unsubstituted or substituted;
A^{3A} represents a divalent aryl group having 6 to 30 ring carbon atoms, which may be unsubstituted or substituted; or a divalent heterocyclic group having 5 to 30 ring atoms, which may be unsubstituted or substituted;
mA represents an integer of 0 to 3;
each of X^{1A} to X^{8A} and Y^{1A} to Y^{8A} independently represents N or CR^{a};
each of R^{a} independently represents a hydrogen atom, an aryl group having 6 to 30 ring carbon atoms, which may be unsubstituted or substituted; a heterocyclic group having 5 to 30 ring atoms, which may be unsubstituted or substituted; an alkyl group having 1 to 30 carbon atoms, which may be unsubstituted or substituted for example by E; a silyl group, which may be unsubstituted or substituted; a halogen atom, or a cyano group, provided that when two or more R^{a} groups exist, the R^{a} groups may be the same or different and one of X^{5A} to X^{8A} and one of Y^{1A} to Y^{4A} are bonded to each other via A^{3A}; and
the formula (A) satisfies at least one of the flowing requirements (i) to (v);
   (i) at least one of A^{1A} and A^{2A} represents a cyano-substituted aromatic hydrocarbon group having 6 to 30 ring carbon atoms or a cyano-substituted heterocyclic group having 5 to 30 ring atoms;
   (ii) at least one of X^{1A} to X^{4A} and Y^{5A} to Y^{8A} represents CR^{a}, and at least one of R^{a} in X^{1A} to X^{4A} and Y^{5A} to Y^{8A} represents a cyano-substituted aromatic hydrocarbon group having 6 to 30 ring carbon atoms or a cyano-substituted heterocyclic group having 5 to 30 ring atoms;
   (iii) mA represents an integer of 1 to 3 and at least one of A³ represents a cyano-substituted divalent aromatic hydrocarbon group having 6 to 30 ring carbon atoms or a cyano-substituted divalent heterocyclic group having 5 to 30 ring atoms;
   (iv) at least one of X^{5A} to X^{8A} and Y^{1A} to Y^{4A} represents CR^{a}, and at least one of R^{a} in X^{5A} to X^{8A} and Y^{1A} to Y^{4A} represents a cyano-substituted aromatic hydrocarbon group having 6 to 30 ring carbon atoms or a cyano-substituted heterocyclic group having 5 to 30 ring atoms; and
   (v) at least one of X^{1A} to X8A and Y^{1A} to Y^{8A} represents C-CN.

The cyano-substituted aromatic hydrocarbon group having 6 to 30 ring carbon atoms and the cyano-substituted heterocyclic group having 5 to 30 ring atoms may be further substituted by a group other than the cyano group.

The subscript mA is preferably 0 to 2 and more preferably 0 or 1. When mA is 0, one of X^{5A} to X^{8A} and one of Y^{1A} to Y^{4A} are bonded to each other via a single bond.

In formula (A), the groups mentioned above have the following meanings:
The aromatic hydrocarbon group having 6 to 30 ring carbon atoms represented by A^{1A}, A^{2A} and R^{a} may be a non-condensed aromatic hydrocarbon group or a condensed aromatic hydrocarbon group. Specific examples thereof include phenyl group, naphthyl group, phenanthryl group, biphenyl group, terphenyl group, quaterphenyl group, fluoranthenyl group, triphenylenyl group, phenanthrenyl group, fluorenyl group, spirofluorenyl group, 9,9-diphenylfluorenyl group, 9,9'-spirobi[9H-fluorene]-2-yl group, 9,9-dimethylfluorenyl group, benzo[c]phenanthrenyl group, benzo[a]triphenylenyl group, naphtho[1,2-c]phenanthrenyl group, naphtho[1,2-a]triphenylenyl group, dibenzo[a,c]triphenylenyl group, and benzo[b]fluoranthenyl group, with phenyl group, naphthyl group, biphenyl group, terphenyl group, phenanthryl group, triphenylenyl group, fluorenyl group, spirobifluorenyl group, and fluoranthenyl group being preferred, and phenyl group, 1-naphthyl group, 2-naphthyl group, biphenyl-2-yl group, biphenyl-3-yl group, biphenyl-4-yl group, phenanthrene-9-yl group, phenanthrene-3-yl group, phenanthrene-2-yl group, triphenylene-2-yl group, 9,9-dimethylfluorene-2-yl group, fluoranthene-3-yl group being more preferred.

Examples of the divalent aromatic hydrocarbon group having 6 to 30 ring carbon atoms represented by A^{3A} include divalent residues of the above aromatic hydrocarbon groups having 6 to 30 ring carbon atoms.

The heterocyclic group having 5 to 30 ring atoms represented by A^{1A}, A^{2A} and R^{a} may be a non-condensed heterocyclic group or a condensed heterocyclic group. Specific examples thereof include the residues of pyrrole ring, isoindole ring, benzofuran ring, isobenzofuran ring, dibenzothiophene ring, isoquinoline ring, quinoxaline ring, phenanthridine ring, phenanthroline ring, pyridine ring, pyrazine ring, pyrimidine ring, pyridazine ring, triazine ring, indole ring, quinoline ring, acridine ring, pyrrolidine ring, dioxane ring, piperidine ring, morpholine ring, piperazine ring, carbazole ring, furan ring, thiophene ring, oxazole ring, oxadiazole ring, benzoxazole ring, thiazole ring, thiadiazole ring, benzothiazole ring, triazole ring, imidazole ring, benzimidazole ring, pyran ring, dibenzofuran ring, and benzo[c]dibenzofuran ring, and the residues of derivatives of these rings, with the residues of dibenzofuran ring, carbazole ring, dibenzothiophene ring, and derivatives of these rings being preferred, and the residues of dibenzofuran-2-yl group, dibenzofuran-4-yl group, 9-phenylcarbazole-3-yl group, 9-phenylcarbazole-2-yl group, dibenzothiophene-2-yl group, and dibenzothiophene-4-yl group being more preferred.

Examples of the divalent heterocyclic group having 5 to 30 ring atoms represented by A^{3A} include divalent residues of the above heterocyclic group having 5 to 30 ring atoms.

Examples of the alkyl group having 1 to 30 carbon atoms represented by R^{a} include methyl group, ethyl group, n-propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neopentyl group, 1-methylpentyl group, cyclopropyl group, cyclobutyl group, cyclopentyl group, cyclohexyl group, cyclooctyl group, and adamantyl group, with methyl group, ethyl group, n-propyl group, isopropyl group, n-butyl group, s-butyl group, isobutyl group, t-butyl group, cyclopentyl group, and cyclohexyl group being preferred.

Examples of the silyl group , which may be unsubstituted or substituted ; represented by R^{a} include trimethylsilyl group, triethylsilyl group, tributylsilyl group, dimethylethylsilyl group, t-butyldimethylsilyl group, vinyldimethylsilyl group, propyldimethylsilyl group, dimethylisopropylsilyl group, dimethylpropylsilyl group, dimethylbutylsilyl group, dimethyltertiarybutylsilyl group, diethylisopropylsilyl group, phenyldimethylsilyl group, diphenylmethylsilyl group, diphenyltertiarybutylsilyl group, and triphenylsilyl group, with trimethylsilyl group, triethylsilyl group, t-butyldimethylsilyl group, vinyldimethylsilyl group, and propyldimethylsilyl group being preferred.

Examples of the halogen atom represented by R^{a} include fluorine, chlorine, bromine, and iodine, with fluorine being preferred.

Also preferred as R^{a} is a hydrogen atom or an aryl group having 6 to 30 ring carbon atoms, which may be unsubstituted or substituted.

Examples of the optional substituent indicated by "substituted or unsubstituted" and "may be substituted" referred to above or hereinafter include a halogen atom (fluorine, chlorine, bromine, iodine), a cyano group, an alkyl group having 1 to 20, preferably 1 to 6 carbon atoms, a cycloalkyl group having 3 to 20, preferably 5 to 12 carbon atoms, an alkoxyl group having 1 to 20, preferably 1 to 5 carbon atoms, a haloalkyl group having 1 to 20, preferably 1 to 5 carbon atoms, a haloalkoxyl group having 1 to 20, preferably 1 to 5 carbon atoms, an alkylsilyl group having 1 to 10, preferably 1 to 5 carbon atoms, an aromatic hydrocarbon group having 6 to 30, preferably 6 to 18 ring carbon atoms, an aryloxy group having 6 to 30, preferably 6 to 18 ring carbon atoms, an arylsilyl group having 6 to 30, preferably 6 to 18 carbon atoms, an aralkyl group having 7 to 30, preferably 7 to 20 carbon atoms, and a heteroaryl group having 5 to 30, preferably 5 to 18 ring atoms.

The optional substituent mentioned above may be further substituted by the optional group mentioned above.

Examples of the optional alkyl group having 1 to 20 carbon atoms include methyl group, ethyl group, n-propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neopentyl group, and 1-methylpentyl group.

Examples of the optional cycloalkyl group having 3 to 20 carbon atoms include cyclopropyl group, cyclobutyl group, cyclopentyl group, cyclohexyl group, cyclooctyl group, and adamantyl group.

Examples of the optional alkoxyl group having 1 to 20 carbon atoms include those having an alkyl portion selected from the alkyl groups mentioned above.

Examples of the optional haloalkyl group having 1 to 20 carbon atoms include the alkyl groups mentioned above wherein the hydrogen atoms thereof are partly or entirely substituted by halogen atoms.

Examples of the optional haloalkoxyl group having 1 to 20 carbon atoms include the alkoxyl group mentioned above wherein the hydrogen atoms thereof are partly or entirely substituted by halogen atoms.

Examples of the optional alkylsilyl group having 1 to 10 carbon atoms include trimethylsilyl group, triethylsilyl group, tributylsilyl group, dimethylethylsilyl group, t-butyldimethylsilyl group, vinyldimethylsilyl group, propyldimethylsilyl group, dimethylisopropylsilyl group, dimethylpropylsilyl group, dimethylbutylsilyl group, dimethyltertiarybutylsilyl group, and diethylisopropylsilyl group.

Examples of the optional aryl group having 6 to 30 ring carbon atoms include those selected from the aryl groups mentioned above with respect to A^{1A}, A^{2A} and R^{a}.

Examples of the optional aryloxy group having 6 to 30 ring carbon atoms include those having an aryl portion selected from the aromatic hydrocarbon groups mentioned above.

Examples of the optional arylsilyl group having 6 to 30 carbon atoms include phenyldimethylsilyl group, diphenylmethylsilyl group, diphenyltertiarybutylsilyl group, and triphenylsilyl group.

Examples of the optional aralkyl group having 7 to 30 carbon atoms include benzyl group, 2-phenylpropane-2-yl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, 2-β-naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group, and 1-chloro-2-phenylisopropyl group.

Examples of the optional heteroaryl group having 5 to 30 ring atoms include those selected from the heterocyclic groups mentioned above with respect to A^{1A}, A^{2A} and R^{a}.

The "carbon number of a to b" in the expression of "substituted or unsubstituted X group having carbon number of a to b" is the carbon number of the unsubstituted X group and does not include the carbon atom of the optional substituent.

The hydrogen atom referred to herein includes isotopes different from neutron numbers, i.e., light hydrogen (protium), heavy hydrogen (deuterium) and tritium.

In the host material represented by formula (A), the groups represented by formulae (a) and (b) are bonded to each other via -(A³)_{mA}- at one of X^{5A} to X^{8A} and one of Y^{1A} to Y^{4A}. Specific examples of the bonding manner between formulae (a) and (b) are represented by X^{6A}-(A^{3A})_{mA}-Y^{3A}, X^{6A}-(A^{3A})_{mA}-Y^{2A}, X^{6A}-(A^{3A})_{mA}-Y^{4A}, X^{6A}-(A^{3A})_{mA}-Y^{1A}, X^{7A}-(A^{3A})_{mA}-Y^{3A}, X^{5A}-(A^{3A})_{mA}-Y^{3A}, X^{8A}-(A^{3A})_{mA}-Y^{3A}, X^{7A}-(A^{3A})_{mA}-Y^{2A}, X^{7A}-(A^{3A})_{mA}-Y^{4A}, X^{7A}-(A^{3A})_{mA}-Y^{1A}, X^{5A}-(A^{3A})_{mA}-Y^{2A}, X^{8A}-(A^{3A})_{mA}-Y^{2A}, X^{8A}-₍A^{3A}_{)mA}-Y^{4A}, X^{8A}-(A^{3A})_{mA}-Y^{1A}, X^{5A}-(A^{3A})_{mA}-Y^{1A}, and X^{5A}-(A^{3A})_{mA}-Y^{4A}.

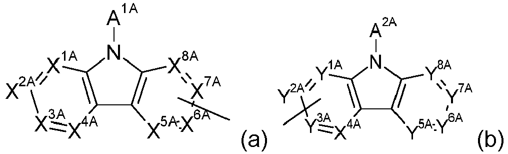

In preferred embodiments of the host material represented by formula (A), the bonding manner between formulae (a) and (b) are represented by X^{6A}-(A^{3A})_{mA}-Y^{3A}, X^{6A}-(A^{3A})_{mA}-Y^{2A}, or X^{7A}-(A^{3A})_{mA}-Y^{3A}, namely the material for organic electroluminescence device is preferably represented by formula (II), (III), or (IV): 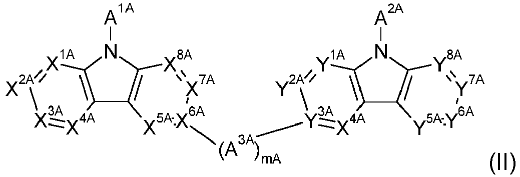 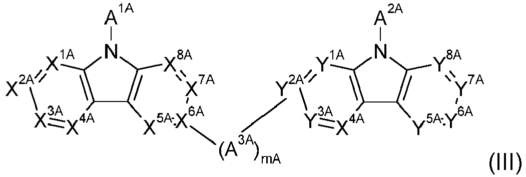 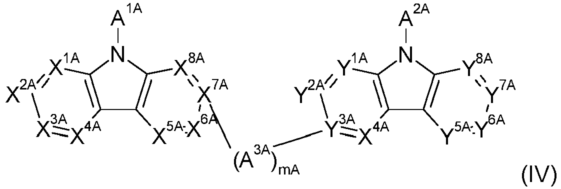 wherein X^{1A} to X^{8A}, Y^{1A} to Y^{8A,} A^{1A} to A^{3A}, and mA are the same as X^{1A} to X^{8A}, Y^{1A} to Y^{8A}, A^{1A} to A^{3A}, mA in formula (A), and each of formulae (II), (III), and (IV) satisfies at least one of the requirements (i) to (v) as specified in the definition of formula (A).

The host material represented by formula (A) satisfies at least one of the requirements (i) to (v), namely, the host material is a cyano group-introduced biscarbazole derivative having a group represented by formula (a) and a group represented by formula (b) which are linked to each other.

A^{3A} of formula (A) preferably represents a single bond, a substituted or unsubstituted divalent monocyclic hydrocarbon group having 6 or less ring carbon atoms, or a substituted or unsubstituted divalent monocyclic heterocyclic group having 6 or less ring atoms.

Examples of the monocyclic hydrocarbon group having 6 or less ring carbon atoms represented by A^{3A} include phenylene group, cyclopentenylene group, cyclopentadienylene group, cyclohexylene group, and cyclopentylene group, with phenylene group being preferred.

Examples of the monocyclic heterocyclic group having 6 or less ring atoms represented by A^{3A} include pyrrolylene group, pyrazinylene group, pyridinylene group, furylene group, and thiophenylene group.

In a preferred embodiment of formulae (A), (II), (III), and (IV), mA is 0 and one of X^{5A} to X^{8A} and one of Y^{1A} to Y^{4A} are bonded to each other via a single bond; or A^{3A} represents the substituted or unsubstituted monocyclic hydrocarbon group having 6 or less ring carbon atoms or the substituted or unsubstituted monocyclic heterocyclic group having 6 or less ring atoms.

In more preferred embodiment, mA is 0 and one of X^{5A} to X^{8A} and one of Y^{1A} to Y^{4A} are bonded to each other via a single bond; or A^{3A} represents a substituted or unsubstituted phenylene group.

The host material of formula (A) satisfies preferably at least one of the requirements (i) and (ii);
(i) at least one of A^{1A} and A^{2A} represents a cyano-substituted aromatic hydrocarbon group having 6 to 30 ring carbon atoms or a cyano-substituted heterocyclic group having 5 to 30 ring atoms; and
(ii) at least one of X^{1A} to X^{4A} and Y^{5A} to Y^{8A} represents CR^{a}, and at least one of R^{a} in X^{1A} to X^{4A} and Y^{5A} to Y^{8A} represents a cyano-substituted aromatic hydrocarbon group having 6 to 30 ring carbon atoms or a cyano-substituted heterocyclic group having 5 to 30 ring atoms.

Namely, the host material of formula (A) is preferably any one of the compounds;
(1) satisfying the requirement (i), but not satisfying the requirements (ii) to (v);
(2) satisfying the requirement (ii), but not satisfying the requirements (i) and (iii) to (v); and
(3) satisfying both the requirements (i) and (ii), but not satisfying the requirements (iii) to (v).

The host material of formula (A) satisfying the requirement (i) and/or (ii) has a structure wherein the cyano group-containing aromatic hydrocarbon group or the cyano group-containing heterocyclic group is introduced to the terminal end of the central skeleton comprising the groups represented by formulae (a) and (b).

When the host material of formula (A) satisfies the requirement (i), at least one of A^{1A} and A^{2A} is preferably a cyano-substituted phenyl group, a cyano-substituted naphthyl group, a cyano-substituted phenanthryl group, a cyano-substituted dibenzofuranyl group, a cyano-substituted dibenzothiophenyl group, a cyano-substituted biphenyl group, a cyano-substituted terphenyl group, a cyano-substituted 9,9-diphenylfluorenyl group, a cyano-substituted 9,9'-spirobi[9H-fluorene]-2-yl group, a cyano-substituted 9,9'-dimethylfluorenyl group, or a cyano-substituted triphenylenyl group, and more preferably 3'-cyanobiphenyl-2-yl group, 3'-cyanobiphenyl-3-yl group, 3'-cyanobiphenyl-4-yl group, 4'-cyanobiphenyl-3-yl group, 4'-cyanobiphenyl-4-yl group, 4'-cyanobiphenyl-2-yl group, 6-cyanonaphthalene-2-yl group, 4-cyanonaphthalene-1-yl group, 7-cyanonaphthalene-2-yl group, 8-cyanodibenzofuran-2-yl group, 6-cyanodibenzofuran-4-yl group, 8-cyanodibenzothiophene-2-yl group, 6-cyanodibenzothiophene-4-yl group, 7-cyano-9-phenylcarbazole-2-yl group, 6-cyano-9-phenylcarbazole-3-yl group, 7-cyano-9,9-dimethylfluorene-2-yl group, or 7-cyanotriphenylene-2-yl group.

The host material of formula (A) wherein A^{1A} is substituted by a cyano group and A^{2A} is not substituted by a cyano group is preferred. In this case, the first host material which does not satisfy the requirement (ii) is more preferred.

When the host material of formula (A) satisfies the requirement (ii), at least one of X^{1A} to X^{4A} and Y^{5A} to Y^{8A} is preferably CR^{a}, and one of R^{a} in X^{1A} to X^{4A} and Y^{5A} to Y^{8A} is preferably a cyano-substituted phenyl group, a cyano-substituted naphthyl group, a cyano-substituted phenanthryl group, a cyano-substituted dibenzofuranyl group, a cyano-substituted dibenzothiophenyl group, a cyano-substituted biphenyl group, a cyano-substituted terphenyl group, a cyano-substituted 9,9-diphenylfluorenyl group, a cyano-substituted 9,9'-spirobi[9H-fluorene]-2-yl group, a cyano-substituted 9,9'-dimethylfluorenyl group, or a cyano-substituted triphenylenyl group, and more preferably 3'-cyanobiphenyl-2-yl group, 3'-cyanobiphenyl-3-yl group, 3'-cyanobiphenyl-4-yl group, 4'-cyanobiphenyl-3-yl group, 4'-cyanobiphenyl-4-yl group, 4'-cyanobiphenyl-2-yl group, 6-cyanonaphthalene-2-yl group, 4-cyanonaphthalene-1-yl group, 7-cyanonaphthalene-2-yl group, 8-cyanodibenzofuran-2-yl group, 6-cyanodibenzofuran-4-yl group, 8-cyanodibenzothiophene-2-yl group, 6-cyanodibenzothiophene-4-yl group, 7-cyano-9-phenylcarbazole-2-yl group, 6-cyano-9-phenylcarbazole-3-yl group, 7-cyano-9,9-dimethylfluorene-2-yl group, or 7-cyanotriphenylene-2-yl group.

It is preferred for the host material of formula (A) to satisfy the requirement (ii), but not satisfy the requirement (i).

In formulae (A) and (II) to (IV), A^{1A} and A^{2A}are preferably different from each other, and more preferably, A^{1A} is substituted by a cyano group but A^{2A} is not substituted by a cyano group. Namely, the host material of formula (A) is preferably structurally asymmetric.

The production method of the first host material is not particularly limited and it is produced according to a known method, for example, by a coupling reaction of a carbazole derivative and an aromatic halogenated compound in the presence of a copper catalyst described in Tetrahedron 40 (1984) 1435 to 1456 or a palladium catalyst described in Journal of American Chemical Society 123 (2001) 7727 to 7729.

Examples of the host material of formula (A) are mentioned in [0145] in US2013234119.

Examples for preferred host materials used as co-hosts mentioned in US2013234119 WO2012108388 and WO2014009317 are:

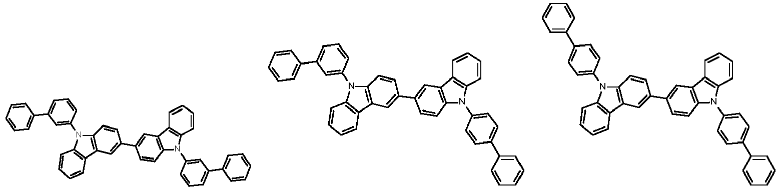

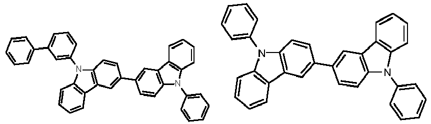

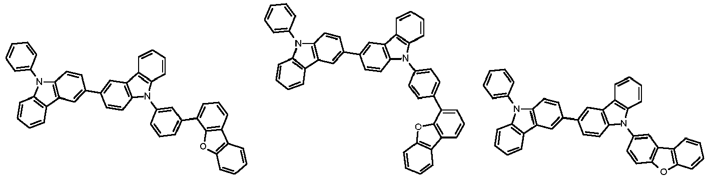

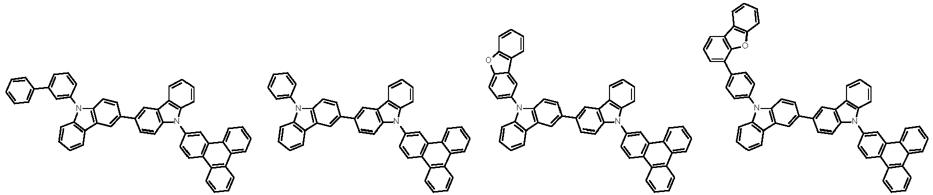

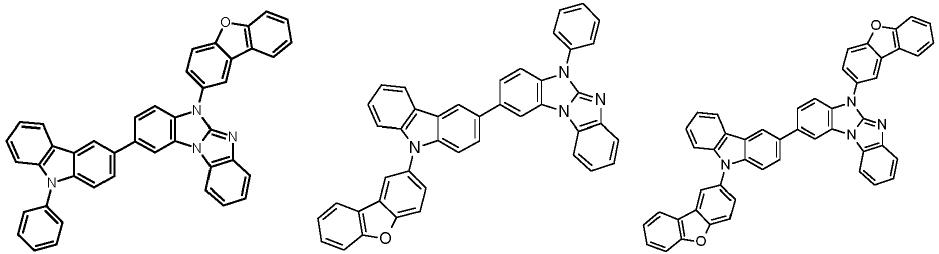

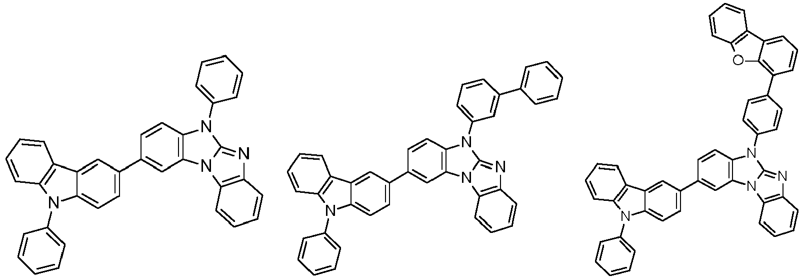

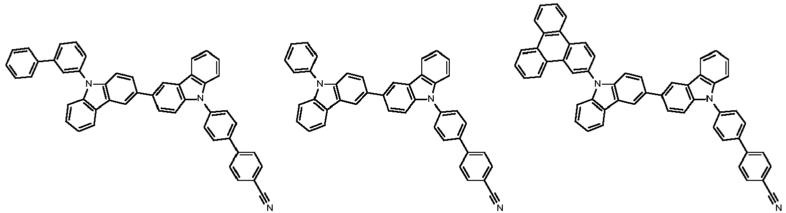

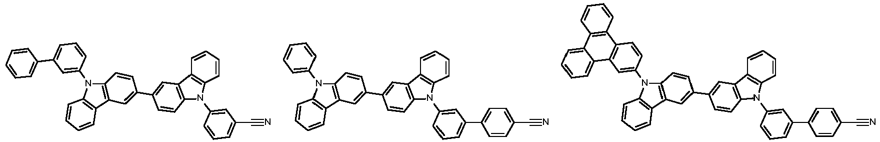

It is further possible to employ the compound of formula (1) to the present invention as host material in an OLED, preferably in the light emitting layer, together with at least one second host material described in US 2013234119, especially in paragraphs [0146] to [0195] in US 2013234119.

Examples for preferred second host materials used as co-hosts mentioned in US2013234119 are:

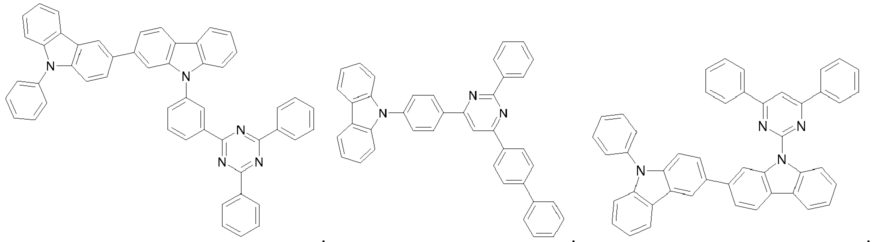

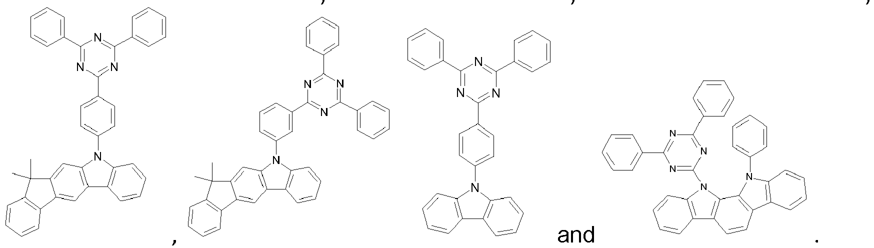

### Hole/exciton blocking layer (f):

Blocking layers may be used to reduce the number of charge carriers (electrons or holes) and/or excitons that leave the emissive layer. The hole blocking layer may be disposed between the emitting layer (e) and electron transport layer (g), to block holes from leaving layer (e) in the direction of electron transport layer (g). Blocking layers may also be used to block excitons from diffusing out of the emissive layer.

Additional hole blocker materials typically used in OLEDs are 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAIq), phenothiazine *S,S*-dioxide derivates and 1,3,5-tris(N-phenyl-2-benzylimidazolyl)benzene) (TPBI), TPBI also being suitable as electron-transport material. Further suitable hole blockers and/or electron conductor materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]-phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, disilylbenzothiophenes, disilylbenzophospholes, disilylbenzothiophene S-oxides and disilylbenzothiophene S,S-dioxides, as specified, for example, in PCT applications WO2009/003919 and WO2009003898 and disilyl compounds as disclosed in WO2008/034758, as a blocking layer for holes/excitons (f).

In another preferred embodiment compounds **(SH-1), (SH-2), (SH-3), SH-4, SH-5, SH-6, (SH-7), (SH-8), (SH-9), (SH-10)** and **(SH-11)** may be used as hole/exciton blocking materials.

### Electron transport layer (g):

Electron transport layer may include a material capable of transporting electrons. Electron transport layer may be intrinsic (undoped), or doped. Doping may be used to enhance conductivity.

The compound of the formula (1) is suitable as electron transport material, either alone or in combination with one or more of the electron transport materials mentioned below.

Further suitable electron-transporting materials for layer (g) of the inventive OLEDs, which may be used in combination with the compound of formula (1) or in absence of the compound of formula (1) as electron transport material, comprise metals chelated with oxinoid compounds, such as tris(8-hydroxyquinolato)aluminum (Alq₃), compounds based on phenanthroline such as 2,9-dimethyl-4,7-diphenyl-1,10-phenanthroline (DDPA = BCP), 4,7-diphenyl-1,10-phenanthroline (Bphen), 2,4,7,9-tetraphenyl-1,10-phenanthroline, 4,7-diphenyl-1,10-phenanthroline (DPA) or phenanthroline derivatives disclosed in EP1786050, in EP1970371, or in EP1097981, and azole compounds such as 2-(4-biphenylyl)-5-(4-t-butylphenyl)-1,3,4-oxadiazole (PBD) and 3-(4-biphenylyl)-4phenyl-5-(4-t-butylphenyl)-1,2,4-triazole (TAZ).

Further suitable electron transport materials, which may be used in combination with the compound of formula (1) or in absence of the compound of formula (1) as electron transport material, are mentioned in Abhishek P. Kulkarni, Christopher J. Tonzola, Amit Babel, and Samson A. Jenekhe, Chem. Mater. 2004, 16, 4556-4573; G. Hughes, M. R. Bryce, J. Mater. Chem. 2005, 15, 94-107 and Yasuhiko Shirota and Hiroshi Kageyama, Chem. Rev. 2007, 107, 953-1010 *(ETM, HTM).*

It is likewise possible to use mixtures of at least two materials in the electron-transporting layer, in which case at least one material is electron-conducting. Preferably, in such mixed electron-transport layers, at least one phenanthroline compound is used, preferably BCP, or at least one pyridine compound according to the formula **(XVI)** below, preferably a compound of the formula **(XVIa)** below. More preferably, in mixed electron-transport layers, in addition to at least one phenanthroline compound, alkaline earth metal or alkali metal hydroxyquinolate complexes, for example Liq, are used. Suitable alkaline earth metal or alkali metal hydroxyquinolate complexes are specified below **(formula XVII).** Reference is made to WO2011/157779.

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, Cs₂CO₃, with alkali metal complexes, for example 8-hydroxyquinolatolithium (Liq), and with Y, Ce, Sm, Gd, Tb, Er, Tm, Yb, Li₃N, Rb₂CO₃, dipotassium phthalate, W(hpp)₄ from EP1786050, or with compounds described in EP1837926B1, EP1837927, EP2246862 and WO2010132236.

In a preferred embodiment, the electron-transport layer comprises at least one compound of the general formula **(XVII)** 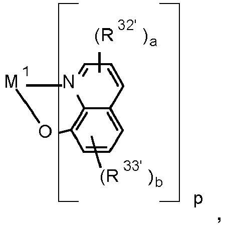 in which
R^{32'} and R^{33'} are each independently F, C₁-C₈-alkyl, or C₆-C₁₄-aryl, which is optionally substituted by one or more C₁-C₈-alkyl groups, or
two R^{32'} and/or R^{33'} substituents together form a fused benzene ring which is optionally substituted by one or more C₁-C₈-alkyl groups;
a and b are each independently 0, or 1, 2 or 3,
M¹ is an alkaline metal atom or alkaline earth metal atom,
p is 1 when M¹ is an alkali metal atom, p is 2 when M¹ is an earth alkali metal atom.

A very particularly preferred compound of the formula **(XVII)** is 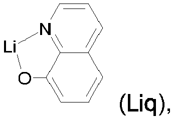 which may be present as a single species, or in other forms such as Li_{g}Q_{g} in which g is an integer, for example Li₆Q₆. Q is an 8-hydroxyquinolate ligand or an 8-hydroxyquinolate derivative.

In a further preferred embodiment, the electron-transport layer comprises at least one compound of the formula **(XVI),** 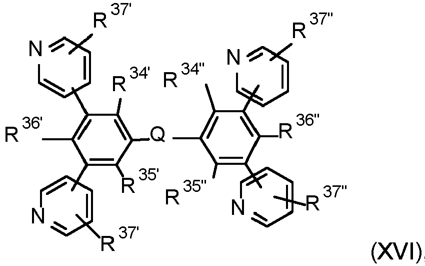 , in which
R^{34"}, R^{35"}, R^{36"}, R^{37"}, R^{34'}, R^{35'}, R^{36'} and R^{37'}are each independently H, C₁-C₁₈-alkyl, C₁-C₁₈-alkyl which is substituted by E' and/or interrupted by D', C₆-C₂₄-aryl, C₆-C₂₄-aryl which is substituted by G', C₂-C₂₀-heteroaryl or C₂-C₂₀-heteroaryl which is substituted by G',
Q is an arylene or heteroarylene group, each of which is optionally substituted by G';
D' is -CO-; -COO-; -S-; -SO-; -SO₂-; -O-; -NR^{40'}-; -SiR^{45'}R^{46'}-; -POR^{47'}-; -CR^{38'}=CR^{39'}-; or -C≡C-;
E' is -OR^{44'}; -SR^{44'}; -NR^{40'}R^{41'}; -COR^{43'}; -COOR^{42'}; -CONR^{40'}R^{41'}; -CN; or F;
G' is E', C₁-C₁₈-alkyl, C₁-C₁₈-alkylwhich is interrupted by D', C₁-C₁₈-perfluoroalkyl, C₁-C₁₈-alkoxy, or C₁-C₁₈-alkoxy which is substituted by E' and/or interrupted by D', in which
R^{38'} and R^{39'} are each independently H, C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-;
R^{40'} and R^{41'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-; or
R^{40'} and R^{41'} together form a 6-membered ring;
R^{42'} and R^{43'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R^{44'} is C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R^{45'} and R^{46'} are each independently C₁-C₁₈-alkyl, C₆-C₁₈-aryl or C₆-C₁₈-aryl which is substituted by C₁-C₁₈-alkyl,
R^{47'} is C₁-C₁₈-alkyl, C₆-C₁₈-aryl or C₆-C₁₈-aryl which is substituted by C₁-C₁₈-alkyl.

Preferred compounds of the formula **(XVI)** are compounds of the formula **(XVIa)** 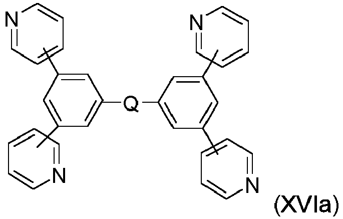 in which Q is: 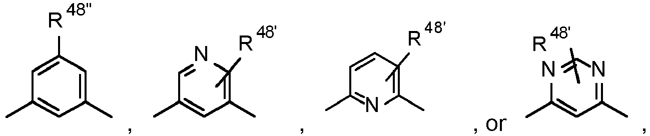
R^{48'} is H or C₁-C₁₈-alkyl and
R^{48"} is H, C₁-C₁₈-alkyl or

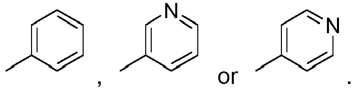

Particular preference is given to a compound of the formula

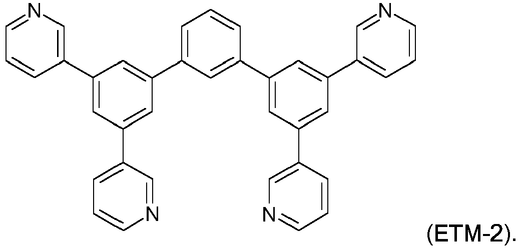

In a further, very particularly preferred embodiment, the electron-transport layer comprises a compound Liq and a compound **ETM-2.**

In a preferred embodiment, the electron-transport layer comprises at least one compound of the formula **(XVII)** in an amount of 99 to 1% by weight, preferably 75 to 25% by weight, more preferably about 50% by weight, and at least one compound of the formula **(XVI)** in an amount of 1 to 99% by weight, preferably 25 to 75% by weight, more preferably about 50% by weight, where the amount of the compounds of the formulae **(XVII)** and the amount of the compounds of the formulae **(XVI)** adds up to a total of 100% by weight.

The preparation of the compounds of the formula (XVI) is described in J. Kido et al., Chem. Commun. (2008) 5821-5823, J. Kido et al., Chem. Mater. 20 (2008) 5951-5953 and JP2008/127326, or the compounds can be prepared analogously to the processes disclosed in the aforementioned documents.

It is likewise possible to use mixtures of alkali metal hydroxyquinolate complexes, preferably Liq, and dibenzofuran compounds in the electron-transport layer. Reference is made to WO2011/157790. Dibenzofuran compounds **A-1** to **A-36** and **B-1** to **B-22** described in WO2011/157790 are preferred, wherein dibenzofuran compound 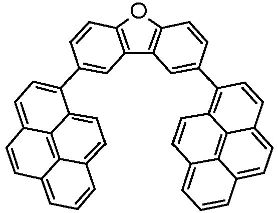 **(A-10; = ETM-1)** is most preferred.

In a preferred embodiment, the electron-transport layer comprises **Liq** in an amount of 99 to 1% by weight, preferably 75 to 25% by weight, more preferably about 50% by weight, and at least one dibenzofuran compound in an amount of 1 to 99% by weight, preferably 25 to 75% by weight, more preferably about 50% by weight, where the amount of **Liq** and the amount of the dibenzofuran compound(s), especially **ETM-1,** adds up to a total of 100% by weight.

In a preferred embodiment, the electron-transport layer comprises at least one phenanthroline derivative and/or pyridine derivative.

In a further preferred embodiment, the electron-transport layer comprises at least one phenanthroline derivative and/or pyridine derivative and at least one alkali metal hydroxyquinolate complex.

In a further preferred embodiment, the electron-transport layer comprises at least one of the dibenzofuran compounds **A-1** to **A-36** and **B-1** to **B-22** described in WO2011/157790, especially **ETM-1.**

In a further preferred embodiment, the electron-transport layer comprises a compound described in WO2012/111462, WO2012/147397, WO2012014621, such as, for example, a compound of formula 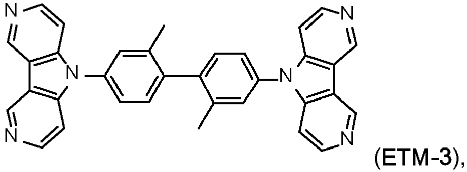 US2012/0261654 , such as, for example, a compound of formula

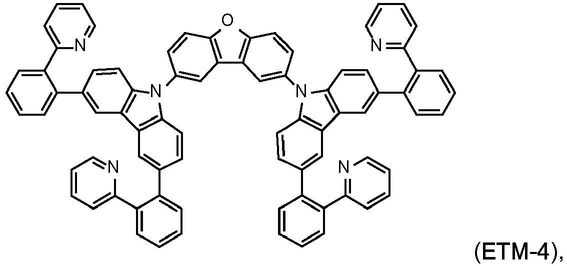

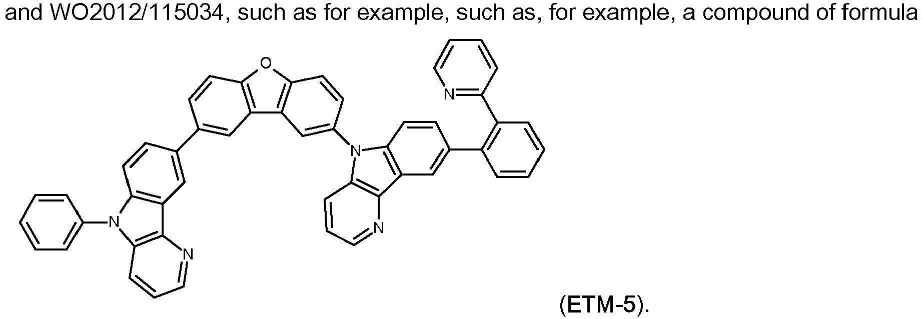

A further suitable electron transport material is:

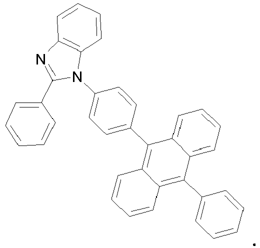

### Electron injection layer (h):

The electron injection layer may be any layer that improves the injection of electrons into an adjacent organic layer.

The compound of the formula (1) is suitable as electron injection material, either alone or in combination with one or more of the electron injection materials mentioned below.

Further lithium-comprising organometallic compounds such as 8-hydroxyquinolatolithium (Liq), CsF, NaF, KF, Cs₂CO₃ or LiF may be applied between the electron transport layer (g) and the cathode (i) as an electron injection layer (h) in order to reduce the operating voltage.

### Cathode (i):

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (angstrom), preferably 1000 to 2000 Å;
hole injection layer (b): 50 to 1000 Å, preferably 200 to 800 Å,
hole-transport layer (c): 50 to 1000 Å, preferably 100 to 800 Å,
exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 Å,
electron injection layer (h): 10 to 500 Å, preferably 20 to 100 Å,
cathode (i): 200 to 10 000 Å, preferably 300 to 5000 Å.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds of the formula (1) in at least one layer of the OLED, preferably in the light-emitting layer (preferably as a matrix material), in a charge transport layer, i.e. electron transport layer or hole transport layer, preferably electron transport layer and/or in the electron injection layer makes it possible to obtain OLEDs with high efficiency and with low use and operating voltage. Frequently, the OLEDs obtained by the use of the compounds of the formula (1) additionally have high lifetimes. The efficiency of the OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### I Preparation Example

### Example 1

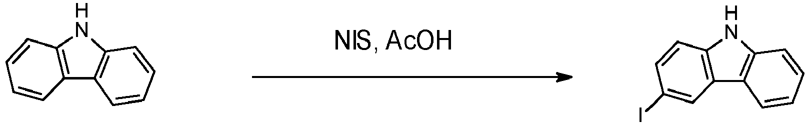

a) 76.9 g (0.460 mol) carbazole and 104 g (0.460 mol) 1-iodopyrrolidine-2,5-dione (NIS) in 100m ml acetic acid are stirred under nitrogen at 20 °C. After 5 h the product is filtered off. The product is crystalized from 900 ml ethanol using 2 g charcoal. The ethanol solution is filtered hot. The ethanol solution is cooled to 20 °C and the product is filtered off (yield: 59.5 g (44 %)). 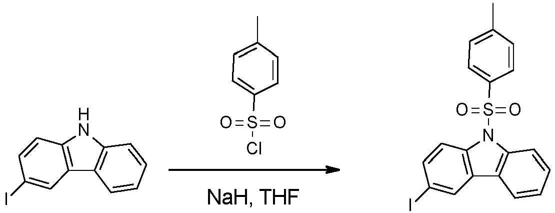
b) 19.7 g (67.0 mmol) 3-iodo-9H-carbazole and 2.95 g (73.7 mmol) sodium hydride 60 % dispersion in mineral oil in 500 ml tetrahydrofuran (THF) are stirred at 50 °C under nitrogen for 1h. 12.8 g (67.0 mmol) 4-methylbenzenesulfonyl chloride in 100 ml THF are added at 20 °C. The reaction mixture is stirred for 1 h at 20 °C and is then stirred for 1 h at 50 °C. The solution is filtered and the solvent is distilled off. 200 ml ethyl acetate are added and the organic phase is washed with a solution of citric acid, sodium hydrogen carbonate and water. The solvent is partly removed until the product starts to crystalize. The product is filtered off and washed with methanol (yield: 23 g (79 %)). 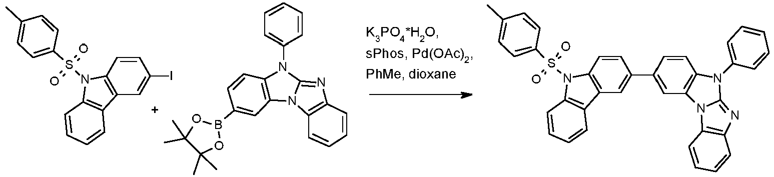
c) To 7.75 g (17.3 mmol) 3-iodo-9-(p-tolylsulfonyl)carbazole, 7.80 (19.1 mmol) 5-phenyl-2-(4,4,5,5-tetramethyl-1,3,2-dioxaborolan-2-yl)benzimidazolo[1,2-a] benzimidazole, 18.4 g (86.6 mmol) potassium phosphate tribasic monohydrate, 25 ml dioxane, 60 ml toluene and 25 ml water are added.
   The mixture is degassed with argon. 426 mg (0.250 mmol) 2-dicyclohexylphosphino-2',6'-dimethoxybiphenyl (SPhos) and 39 mg (0.17 mmol) palladium(II) acetate are added. The reaction mixture is degassed with argon and is stirred for 16 h at 90 °C under argon. 30 ml of a 1 % sodium cyanide solution are added and the reaction mixture is refluxed for 2 h. Toluene is added and organic phase is separated. The organic phase is dried with magnesium sulfate. The solvent is removed in vacuum. The product is crystallized from diethyl ether. 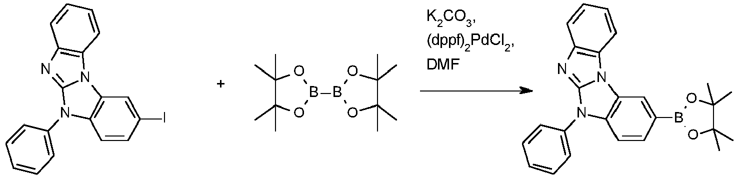
d) 2-iodo-5-phenyl-benzimidazolo[1,2-a]benzimidazole is prepared as describe in WO 2014/009317
   3.00 g (7.33 mmol) 2-iodo-5-phenyl-benzimidazolo[1,2-a]benzimidazole, 2.88 g (29.3 mmol) potassium acetate and 2.23 (8.80 mmol) 4,4,5,5-tetramethyl-2-(4,4,5,5 -tetramethyl-1,3,2-dioxaborolan- 2-yl)-1,3,2-dioxaborolane are degassed 3 times with argon The water free DMF is added and the reaction mixture is degassed 7 times with argon under stirring and at 25 °C. The catalyst is added and the reaction mixture is degassed 2 times with argon at 25 °C. Reaction mixture is stirred at 65 °C under argon for 18 h.
   The reaction mixture is cooled to 35 °C 5 ml diethyl ether and 400mg NaCN in 2 ml water is added simultaneously. The reaction mixture is stirred 10 min. The reaction mixture is poured in dichloromethane containing 20 % diethyl ether. The organic phase is washed with water and dried with magnesium sulfate and filtered on Hyflo. The solvent is removed in vacuum. Yield 2.93 g (97.7 %). 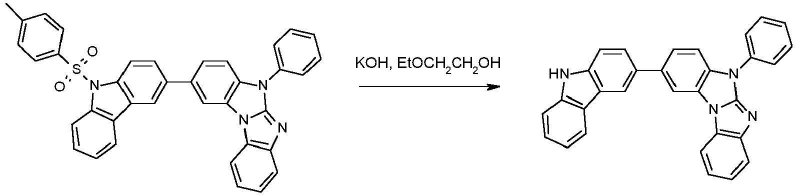
e) 9.30 g (15.4 mmol) 5-phenyl-2-[9-(p-tolylsulfonyl)carbazol-3-yl]benzimidazolo [1,2-a]benzimidazole and 2.14 g (32.4 mmol) potassium hydroxide in 200 ml 2-ethoxyethanol is refluxed for 2 h. The solvent is removed in vacuum. The product is decocted in ethanol and the product is filtered off. 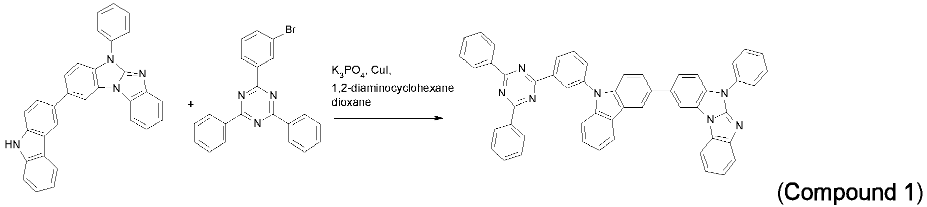
f) The synthesis of 2-(3-bromophenyl)-4,6-diphenyl-1,3,5-triazine is described in WO2012099219 and WO2013172255

2.00 g (5.15 mmol) 2-(3-bromophenyl)-4,6-diphenyl-1,3,5-triazine, 2.77 g (6.18 mmol) 2-(9H-carbazol-3-yl)-5-phenyl-benzimidazolo[1,2-a]benzimidazole, 3.28 g (15.5 mmol) potassium phosphate tribasic, 196 mg (1.03 mmol) copper iodide in 50 ml dioxane are stirred under nitrogen at 100 °C. 4.12 g (36.1 mmol) cis,trans 1,2-diaminocyclohexane are added. The reaction mixture is stirred for 22 h. 196 mg (1.03 mmol) copper iodide and 4.12 g (36.1 mmol) cis,trans 1,2-diaminocyclohexane are added. The reaction mixture is stirred for 48 h at 100 °C under nitrogen.

The reaction mixture is poured in 200 ml methanol. The product is filtered off and is washed with water and methanol. The product is decocted in THF and filtered off. The product is decocted in acetic acid. The product is washed with ethanol.

Yield: 0.62 g (16 %).

**¹H NMR** (400 MHz, TFA-d1): δ = 9.29-9.30 (m, 1H), 9.12 (d, 1H), 8.83-8.86 (m, 4H), 8.73 (m,1H), 8.66 (m, 1H), 8.41-8.51 (m, 3H), 8.24-8.32 (m, 2H), 8.13 (t, 2H), 7.90-8.05 (m, 14H), 7.67-7.88 (m, 4H)

### Example 2

### Example 2a

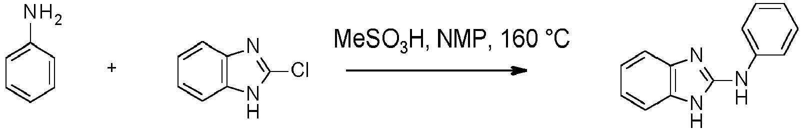

To 38.1 g (0.250 mol) 2-chlorobenzimidazole, 25.6 g 0.275 mol) aniline in 250 ml NMP 26.4 g (0.275 mmol) methane sulfuric acid is added. The reaction mixture is stirred at 100 °C for 3 h under nitrogen. The reaction mixture is poured on a saturated solution of sodium hydrogen carbonate in water. The water phase is extracted with ethyl acetate. The organic phase is 3 times washed with water and the organic phase is dried with magnesium sulfate. The solvent is removed in vacuum. The product is decocted in 100 ml dichloromethane.

Yield 43.6 g (83%)

The above reaction is carried out according to a procedure given in US20090186879 (page 57).

**¹H NMR** (400 MHz, DMSO-d6): δ = 10.9 (s, 1H), 9.38 (s, 1H), 7.29-7.36 (d, 2H), 2.29-7.36 (m, 4H), 6.96-7.02(m, 2H), 6.91-6.94 (m, 1H).

### Example 2b

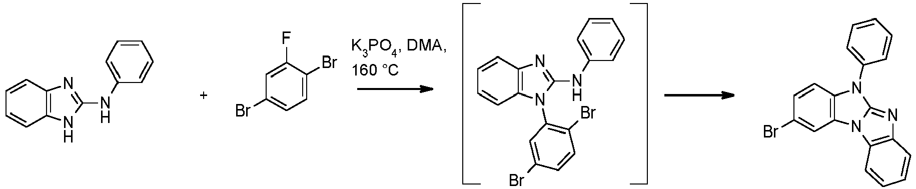

To 6.35 g (25.0 mmol) 1,2-dibromo-3-fluoro-benzene, 5.23 g (25.0 mmol) N-phenyl-1H-benzimidazol-2-amine and 15.9 g (75.0 mmol) potassium phosphate tribasic in 50 ml DMA are stirred at 160 °C for 4 h under nitrogen. The reaction mixture is poured on water. The product is filtered off washed with water.

The product is dissolved in dichloromethane and is 3 times washed with water. The organic phase is dried with magnesium sulfate and the solvent is removed in vacuum.

Yield 7.67 g (85 %)

**¹H NMR** (400 MHz, CDCl₃): δ = 7.98 (d, 1H), 7.78-7.84 (m, 4H), 7.63-7.67 (m, 2H), 7.32-7.50 (m, 5H).

### Example 2c

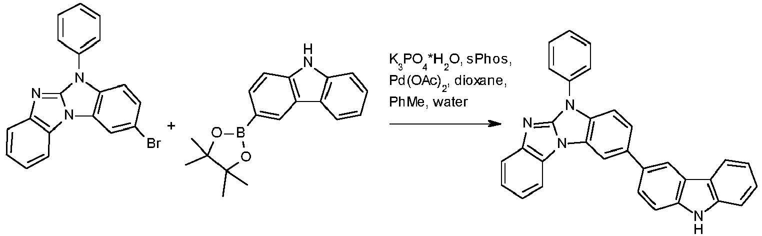

5.00 g (13.8 mmol) 2-bromo-5-phenyl-benzimidazolo[1,2-a]benzimidazole, 4.86 g (16.6 mmol) 3-(4,4,5,5-tetramethyl-1,3,2-dioxaborolan-2-yl)-9H-carbazole and 14.7 g (69.0 mmol) potassium phosphate tribasic in 25 ml dioxane, 70 ml toluene and 25 ml water are degassed with argon. 340 mg (0.84 mmol) 2-Dicyclohexylphosphino-2',6'-di-methoxybiphenyl (sPhos) and 31 mg (0.14 mmol) palladium (II) acetate is added. The reaction mixture is degassed with argon. The reaction mixture is stirred for 17 h at 90 °C under argon.

40 ml of a 1 % solution of sodium cyanide in water is added and the reaction mixture is stirred at 100 °C for 1 h. The product is filtered off, is washed with water and ethanol. The product is decocted with methyl ethyl ketone. Yield 4.65 g (75%).

**¹H NMR** (400 MHz, CDCl3): δ = 8.39 (s, 1H), 8.24 (s, 1H), 8.22 (d, 1H), 8.13 (s, 1H), 7.99 (d, 1H), 7.91-7.94 (m, 2H), 7.84 (d, 1H), 7,78 (dd, 1H), 7.65-7.70 (m, 4H), 7.58 (d, 1H), 7.47-7.52 (m, 3H), 7.30-7.43 (m, 3H).

**The synthesis of** 2-(4,4,5,5-tetramethyl-1,3,2-dioxaborolan-2-yl)-9H-carbazole is described in WO2013084881**.**

### Example 3

### Example 3a

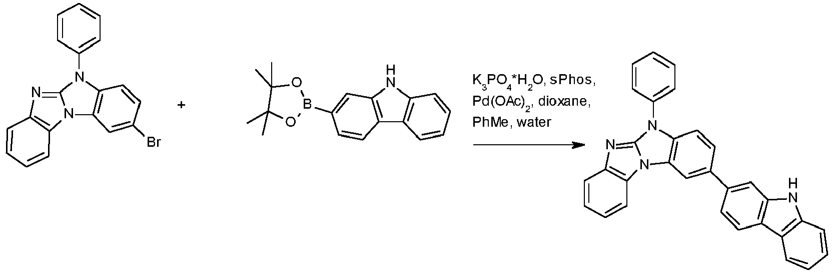

The reaction is carried out as described in example 2c except that 2-(4,4,5,5-tetramethyl-1,3,2-dioxaborolan-2-yl)-9H-carbazole instead of 3-(4,4,5,5-tetramethyl-1,3,2-dioxaborolan-2-yl)-9H-carbazole is used.

### Example 3b

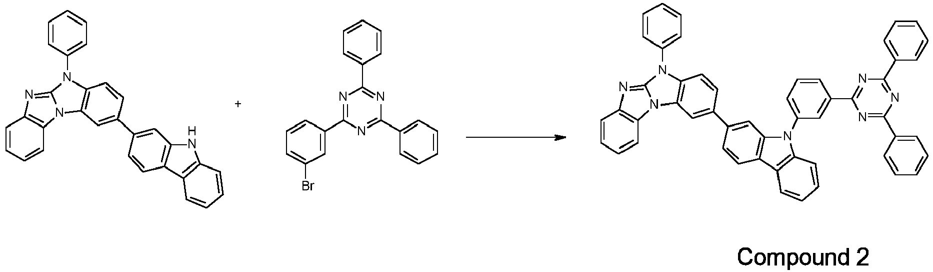

The reaction is carried out as described in patent example 1f except that 2-(9H-carbazol-2-yl)-5-phenyl-benzimidazolo[1,2-a]benzimidazole instead of 2-(9H-carbazol-3-yl)-5-phenyl-benzimidazolo[1,2-a]benzimidazole is used.

The synthesis of 1-(4,4,5,5-tetramethyl-1,3,2-dioxaborolan-2-yl)-9H-carbazole is described in Chem. Commun., 2015, 51, 10672-10675.

### Example 4

### Example 4a

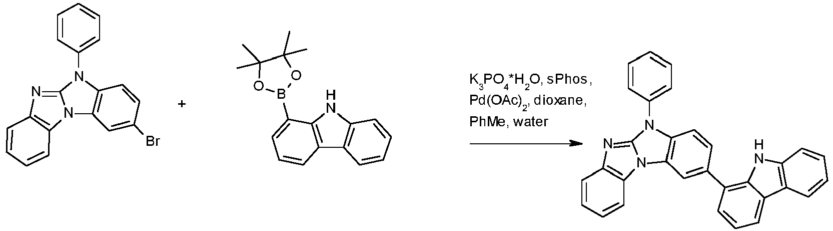

The reaction is carried out as described in example 2c except that 1-(4,4,5,5-tetramethyl-1,3,2-dioxaborolan-2-yl)-9H-carbazole instead of 3-(4,4,5,5-tetramethyl-1,3,2-dioxaborolan-2-yl)-9H-carbazole is used.

### Example 4b

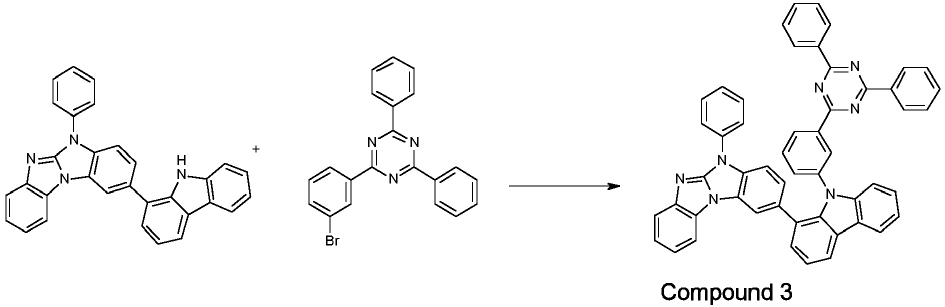

The reaction is carried out as described in patent example 1f except that 2-(9H-carbazol-1-yl)-5-phenyl-benzimidazolo[1,2-a]benzimidazole instead of 2-(9H-carbazol-3-yl)-5-phenyl-benzimidazolo[1,2-a]benzimidazole is used.

**The synthesis of** 4-(4,4,5,5-tetramethyl-1,3,2-dioxaborolan-2-yl)-9H-carbazole is described in WO2010131855.

### Example 5

### Example 5a

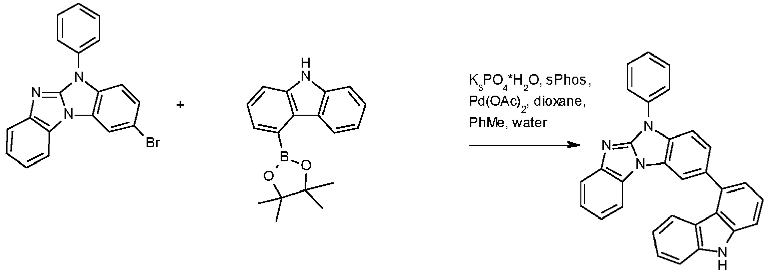

The reaction is carried out as described in example 2c except that 4-(4,4,5,5-tetramethyl-1,3,2-dioxaborolan-2-yl)-9H-carbazole instead of 3-(4,4,5,5-tetramethyl-1 ,3,2-dioxaborolan-2-yl)-9H-carbazole is used.

### Example 5b

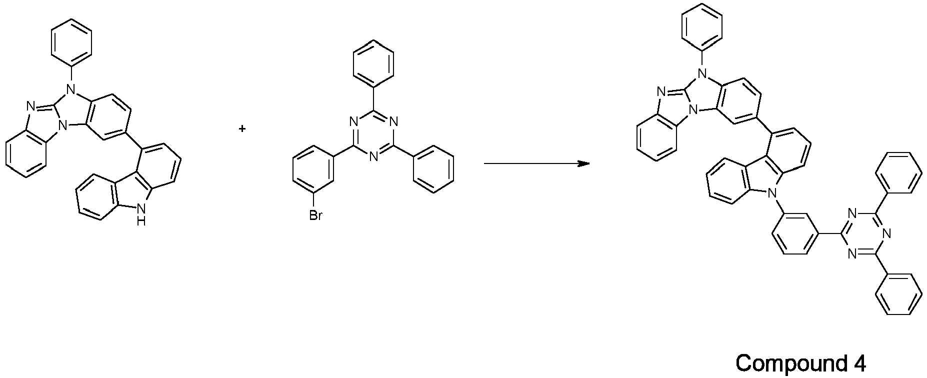

The reaction is carried out as described in patent example 1f except that 2-(9H-carbazol-4-yl)-5-phenyl-benzimidazolo[1,2-a]benzimidazole instead of 2-(9H-carbazol-3-yl)-5-phenyl-benzimidazolo[1,2-a]benzimidazole is used.

### Example 6

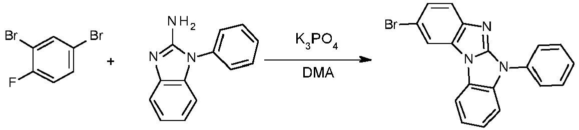

a.) To 10. g (39.4 mmol) 1,3-dibromo-4-fluorobezene, 8.20 g (39.4 mmol) 1-phenylbenzimidazol-2-amine and 25.1 g (11.8 mmol) potassium phosphate tribasic in 200 ml DMA are stirred at 110 °C for 4 h under nitrogen. The reaction mixture is poured on water. The water phase is extracted with dichloromethane. The organic phase is washed with 3 times water and dried with magnesium sulfate. The solvent is distilled off. The product is decocted in diethyl ether.
   Yield 5.30 g (36 %).
   **¹H NMR** (400 MHz, CDCl₃): δ = 8.01 (d, 1H), 7.81-7.86 (m, 3H), 7.63-7.7.67 (m, 3H), 7.57-5.59 (m, 1H), 7.46-7.51 (m, 2H), 7.36-7.44 (m, 2H). 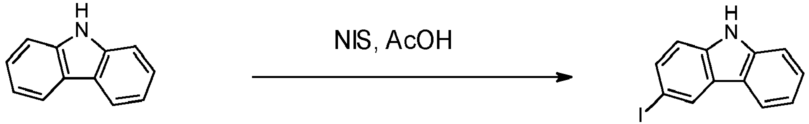
b) 76.9 g (0.460 mol) carbazole and 104 g (0.460 mol) 1-iodopyrrolidine-2,5-dione (NIS) in 100m ml acetic acid are stirred under nitrogen at 20 °C. After 5 h the product is filtered off. The product is crystalized from 900 ml ethanol using 2 g charcoal. The ethanol solution is filtered hot. The ethanol solution is cooled to 20 °C and the product is filtered off (yield: 59.5 g (44 %)). 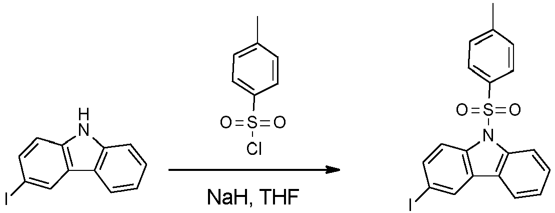
c) 19.7 g (67.0 mmol) 3-iodo-9H-carbazole and 2.95 g (73.7 mmol) sodium hydride 60 % dispersion in mineral oil in 500 ml tetrahydrofuran (THF) are stirred at 50 °C under nitrogen for 1h. 12.8 g (67.0 mmol) 4-methylbenzenesulfonyl chloride in 100 ml THF are added at 20 °C. The reaction mixture is stirred for 1 h at 20 °C and is then stirred for 1 h at 50 °C. The solution is filtered and the solvent is distilled off. 200 ml ethyl acetate are added and the organic phase is washed with a solution of citric acid, sodium hydrogen carbonate and water. The solvent is partly removed until the product starts to crystalize. The product is filtered off and washed with methanol (yield: 23 g (79 %)). 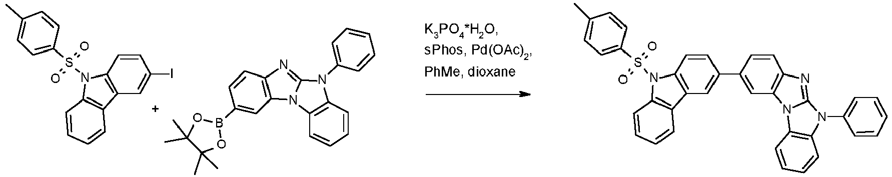
d.)To 7.75 g (17.3 mmol) 3-iodo-9-(p-tolylsulfonyl)carbazole, 7.80 (19.1 mmol) 5-phenyl-2-(4,4,5,5-tetramethyl-1,3,2-dioxaborolan-2-yl)benzimidazolo[1,2-a] benzimidazole, 18.4 g (86.6 mmol) potassium phosphate tribasic monohydrate, 25 ml dioxane, 60 ml toluene and 25 ml water are added.
   The mixture is degassed with argon. 426 mg (0.250 mmol) 2-dicyclohexylphosphino-2',6'-dimethoxybiphenyl (SPhos) and 39 mg (0.17 mmol) palladium(II) acetate are added. The reaction mixture is degassed with argon and is stirred for 16 h at 90 °C under argon. 30 ml of a 1 % sodium cyanide solution are added and the reaction mixture is refluxed for 2 h. Toluene is added and organic phase is separated. The organic phase is dried with magnesium sulfate. The solvent is removed in vacuum. The product is crystalized from diethyl ether. 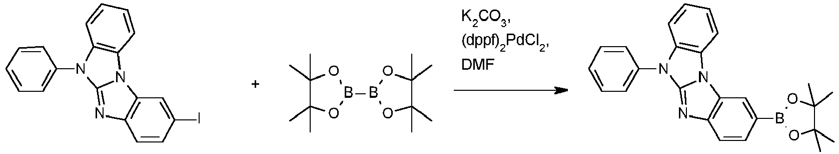
e.) 3.00 g (7.33 mmol) 2-iodo-5-phenyl-benzimidazolo[1,2-a]benzimidazole, 2.88 g (29.3 mmol) potassium acetate and 2.23 (8.80 mmol) 4,4,5,5-tetramethyl-2-(4,4,5,5 -tetramethyl-1,3,2-dioxaborolan- 2-yl)-1,3,2-dioxaborolane are degassed 3 times with argon The water free DMF is added and the reaction mixture is degassed 7 times with argon under stirring and at 25 °C. The catalyst is added and the reaction mixture is degassed 2 times with argon at 25 °C. Reaction mixture is stirred at 65 °C under argon for 18 h.
   The reaction mixture is cooled to 35 °C 5 ml diethyl ether and 400mg NaCN in 2 ml water is added simultaneously. The reaction mixture is stirred 10 min. The reaction mixture is poured in dichloromethane containing 20 % diethyl ether. The organic phase is washed with water and dried with magnesium sulfate and filtered on Hyflo. The solvent is removed in vacuum. Yield 2.93 g (97.7 %). 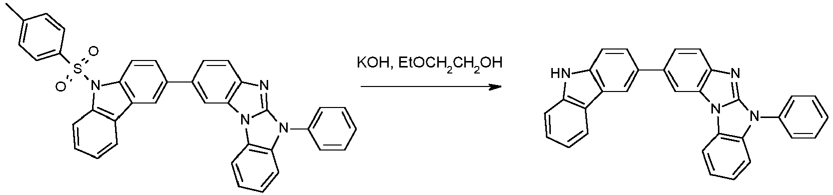
f.) 9.30 g (15.4 mmol) 5-phenyl-2-[9-(p-tolylsulfonyl)carbazol-3-yl]benzimidazolo [1,2-a]benzimidazole and 2.14 g (32.4 mmol) potassium hydroxide in 200 ml 2-ethoxyethanol is refluxed for 2 h. The solvent is removed in vacuum. The product is decocted in ethanol and the product is filtered off. 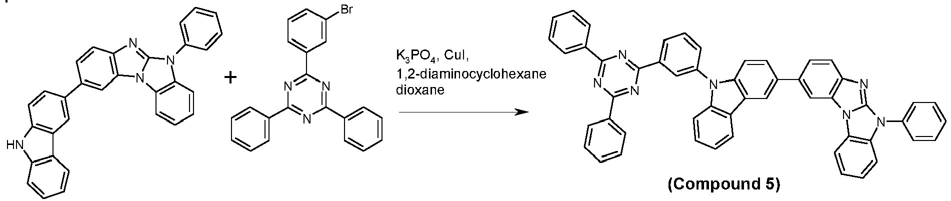
g.) The synthesis of 2-(3-bromophenyl)-4,6-diphenyl-1,3,5-triazine is described in WO2012099219 and WO2013172255

2.00 g (5.15 mmol) 2-(3-bromophenyl)-4,6-diphenyl-1,3,5-triazine, 2.77 g (6.18 mmol) 2-(9H-carbazol-3-yl)-5-phenyl-benzimidazolo[1,2-a]benzimidazole, 3.28 g (15.5 mmol) potassium phosphate tribasic, 196 mg (1.03 mmol) copper iodide in 50 ml dioxane are stirred under nitrogen at 100 °C. 4.12 g (36.1 mmol) cis,trans 1,2-diaminocyclohexane are added. The reaction mixture is stirred for 22 h. 196 mg (1.03 mmol) copper iodide and 4.12 g (36.1 mmol) cis,trans 1,2-diaminocyclohexane are added. The reaction mixture is stirred for 48 h at 100 °C under nitrogen.

The reaction mixture is poured in 200 ml methanol. The product is filtered off and is washed with water and methanol. The product is decocted in THF and filtered off. The product is decocted in acetic acid. The product is washed with ethanol.

0.62 g (16 %).

**¹H NMR** (400 MHz, TFA-d1): δ = 9.29-9.30 (m, 1H), 9.12 (d, 1H), 8.83-8.86 (m, 4H), 8.73 (m,1H), 8.66 (m, 1H), 8.41-8.51 (m, 3H), 8.24-8.32 (m, 2H), 8.13 (t, 2H), 7.90-8.05 (m, 14H), 7.67-7.88 (m, 4H)

### Example 7

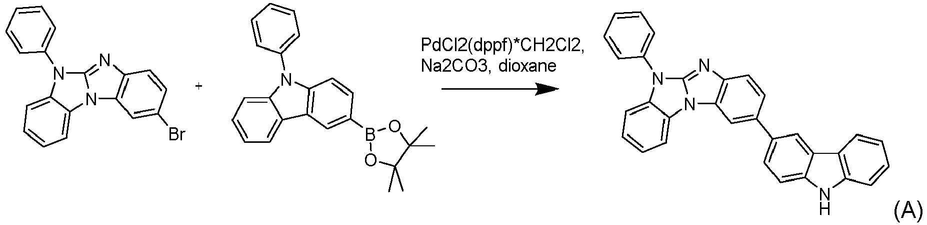

a.) The reaction was carried out according to example 6d except that PdCl₂(dppf)*CH₂Cl₂ and Na₂CO₃ is used instead of SPhos, Pd(OAc)₂ and K₃PO₄ as catalyst and base. 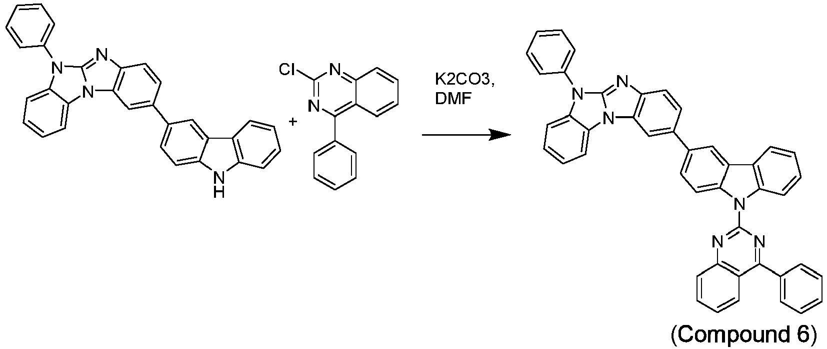
b.) In an argon atmosphere, 56.0 g of intermediate (A), 30.0 g of 2-chloro-4-phenylquinazoline, that had been prepared by a known method, 19.1 g of potassium carbonate, 900 mL of N,N-dimethylfolmamide were charged into flask and stirred at 120°C for 70 hours. After cooling to room temperature, 1000mL water was added and precipitated solids were separated by filtration. Purification of precipitated solids by silica gel column chromatography afforded yellow solids of compound 3 (yield: 33.0g( 40%)).

**¹H NMR** (300 MHz, CDCl₃): δ 9.18 (d, 1H), 9.13 (d, 1H), 8.41 (s, 1H), 8.16-8-23 (m, 4H), 7.85-8.01 (m, 8H), 7.30-7-83 (m, 13H).

### Example 8

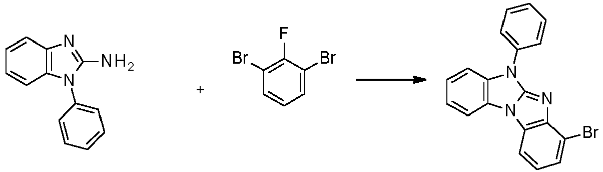

The reaction is carried out as described in example 6a, except that 1,3-dibromo-2-fluorobenzene is used instead of 1,3-dibromo-4-fluorobezene.

**¹H NMR** (400 MHz, CDCl₃): δ = 7.81-7.89 (m, 4H), 7.57-7.67 (m. 4H), 7.46-7.50 (m, 1H), 7.36-7.44 (m, 2H), 7.20 (t, 1H).

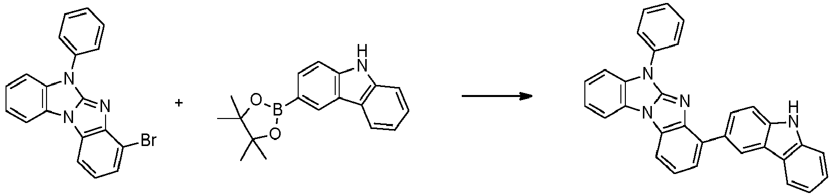

The reaction is carried out as described in example 7a

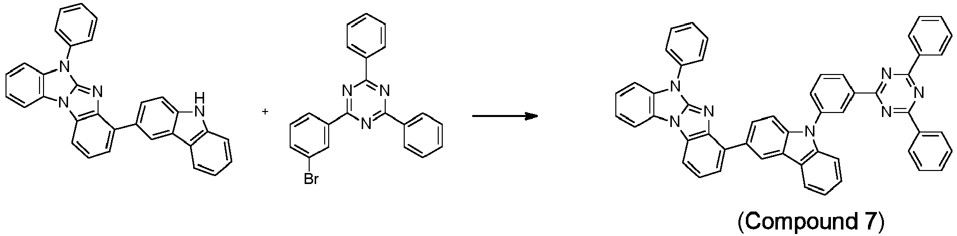

The reaction is carried out as described in example 6g

### Example 9

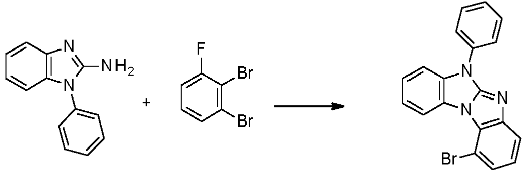

The reaction is carried out as described in example 6a, except that 1,2-dibromo-3-fluorobenzene is used instead of 1,3-dibromo-4-fluorobezene.

**¹H NMR** (400 MHz, CDCl₃): δ = 8.99-8.94 (m, 1H) 7.79-7.82 (m, 2H), 7.61-7.72 (m, 3H), 7.47-7.53 (m, 2H), 7.44 (d, 1H), 7.34-7.39 (m, 2H), 7.23 (t, 1H).

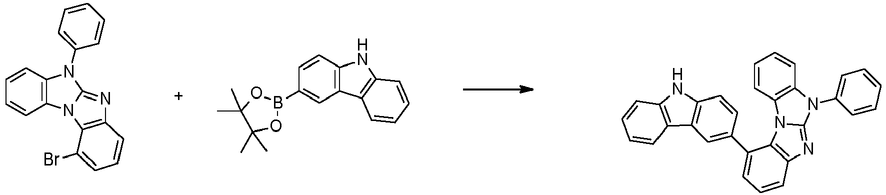

The reaction is carried out as described in example 7a

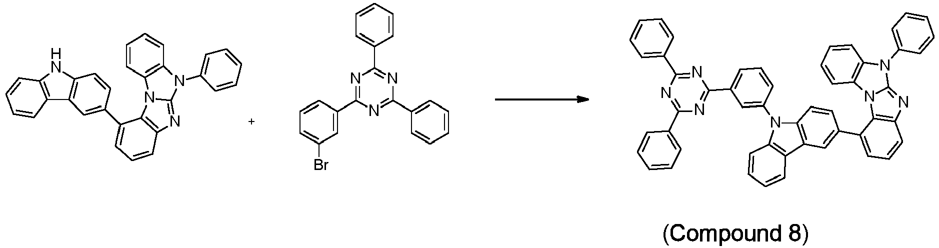

The reaction is carried out as described in example 6g.

### Example 10

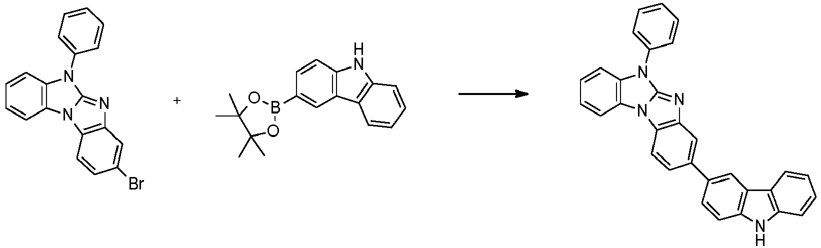

The reaction is carried out as described in example 7a

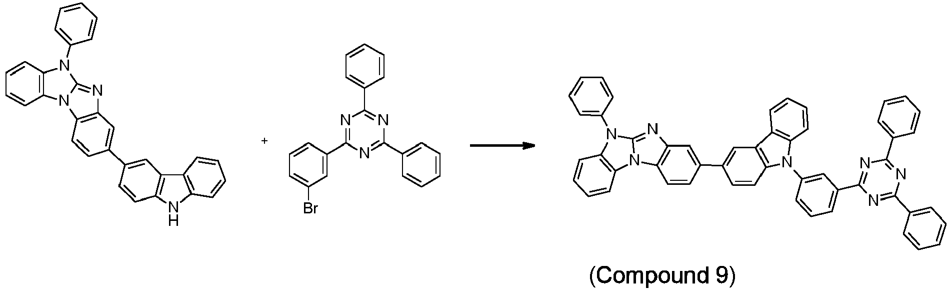

The reaction is carried out as described in example 6g.

### Example 11

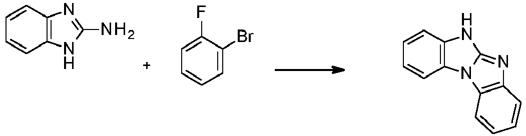

The reaction is carried out as described in example 2b, except that 1-bromo-2-fluoro-benzene is used instead of 1,4-dibromo-2-fluoro-benzene and the reaction is carried out at 160 °C.

**¹H NMR** (400 MHz, DMSO-d6): δ = 12.04 (s, 1H (NH)), 8.10 (d, 2H), 7.51 (d, 2H), 7.22-7.32 (m, 4H)

### II Application Examples

### 1. Comparative Application Example 1C

A glass substrate with 120 nm-thick indium-tin-oxide (ITO) transparent electrode (manufactured by Geomatec Co., Ltd.) used as an anode is first cleaned with isopropanol in an ultrasonic bath for 10 min. To eliminate any possible organic residues, the substrate is exposed to an ultraviolet light and ozone for further 30 min. This treatment also improves the hole injection properties of the ITO. The cleaned substrate is mounted on a substrate holder and loaded into a vacuum chamber. Thereafter, the organic materials specified below are applied by vapor deposition to the ITO substrate at a rate of approx. 0.2-1 Å/sec at about 10⁻⁶ -10⁻⁸ mbar. As a hole injection layer, 40 nm-thick of compound A is applied. Then 20 nm-thick of compound B is applied as a hole transporting layer. Subsequently, a mixture of 20% by weight of an emitter compound, (Ir(Ph-ppy)₃), and 80% by weight of a host **(Comparative compound 1C)** are applied to form a 40 nm-thick phosphorescent-emitting layer. On the emitting layer, 30 nm-thick compound C is applied as an electron transport layer. Finally, 1 nm-thick LiF is deposited as an electron injection layer and 80 nm-thick Al is then deposited as a cathode to complete the device. The device is sealed with a glass lid and a getter in an inert nitrogen atmosphere with less than 1 ppm of water and oxygen.

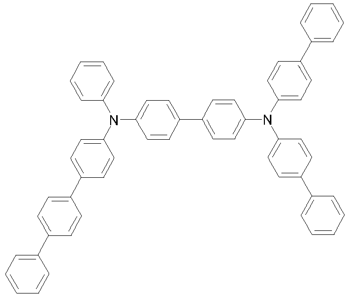

### Compound A (described in WO2006073054, page 82 and US2012112176, H24, page 87)

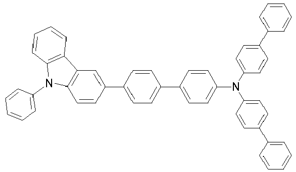

### Compound B (described in WO09072587, page181)

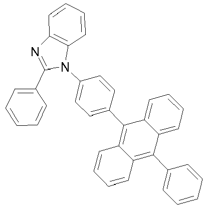

### Compound C (described in US 2014/0197386, page 44)

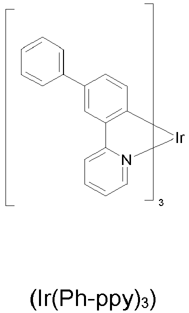

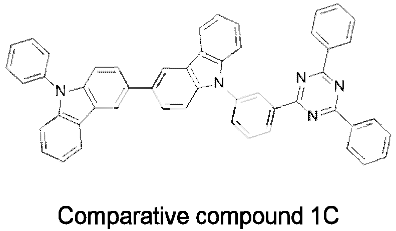

### 2. Inventive Application Example 1

Comparative Application Example 1 is repeated except that the host **(Comparative compound 1C)** is replaced by inventive **Compound 1.** The device results are shown in Table 1.

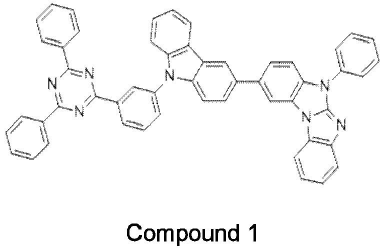

### OLED characterization

To characterize the OLED, electroluminescence spectra are recorded at various currents and voltages. In addition, the current-voltage characteristic is measured in combination with the luminance to determine luminous efficiency and external quantum efficiency (EQE). Driving voltage U, EQE and Commission Internationale de I'Eclairage (CIE) coordinate are given at 10mA/cm² except otherwise stated.

**Table 1**

| **Appl. Ex.** | **Host** | **U [V]** | **LT80 [hrs]** |
|---|---|---|---|
| **Comparative Appl. Ex. 1C** | **Comparative compound 1 C** | **4.9** | **60** |
| **Inventive Appl. Ex. 1** | **Compound 1** | **4.4** | **90** |

The results shown in Table 1 demonstrate that in an OLED comprising the inventive compound of formula **(1),** the driving voltage U is reduced and the lifetime is prolonged.

### 3. Inventive Application Example 2

Comparative Application Example 1C is repeated except that the host **(Comparative compound 1)** is replaced by a combination of 40% of inventive **compound 1** and 40% of compound D by co-deposition. The device results are shown in Table 2.

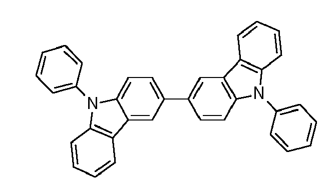

### Compound D (described in US2009009067, page 101 and US 20090009065, page 79)

### Compound D

**Table 2**

| **Appl. Ex.** | **Host** | **LT80 [hrs]** |
|---|---|---|
| **Inventive Appl. Ex. 1** | **Compound 1** | **90** |
| **Inventive Appl. Ex. 2** | **Compound 1 + compound D** | **170** |

The results shown in Table 2 demonstrate that the lifetime is further improved in the case that an inventive compound of formula (1) is used as a host together with a co-host in an OLED.

## Claims

1. A heterocyclic derivative of formula (1);
**A-[(B₁)ₒ-(B₂)ₚ-(B₃)_{q}-(B₄)ᵣ-Az]_{z}** **(1)**
wherein
B₁, B₂, B₃ and B₄
are independently of each other a C₆-C₂₄arylene group, which can optionally be substituted by G, or a C₁-C₂₄ heteroarylene group, which can optionally be substituted by G; o is 0 or 1, p is 0 or 1, q is 0 or 1, r is 0 or 1;
Az is represented by one of the following formulae (16), (17) or (18) 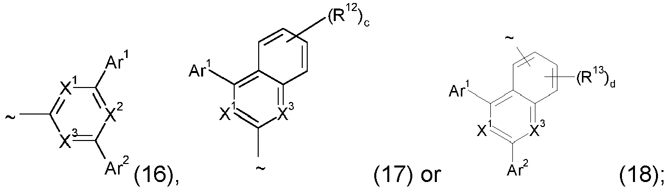
A is a heterocyclic group represented by formula (2) or formula (3); 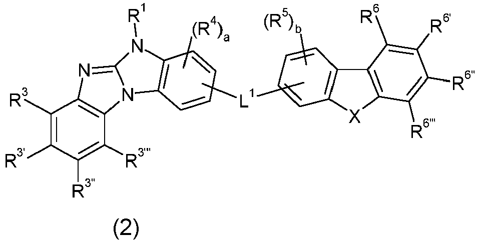 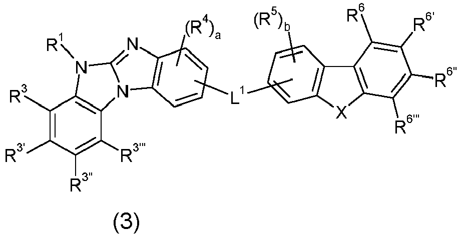 wherein X is NR⁷;
L¹ is single bond, a C₆-C₂₄arylene group, which can optionally be substituted by G, or a C₁-C₂₄heterocyclic group, which can optionally be substituted by G;
R¹ is phenyl or a group of the following formula 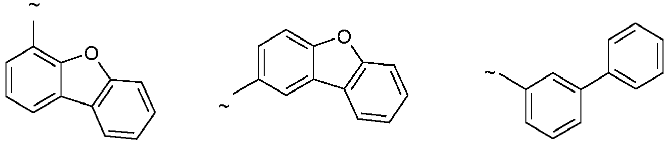 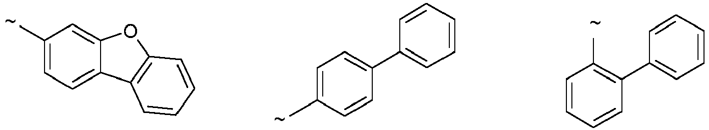 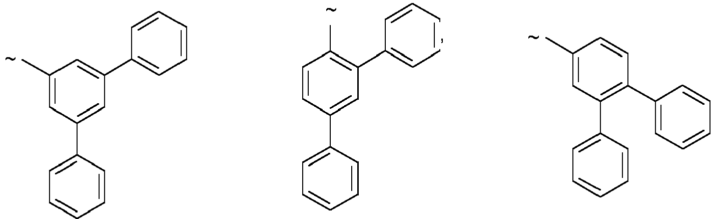 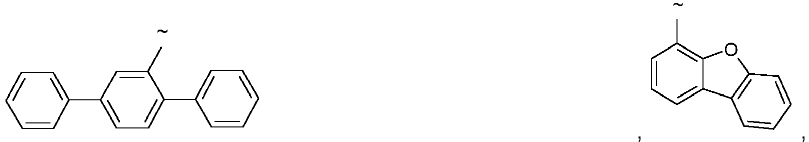 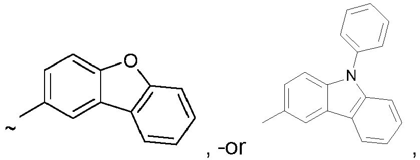 wherein ~ are bonding sites;
R³, R^{3'}, R^{3"}, R^{3"}, R⁴, R⁵, R⁶, R^{6'}, R^{6"} and R^{6‴}
are H;
G is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen,, or a C₁-C₂₄alkyl group, a C₆-C₆₀aryl group, a C₆-C₆₀aryl group, which is substituted by F, C₁-C₂₄alkyl, or C₁-C₂₄alkyl which is interrupted by O; a C₂-C₆₀heteroaryl group, or a C₂-C₆₀heteroaryl group, which is substituted by F, C₁-C₁₈alkyl, or C₁-C₁₈alkyl which is interrupted by O;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ may form together with the atom to which they are bonded a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
R⁷⁰ is a C₁-C₁₈alkyl group, a C₆-C₁₈aryl group, or a C₆-C₁₈aryl group, which is substituted by C₁-C₁₈alkyl, and
z is 1;
X¹, X² and X³ are independently of each other CR¹¹ or N, wherein in formula (16) at least one of X¹ to X³ is N, and wherein in formulae (17) and (18) at least one of X¹ and X³ is N; Ar₁ and Ar₂ are independently of each other a C₆-C₂₄ aryl group, which is optionally substituted by G, or a C₁-C₂₄ heteroaryl group, which is optionally substituted by G;
R¹¹, R¹² and R¹³ are independently of each other H, a C₆-C₂₄ aryl group which can be substituted by G, a C₁-C₂₄ heteroaryl group which can be substituted by G or a C₁-C₂₅alkyl group, which can optionally be substituted by E and/or interrupted by D; preferably, H;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or-C≡C;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen; R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-; R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁷¹ is a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl;
c is 0, 1, 2, 3 or 4; and
d is 0, 1, 2 or 3;
wherein R⁷ is replaced by -(B₁)ₒ-(B₂)ₚ-(B₃)_{q}-(B₄)ᵣ-Az.

2. The heterocyclic derivative according to claim 1, wherein L¹ is single bond.

3. The heterocyclic derivative according to claim 1 or 2, wherein at least one of o, p, q and r is 1.

4. The heterocyclic derivative according to any one of claims 1 to 3, wherein A is a heterocyclic group represented by formula (4), formula (5), formula (6), formula (7), formula (8), formula (9), formula (10), formula (11), formula (12), formula (13), formula (14), formula (15) or formula (38): 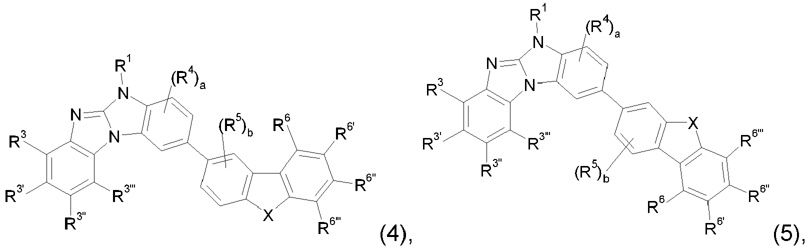 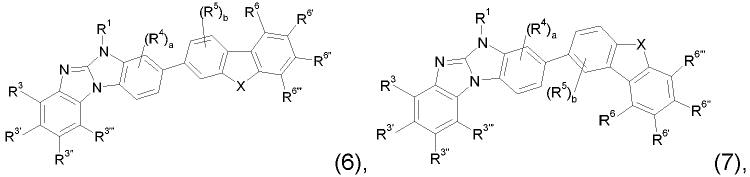 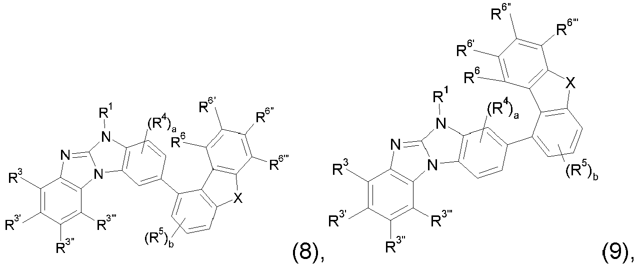 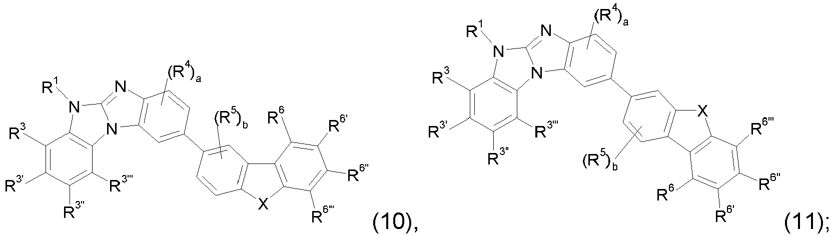 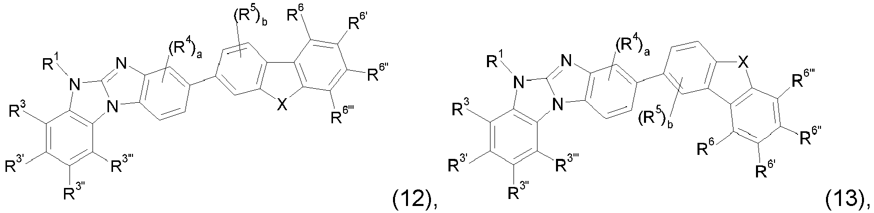 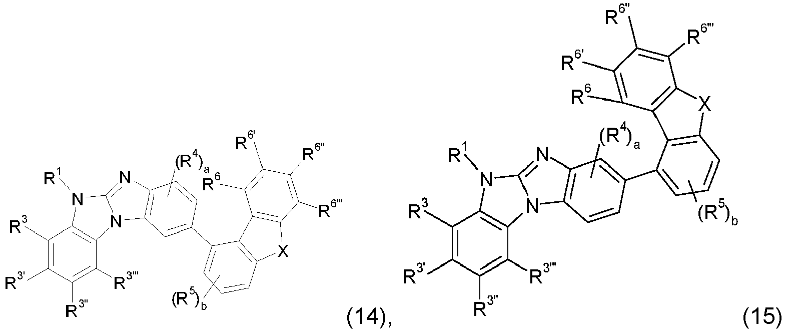 or 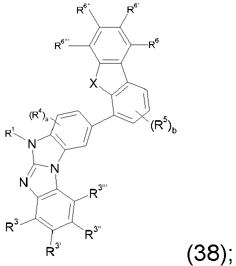 wherein the residues R¹, R³, R³, R^{3"}, R^{3‴}, R⁴, R⁵, R⁶, R^{6'}, R^{6"}, R^{6‴}, the indices a and b and the group X are defined in claim 1.

5. The heterocyclic derivative according to any one of claims 1 to 4, wherein A is a heterocyclic group represented by formula (4') or formula (5') 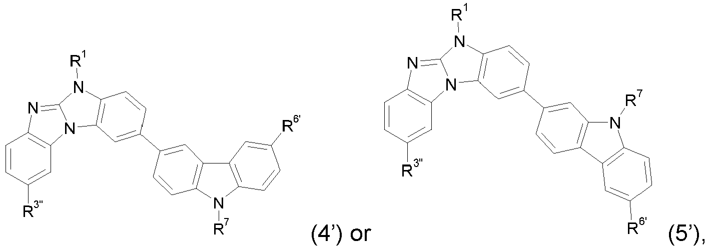 wherein the residues R¹, R^{3"}, R^{6'} and R⁷ are defined in claim 1.

6. The heterocyclic derivative according to any one of claims 1 to 5, wherein B₁, B₂, B₃ and B₄
are independently of each other C₆-C₂₄arylene groups, which optionally can be substituted by G, selected from the group consisting of phenylene, naphthylene, especially 1-naphthylene, or 2-naphthylene, biphenylylene, triphenylylene, terphenylylene, pyrenylene, 2- or 9-fluorenylene, phenanthrylene, or anthrylene, which may be unsubstituted or substituted by G; or
C₅-C₂₄heteroarylen groups, which optionally can be substituted by G, **characterized by** a ring with five to seven ring atoms or a condensed ring system, wherein nitrogen, oxygen or sulfur are the possible heteroatoms, and having at least six conjugated-electrons, preferably selected from benzothiophenylene, thianthrenylene, furylene, furfurylene, 2H-pyranylene, benzofuranylene, isobenzofuranylene, dibenzofuranylene ( 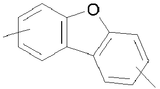 ), dibenzothiophenylene ( 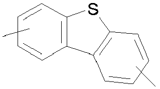 ), carbazolylene ( 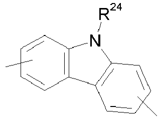 or 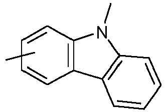 ), imidazolylene, pyrazolylene, pyridylene, bipyridylene, triazinylene, pyrimidinylene, pyrazinylene, pyridazinylene, indolizinylene, isoindolylene, indolylene, inda-zolylene, purinylene, quinolizinylene, chinolylene, isochinolylene, phthalazinylene, naph-thyridinylene, chinoxalinylene, chinazolinylene, cinnolinylene, pteridinylene, carbolinylene, benzotriazolylene, benzoxazolylene, phenanthridinylene, pyrimidinylene, benzimidazo[1,2-a]benzimidazo-2,5-ylene ( 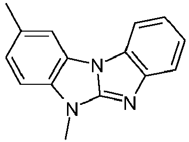 ),which can be unsubstituted or substituted by G; R²⁴ is a C₆-C₂₄aryl group, or a C₂-C₃₀heteroaryl group, which can optionally be substituted by G; wherein the lines are bonding sites; 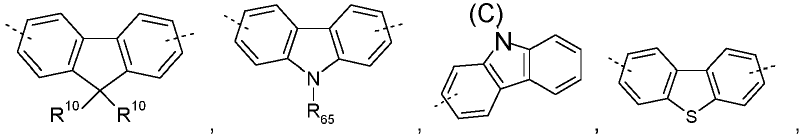 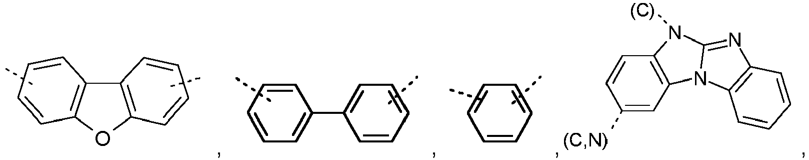 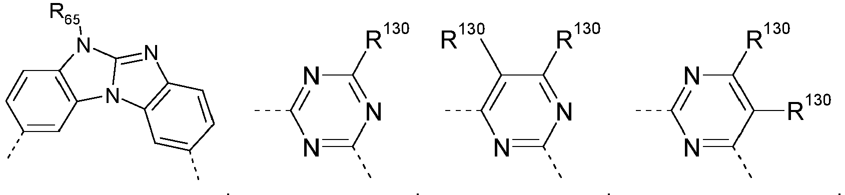 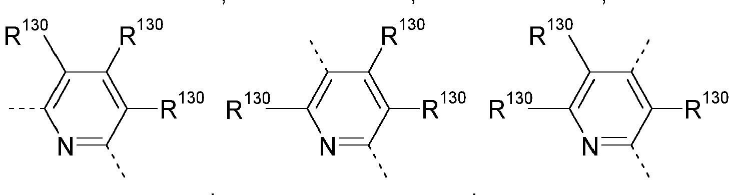 which can be unsubstituted or substituted by G;
R⁶⁵ is a C₆-C₁₈aryl group; a C₆-C₁₈aryl which is substituted by C₁-C₁₈alkyl or C₁-C₁₈alkoxy; a C₁-C₁₈alkyl group; or a C₁-C₁₈alkyl group, which is interrupted by -O-, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl;
R¹⁰ a C₁-C₂₅alkyl group, which can optionally be substituted by E and or interrupted by D; a C₆-C₂₄aryl group, which can optionally be substituted by G, or a C₁-C₂₄heteroaryl group, which can optionally be substituted by G; and/or two adjacent groups of the groups R¹⁰ may form together with the atom to which they are bonded a ring structure, which can optionally be substituted by G; R¹³⁰ is independently in each occurrence H or C₆-C₂₄arylene group, which can optionally be substituted by G, or a C₂-C₃₀heteroarylene group, which can optionally be substituted by G; wherein G is as defined in above; wherein the dotted lines are bonding sites;
wherein (C)- has the meaning that the bonding site of the group B₁, B₂, B₃ and B₄ is linked to a C-atom, and (N)- has the meaning that the bonding site of the group B₁, B₂, B₃ and B₄ is linked to a N-atom, and (C,N) has the meaning that the bonding site of the group B₁, B₂, B₃ and B₄ is linked to a C or N-atom.

7. The heterocyclic derivative according to any one of claims 1 to 6, wherein Az is selected from the group consisting of the following groups pyridine, pyrazine, pyrimidine, triazine, quinolone, isoquinoline, quinoxaline, quinazoline, phenanthroline, phenanthridine, ben-zo[h]quinolone, benz[h]isoquinoline, benzo[f]isoquinoline, benzo[f]quinoline, ben-zo[h]quinazoline, benzo[f]quinazoline, dibenzo[f,h]quinolone, dibenzo[f,h]isoquinolone, dibenzo[f,h]quinoxaline and dibenzo[f,h]quinazoline; which groups can be unsubstituted or substituted by G.

8. An organic electronic device, comprising the heterocyclic derivative according to any one of claims 1 to 7.

9. The organic electronic device according to claim 8, which is an organic electroluminescent device, wherein the organic electroluminescent device comprises an organic thin film layer between a cathode and an anode, wherein the organic thin film layer comprises one or more layers and comprises a light emitting layer, and at least one layer of the organic thin film layer comprises the heterocyclic derivative according to any one of claims 1 to 7.

10. The organic electronic device according to claim 9, wherein the light emitting layer comprises the heterocyclic derivative according to any one of claims 1 to 7.

11. The organic electronic device according to claim 8 or 10, wherein the light emitting layer comprises a phosphorescent material, which is an ortho-metallated complex comprising a metal atom selected from iridium (Ir), osmium (Os) and platinum (Pt).

12. An electron transport layer, an electron injection layer, or an emitting layer comprising the heterocyclic derivative according to any one of claims 1 to 7.

13. The emitting layer according to claim 12, comprising the heterocyclic derivative according to any one of claims 1 to 7 as host material in combination with a phosphorescent emitter.

14. An apparatus selected from the group consisting of stationary visual display units; mobile visual display units; illumination units; keyboards; items of clothing; furniture; wallpaper, comprising the organic electronic device according to claim 8 or 11, or the electron transport layer, the electron injection layer, or the emitting layer according to claim 13.

15. Use of the heterocyclic derivatives of formula (1) according to any one of claims 1 to 7 for organic electroluminescent devices, electrophotographic photoreceptors, photoelectric converters, organic solar cells, switching elements, organic light emitting field effect transistors, image sensors or dye lasers.

16. A process for the preparation of the heterocyclic derivative of formula (1) according to any one of claims 1 to 7, comprising:
a) Coupling of a group 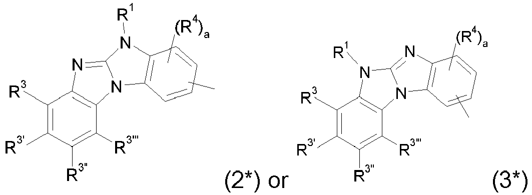 with a group 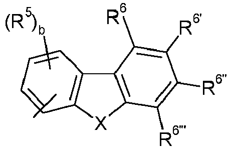 via a group L¹,
whereby a heterocyclic group A of formula (2) or formula (3) is obtained 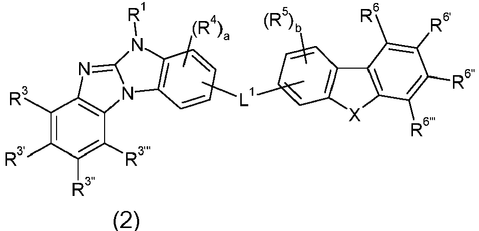 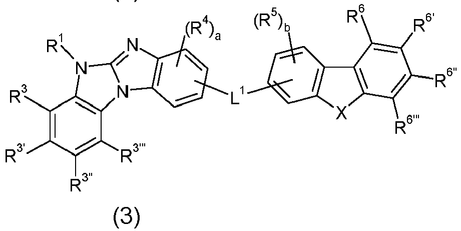 and
b) Introduction of one group -(B₁)ₒ₋(B₂)ₚ-(B₃)_{q}-(B₄)ᵣ-Az into the heterocyclic group A of formula (2) or formula (3),
whereby a heterocyclic derivative of formula (1)
A-[(B₁)ₒ-(B₂)ₚ-(B₃)_{q}-(B₄)ᵣ-Az]_{z} (1)
is obtained.

17. A process for the preparation of the heterocyclic derivative of formula **(1)** according to any one of claims 1 to 7,
comprising the step of coupling a compound of formula **(1")** or (**1‴**) 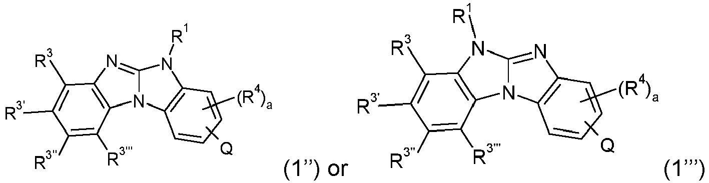 wherein
Q is H, F, CI, Br, or I, preferably Cl or Br, more preferably Br;
R¹ is phenyl or a group of the following formula 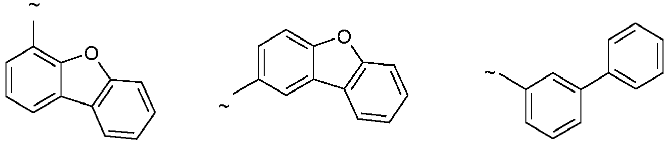 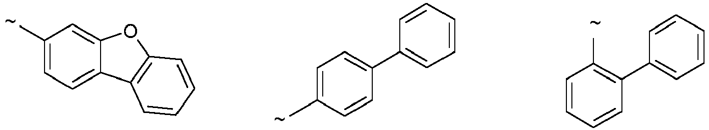 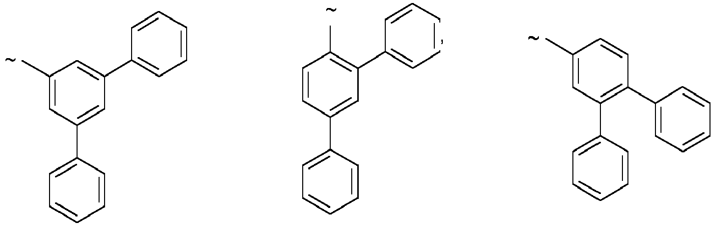 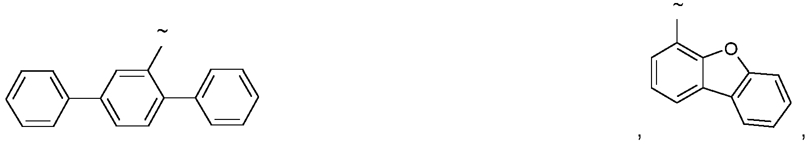 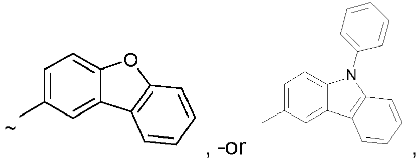 wherein ~ are bonding sites;
R³, R^{3'}, R^{3"}, R^{3‴} and R⁴
are H,
with at least one group of formula 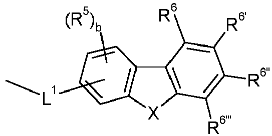 , wherein X is NR⁷;
L¹ is single bond, a C₆-C₂₄arylene group, which can optionally be substituted by G, or a C₁-C₂₄heterocyclic group, which can optionally be substituted by G;
R⁵, R⁶, R^{6'}, R^{6"} and R^{6‴} are H.

18. A process for the preparation of the heterocyclic derivative of formula (1) according to any one of claims 1 to 7, comprising the step:
Reaction of a compound of formula (31) with a compound of formula (32) in the presence of a base, whereby a compound of formula (1") is obtained: 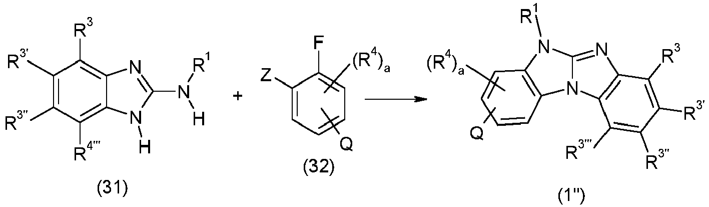 or
Reaction of a compound of formula (31') with a compound of formula (32') in the presence of a base, whereby a compound of formula (1'") is obtained: 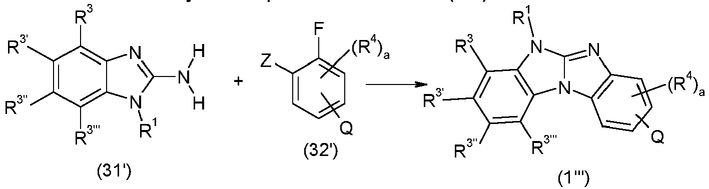 wherein
Q is H, F, CI, Br, or I, preferably Cl or Br, more preferably Br;
Z is F, CI, Br, or I, preferably Cl or Br, more preferably Br.
R¹ is phenyl or a group of the following formula 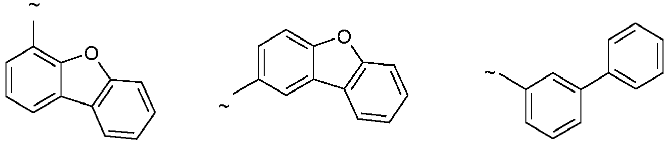 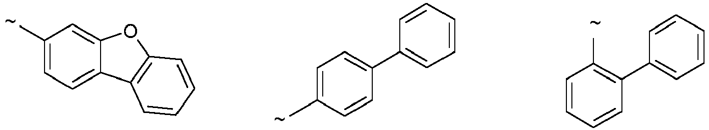 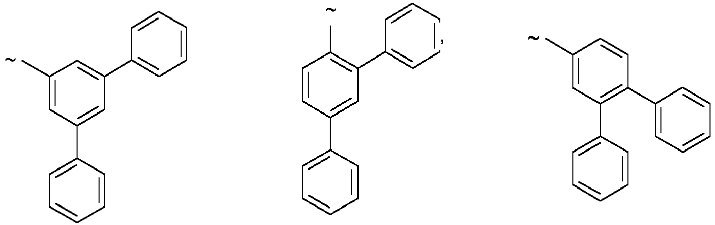 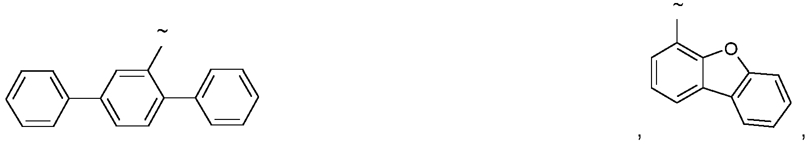 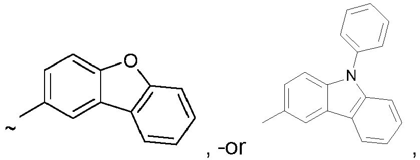
wherein ~ are bonding sites;
R³, R^{3'}, R^{3"}, R^{3‴} and R⁴
are H.

## Patentansprüche

1. Heterocyclisches Derivat der Formel **(1);**
**A-[(B₁)ₒ-(B₂)ₚ-(B₃)_{q}-(B₄)ᵣ-Az]_{z}** **(1)**
wobei
B₁, B₂, B₃ und B₄
unabhängig voneinander für eine C₆-C₂₄-Arylen-gruppe, die gegebenenfalls durch G substituiert sein kann, oder eine C₁-C₂₄-Heteroarylengruppe, die gegebenenfalls durch G substituiert sein kann, stehen;
o für 0 oder 1 steht, p für 0 oder 1 steht, q für 0 oder 1 steht, r für 0 oder 1 steht;
Az durch eine der folgenden Formeln (16), (17) oder (18) wiedergegeben wird: 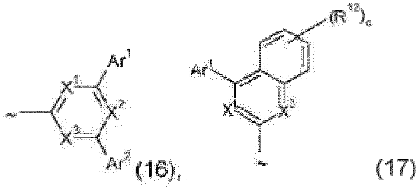 oder 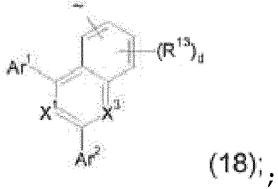
A für eine heterocyclische Gruppe steht, die durch Formel (2) oder Formel (3) wiedergegeben wird; 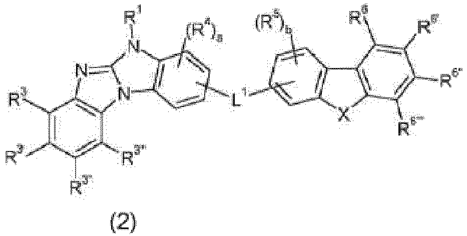 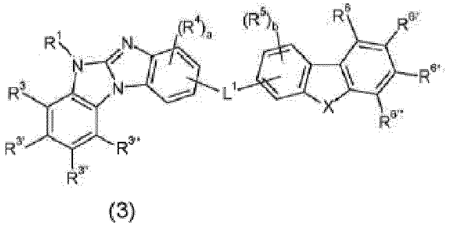 wobei X für NR⁷ steht;
L¹ für eine Einfachbindung, eine C₆-C₂₄-Arylen-gruppe, die gegebenenfalls durch G substituiert sein kann, oder eine C₁-C₂₄-heterocyclische Gruppe, die gegebenenfalls durch G substituiert sein kann, steht;
R¹ für Phenyl oder eine Gruppe der folgenden Formel steht: 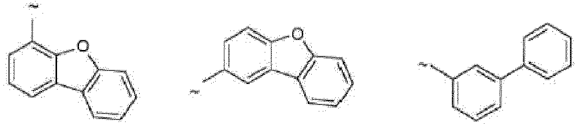 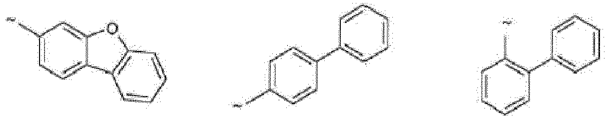 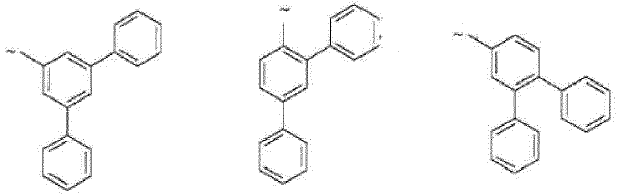 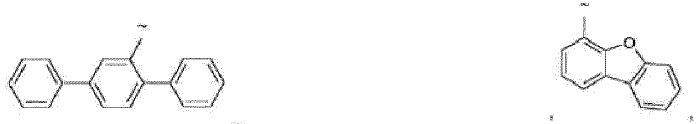 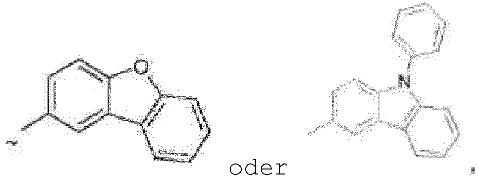 wobei die Symbole ~ für Bindungsstellen stehen;
R³, R^{3'}, R^{3"}, R^{3‴}, R⁴, R⁵, R⁶, R^{6'}, R^{6"} und R^{6‴} für H stehen;
G für -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ oder Halogen oder eine C₁-C₂₄-Alkylgruppe, C₆-C₆₀-Arylgruppe, eine C₆-C₆₀-Arylgruppe, die durch F, C₁-C₂₄-Alkyl oder C₁-C₂₄-Alkyl, das durch O unterbrochen ist, substituiert ist, eine C₂-C₆₀-Heteroarylgruppe oder eine C₂-C₆₀-Heteroarylgruppe, die durch F, C₁-C₁₈-Alkyl oder C₁-C₁₈-Alkyl, das durch O unterbrochen ist, substituiert ist, steht;
R⁶⁵ und R⁶⁶ unabhängig voneinander für eine C₆-C₁₈-Arylgruppe, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, stehen; oder
R⁶⁵ und R⁶⁶ zusammen mit dem Atom, an das sie gebunden sind, einen fünf- oder sechsgliedrigen Ring bilden können;
R⁶⁷ für eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht;
R⁶⁸ für H, eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht;
R⁶⁹ für ein C₆-C₁₈-Aryl, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht;
R⁷⁰ für eine C₁-C₁₈-Alkylgruppe, eine C₆-C₁₈-Arylgruppe oder eine C₆-C₁₈-Arylgruppe, die durch C₁-C₁₈-Alkyl substituiert ist, steht und
z für 1 steht;
X¹, X² und X³ unabhängig voneinander für CR¹¹ oder N stehen, wobei in Formel (16) mindestens eines von X¹ bis X³ für N steht und wobei in den Formeln (17) und (18) mindestens eines von X¹ und X³ für N steht;
Ar₁ und Ar₂ unabhängig voneinander für eine C₆-C₂₄-Arylgruppe, die gegebenenfalls durch G substituiert sein kann, oder eine C₁-C₂₄-Heteroarylgruppe, die gegebenenfalls durch G substituiert sein kann, stehen;
R¹¹, R¹² und R¹³ unabhängig voneinander für H, eine C₆-C₂₄-Arylgruppe, die gegebenenfalls durch G substituiert sein kann, eine C₁-C₂₄-Heteroaryl-gruppe, die gegebenenfalls durch G substituiert sein kann, oder eine C₁-C₂₅-Alkylgruppe, die gegebenenfalls durch E substituiert und/oder durch D unterbrochen sein kann; vorzugsweise H; steht;
D für -CO-, -COO-, -S-, -SO-, -SO₂-, -O-,-NR⁶⁵-, -SiR⁷⁰R⁷¹-, -POR⁷²-, -CR⁶³=CR⁶⁴- oder -C=C- steht;
E für -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ oder Halogen steht;
R⁶³ und R⁶⁴ unabhängig voneinander für H, C₆-C₁₈-Aryl, C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -O- unterbrochen ist, stehen; R⁶⁵ und R⁶⁶ unabhängig voneinander für eine C₆-C₁₈-Arylgruppe, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, stehen; oder
R⁷¹ für eine C₁-C₁₈-Alkylgruppe, eine C₆-C₁₈-Arylgruppe oder eine C₆-C₁₈-Arylgruppe, die durch C₁-C₁₈-Alkyl substituiert ist, steht und
R⁷² für eine C₁-C₁₈-Alkylgruppe, eine C₆-C₁₈-Arylgruppe oder eine C₆-C₁₈-Arylgruppe, die durch C₁-C₁₈-Alkyl substituiert ist, steht;
c für 0, 1, 2, 3 oder 4 steht und
d für 0, 1, 2 oder 3 steht;
wobei R⁷ durch - (B₁)ₒ-(B₂)ₚ-(B₃)_{q}-(B₄)ᵣ-Az ersetzt ist.

2. Heterocyclisches Derivat nach Anspruch 1, wobei L¹ für eine Einfachbindung steht.

3. Heterocyclisches Derivat nach Anspruch 1 oder 2, wobei mindestens eine der Variablen o, p, q und r für 1 steht.

4. Heterocyclisches Derivat nach Anspruch 2, wobei A für eine heterocyclische Gruppe steht, die durch Formel (4), Formel (5), Formel (6), Formel (7), Formel (8), Formel (9), Formel (10), Formel (11), Formel (12), Formel (13), Formel (14), Formel (15) oder Formel (38) wiedergegeben wird: 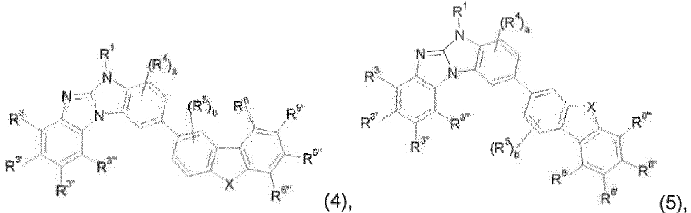 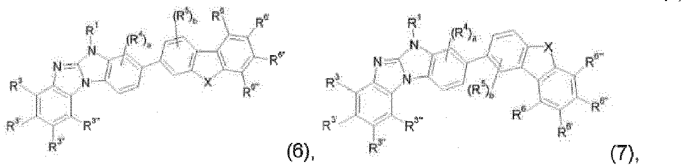 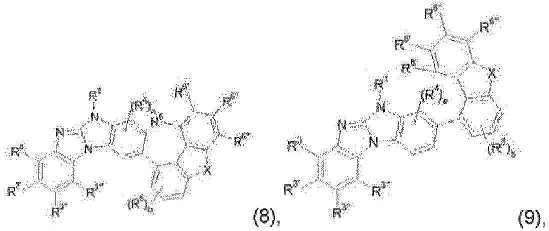 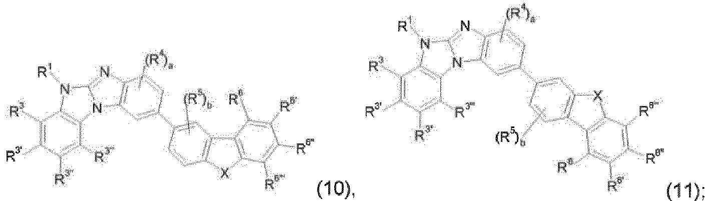 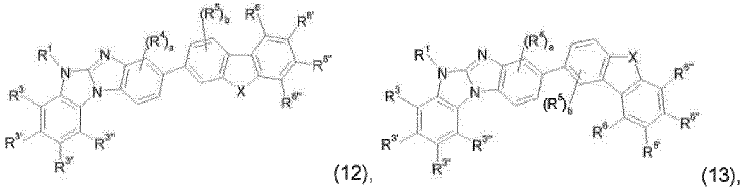 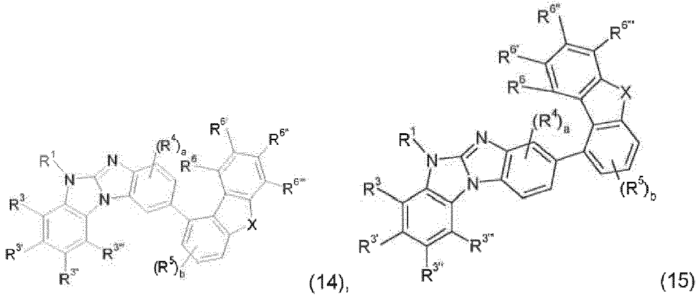 oder 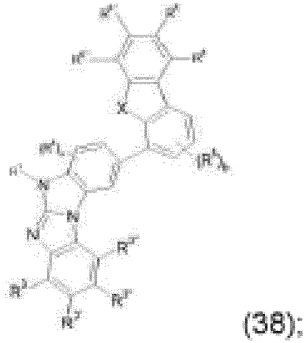 wobei die Reste R¹, R³, R^{3′}, R^{3ʺ} R^{3‴}, R⁴, R⁵, R⁶, R^{6′}, R^{6ʺ}, R^{6‴}, die Indizes a und b und die Gruppe X wie in Anspruch 1 definiert sind.

5. Heterocyclisches Derivat nach einem der Ansprüche 1 bis 4, wobei A für eine heterocyclische Gruppe steht, die durch Formel (4') oder Formel (5') wiedergegeben wird: 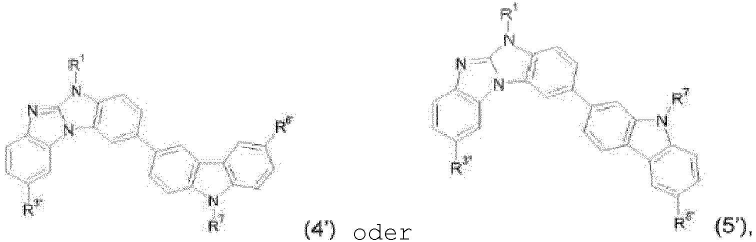 wobei die Reste R¹, R^{3ʺ} R^{6′} und R⁷ wie in Anspruch 1 definiert sind.

6. Heterocyclisches Derivat nach einem der Ansprüche 1 bis 5, wobei B₁, B₂, B₃ und B₄
unabhängig voneinander für C₆-C₂₄-Arylengruppen, die gegebenenfalls durch G substituiert sein können, ausgewählt aus der Gruppe bestehend aus Phenylen, Naphthylen, insbesondere 1-Naphthylen oder 2-Naphthylen, Biphenylylen, Triphenylylen, Terphenylylen, Pyrenylen, 2- oder 9-Fluorenylen, Phenanthrylen oder Anthrylen, die unsubstituiert oder durch G substituiert sein können; oder C₅-C₂₄-Heteroarylengruppen, die gegebenenfalls durch G substituiert sein können, **gekennzeichnet durch** einen Ring mit fünf bis sieben Ringatomen oder ein kondensiertes Ringsystem, wobei Stickstoff, Sauerstoff oder Schwefel die möglichen Heteroatome sind, und mit mindestens sechs konjugierten Elektronen, vorzugsweise ausgewählt aus Benzothiophenylen, Thianthrenylen, Furylen, Furfurylen, 2H-Pyranylen, Benzofuranylen, Isobenzofuranylen, Dibenzofuranylen ( 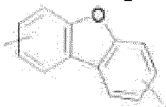 ), Dibenzothiophenylen ( 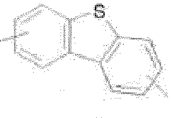 ), Carbazolylen ( 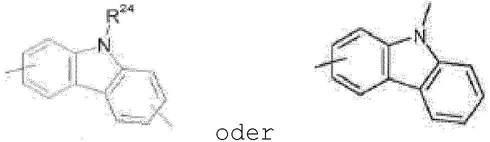 ), Imidazolylen, Pyrazolylen, Pyridylen, Bipyridylen, Triazinylen, Pyrimidinylen, Pyrazinylen, Pyridazinylen, Indolizinylen, Isoindolylen, Indolylen, Indazolylen, Purinylen, Chinolizinylen, Chinolylen, Isochinolylen, Phthalazinylen, Naphthyridinylen, Chinoxalinylen, Chinazolinylen, Cinnolinylen, Pteridinylen, Carbolinylen, Benzotriazolylen, Benzoxazolylen, Phenanthridinylen, Pyrimidinylen, Benzimidazo[1,2-a]benzimidazo-2,5-ylen ( 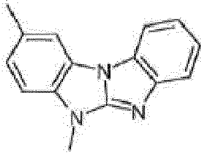 ), die unsubstituiert oder durch G substituiert sein können; R²⁴ für eine C₆-C₂₄-Arylgruppe oder eine C₂-C₃₀-Heteroarylgruppe, die gegebenenfalls durch G substituiert sein kann, steht; wobei die Linien für Bindungsstellen stehen; 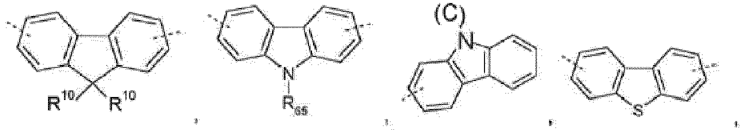 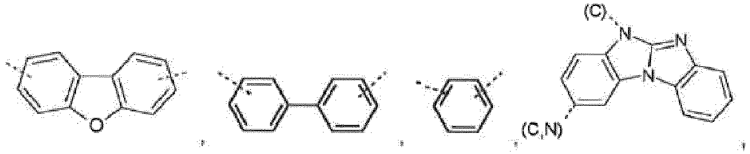 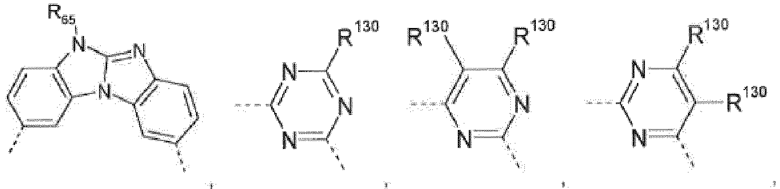 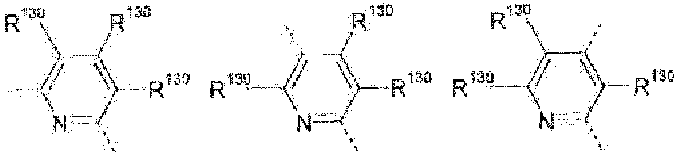 die unsubstituiert oder durch G substituiert sein können;
R⁶⁵ für eine C₆-C₁₈-Arylgruppe, ein C₆-C₁₈-Aryl, das durch C₁-C₁₈-Alkyl oder C₁-C₁₈-Alkoxy unterbrochen ist, eine C₁-C₁₈-Alkylgruppe oder eine C₁-C₁₈-Alkylgruppe, die durch -O- unterbrochen ist, vorzugsweise C₁-C₁₈-Alkyl, wie Methyl, Ethyl, n-Propyl, Isopropyl, n-Butyl, Isobutyl, sec-Butyl, Hexyl, Octyl oder 2-Ethylhexyl, oder C₆-C₁₄-Aryl, wie Phenyl, Tolyl, Naphthyl oder Biphenylyl, steht;
R¹⁰ für eine C₁-C₂₅-Alkylgruppe, die gegebenenfalls durch E substituiert und/oder durch D unterbrochen sein kann, eine C₆-C₂₄-Arylgruppe, die gegebenenfalls durch G substituiert sein kann, oder eine C₁-C₂₄-Heteroarylgruppe, die gegebenenfalls durch G substituiert sein kann, steht und/oder zwei benachbarte Gruppen der Gruppen R¹⁰ zusammen mit dem Atom, an das sie gebunden sind, eine Ringstruktur, die gegebenenfalls durch G substituiert sein kann, bilden können; R¹³⁰ unabhängig bei jedem Vorkommen für H oder eine C₆-C₂₄-Arylengruppe, die gegebenenfalls durch G substituiert sein kann, oder eine C₂-C₃₀-Heteroarylengruppe, die gegebenenfalls durch G substituiert sein kann, steht; wobei G wie oben definiert ist; wobei die gestrichelten Linien für Bindungsstellen stehen;
wobei (C)- die Bedeutung hat, dass die Bindungsstelle der Gruppe B₁, B₂, B₃ und B₄ an ein C-Atom gebunden ist, und (N)- die Bedeutung hat, dass die Bindungsstelle der Gruppe B₁, B₂, B₃ und B₄ an ein N-Atom gebunden ist, und (C,N) die Bedeutung hat, dass die Bindungsstelle der Gruppe B₁, B₂, B₃ und B₄ an ein C- oder N-Atom gebunden ist.

7. Heterocyclisches Derivat nach einem der Ansprüche 1 bis 6, wobei Az aus der Gruppe bestehend aus den folgenden Gruppen ausgewählt ist: Pyridin, Pyrazin, Pyrimidin, Triazin, Chinolon, Isochinolin, Chinoxalin, Chinazolin, Phenanthrolin, Phenanthridin, Benzo[h]chinolon, Benz[h]isochinolin, Benzo[f]isochinolin, Benzo[f]chinolin, Benzo[h]chinazolin, Benzo[f]chinazolin, Dibenzo-[f,h]chinolon, Dibenzo[f,h]isochinolon, Dibenzo-[f,h]chinoxalin und Dibenzo[f,h]chinazolin; wobei diese Gruppen unsubstituiert oder durch G substituiert sein können.

8. Organische elektronische Vorrichtung, umfassend eine Verbindung nach einem der Ansprüche 1 bis 7.

9. Organische elektronische Vorrichtung nach Anspruch 8, bei der es sich um eine organische Elektrolumineszenzvorrichtung handelt, wobei die organische Elektrolumineszenzvorrichtung eine organische Dünnfilmschicht zwischen einer Kathode und einer Anode umfasst, wobei die organische Dünnfilmschicht ein oder mehrere Schichten umfasst und eine lichtemittierende Schicht umfasst und mindestens eine Schicht der organischen Dünnfilmschicht das heterocyclische Derivat nach einem der Ansprüche 1 bis 7 umfasst.

10. Organische elektronische Vorrichtung nach Anspruch 9, wobei die lichtemittierende Schicht das heterocyclische Derivat nach einem der Ansprüche 1 bis 7 umfasst.

11. Organische elektronische Vorrichtung nach Anspruch 8 oder 10, wobei die lichtemittierende Schicht ein phosphoreszierendes Material umfasst, bei dem es sich um einen ortho-metallierten Komplex mit einem aus Iridium (Ir), Osmium (Os) und Platin (Pt) ausgewählten Metallatom handelt.

12. Elektronentransportschicht, Elektroneninjektionsschicht oder Emissionsschicht, umfassend das heterocyclische Derivat nach einem der Ansprüche 1 bis 7.

13. Emissionsschicht nach Anspruch 12, umfassend das heterocyclische Derivat nach einem der Ansprüche 1 bis 7 als Wirtsmaterial in Kombination mit einem phosphoreszierenden Emitter.

14. Apparatur, ausgewählt aus der Gruppe bestehend aus stationären Bildschirmen; mobilen Bildschirmen; Beleuchtungseinheiten; Tastaturen; Kleidungsstücken; Möbeln; Tapeten, umfassend die organische elektronische Vorrichtung nach Anspruch 8 oder 11 oder die Elektronentransportschicht, die Elektroneninjektionsschicht oder die Emissionsschicht nach Anspruch 13.

15. Verwendung der heterocyclischen Derivate der Formel (1) nach einem der Ansprüche 1 bis 7 für organische Elektrolumineszenzvorrichtungen, elektrophotographische Photorezeptoren, photoelektrische Umwandler, organische Solarzellen, Schaltelemente, organische lichtemittierende Feldeffekttransistoren, Bildsensoren oder Farbstofflaser.

16. Verfahren zur Herstellung des heterocyclischen Derivats der Formel (1) nach einem der Ansprüche 1 bis 7, umfassend:
a) Kuppeln einer Gruppe 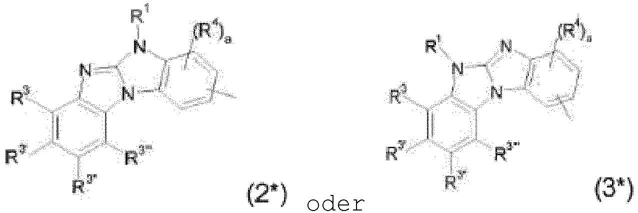 mit einer Gruppe 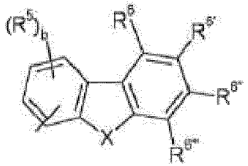 über eine Gruppe L¹,
wodurch eine heterocyclische Gruppe A der Formel (2) oder Formel (3) erhalten wird, 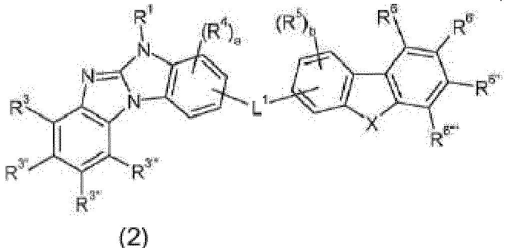 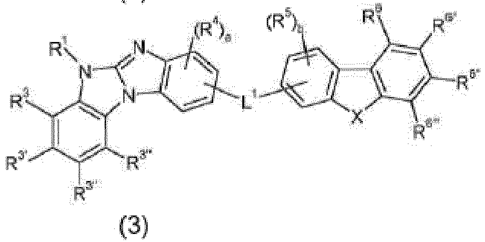 und
b) Einführung einer Gruppe -(B₁)ₒ-(B₂)ₚ-(B₃)_{q}-(B₄)ᵣ-Az in die heterocyclische Gruppe A der Formel (2) oder Formel (3),
wodurch ein heterocyclisches Derivat der Formel (1)
A-[(B₁)ₒ-(B₂)ₚ-(B₃)_{q}-(B₄)ᵣ-Az] (1)
erhalten wird.

17. Verfahren zur Herstellung des heterocyclischen Derivats der Formel (1) nach einem der Ansprüche 1 bis 7,
umfassend den Schritt des Kuppelns einer Verbindung der Formel (1ʺ) oder (1‴), 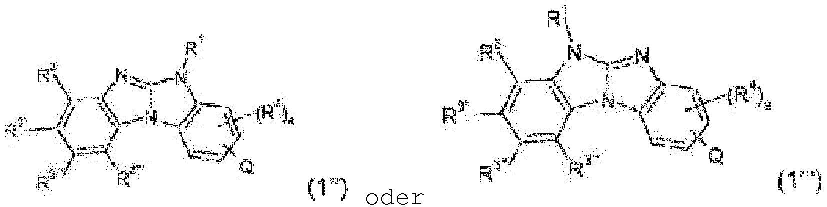 wobei
Q für H, F, Cl, Br oder I, vorzugsweise Cl oder Br, weiter bevorzugt Br, steht;
R¹ für Phenyl oder eine Gruppe der folgenden Formel steht: 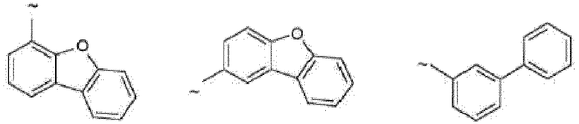 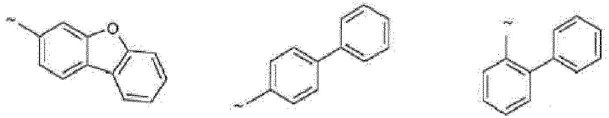 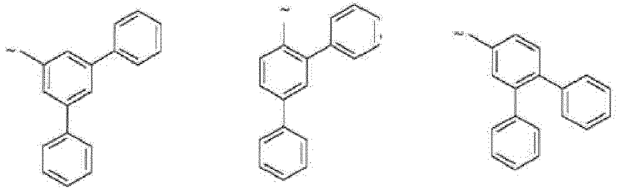 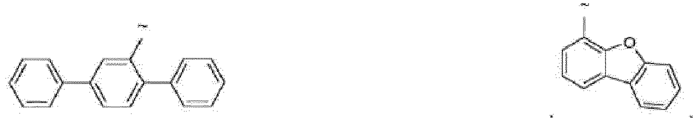 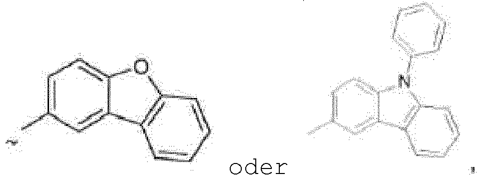 wobei die Symbole ~ für Bindungsstellen stehen;
R³, R³', R^{3ʺ} R^{3‴} und R⁴ für H stehen,
mit mindestens einer Gruppe der Formel 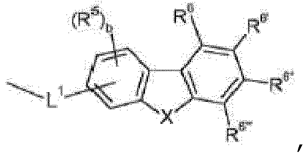 wobei
X für NR⁷ steht;
L¹ für eine Einfachbindung, eine C₆-C₂₄-Arylen-gruppe, die gegebenenfalls durch G substituiert sein kann, oder eine C₁-C₂₄-heterocyclische Gruppe, die gegebenenfalls durch G substituiert sein kann, steht;
R⁵, R⁶, R⁶, R^{6ʺ} und R^{6‴} für H stehen.

18. Verfahren zur Herstellung des heterocyclischen Derivats der Formel (1) nach einem der Ansprüche 1 bis 7, umfassend den Schritt:
Umsetzung einer Verbindung der Formel (31) mit einer Verbindung der Formel (32) in Gegenwart einer Base, wodurch eine Verbindung der Formel (1ʺ) erhalten wird: 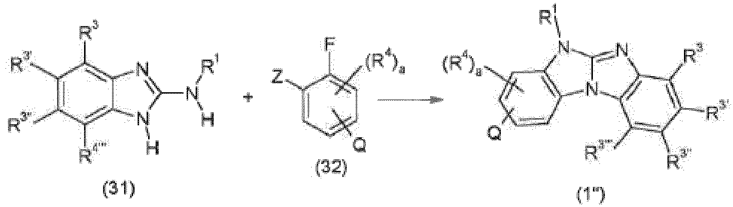 oder
Umsetzung einer Verbindung der Formel (31') mit einer Verbindung der Formel (32') in Gegenwart einer Base, wodurch eine Verbindung der Formel (1‴) erhalten wird: 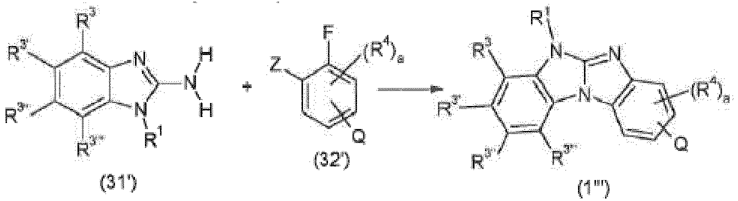 wobei
Q für H, F, Cl, Br oder I, vorzugsweise Cl oder Br, weiter bevorzugt Br, steht;
Z für F, Cl, Br oder I, vorzugsweise Cl oder Br, weiter bevorzugt Br, steht;
R¹ für Phenyl oder eine Gruppe der folgenden Formel steht: 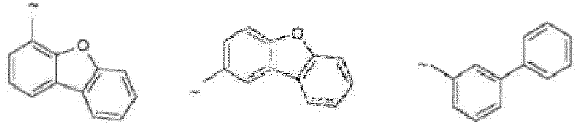 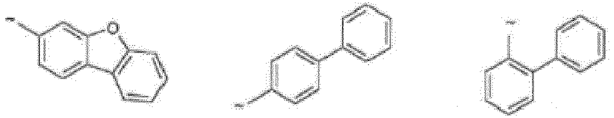 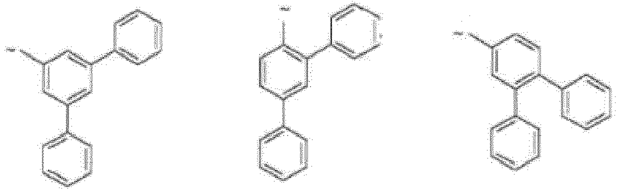 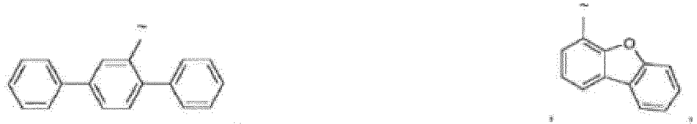 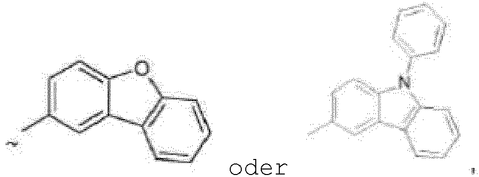 wobei die Symbole ~ für Bindungsstellen stehen;
R³, R³', R^{3ʺ} R^{3‴} und R⁴
für H stehen.

## Revendications

1. Dérivé hétérocyclique de formule (1) :
A-[(B₁)ₒ-(B₂)ₚ-(B₃)_{q}-(B₄)ᵣ-Az]_{z} (1)
B₁, B₂, B₃ et B₄
étant indépendamment les uns des autres un groupe C₆-C₂₄arylène, qui peut éventuellement être substitué par G, ou un groupe C₁-C₂₄ hétéroarylène, qui peut éventuellement être substitué par G ; o étant 0 ou 1, p étant 0 ou 1, q étant 0 ou 1, r étant 0 ou 1 ;
Az étant représenté par l'une des formules suivantes (16), (17) ou (18) 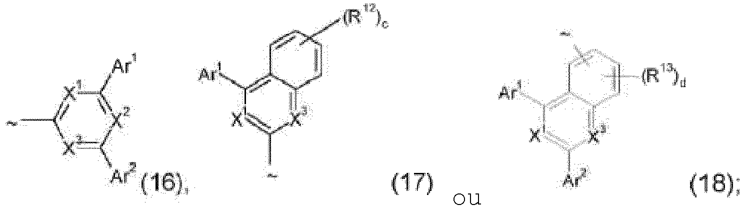
A étant un groupe hétérocyclique représenté par la formule (2) ou la formule (3) ; 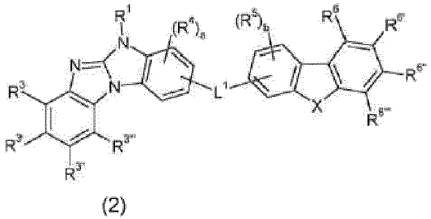 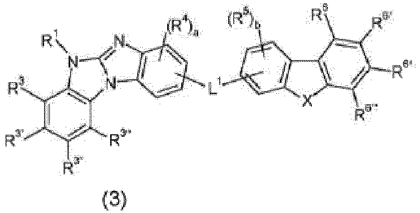
X étant NR⁷ ;
L¹ étant une simple liaison, un groupe C₆-C₂₄arylène, qui peut éventuellement être substitué par G, ou un groupe C₁-C₂₄hétérocyclique, qui peut éventuellement être substitué par G ;
R¹ étant phényle ou un groupe parmi les formules suivantes 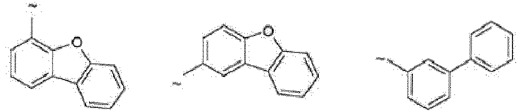 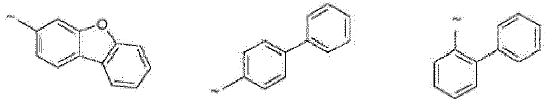 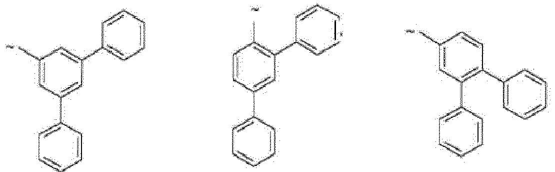 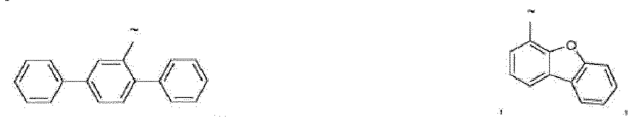 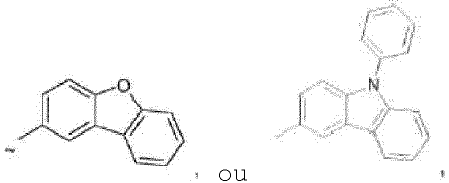
~ étant des sites de liaison ;
R³, R³', R^{3ʺ} R^{3‴}, R⁴, R⁵, R⁶, R⁶, R^{6ʺ} et R^{6‴} étant H ;
G étant -OR⁶⁹, -SR⁶⁹, -NR⁶⁵R⁶⁶, -COR⁶⁸, -COOR⁶⁷, - CONR⁶⁵R⁶⁶, -CN, -Si(R⁷⁰)₃ ou halogène, ou un groupe C₁-C₂₄alkyle, un groupe C₆-C₆₀aryle, un groupe C₆-C₆₀aryle, qui est substitué par F, C₁-C₂₄alkyle, ou C₁-C₂₄alkyle qui est interrompu par O; un groupe C₂-C₆₀hétéroaryle, ou un groupe C₂-C₆₀hétéroaryle, qui est substitué par F, C₁-C₁₈alkyle, ou C₁-C₁₈alkyle qui est interrompu par O ;
R⁶⁵ et R⁶⁶ étant indépendamment les uns des autres un groupe C₆-C₁₈aryle ; un C₆-C₁₈aryle qui est substitué par C₁-C₁₈alkyle, ou C₁-C₁₈alcoxy ; un groupe C₁-C₁₈alkyle; ou un groupe C₁-C₁₈alkyle, qui est interrompu par -O- ; ou
R⁶⁵ et R⁶⁶ pouvant former conjointement avec l'atome auquel ils sont liés un cycle à cinq ou six chaînons,
R⁶⁷ étant un groupe C₆-C₁₈aryle ; un groupe C₆-C₁₈aryle, qui est substitué par C₁-C₁₈alkyle, ou C₁-C₁₈alcoxy ; un groupe C₁-C₁₈alkyle ; ou un groupe C₁-C₁₈alkyle, qui est interrompu par -O-,
R⁶⁸ étant H ; un groupe C₆-C₁₈aryle ; un groupe C₆-C₁₈aryle, qui est substitué par C₁-C₁₈alkyle, ou C₁-C₁₈alcoxy ; un groupe C₁-C₁₈alkyle ; ou un groupe C₁-C₁₈alkyle, qui est interrompu par -O-,
R⁶⁹ étant un C₆-C₁₈aryle ; un C₆-C₁₈aryle, qui est substitué par C₁-C₁₈alkyle, ou C₁-C₁₈alcoxy ; un groupe C₁-C₁₈alkyle ; ou un groupe C₁-C₁₈alkyle, qui est interrompu par -O-,
R⁷⁰ étant un groupe C₁-C₁₈alkyle, un groupe C₆-C₁₈aryle, ou un groupe C₆-C₁₈aryle, qui est substitué par C₁-C₁₈alkyle, et
z étant 1 ;
X¹, X² et X³ étant indépendamment les uns des autres CR¹¹ ou N, dans la formule (16) au moins l'un parmi X¹ à X³ étant N, et dans les formules (17) et (18) au moins l'un parmi X¹ et X³ étant N ;
Ar₁ et Ar₂ étant indépendamment les uns des autres un groupe C₆-C₂₄aryle, qui est éventuellement substitué par G, ou un groupe C₁-C₂₄ hétéroaryle, qui est éventuellement substitué par G ;
R¹¹, R¹² et R¹³ étant indépendamment les uns des autres H, un groupe C₆-C₂₄ aryle qui peut être substitué par G, un groupe C₁-C₂₄ hétéroaryle qui peut être substitué par G ou un groupe C₁-C₂₅alkyle, qui peut éventuellement être substitué par E et/ou interrompu par D ; préférablement, H ; D étant -CO-, -COO-, -S-, -SO-, -SO₂-, -O-,-NR⁶⁵-, -SiR⁷⁰R⁷¹-, -POR⁷²-, -CR⁶³=CR⁶⁴-, ou -C≡C ;
E étant -OR⁶⁹, -SR⁶⁹, -NR⁶⁵R⁶⁶, -COR⁶⁸, -COOR⁶⁷, - CONR⁶⁵R⁶⁶, -CN, -Si(R⁷⁰)₃ ou halogène ; R⁶³ et R⁶⁴ étant indépendamment les uns des autres H, C₆-C₁₈aryle ; C₆-C₁₈aryle qui est substitué par C₁-C₁₈alkyle, ou C₁-C₁₈alcoxy ; C₁-C₁₈alkyle; ou C₁-C₁₈alkyle qui est interrompu par -O- ;
R⁶⁵ et R⁶⁶ étant indépendamment les uns des autres un groupe C₆-C₁₈aryle ; un C₆-C₁₈aryle qui est substitué par C₁-C₁₈alkyle, ou C₁-C₁₈alcoxy ; un groupe C₁-C₁₈alkyle; ou un groupe C₁-C₁₈alkyle, qui est interrompu par -O- ; ou
R⁷¹ étant un groupe C₁-C₁₈alkyle, un groupe C₆-C₁₈aryle, ou un groupe C₆-C₁₈aryle, qui est substitué par C₁-C₁₈alkyle, et
R⁷² étant un groupe C₁-C₁₈alkyle, un groupe C₆-C₁₈aryle, ou un groupe C₆-C₁₈aryle, qui est substitué par C₁-C₁₈alkyle;
c étant 0, 1, 2, 3 ou 4 ; et
d étant 0, 1, 2 ou 3 ;
R⁷ étant remplacé par -A-(B₁)ₒ-(B₂)ₚ-(B₃)_{q}-(B₄)ᵣ-Az.

2. Dérivé hétérocyclique selon la revendication 1, L¹ étant une simple liaison.

3. Dérivé hétérocyclique selon la revendication 1 ou 2, au moins l'un parmi o, p, q et r étant 1.

4. Dérivé hétérocyclique selon l'une quelconque des revendications 1 à 3, A étant un groupe hétérocyclique représenté par la formule (4), la formule (5), la formule (6), la formule (7), la formule (8), la formule (9), la formule (10), la formule (11), la formule (12), la formule (13), la formule (14), la formule (15) ou la formule (38) : 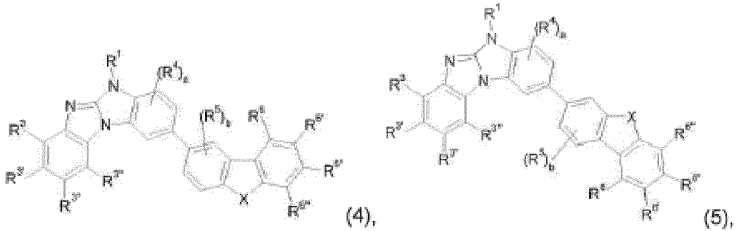 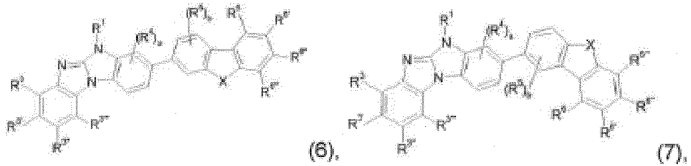 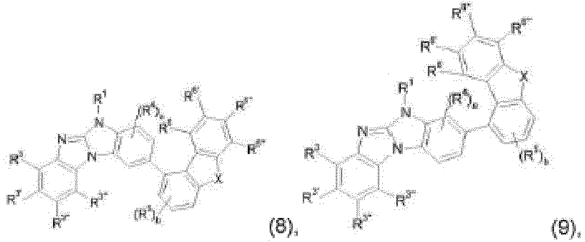 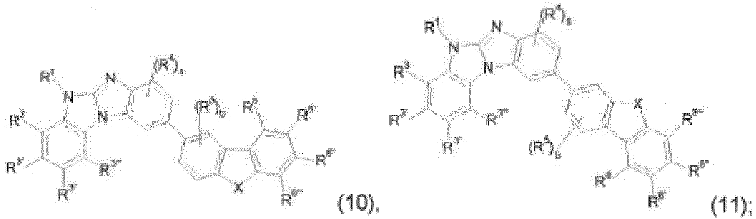 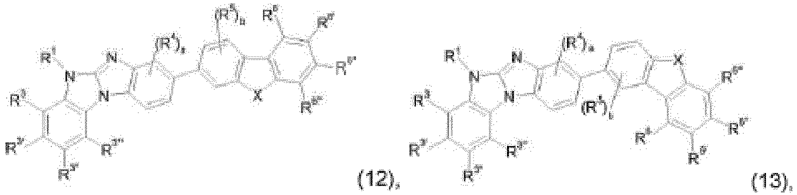 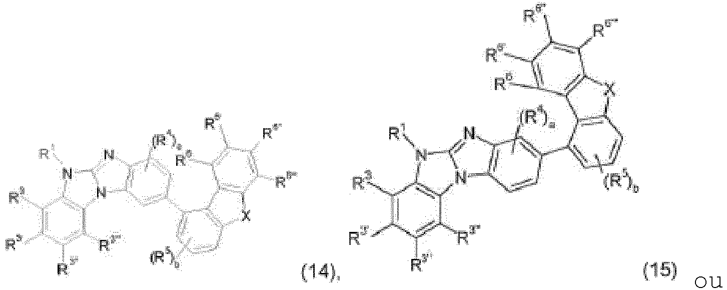 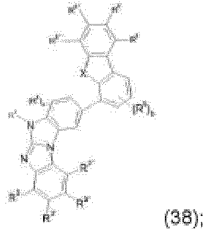 dans lequel dans les radicaux R¹, R³, R³', R^{3ʺ}, R3^{‴} , R⁴, R⁵, R⁶, R^{6′}, R^{6ʺ}, R^{6‴}, les indices a et b et le groupe X sont définis dans la revendication 1.

5. Dérivé hétérocyclique selon l'une quelconque des revendications 1 à 4, A étant un groupe hétérocyclique représenté par la formule (4') ou la formule (5') 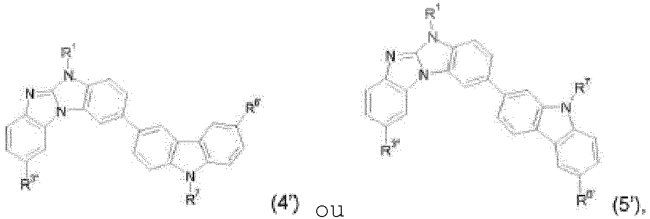 les radicaux R¹, R^{3ʺ} R^{6′} et R⁷ étant définis dans la revendication 1.

6. Dérivé hétérocyclique selon l'une quelconque des revendications 1 à 5, B₁, B₂, B₃ et B₄ étant indépendamment les uns des autres des groupes C₆-C₂₄arylène, qui éventuellement peut être substitué par G, choisi dans le groupe constitué par phénylène, naphtylène, notamment 1-naphtylène, ou 2-naphtylène, biphénylylène, triphénylylène, terphénylylène, pyrénylène, 2- ou 9-fluorénylène, phénanthrylène, ou anthrylène, qui peut être non substitué ou substitué par G ; ou
des groupes C₅-C₂₄hétéroarylène, qui éventuellement peuvent être substitués par G, **caractérisé par** un cycle comportant cinq à sept atomes de cycle ou un système cyclique condensé, l'azote, l'oxygène ou le soufre étant les hétéroatomes possibles, et possédant au moins six électrons conjugués, préférablement choisis parmi benzothiophénylène, thianthrénylène, furylène, furfurylène, 2H-pyrannylène, benzofurannylène, isobenzofurannylène, dibenzofurannylène ( 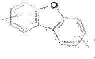 ), dibenzothiophénylène ( 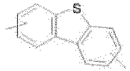 ), carbazolylène ( 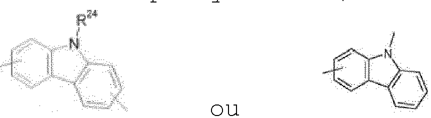 ), imidazolylène, pyrazolylène, pyridinylène, bipyridinylène, triazinylène, pyrimidinylène, pyrazinylène, pyridazinylène, indolizinylène, isoindolylène, indolylène, indazolylène, purinylène, quinoléizinylène, quinolylène, isoquinolylène, phtalazinylène, naphtyridinylène, quinoxalinylène, quinazolinylène, cinnolinylène, ptéridinylène, carbolinylène, benzotriazolylène, benzoxazolylène, phénanthridinylène, pyrimidinylène, benzimidazo[1,2-a]benzimidazo-2,5-lène ( 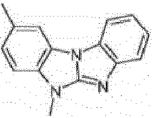 ), qui peut être non substitué ou substitué par G ; R²⁴ étant un groupe C₆-C₂₄aryle, ou un groupe C₂-C₃₀hétéroaryle, qui peut éventuellement être substitué par G ; les lignes étant des sites de liaison ; 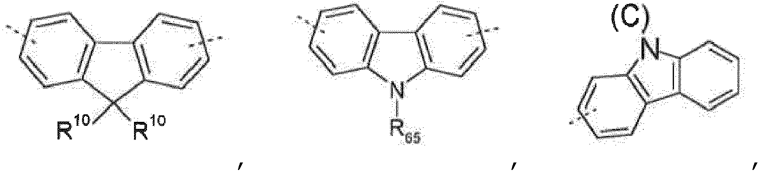 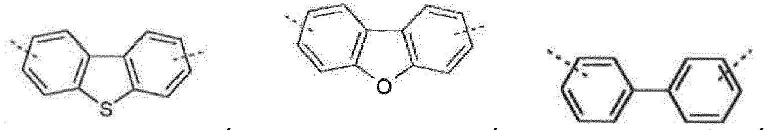 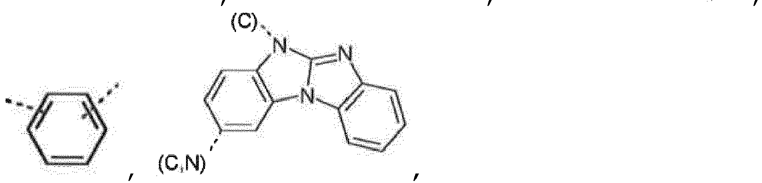 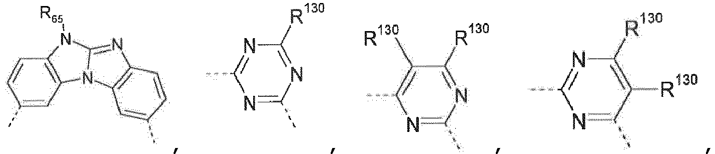 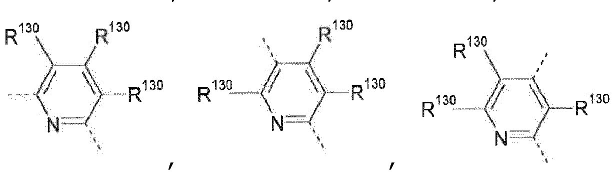 qui peut être non substitué ou substitué par G ; R⁶⁵ étant un groupe C₆-C₁₈aryle ; un C₆-C₁₈aryle qui est substitué par C₁-C₁₈alkyleou C₁-C₁₈alcoxy ; un groupe C₁-C₁₈alkyle; ou un groupe C₁-C₁₈alkyle, qui est interrompu par -O-,préférablement C₁-C₁₈alkyle, tel que méthyle, éthyle, n-propyle, iso-propyle, n-butyle, isobutyle, sec-butyle, hexyle, octyle, ou 2-éthyl-hexyle, ou C₆-C₁₄aryle, tel que phényle, tolyle, naphtyle, ou biphénylyle ;
R¹⁰ un groupe C₁-C₂₅alkyle, qui peut éventuellement être substitué par E et ou interrompu par D ; un groupe C₆-C₂₄aryle, qui peut éventuellement être substitué par G, ou un groupe C₁-C₂₄hétéroaryle, qui peut éventuellement être substitué par G ; et/ou deux groupes adjacents parmi les groupes R¹⁰ pouvant former une structure cyclique conjointement avec l'atome auquel ils sont liés, qui peut éventuellement être substitué par G ; R¹³⁰ étant indépendamment en chaque occurrence H ou C₆-C₂₄arylène, qui peut éventuellement être substitué par G, ou un groupe C₂-C₃₀hétéroarylène, qui peut éventuellement être substitué par G ; G étant tel que défini ci-dessus ; les lignes en pointillés étant des sites de liaison ;
(C)- possédant la signification que le site de liaison du groupe B₁, B₂, B₃ et B₄ est lié à un atome de C, et (N)- possédant la signification que le site de liaison du groupe B₁, B₂, B₃ et B₄ est lié à un atome N, et (C,N) possédant la signification que le site de liaison du groupe B₁, B₂, B₃ et B₄ est lié à un atome de C ou de N.

7. Dérivé hétérocyclique selon l'une quelconque des revendications 1 à 6, Az étant choisi dans le groupe constitué par les groupes suivants pyridine, pyrazine, pyrimidine, triazine, quinolone, isoquinoléine, quinoxaline, quinazoline, phénanthroline, phénanthridine, benzo[h]quinolone, benz[h]isoquinoléine, benzo[f]isoquinoléine, benzo[f]quinoléine, benzo[h]quinazoline, benzo[f]quinazoline, dibenzo[f,h]quinolone, dibenzo[f,h]isoquinolone, dibenzo[f,h]quinoxaline et dibenzo[f,h]quinazoline ; lesquels groupes pouvant être non substitués ou substitués par G.

8. Dispositif électronique organique, comprenant le dérivé hétérocyclique selon l'une quelconque des revendications 1 à 7.

9. Dispositif électronique organique selon la revendication 8, qui est un dispositif électroluminescent organique, le dispositif électroluminescent organique comprenant une couche de film mince organique entre une cathode et une anode, la couche de film mince organique comprenant une ou plusieurs couches et comprenant une couche luminescente, et au moins une couche parmi la couche de film mince organique comprenant le dérivé hétérocyclique selon l'une quelconque des revendications 1 à 7.

10. Dispositif électronique organique selon la revendication 9, la couche luminescente comprenant le dérivé hétérocyclique selon l'une quelconque des revendications 1 à 7.

11. Dispositif électronique organique selon la revendication 8 ou 10, la couche luminescente comprenant un matériau phosphorescent, qui est un complexe ortho-métallé comprenant un atome de métal choisi parmi l'iridium (Ir), l'osmium (Os) et le platine (Pt).

12. Couche de transport d'électrons, couche d'injection d'électrons, ou couche émettrice comprenant le dérivé hétérocyclique selon l'une quelconque des revendications 1 à 7.

13. Couche émettrice selon la revendication 12, comprenant le dérivé hétérocyclique selon l'une quelconque des revendications 1 à 7 en tant que matériau hôte en combinaison avec un émetteur phosphorescent.

14. Appareil choisi dans le groupe constitué par des unités d'affichage visuel fixes ; des unités d'affichage visuel mobiles ; des unités d'illumination ; des claviers ; des objets d'habillement ; un meuble ; du papier peint, comprenant le dispositif électronique organique selon la revendication 8 ou 11, ou la couche de transport d'électrons, la couche d'injection d'électrons, ou la couche émettrice selon la revendication 13.

15. Utilisation des dérivés hétérocycliques de formule (1) selon l'une quelconque des revendications 1 à 7 pour des dispositifs électroluminescents organiques, des photorécepteurs électrophotographiques, des convertisseurs photoélectriques, des cellules solaires organiques, des éléments de commutation, les transistors à effet de champ luminescents organiques, des capteurs d'images ou des lasers à colorant.

16. Procédé pour la préparation du dérivé hétérocyclique de formule (1) selon l'une quelconque des revendications 1 à 7, comprenant :
a) le couplage d'un groupe 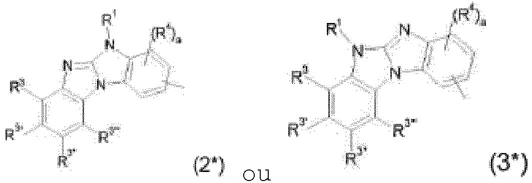 avec un groupe 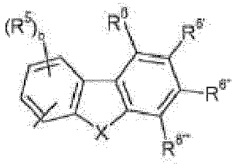 via un groupe L¹,
moyennant quoi un groupe hétérocyclique A de formule (2) ou de formule (3) est obtenu 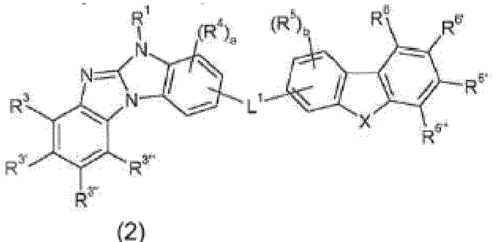 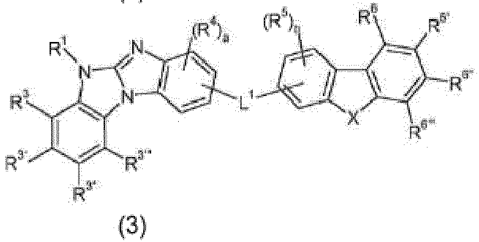 et
b) l'introduction d'un groupe -(B₁)ₒ-(B₂)ₚ-(B₃)_{q}-(B₄)ᵣ-Az dans le groupe hétérocyclique A de formule (2) ou de formule (3),
moyennant quoi un dérivé hétérocyclique de formule (1)
A-[(B₁)ₒ-(B₂)ₚ-(B₃)_{q}-(B₄)ᵣ-Az]_{z} (1)
est obtenu.

17. Procédé pour la préparation du dérivé hétérocyclique de formule (1) selon l'une quelconque des revendications 1 à 7,
comprenant l'étape de couplage d'un composé de formule (1ʺ) ou (1‴) 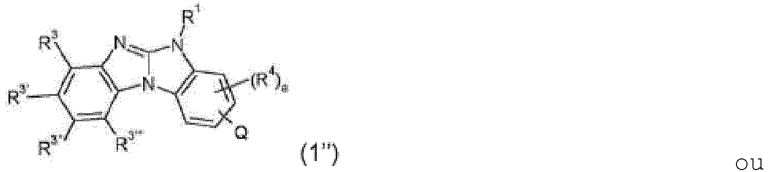 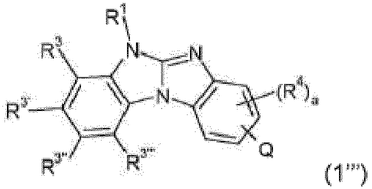
Q étant H, F, Cl, Br, ou I, préférablement Cl ou Br, plus préférablement Br ;
R¹ étant phényle ou un groupe parmi les formules suivantes 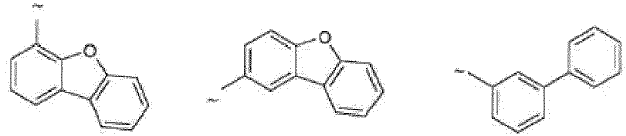 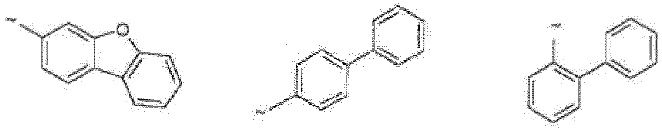 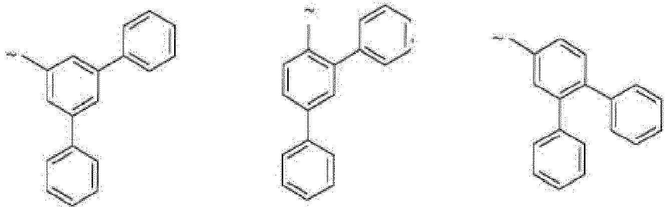 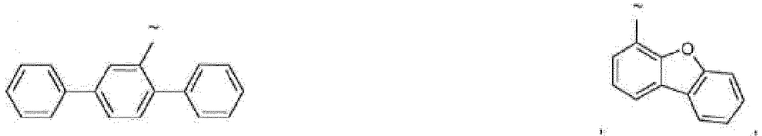 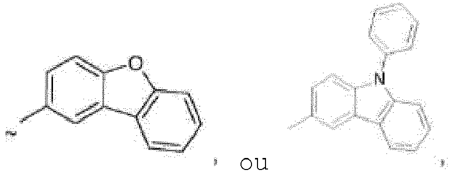
∼ étant des sites de liaison ;
R³, R³', R^{3ʺ}, R^{3‴} et R⁴
étant H,
avec au moins un groupe de formule 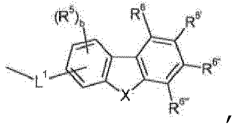
X étant NR⁷ ;
L¹ étant une simple liaison, un groupe C₆-C₂₄arylène, qui peut éventuellement être substitué par G, ou un groupe C₁-C₂₄hétérocyclique, qui peut éventuellement être substitué par G ;
R⁵, R⁶, R⁶, R^{6ʺ} et R^{6‴} étant H.

18. Procédé pour la préparation du dérivé hétérocyclique de formule (1) selon l'une quelconque des revendications 1 à 7, comprenant l'étape :
réaction d'un composé de formule (31) avec un composé de formule (32) en la présence d'une base,
moyennant quoi un composé de formule (1ʺ) est obtenu : 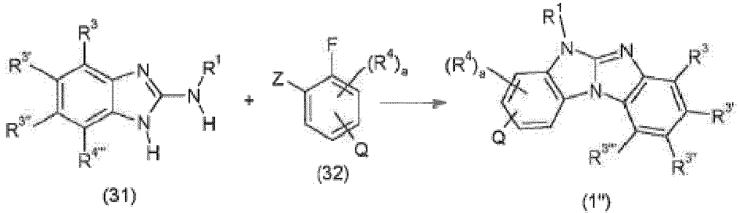 ou
réaction d'un composé de formule (31') avec un composé de formule (32') en la présence d'une base, moyennant quoi un composé de formule (1‴) est obtenu : 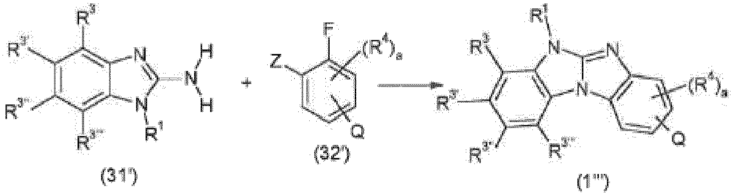
Q étant H, F, Cl, Br, ou I, préférablement Cl ou Br, plus préférablement Br ;
Z étant F, Cl, Br, ou I, préférablement Cl ou Br, plus préférablement Br,
R¹ étant phényle ou un groupe parmi les formules suivantes 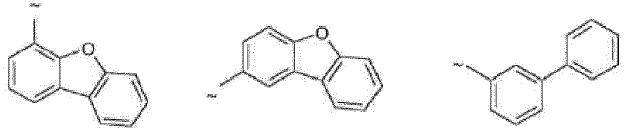 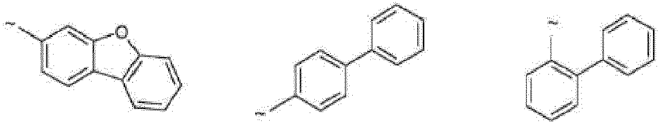 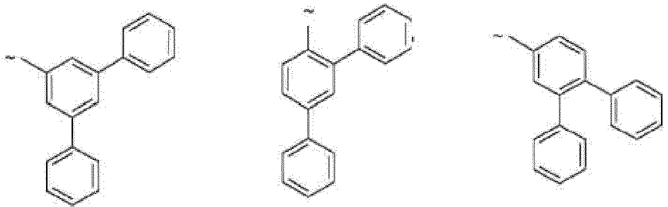 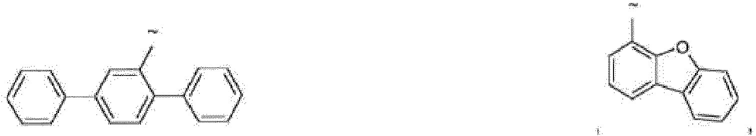 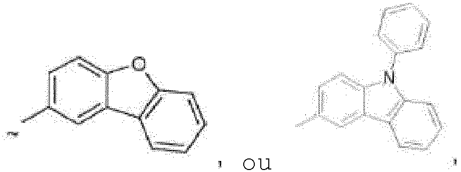
~ étant des sites de liaison ;
R³, R³', R^{3ʺ} R^{3‴} et R⁴
étant H.
